# EUROPEAN PATENT APPLICATION

(11) **EP 2 942 357 A1**
(43) Date of publication of application: **11.11.2015**
(21) Application number: 15169327.2
(22) Date of filing: 04.08.2009
(51) Int. Cl.: C07K 14/47, A61K 38/00, C12N 15/113

(54) **STING (STIMULATOR OF INTEFERON GENES), A REGULATOR OF INNATE IMMUNE RESPONSES**

(30) Priority: 04.08.2008 US 129975 P
(62) Divisional of application: 09805473.7
(71) Applicant: Barber, Glen N., Palmetto Bay, FL 33157 (US)
(72) Inventor: Barber, Glen N., Palmetto Bay, FL 33157 (US)
(74) Representative: Inspicos A/S

(57) **Abstract**

Novel molecules termed STING which include nucleic acids, polynucleotides, oligonucleotides, peptides, mutants, variants and active fragments thereof, modulate innate and adaptive immunity in a subject. STING compositions are useful for the treatment of an immune-related disorder, including treating and preventing infection by modulating immunity.

SEQUENCE LISTING

<110> UNIVERSITY OF MIAMI

<120> STING (STIMULATOR OF INTERFERON GENES), A REGULATOR OF INNATE
      IMMUNE RESPONSES

<130> 21331/2212101-WO0

<140> PCT/US09/52767
<141> 2009-08-04

<150> 61/129,975
<151> 2008-08-04

<160> 14

<170> PatentIn version 3.5

<210> 1
<211> 379
<212> PRT
<213> Homo sapiens

<400> 1

<210> 2
<211> 378
<212> PRT
<213> Mus sp.

<400> 2
Met Pro Tyr Ser Asn Leu His Pro Ala Ile Pro Arg Pro Arg Gly His
1               5                   10                  15

Arg Ser Lys Tyr Val Ala Leu Ile Phe Leu Val Ala Ser Leu Met Ile
            20                  25                  30

Leu Trp Val Ala Lys Asp Pro Pro Asn His Thr Leu Lys Tyr Leu Ala
        35                  40                  45

Leu His Leu Ala Ser His Glu Leu Gly Leu Leu Leu Lys Asn Leu Cys
    50                  55                  60

Cys Leu Ala Glu Glu Leu Cys His Val Gln Ser Arg Tyr Gln Gly Ser
65                  70                  75                  80

Tyr Trp Lys Ala Val Arg Ala Cys Leu Gly Cys Pro Ile His Cys Met
                85                  90                  95

Ala Met Ile Leu Leu Ser Ser Tyr Phe Tyr Phe Leu Gln Asn Thr Ala
            100                 105                 110

Asp Ile Tyr Leu Ser Trp Met Phe Gly Leu Leu Val Leu Tyr Lys Ser
        115                 120                 125

Leu Ser Met Leu Leu Gly Leu Gln Ser Leu Thr Pro Ala Glu Val Ser
    130                 135                 140

Ala Val Cys Glu Glu Lys Lys Leu Asn Val Ala His Gly Leu Ala Trp
145                 150                 155                 160

Ser Tyr Tyr Ile Gly Tyr Leu Arg Leu Ile Leu Pro Gly Leu Gln Ala
                165                 170                 175

Arg Ile Arg Met Phe Asn Gln Leu His Asn Asn Met Leu Ser Gly Ala
            180                 185                 190

Gly Ser Arg Arg Leu Tyr Ile Leu Phe Pro Leu Asp Cys Gly Val Pro
        195                 200                 205

Asp Asn Leu Ser Val Val Asp Pro Asn Ile Arg Phe Arg Asp Met Leu
    210                 215                 220

Pro Gln Gln Asn Ile Asp Arg Ala Gly Ile Lys Asn Arg Val Tyr Ser
225                 230                 235                 240

Asn Ser Val Tyr Glu Ile Leu Glu Asn Gly Gln Pro Ala Gly Val Cys
                245                 250                 255

Ile Leu Glu Tyr Ala Thr Pro Leu Gln Thr Leu Phe Ala Met Ser Gln
            260                 265                 270

Asp Ala Lys Ala Gly Phe Ser Arg Glu Asp Arg Leu Glu Gln Ala Lys
        275                 280                 285

Leu Phe Cys Arg Thr Leu Glu Glu Ile Leu Glu Asp Val Pro Glu Ser
    290                 295                 300

Arg Asn Asn Cys Arg Leu Ile Val Tyr Gln Glu Pro Thr Asp Gly Asn
305                 310                 315                 320

Ser Phe Ser Leu Ser Gln Glu Val Leu Arg His Ile Arg Gln Glu Glu
                325                 330                 335

Lys Glu Glu Val Thr Met Asn Ala Pro Met Thr Ser Val Ala Pro Pro
            340                 345                 350

Pro Ser Val Leu Ser Gln Glu Pro Arg Leu Leu Ile Ser Gly Met Asp
        355                 360                 365

Gln Pro Leu Pro Leu Arg Thr Asp Leu Ile
    370                 375

<210> 3
<211> 18
<212> RNA
<213> Homo sapiens

<400> 3
gcaucaagga ucggguuu 18

<210> 4
<211> 18
<212> RNA
<213> Homo sapiens

<400> 4
ggucauauua caucggau 18

<210> 5
<211> 18
<212> RNA
<213> Mus sp.

<400> 5
ccaacagcgu cuacgaga 18

<210> 6
<211> 21

<212> RNA
<213> Mus sp.

<400> 6
ugaaagagag gacggguuau u                                                21


<210> 7
<211> 19
<212> RNA
<213> Mus sp.

<400> 7
agaaguauua ggucggaaa                                                   19


<210> 8
<211> 19
<212> RNA
<213> Mus sp.

<400> 8
ucaacguacu ucagcgauu                                                   19


<210> 9
<211> 8
<212> PRT
<213> Artificial Sequence

<220>
<223> Description of Artificial Sequence: Synthetic peptide

<400> 9
Ser Ile Ile Asn Phe Glu Lys Leu
1               5


<210> 10
<211> 19
<212> RNA
<213> Mus sp.

<400> 10
cagcagagau uacacguca                                                   19


<210> 11
<211> 19
<212> RNA
<213> Mus sp.

<400> 11
gugaguggcu ugcgcagua                                                   19


<210> 12
<211> 97
<212> PRT
<213> Yellow fever virus

<400> 12
Asn Glu Leu Gly Met Leu Glu Lys Thr Lys Glu Asp Leu Phe Gly Lys

1               5                   10                  15

Lys Asn Leu Ile Pro Ser Ser Ala Ser Pro Trp Ser Trp Pro Asp Leu
            20                  25                  30

Asp Leu Lys Pro Gly Ala Ala Trp Thr Val Tyr Val Gly Ile Val Thr
        35                  40                  45

Met Leu Ser Pro Met Leu His His Trp Ile Lys Val Glu Tyr Gly Asn
    50                  55                  60

Leu Ser Leu Ser Gly Ile Ala Gln Ser Ala Ser Val Leu Ser Phe Met
65                  70                  75                  80

Asp Lys Gly Ile Pro Phe Met Lys Met Asn Ile Ser Val Ile Met Leu
                85                  90                  95

Leu

<210> 13
<211> 94
<212> PRT
<213> Dengue virus

<400> 13
Asn Glu Met Gly Phe Leu Glu Lys Thr Lys Lys Asp Leu Gly Leu Gly
1               5                   10                  15

Ser Ile Ala Thr Gln Gln Pro Glu Ser Asn Ile Leu Asp Ile Asp Leu
            20                  25                  30

Arg Pro Ala Ser Ala Trp Thr Leu Tyr Ala Val Ala Thr Thr Phe Val
        35                  40                  45

Thr Pro Met Leu Arg His Ser Ile Glu Asn Ser Ser Val Asn Val Ser
    50                  55                  60

Leu Thr Ala Ile Ala Asn Gln Ala Thr Val Leu Met Gly Leu Gly Lys
65                  70                  75                  80

Gly Trp Pro Leu Ser Lys Met Asp Ile Gly Val Pro Leu Leu
                85                  90

<210> 14
<211> 84
<212> PRT
<213> Hepatitis C virus

<400> 14

Phe Lys Gln Lys Ala Leu Gly Leu Leu Gln Thr Ala Thr Lys Gln Ala
1               5                   10                  15

Glu Ala Ala Ala Pro Val Val Glu Ser Lys Trp Arg Ala Leu Glu Thr
            20                  25                  30

Phe Trp Ala Lys His Met Trp Asn Phe Ile Ser Gly Ile Gln Tyr Leu
        35                  40                  45

Ala Gly Leu Ser Thr Leu Pro Gly Asn Pro Ala Ile Ala Ser Leu Met
    50                  55                  60

Ala Phe Thr Ala Ser Ile Thr Ser Pro Leu Ala Thr Gln Tyr Thr Leu
65                  70                  75                  80

Leu Phe Asn Ile

## Description

### RELATED APPLICATIONS

This application claims priority under 35 USC § 119 to U.S. Provisional Patent Application No. 61/129,975 filed August 4, 2008, the disclosure of which is incorporated by reference in its entirety.

### FIELD OF THE INVENTION

Embodiments of the invention relate to compositions and methods for modulating innate and adaptive immunity in a subject and/or for the treatment of an immune-related disorder, cancer, autoimmunity, treating and preventing infections.

### BACKGROUND

Cellular host defense responses to pathogen invasion principally involves the detection of pathogen associated molecular patterns (PAMPs) such as viral nucleic acid or bacterial cell wall components including lipopolysaccharide or flagellar proteins that results in the induction of anti-pathogen genes. For example, viral RNA can be detected by membrane bound Toll-like receptors (TLR's) present in the endoplasmic reticulum (ER) and/or endosomes (e.g. TLR 3 and 7/8) or by TLR-independent intracellular DExD/H box RNA helicases referred to as retinoic acid inducible gene 1 (RIG-I) or melanoma differentiation associated antigen 5 (MDA5, also referred to as IFIH1 and helicard). These events culminate in the activation of downstream signaling events, much of which remains unknown, leading to the transcription of NF-κB and IRF3/7- dependent genes, including type IIFN.

### SUMMARY

This Summary is provided to present a summary of the invention to briefly indicate the nature and substance of the invention. It is submitted with the understanding that it will not be used to interpret or limit the scope or meaning of the claims.

STING molecules (Stimulator of Interferon Genes) modulate the immune system, in particular the innate immune system. Compositions comprising STING and/or other agents which modulate STING expression, activity and/or functions treat diseases such as cancer, infections, autoimmune diseases or disorders, inflammation and the like are administered to patients at risk of developing or for the treatment of patients afflicted with such diseases.

Embodiments of the invention are also directed to molecules and pathways which directly or indirectly interact or associate with STING.

Other aspects of the invention are described *infra.*

### BRIEF DESCRIPTION OF THE DRAWINGS

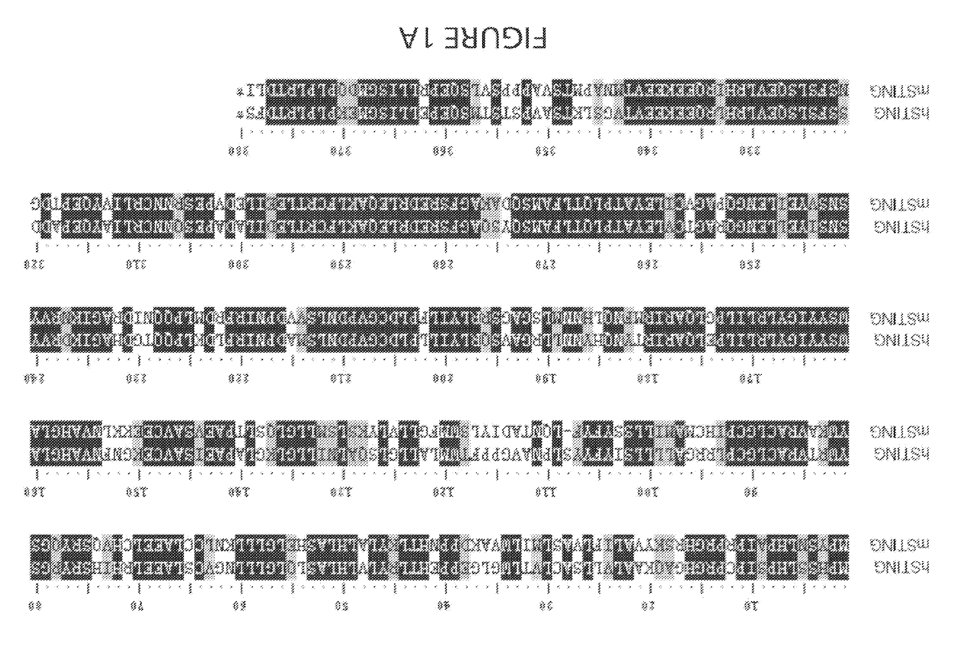

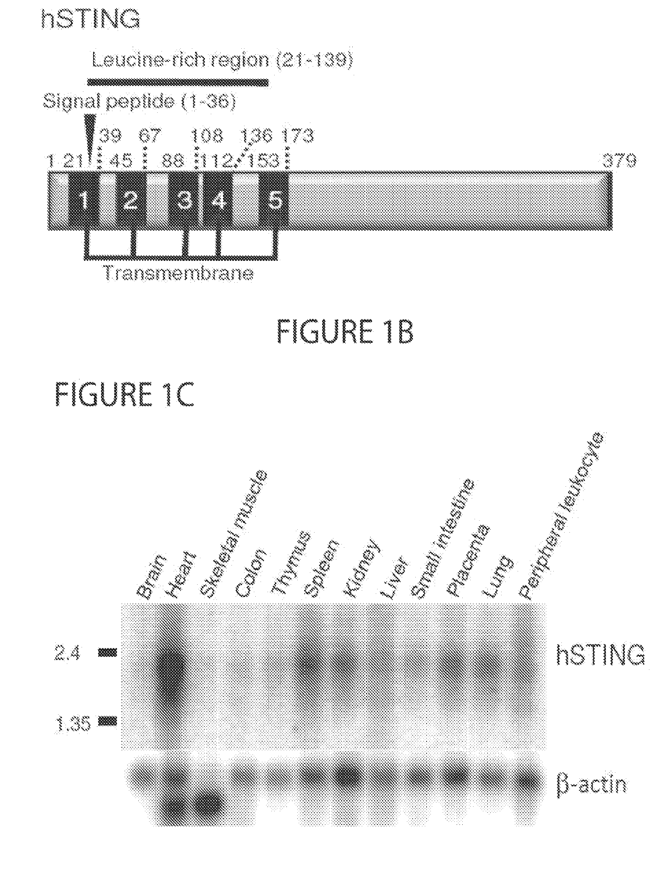

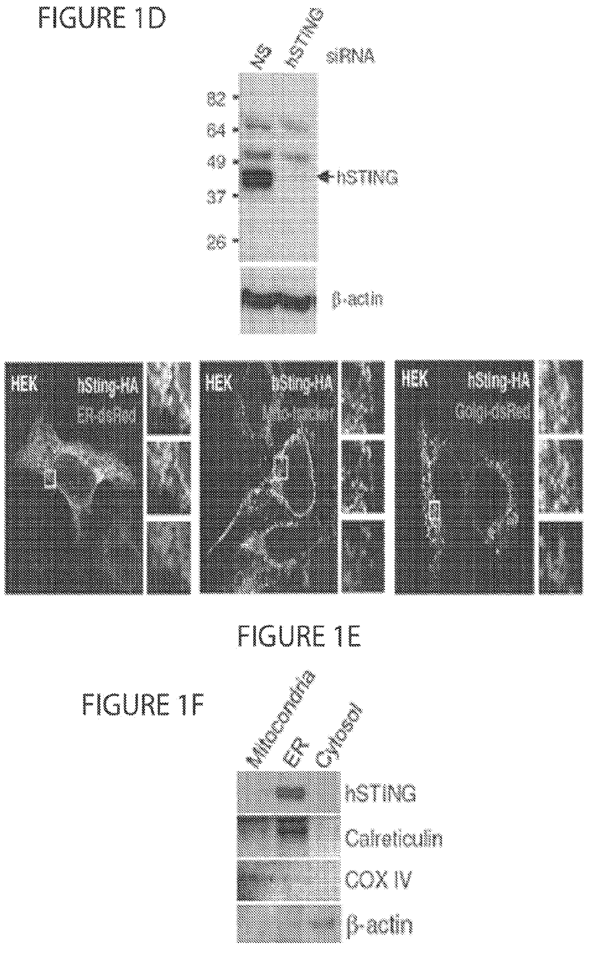

Figure 1A shows the amino acid sequence of human and mouse STING (SEQ ID NOS: 1 and 2 respectively). Figure 1B shows a schematic representation of hSTING indicating TM and leucine rich regions. Figure 1C shows a Northern blot analysis of human STING. Figure 1D shows an immunoblot analysis of STING in HEK 293 cells. RNAi to STING (hSTING) or control RNAi (NS) was used to confirm specificity of the antibody. Figure 1E shows a confocal analysis of HEK 293 cells transfected with hSTING tagged at the carboxyl end with HA. Transfected cells were also analyzed using ER-dsRed, Mitotracker or Golgi-dsRed. Figure 1F shows that fractionation experiments confirm that STING resides in the ER. Control antibodies indicate accuracy of fractionation (Calreticulin- ER, COX IV-mitochondria, beta actin- cytosol).

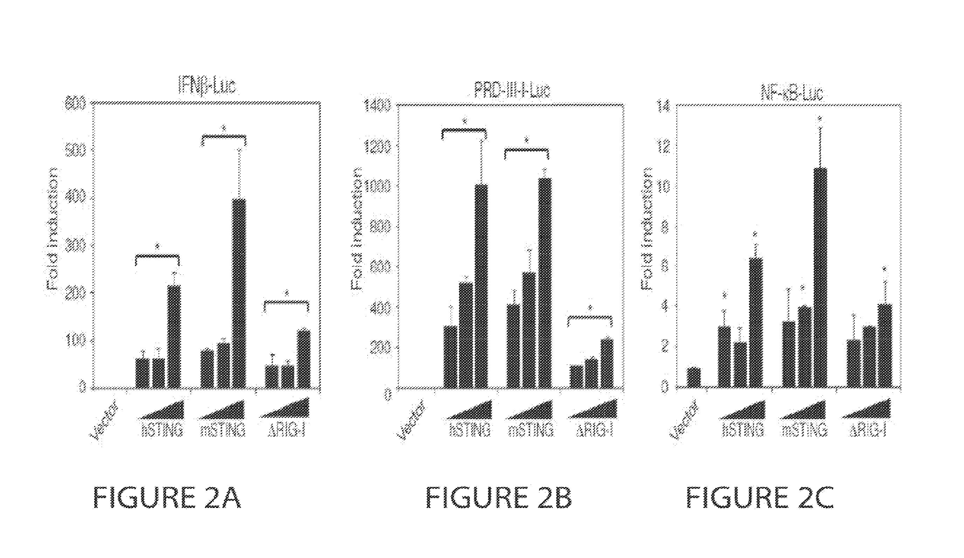

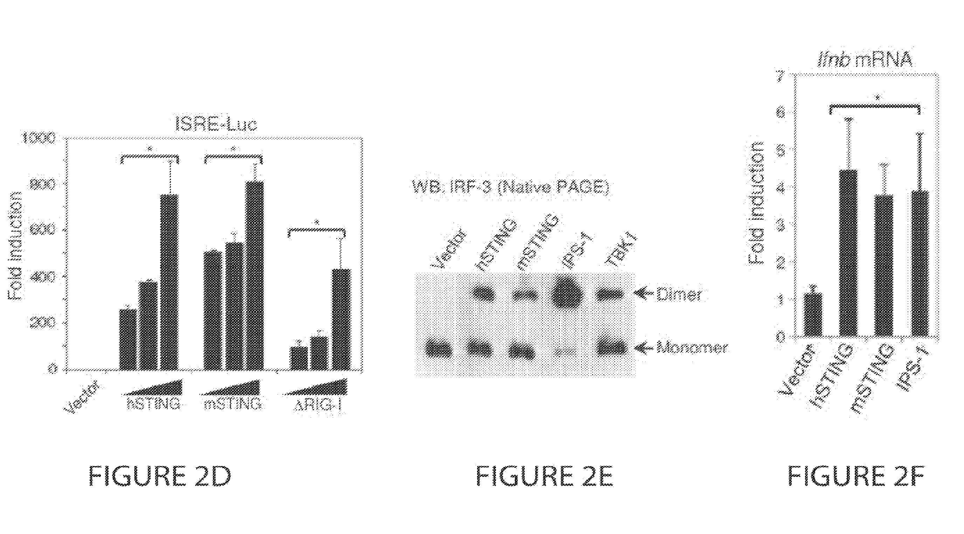

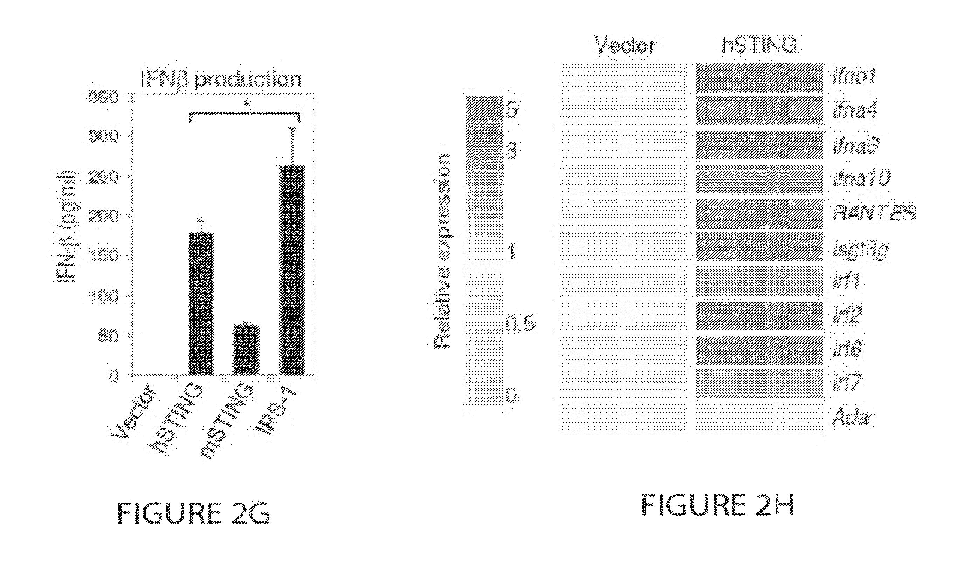

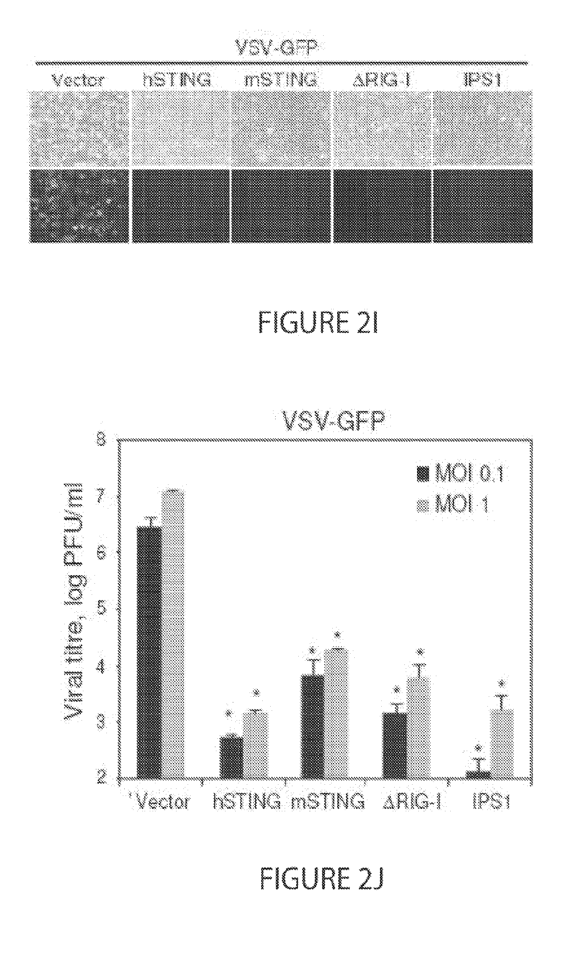

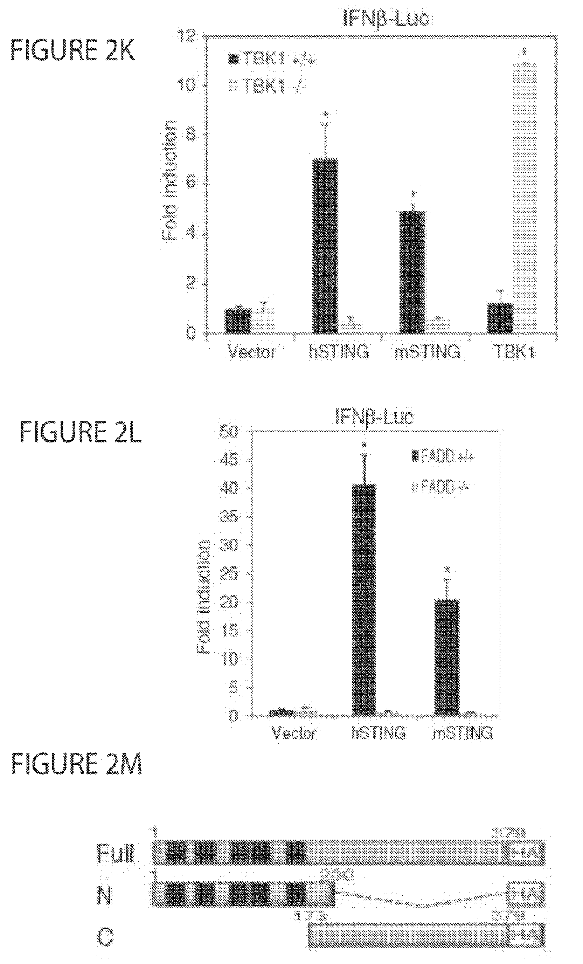

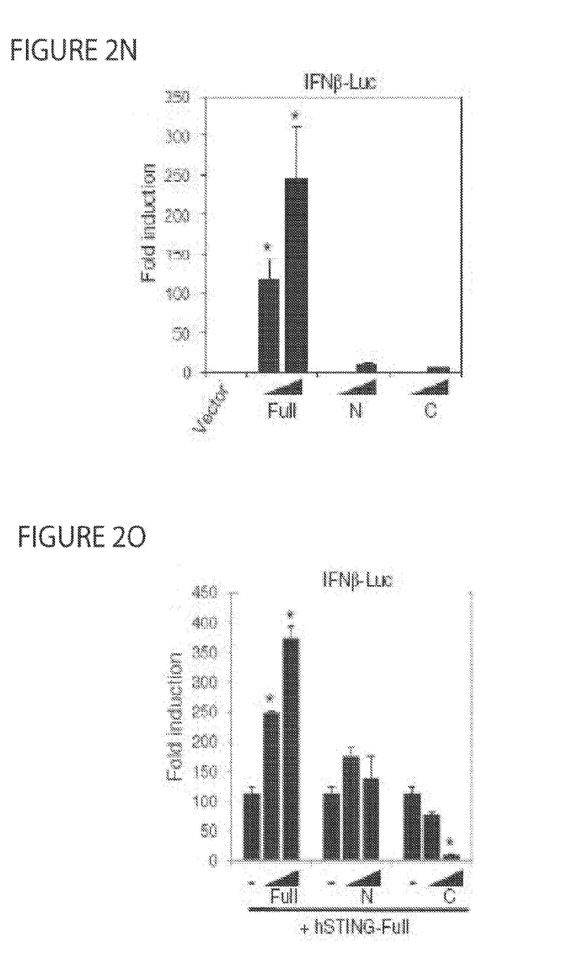

Figure 2A: 293T cells were transfected with 50ng p110-Luc plasmid and 10ng pRL-TK normalization plasmid with increasing amounts of (50ng, 150ng, 250ng) human hSTING, murine mSTING or control ΔRIG-I. Luciferase assays indicating IFNβ promoter activity were taken 36 hours post-transfection. 293T cells transfected as in (Figure 2A) with either PRDIII-I-Luc (Figure 2B), NF-κB-Luc (Figure 2C) or ISRE-Luc (Figure 2D) responsive plasmids were analyzed similarly. Figure 2E: 293T cells were transfected with 250 ng of vector alone or STING, IPS-1 or TBK-1 expressing plasmids for 24hrs and lysed cells analyzed by native gel electrophoresis and by subsequent immunoblot using antibody to detect IRF3 dimerization. Figure 2F: MEF's were transfected with vector alone or hSTING or mSTING or IPS-1 expressing plasmid (500 ng) and mRNA retrieved after 24hrs post-transfection. IFNβ mRNA was analyzed by qRT-PCR. Figure 2G: Medium from transfected MEFs was analyzed for IFNβ protein by ELISA. Figure 2H: 293T cells were transfected with 250ng of control or hSTING expressing plasmid and mRNA retrieved after 36 hrs for analysis by DNA microarray. Figure 2I: MEF's were transfected with vector alone or hSTING or mSTING or ΔRIG-I or IPS-1 expressing plasmid (500 ng) and after 36 hrs post-transfection were infected at an MOI of 1 with VSV-GFP. Figure 2J: Viral replication from experiment (Figure 2H) was measured by plaque assay. Figure 2K: Normal or TBK-1 deficient MEFs were transfected with vector alone, hSTING, mSTING or TBK-1 expressing plasmids (500ng) and 100 ng murine IFNβ-Luc reporter plasmid with 10ng PRL-TK for 24 hrs and luciferase measured. Figure 2L: Normal or FADD -/- MEFs were treated as in (Figure 2K) and luciferase measured. Figure 2M: Schematic of hSTING variants. Figure 2N: 293T cells were transfected as in (Figure 2A) with hSTING full-length or variants and luciferase measured. Figure 2O: 293T cells were transfected with 100ng full length STING and increasing amounts of hSTING-Full, hSTING-N or hSTING-C (0ng, 150ng, 250ng) with luciferase plasmids as in (Figure 2A). Luciferase was measured after 36 hrs. Asterisks indicate significant difference (P < 0.05) as determined by Student's t-test.

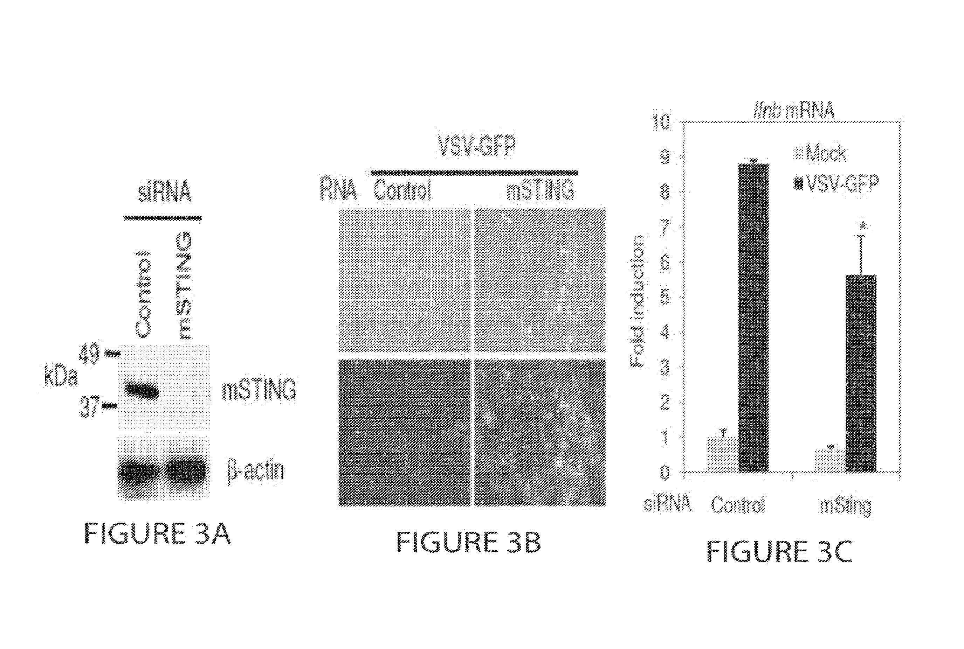

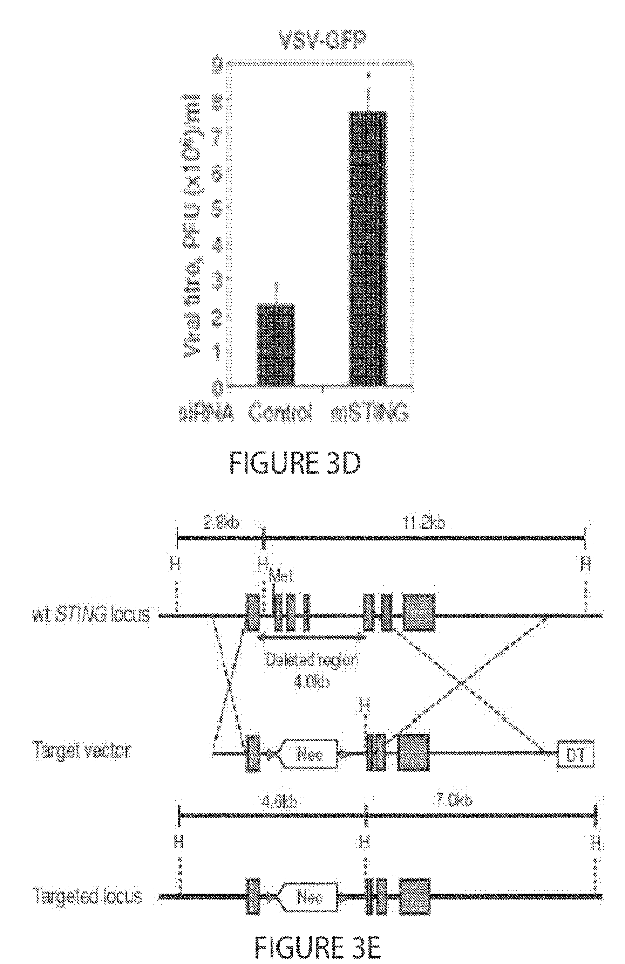

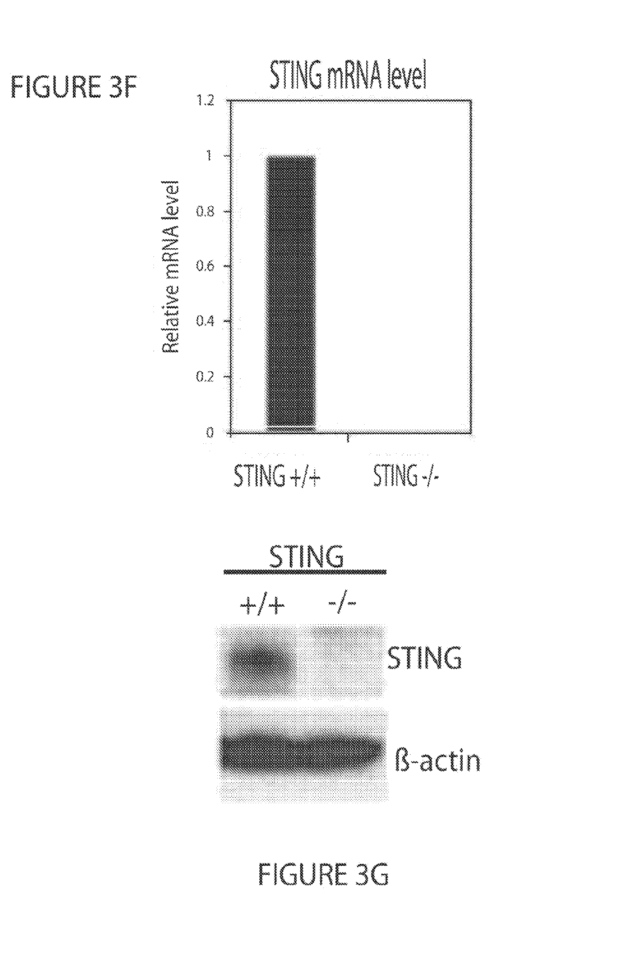

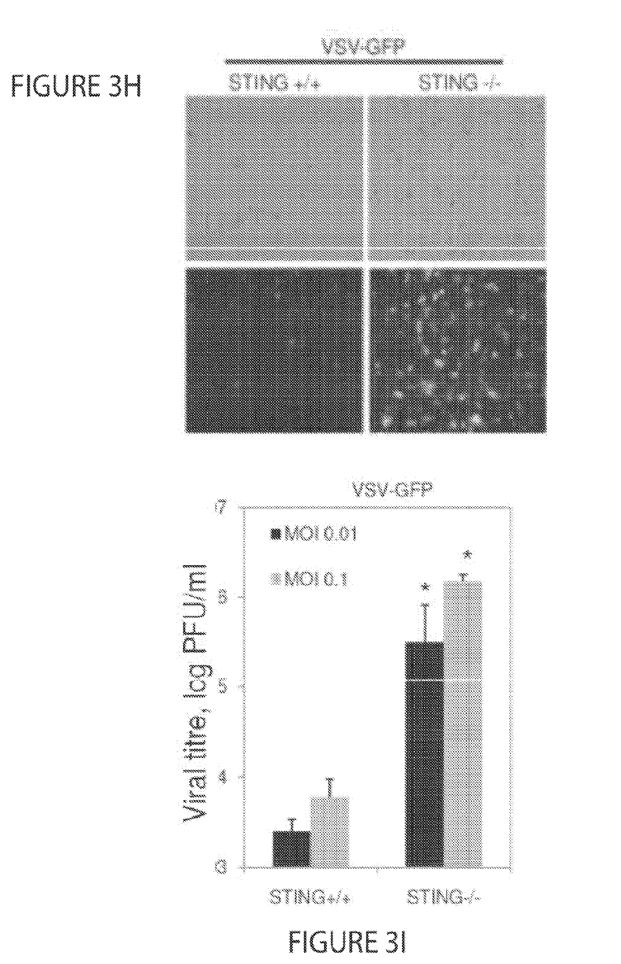

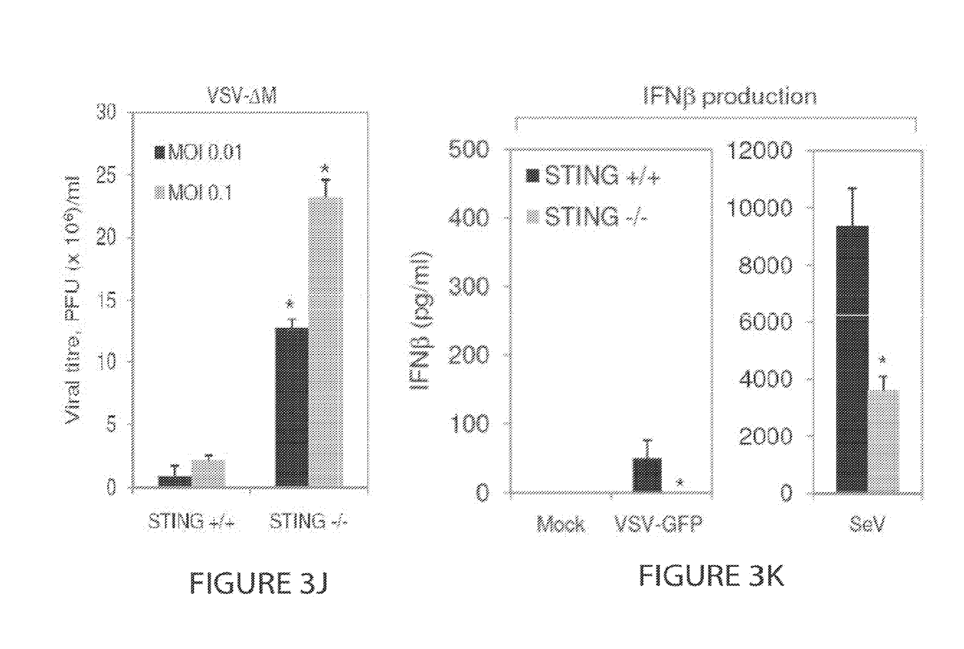

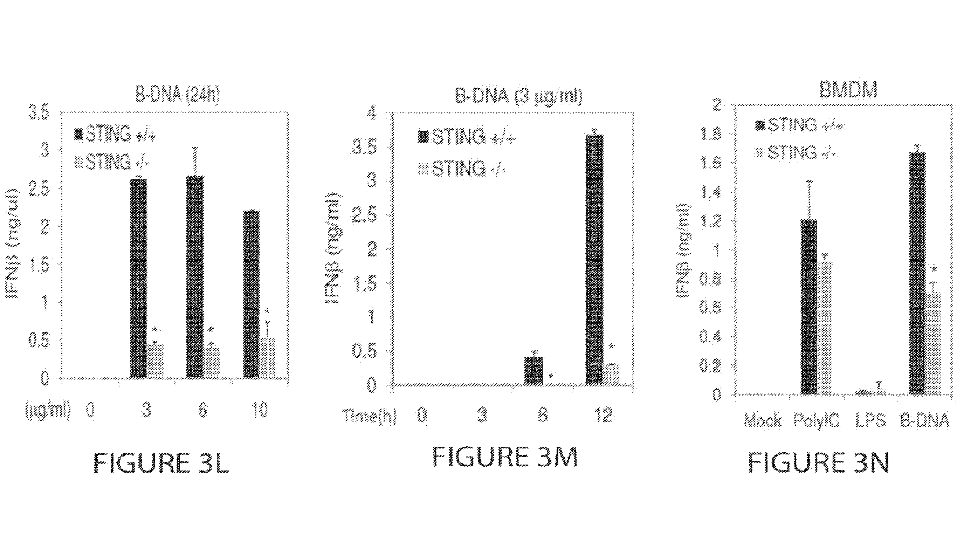

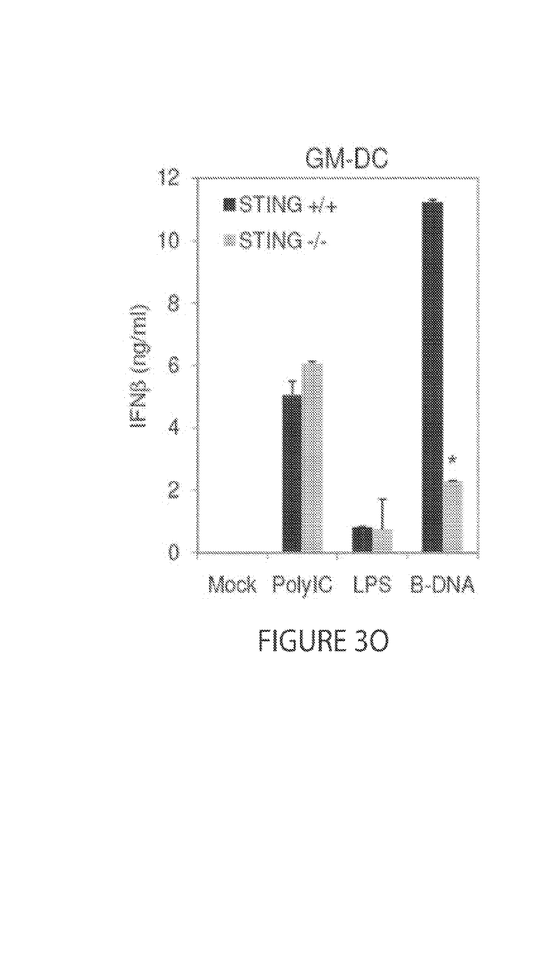

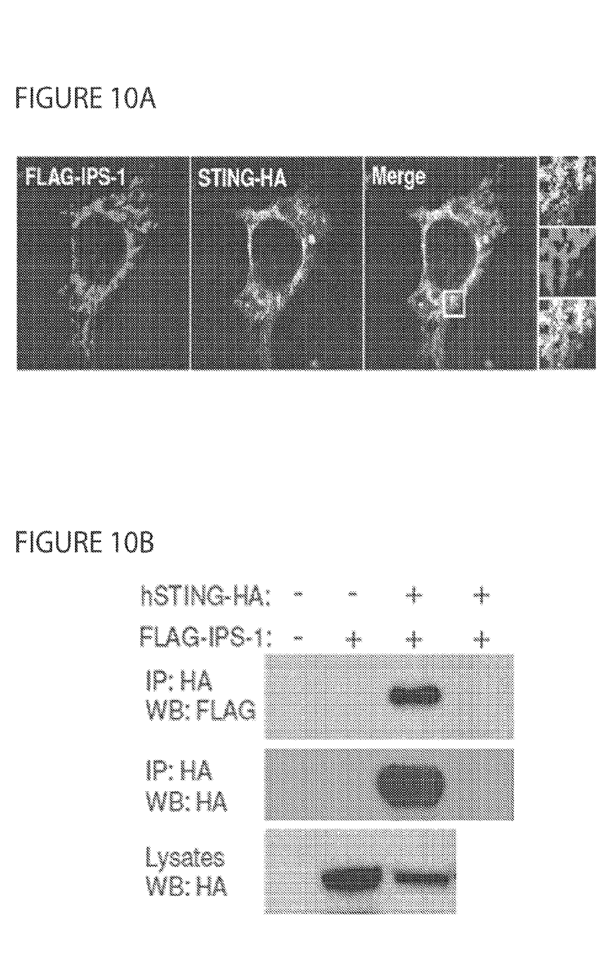

Figure 3A: MEFs (C57/BL6) were treated with RNAi to mSTING and knockdown confirmed after 72 hours by immunoblot using anti-STING rabbit antiserum. Figure 3B: Fluorescence microscopy (GFP) of MEFs treated with RNAi to mSTING following 24 hrs infection with VSV-GFP (MOI 1). Figure 3C: RNAi treated cells were infected with VSVGFP (MOI 1) for 16 hrs and IFNβ mRNA measured using quantitative RT-PCR. Figure 3D: Viral titers taken from RNAi treated or untreated MEFs after 24 hours. Figure 3E schematic diagram depicting targeted homologous recombination strategy of STING in ES cells. Figure 3F: quantitative RT-PCR analysis of STING mRNA in STING -/-or control litter mate MEFs. Figure 3G: Immunoblot of STING -/- cells or control MEFs using antiserum as in (Figure 3A). Figure 3H: Fluorescence microscopy (GFP) of STING -/-or control MEFs following 12 hrs infection with VSV-GFP (MOI 0.1). Figure 3I: Viral titers taken from STING -/- or control MEFs following infection with VSV-GFP after 24 hours. Figure 3J: Viral titers taken from STING -/- or control MEFs following infection with VSVΔM after 24 hours. Figure 3K: Endogenous IFNβ levels measured from STING -/- or control MEFs infected with VSV-GFP (MOI 1) or Sendai Virus (SeV MOI 1) after 24 hours. Figure 3L: STING-/- MEF's or controls were treated with transfected (Lipo 2000 [3µl/ml]) poly dA-dT for 24 hrs and IFNβ measured by ELISA. Figure 3M: Time course analysis of DNA transfected MEFs. Figure 3N: BMDM were treated with exogenous poly I:C (10µg/ml) or LPS (10µg/ml) or transfected poly dA-dT (as in Figure 3L; 10µg/ml) and IFNβ after 24 hours by ELISA. Figure 3O: GM-DC's were treated as in Figure 3N. Asterisks indicate significant difference (P < 0.05) as determined by Student's t-test.

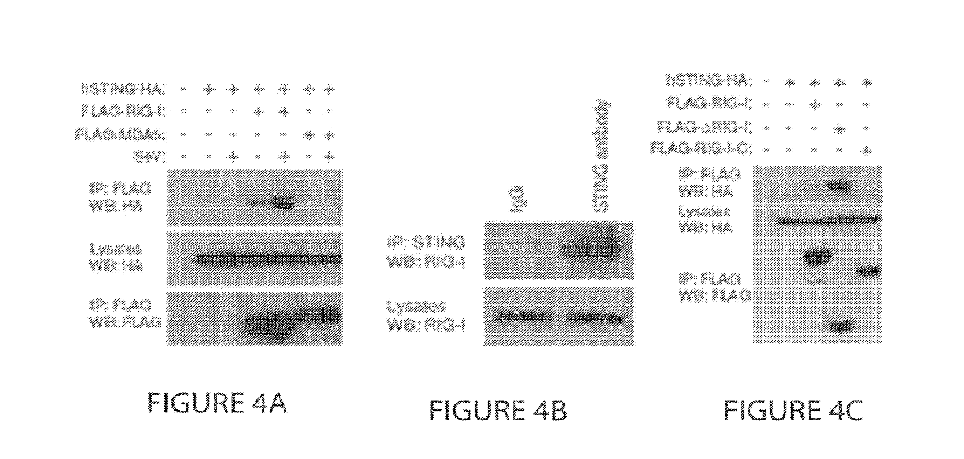

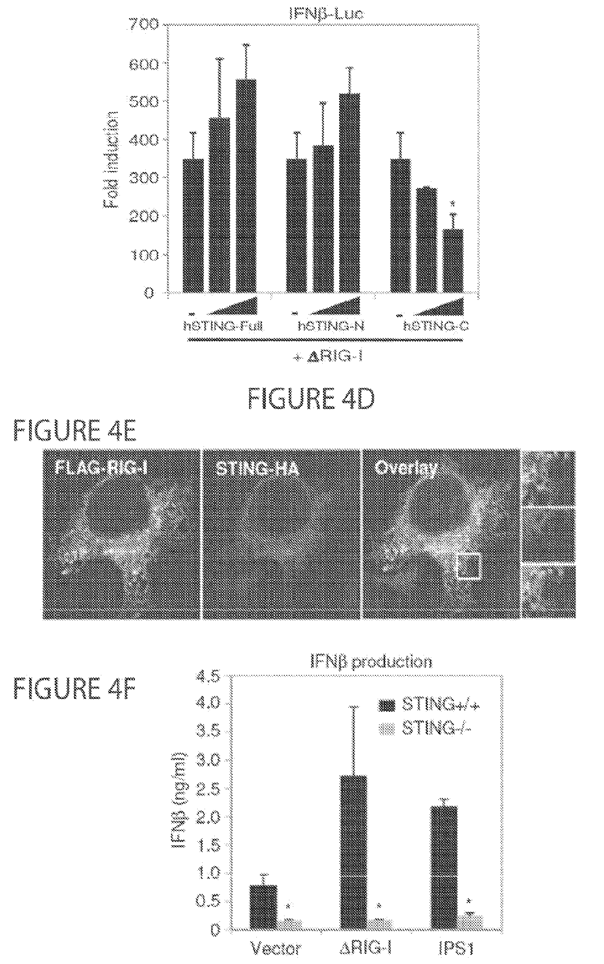

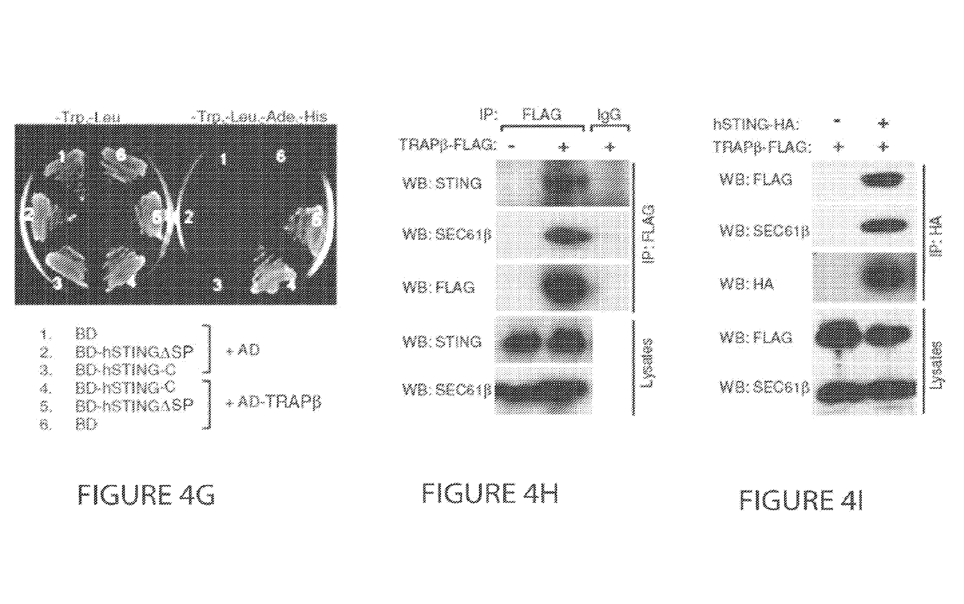

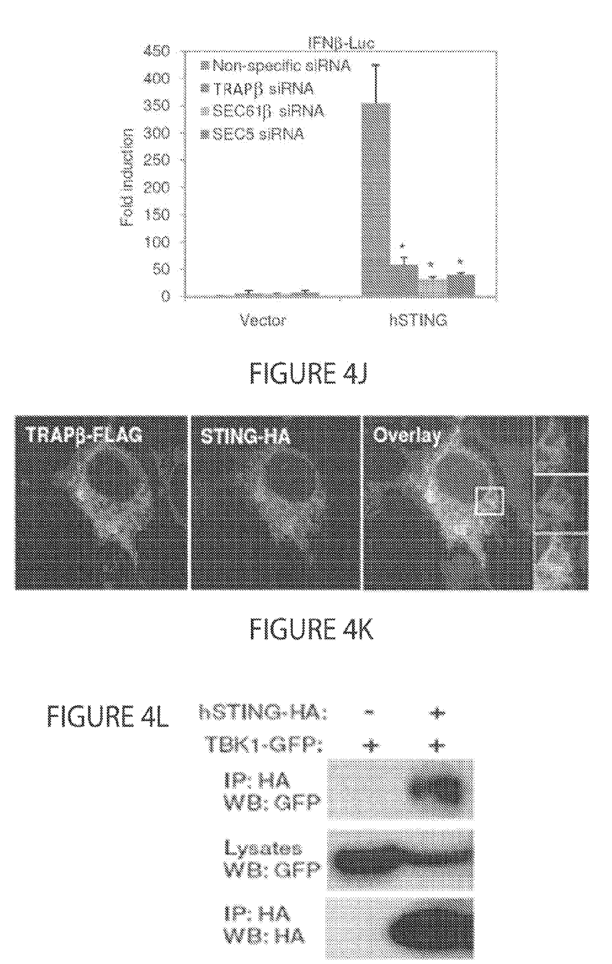

Figure 4A: 293T cells were co-transfected with HA-tagged STING and FLAG-tagged RIG-I or MDA5 for 24 hours. Cells were infected with SeV (MOI 1) for 12 hours. Cells were lysed and co-immunoprecipitated with anti-FLAG antibody and after immunoblotting analyzed using antibody to HA. Figure 4B: HUVECs were lysed and immunoprecipitated using an antibody to endogenous hSTING. Washed precipitates were immunoblotted using an antibody to endogenous RIG-I. Figure 4C: 293T cells were co-transfected with HA-tagged STING and FLAG-tagged RIG-I, ΔRIG-I (aa1-284) or RIG-I-C (aa218-925) for 24 hours. Cells were lysed and co-immunoprecipitated with anti-FLAG antibody and after immunoblotting analyzed using antibody to HA. Figure 4D: 293T cells were co-transfected with control vector (-) or increasing amounts of full-length, amino (aa1-230) or carboxyl (aa173-379) STING (0, 150ng and 250 ng) together with 150ng of ΔRIG-I. Luciferase was measured after 36 hrs. Figure 4E: 293T cells were co-transfected with HA-tagged STING and FLAG-tagged RIG-I for 24 hours. Cells were fixed and stained with anti-FLAG and anti-HA antibody as described in materials and methods. Figure 4F: Control or STING -/- MEFs were transfected with ΔRIG-I (aa1-284) or IPS-1 for 24 hours and endogenous IFNβ measured by ELISA. Figure 4G: STING lacking the first 35 amino acids comprising the signal peptide (amino acids 36-369; BD-hSTINGΔSP) or the carboxyl region of STING (amino acids 173-379; BD-hSTING-C) was fused to the binding domain of yeast GAL4 transcription factor and used to screen a human IFN-inducible fibroblast cDNA library fused to the activation domain of the yeast GAL4 transcription factor as described in materials and methods. BD-hSTING-C was found to interact with Ssr-2/TRAPβ (AD-hTRAPβ) but not control vector (AD) or negative control (AD vector containing). Efficiency of yeast harboring both BD (-Trp) and AD (-Leu) plasmids is shown on left panel (-Trp,-Leu) and high stringency interaction on the right panel (-Trp, -Leu, -Ade, -His). Figure 4H: HEK 293 cells were transfected with FLAG-tagged TRAPβ (+) for 36 hours. Cell lysates were immunoprecipitated with anti-FLAG antibody and immunoblotted using antibody to endogenous STING or endogenous Sec61β. Figure 4I: HEK 293 cells were transfected with FLAG-TRAPβ and HA-STING for 36 hours. Cells were lysed and immunoblotted using antibody to endogenous Sec61β, or FLAG or HA. Figure 4J: 293T cells were treated for 48 hours with RNAi to TRAPβ, SEC61β or Sec5. Cells were then co-transfected with HA-STING and IFNβ-Luc for 24 hours. Luciferase activity was measured as described. Figure 4K: 293T cells were co-transfected with HA-tagged STING and FLAG-tagged TRAPβ for 24 hours. Cells were fixed and stained with anti-FLAG and anti-HA antibody as described in materials and methods. Figure 4L: HEK 293 cells were transfected with HA-STING and GFPTBK-1 for 36 hours. Co-immunoprecipitation experiments were done using and HA-tagged antibody and immunoblot carried out using an antibody GFP. Asterisks indicate significant difference (P < 0.05) as determined by Student's t-test.

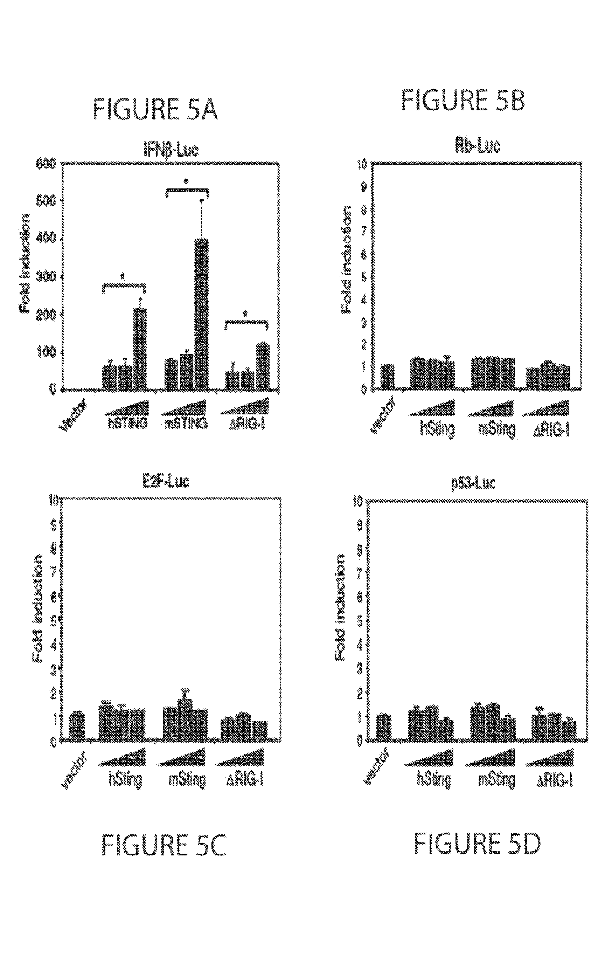

Figures 5A-5D are graphs showing 293T cells were co-transfected with STING or ΔRIG-I as in Figure 2A using an IFNβ (p110), RB, E2F or p53 promoter driving luciferase reporter. 36 hours later, luciferase activity was measured. Asterisks indicate significant difference (P < 0.05) as determined by Student's t-test.

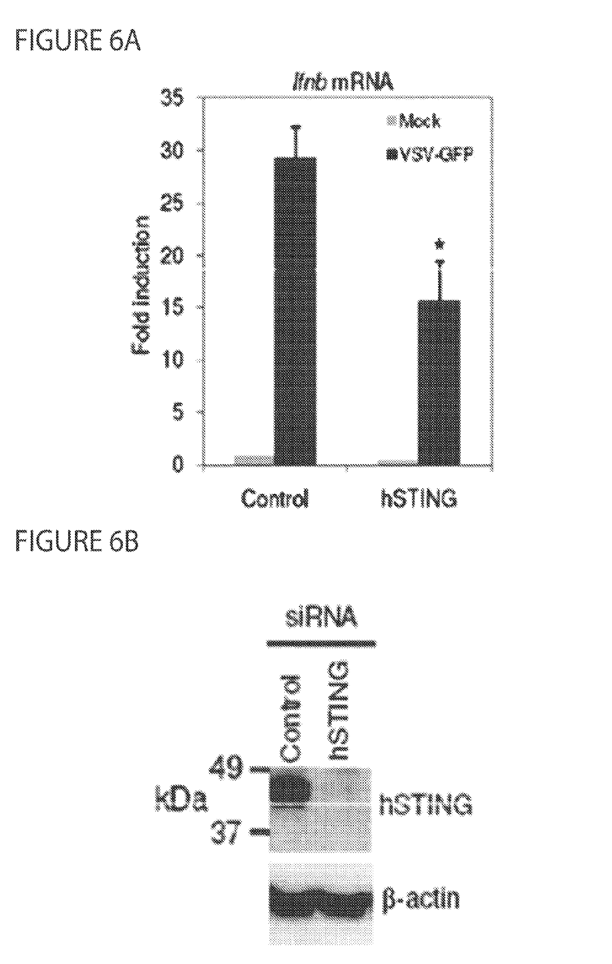

Figure 6A is a blot showing the confirmation of hSTING RNAi knockdown, as described in materials and methods, in HEK 293 cells by immunoblot using anti-STING rabbit antiserum, after 72 hours. Figure 6B: is a graph showing RNAi treated cells, as in (Figure 6A), which were infected for 16 hrs with VSV-GFP (MOI 1) and IFNβ mRNA measured using quantitative RT-PCR. Asterisks indicate significant difference (P < 0.05) as determined by Student's t-test.

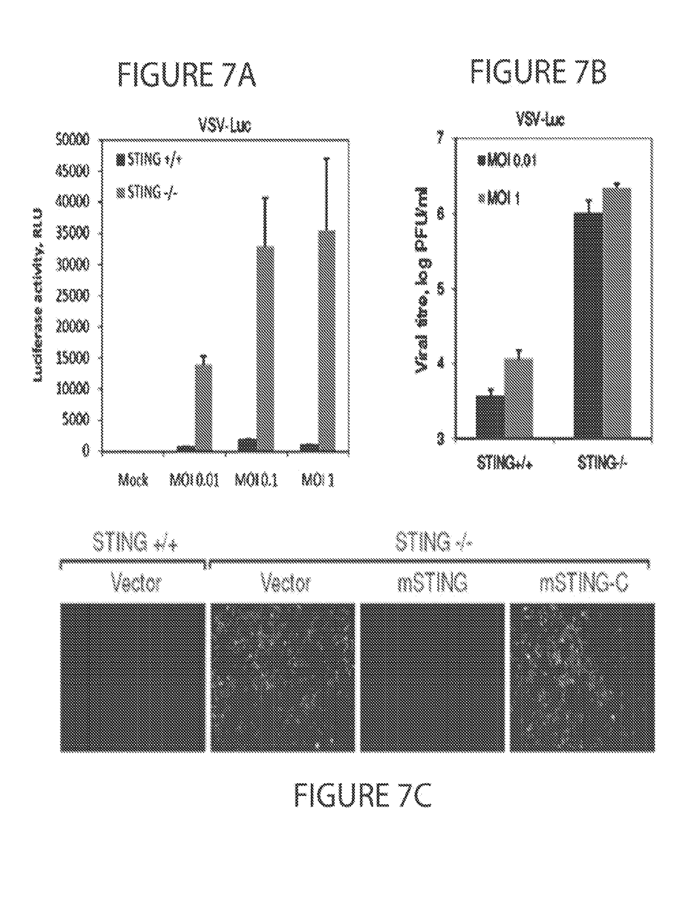

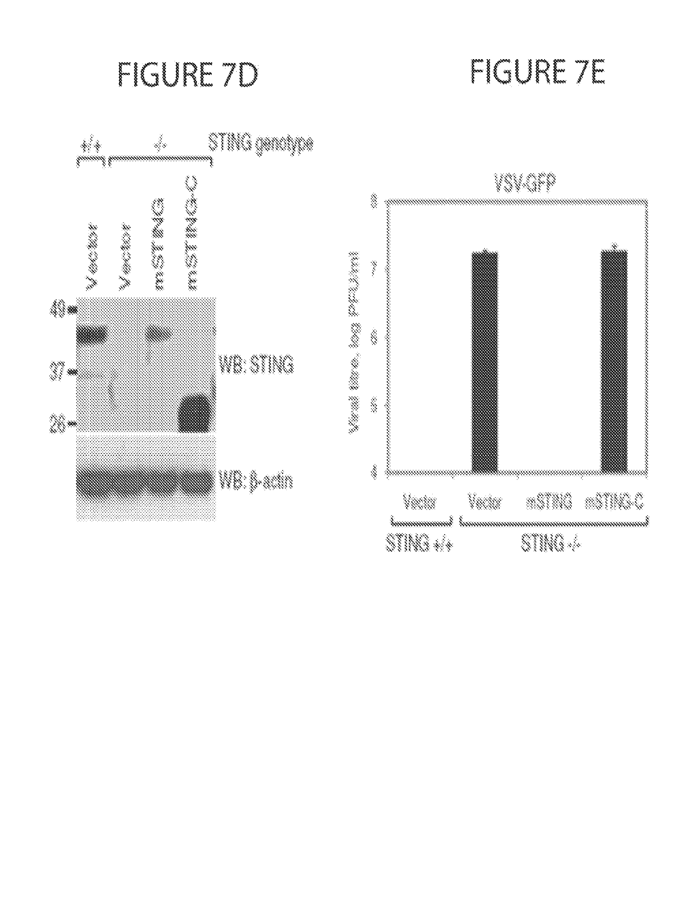

Figure 7A is a graph showing luciferase activity which was measured in STING ^{-/-} or control MEFs following infection with VSV-Luc after 24 hours. Figure 7B is a graph showing the viral titers taken from STING^{-/-} or control MEFs following infection with VSV-Luc after 24 hours. Figure7C is a scan of photographs showing that STING ^{-/-} or control MEF's were transfected with STING or STING carboxyl region (aa173-378) for 24 hours. Cells were then infected with VSV-GFP (MOI 0.1) for 24 hours. Fluorescence was detected using microscopy as in Figure 3B. Figure 7D is an immunoblot confirming expression of reconstituted STING using anti-STING antibody. Figure 7E is a graph showing VSV-GFP titers of Figure 7C which were measured 24 hours post-infection by plaque assay.

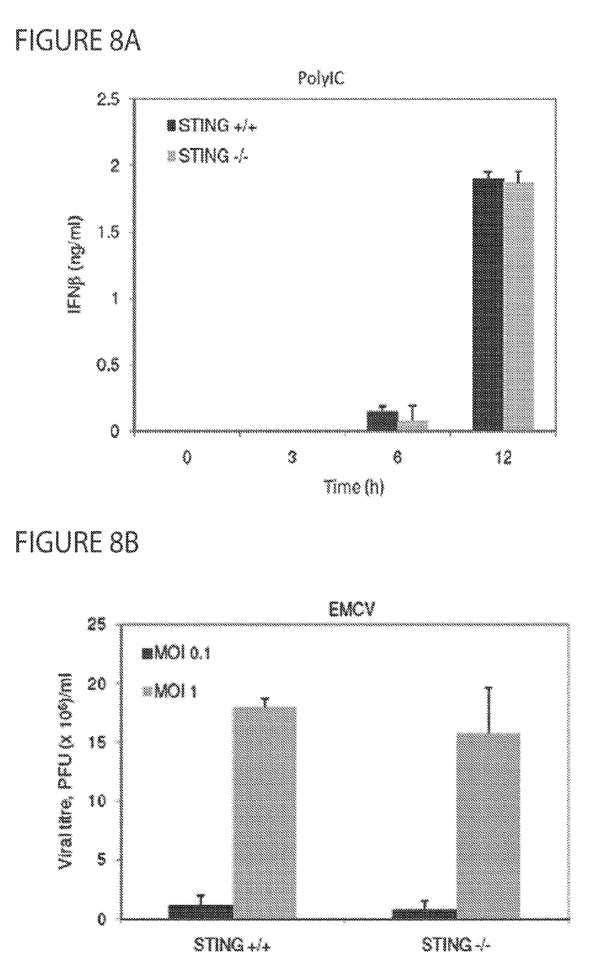

Figure 8A is a graph showing STING ^{-/-} or control MEFs which were transfected with poly I:C (1 µg/ml in Lipo 2000-3µl/ml). IFNβ was measured using ELISA as described in materials and methods. Figure 8B is a graph showing that EMCV was used to inoculate STING ^{-/-} or control MEF's. Virus replication was measured by plaque assay 24 hours later using BHK cells.

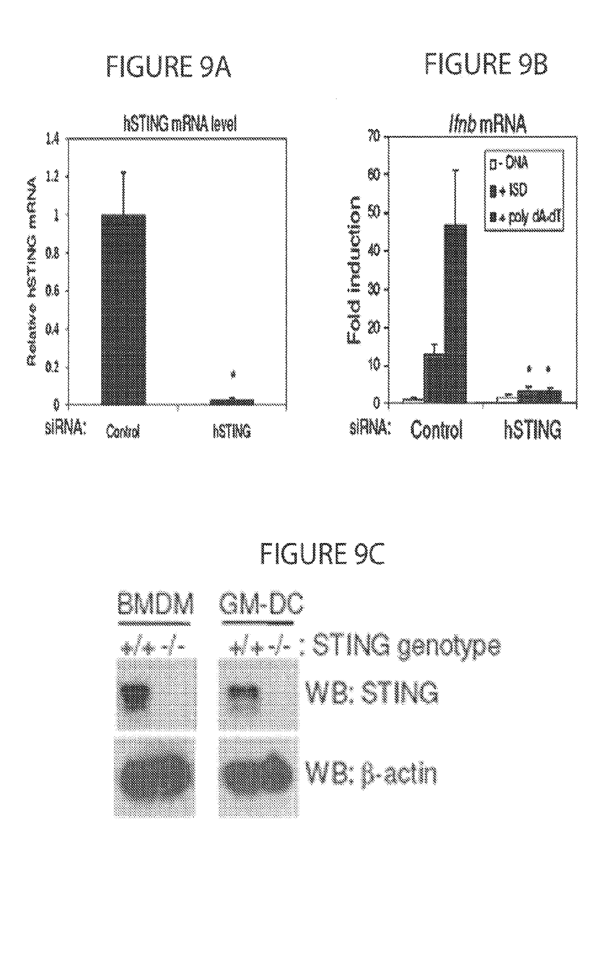

Figure 9A is a graph showing HeLa cells which were treated with RNAi to STING for 72 hours. Quantitative-RT-PCR was used to confirm knockdown. Figure 9B is a graph showing that after 72 hours of RNAi treatment, ISD (3µg/ml with Lipo 2000 3µl/ml) or poly dA-dT (3µg/ml with Lipo 2000 3µl/ml) was used to treat cells for 6 hours. IFNβ mRNA production was measured using quantitative-RT-PCR. Figure 9C showing: BMDM or GM-DC's from STING deficient mice were analyzed by immunoblot using anti-STING antiserum. Figure 9D: is a graph showing BMDM from STING deficient mice which were infected with VSV-GFP (MOI 1) for 24 hours. Virus replication was measured by plaque assay as described in materials and methods. Figure 9E is a graph showing GM-DC's from STING deficient mice which were infected with VSV-GFP (MOI 1) for 24 hours. Virus replication was measured by plaque assay as described in materials and methods. Asterisks indicate significant difference (P <0.05) as determined by Student's t-test.
Figure 10A is a photograph showing 293T cells which were co-transfected with FLAG-IPS-1 and HA-STING or control vector. After 24 hours, cells were fixed as described in materials and methods and examined using confocal microscopy as described in materials and methods. Figure 10B: is a blot showing 293T cells which were co-transfected with FLAG-IPS-1 and HA-STING or control vector (1 µg/ml). After 24 hours, cells were lysed and immunoprecipitated using HA-antibody. Washed immunoprecipitates were separated using SDS/PAGE and immunoblotted using anti-FLAG antibody.

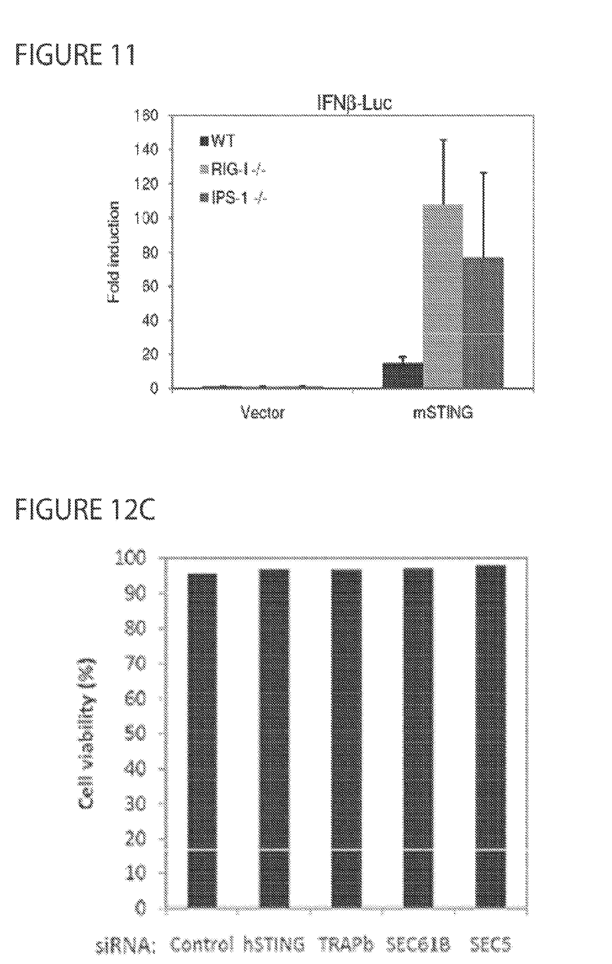

Figure 11, is a graph showing RIG-I or IPS-1 deficient MEFs, or control MEFs which were co-transfected with mSTING (1µg/ml) and IFNβ-Luc (100 ng/ml) for 24 hours. Luciferase activity was measured as described in materials and methods.

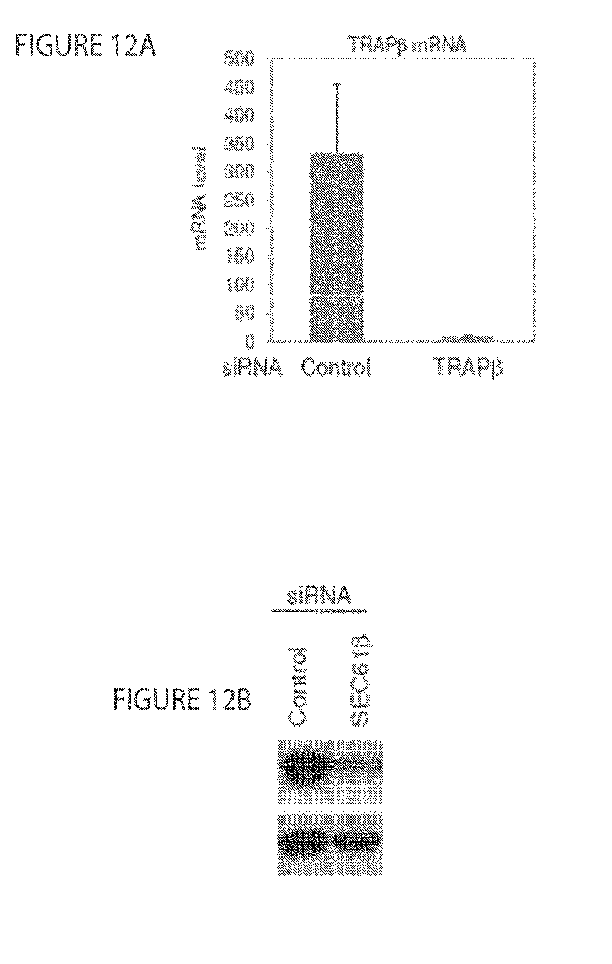

Figure 12A is a graph showing 293T cells which were treated with RNAi to TRAPβ or control RNAi for 72 hours. Confirmation of knockdown was carried out using quantitative-RT-PCR. Figure 12B is a blot showing 293T cells were treated with RNAi to Sec61β or control RNAi for 72 hours. Confirmation of knockdown was carried out using an antibody to Sec61β. Figure 12C is a graph showing that after 72 hours cell viability of the RNAi treated 293T cells was measured using Trypan blue exclusion analysis.

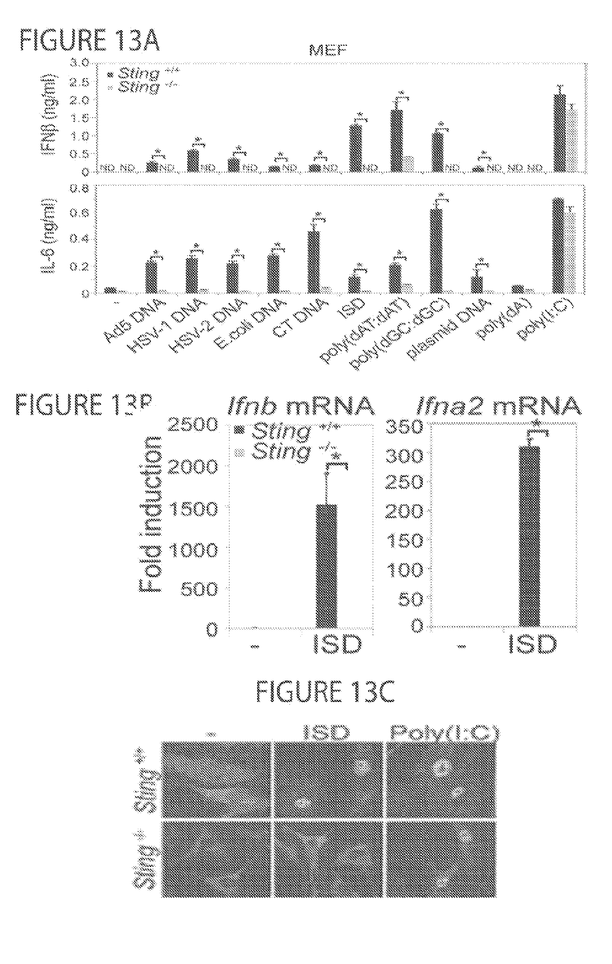

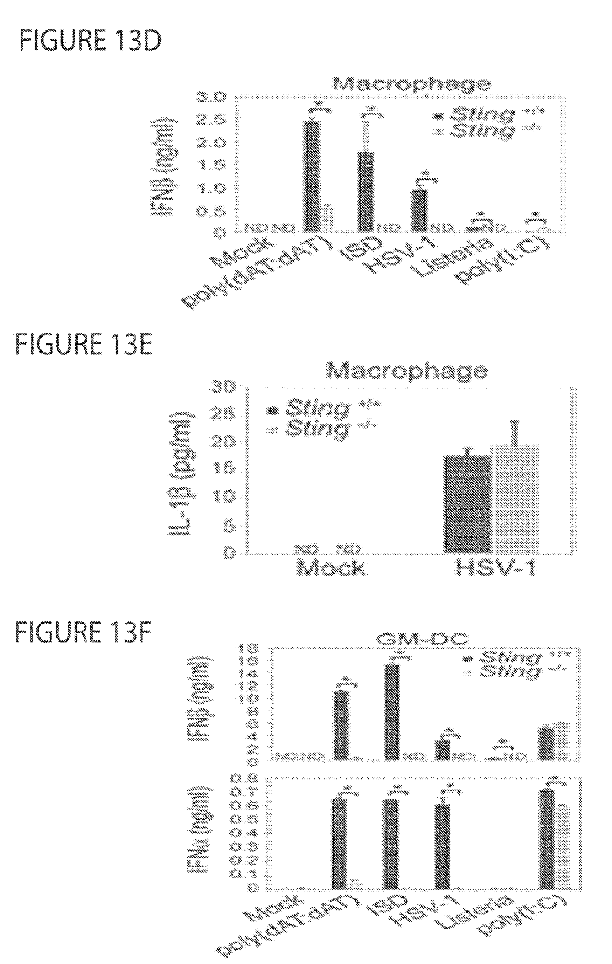

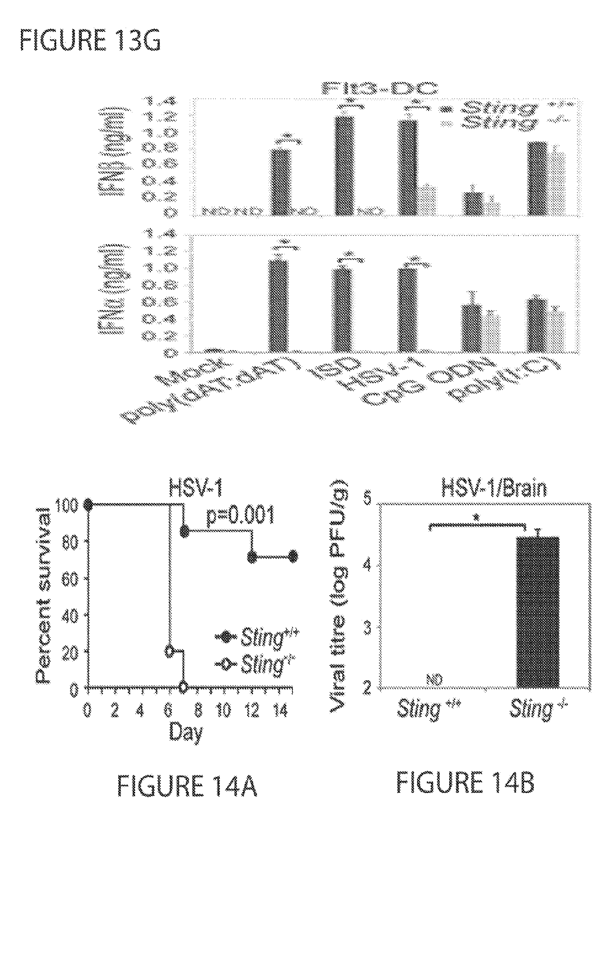

Figures 13A-13G show that STING is essential for intracellular DNA-mediated IFN production. Figure 13A: Murine embryonic fibroblasts (MEFs) were transfected with 1µg/ml of DNA ligands (with Lipofectamine 2000) for 16 hours and IFNβ or IL-6 measured. Figure 13B: MEF's were transfected with ISD (interferon stimulatory DNA) for 4 hours and Ifnβ or Ifna2 mRNA measured. Figure 13C: MEFs treated as in Figure 13B, was stained by antibody for IRF3 translocation. Figure 13D: Bone-marrow derived macrophages were transfected with poly(dAT:dAT), poly(I:C) or ISD or infected with HSV (moi 10) or Listeria (moi 10) for 16 hours and IFNβ measured. Figure 13E: Macrophages was infected with HSV-1 for 16 hours and IL-1β measured. Figure 13F: GM-CSF induced dendritic cells (GM-DC) treated as in Figure 13D, and IFNβ or IFNα measured after 16 hours. Figure 13G: Flt3 stimulated DC's were treated as in Figure 13F (exogenous CpG-ODN (1µg/ml) was additionally used). Asterisks indicate significant difference (P<0.05) as determined by Student's t-test. Error bars indicate s.d.

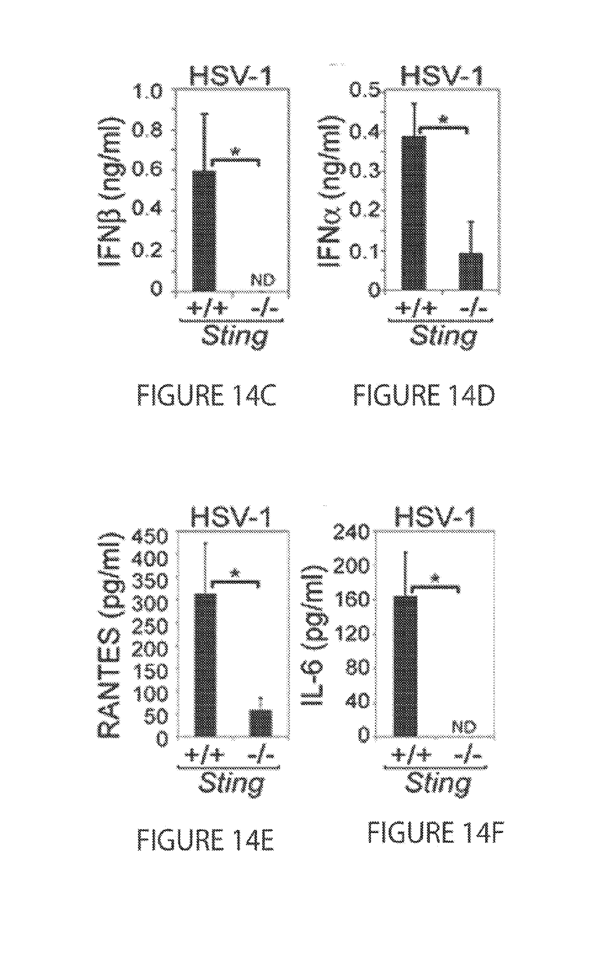

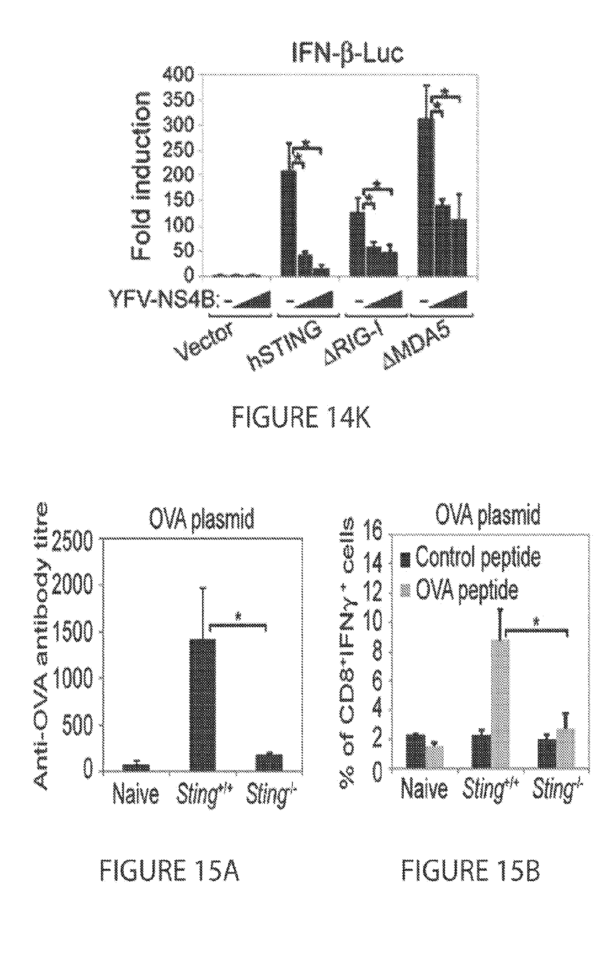

Figures 14A-14K show that STING is required for effective *in vivo* host defense. Figure 14A: STING deficient animals (^{-/-}) or litter mate controls (^{+/+}) (n=7; approximately 8 weeks of age) were infected intravenously (i.v.) with 1 × 10⁷ HSV and survival monitored. Figure 14B: Sting ^{-/-} mice or controls were infected with HSV-1 as in Figure 14A and brain retrieved after 5 days for HSV-1 plaque assays. Figures 14C, 14D: Serum from animals (n=3) infected with HSV (1 × 10⁷ i.v.) were analyzed for IFNβ or IFNα production after 6 hours). Figure 14E, 14F: Serum from animals infected as in Figure 14C, was analyzed for RANTES and IL-6 production. Figure 14G: STING deficient animals or controls (n=6) were infected i.v. with vesicular stomatitis virus (VSV, 5 × 10⁷) and survival monitored. Figures 14H, 14I: Mice (n=3) were treated as in Figure 14G and IFNβ or IFNα measured after 6 hours. Figures 14J: STING deficient animals or controls (n=6) were infected intraperitoneally (i.p) with encephalomyocarditis virus (EMCV, 1 × 10²) and survival monitored. Figure 14K: Increasing amounts of Yellow Fever Virus (YFV) NS4b was co-transfected into 293T cells with STING or the amino terminus of RIG-1 (ΔRIG-I, 1-284) and transfected IFNβ promoter driven luciferase (IFN-β-Luc) measured after 36 hours. Asterisks indicate significant difference (P<0.05) as determined by Student's t-test. Error bars indicate s.d.

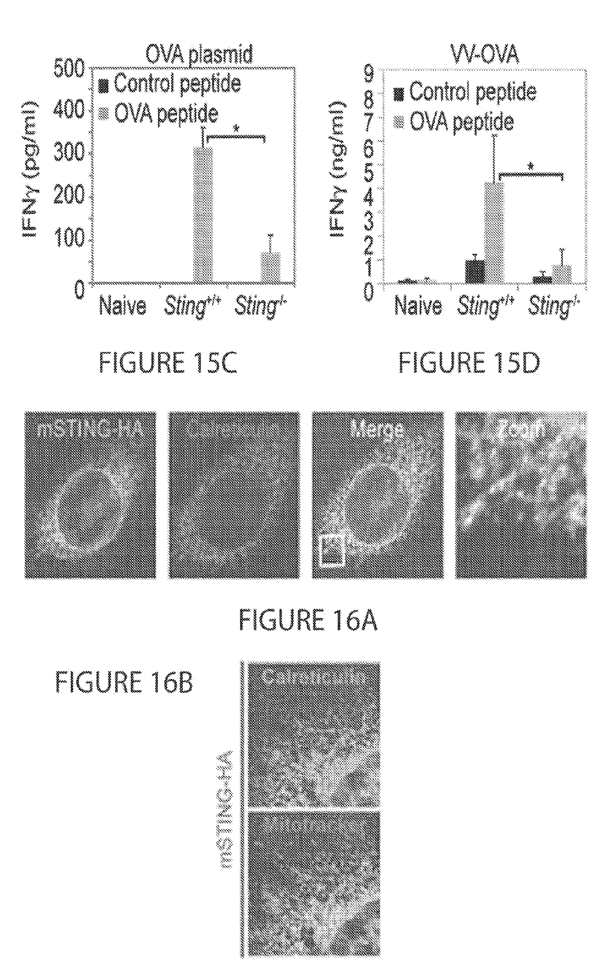

Figures 15A-15D: STING is required for effective DNA-mediated adaptive immune responses. Figure 15A: STING deficient animals (^{-/-}) or controls (^{+/+}) [n=4; approximately 8 weeks of age] were immunized twice (100µg, intramuscularly [i.m.]) by electroporation with a DNA vaccine encoding ovalbumin. Serum was measured for anti-OVA IgG. Figures 15B,15C: Mice were treated as in a and spleen CD8⁺ /IFN-γ⁺ cells measured by FACS and anti-OVA-specific IFN-γ production measured by ELISA. Figure 15D: STING deficient animals (^{-/-}) or controls (^{+/+}) [n=4; approximately 8 weeks of age] were infected with vaccinia expressing ovalbumin (VV-OVA; i.v., 5 × 10⁶) and spleen anti-OVA-specific IFN-γ production measured by ELISA. Asterisks indicate significant difference (P<0.05) as determined by Student's t-test. Error bars indicate s.d.

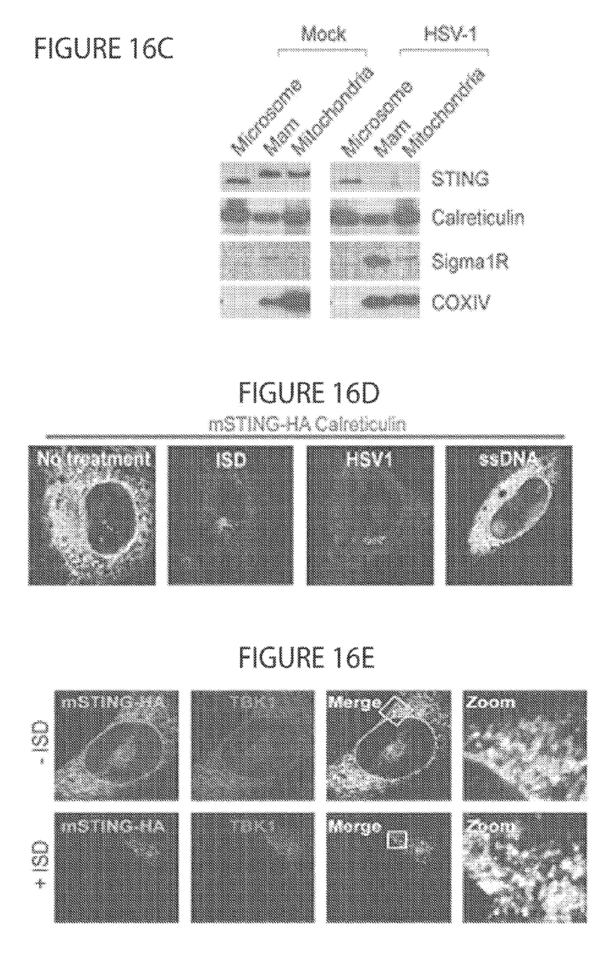

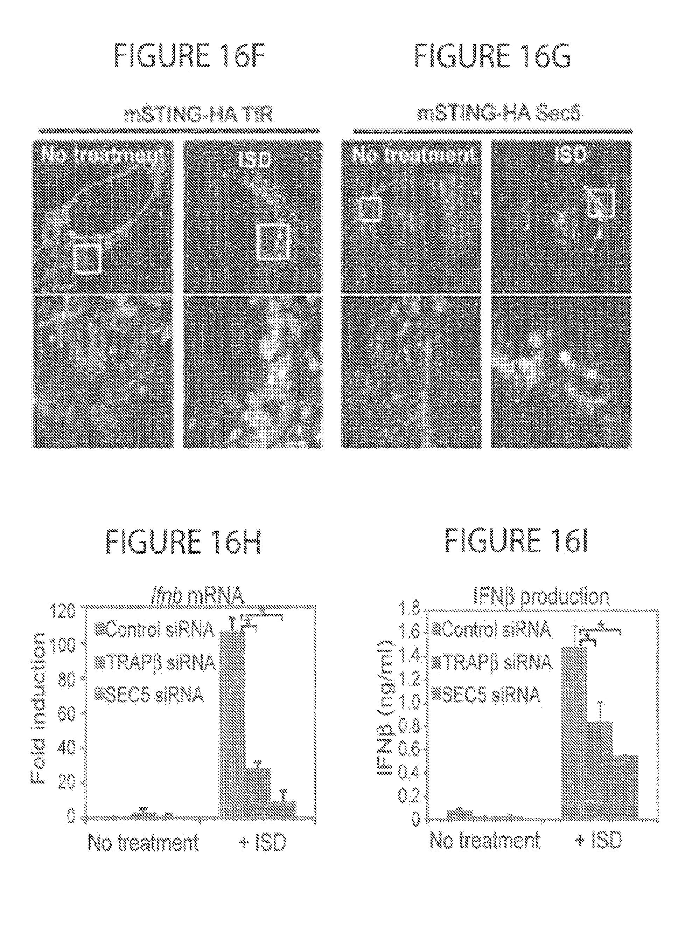

Figures 16A-16I: STING translocates from the ER to Sec5 containing vesicles. Figure 16A: STING ^{-/-} MEFs, stably reconstituted with HA tagged STING (mSTING-HA) were stained using HA (green) and calreticulin (red) antibody. Figure 16B: HA-STING MEFs was stained for HA-Sting (green), calreticulin (blue) or Mitotracker (red). Figure 16C: Immunoblot analysis of fractionation experiments of uninfected or HSV infected (moi 10; 4 hours) HA-STING MEFs (HA for STING, calreticulin, ER; Sigma 1R, mitochondrial associated ER membranes [MAM]; COXIV, mitochondria). Figure 16D: HA (green) or Calreticulin (red) staining of mSTING-HA MEFs following treatment with transfected ISD (1µg/ml), transfected single stranded DNA (ssDNA, 1µg/ml) or HSV infection as in Figure 16C. Figure 16E: mSTING-HA MEFs were transfected with or without ISD and cells stained with HA (green) and TBK-1 (red) antibody. Figure 16F: mSTING-HA MEFs were transfected as in Figure 16E and stained with HA (green) and Transferrin receptor (Tfr, red) antibody. Figure 16G: mSTING-HA MEFs were transfected as in Figure 16E and stained with HA (green) and Sec5 antibody (red). Figures 16H, 16I: MEF's were treated with RNAi to TRAPβ or SEC5 for 72 hours and transfected with ISD. IFNβ mRNA and protein was measured at 4 and 16 hours respectively. Asterisks indicate significant difference (P<0.05) as determined by Student's t-test. Error bars indicate s.d.

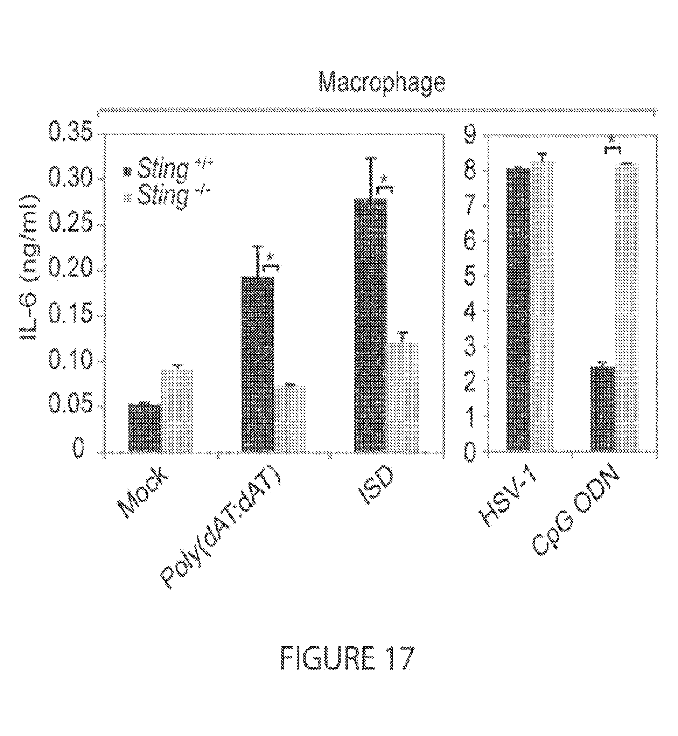

Figure 17: IL-6 production in the absence of STING is defective in response to poly(dAT:dAT) and ISD, but not HSV-1 or CpG ODN. Macrophages were transfected with 1ug/ml of DNA ligands or infected for 16 hours with HSV-1 (moi 10). IL-6 was measured by ELISA. Asterisks indicate significant difference (P<0.05) as determined by Student's t-test. Error bars indicate s.d.

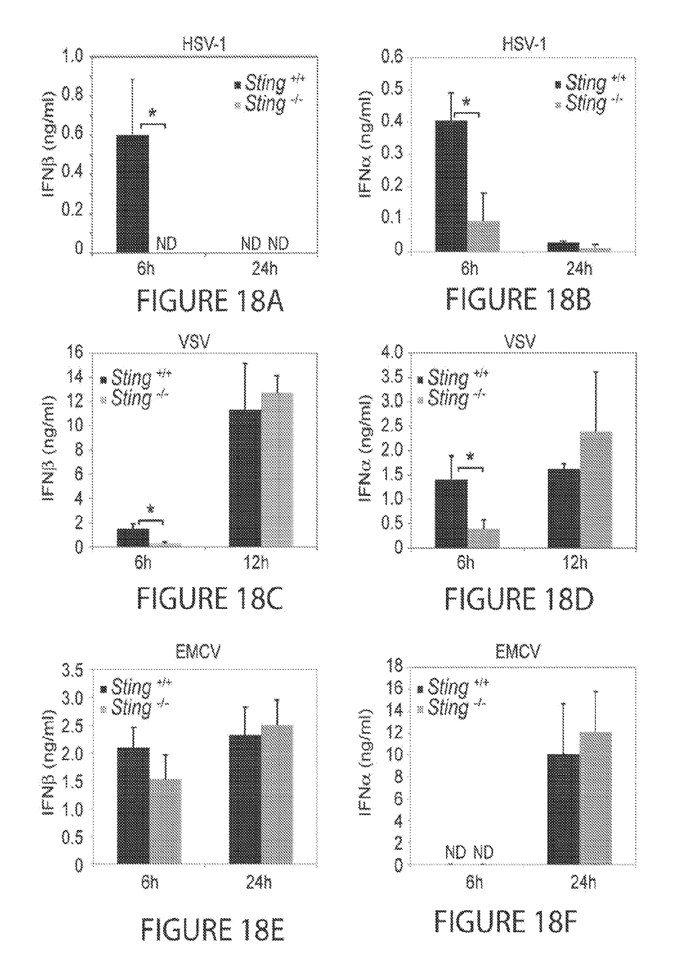

Figures 18A-18F: Defective type I IFN production in viral-infected STING knockout mice. Figures 18A, 18B: STING deficient animals *(Sting*^{*-*/}*⁻)* or litter mate controls (^{+/+}) [n=3; approximately 8 weeks of age] were infected intravenously (i.v.) with 1 × 10⁷ HSV and survival monitored. Serum from animals were analyzed for IFNβ or IFNα production after 24 hours). Figures 18C, 18D: STING deficient animals or controls (n=3) were infected i.v. with vesicular stomatitis virus (VSV, 5 × 10⁷) and IFNβ or IFNα measured after 12 hours). Figures 18E, 18F: STING deficient animals or controls (n=3) were infected intraperitoneally (i.p.) with encephalomyocarditis virus (EMCV, 1 × 10⁵). Serum from animals was analyzed for IFNβ or IFNα production after 6 and 24 hours. Asterisks indicate significant difference (P<0.05) as determined by Student's t-test. Error bars indicate s.d.

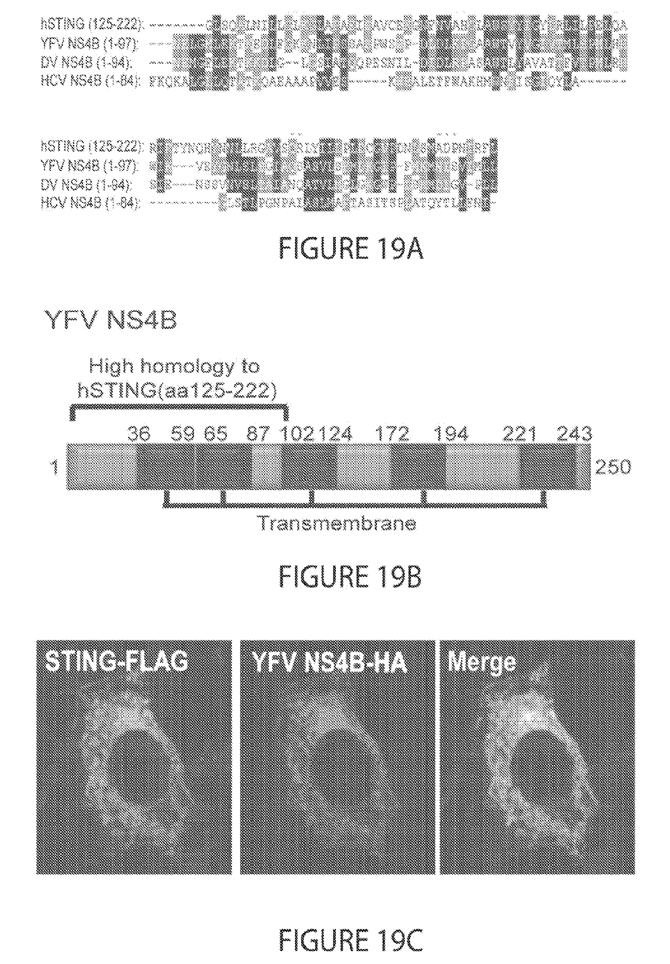

Figures 19A-19C: Human STING (hSTING, amino acids 125-222) exhibits significant homology to flavivirus NS4B proteins. Figure 19A: Homology between Yellow Fever Virus (YFV), Dengue Virus (DV) and hepatitis C virus (HCV) NS4b are shown. Figure 19B: Schematic of YFV NS4b homology versus STING. Figure19C: 293T cells were co-transfected with FLAG-tagged STING and HA tagged YFV-NS4B for 36 hours and observed by confocal microscopy.

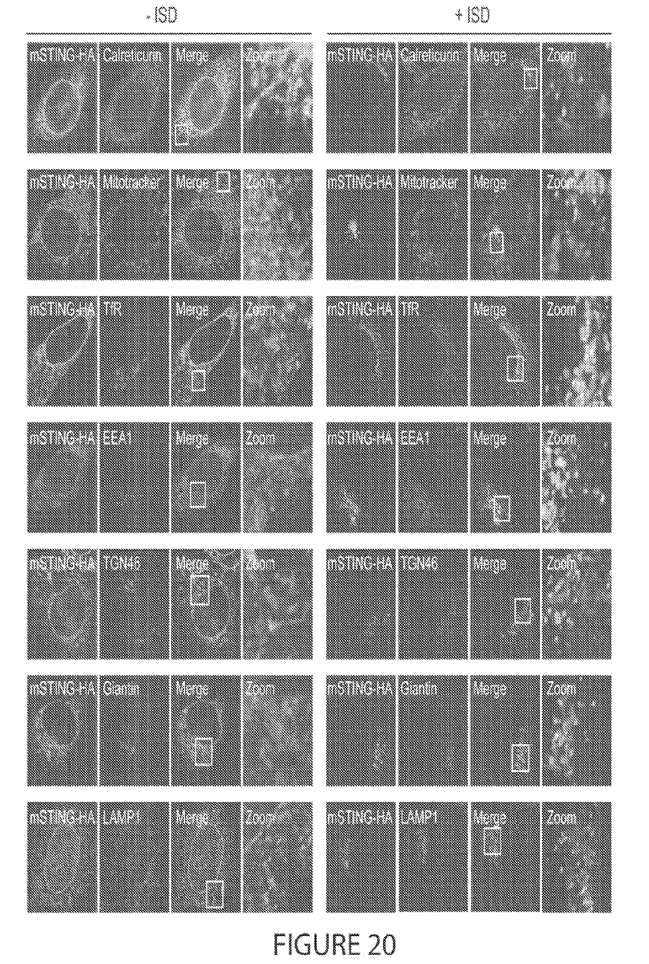

Figure 20: STING translocates to endosomes following exposure to ISD. STING^{-/-} MEFs, stably reconstituted with HA tagged STING (mSTING-HA) were treated with or without transfected ISD (4 hours) and stained using HA antibody (green) and either calreticulin, ER; mitotracker, mitochondria; TfR, recycled endosome; EEA1, early endosome; TGN46, trans-Golgi; Giantin, cis-Golgi; LAMP1, lysosome (red).

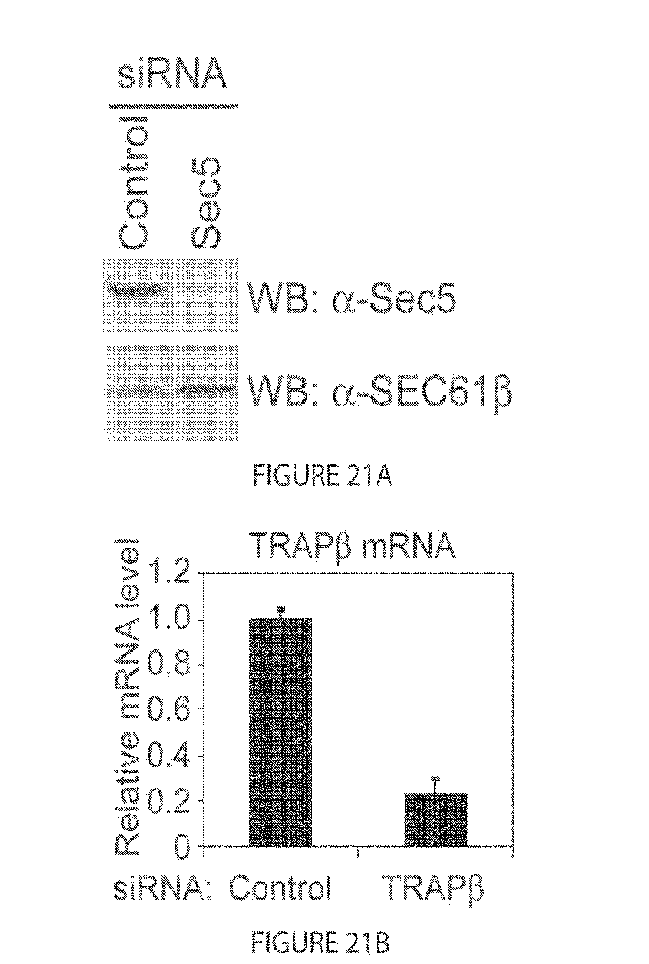

Figures 21A, 21B: Confirmation of RNAi knockdown in MEFs. Figure 21A: Immunoblot of RNAi (to Sec5) treated cells indicating knockdown of Sec5. Figure 21B: RT-PCR measurements of RNAi (Trapβ) treated cells indicating knockdown of Trapβ.

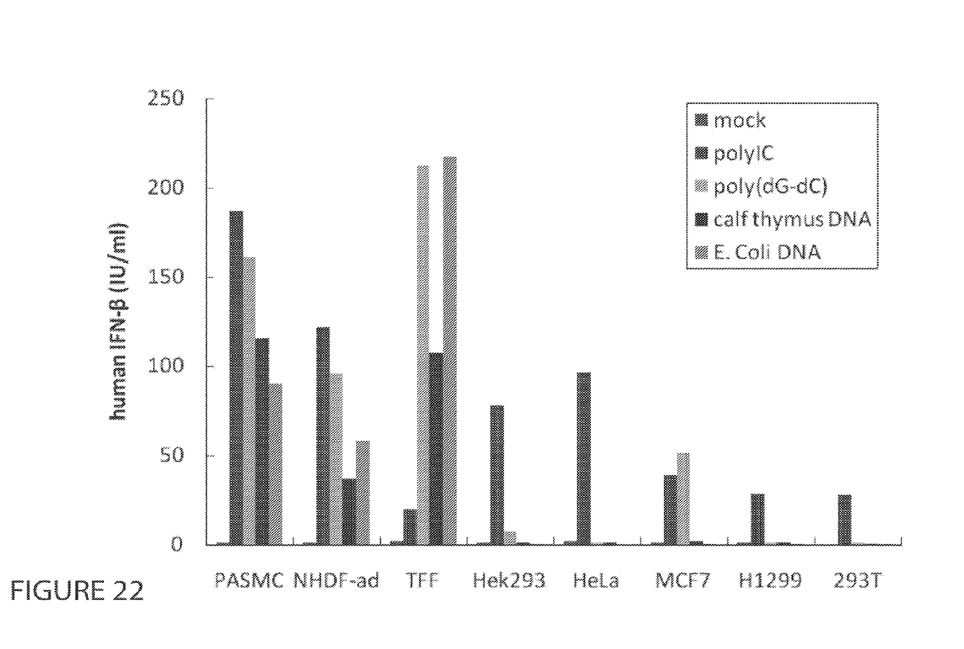

Figure 22 is a graph showing that the intracellular DNA pathway is defective in human cancer cells, but not normal cells. Normal human cells (PASMC, NHDF-ad and TFF) or human cancer cell lines (Hek293, HeLa, MCF7, H1299 and 293T) were either mock transfected or transfected with Lipofectamine and 5 µg/ml of polyIC, poly(dG-dC), calf thymus DNA or *E. coli* DNA. Culture supernatants were collected 16 hours after transfection and endogenous IFN-β was analyzed by ELISA (Invitrogen). The data indicates that dsRNA pathways (polyIC), but not intracellular DNA (poly dG-dC, calf thymus DNA or *E. coli* DNA) pathways which are regulated by STING are functional.

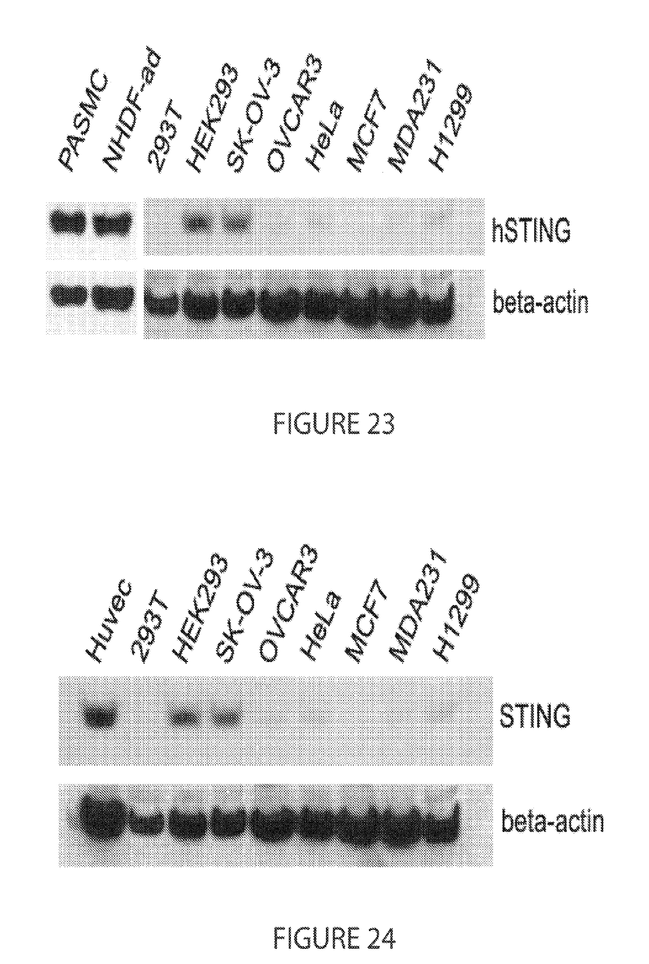

Figure 23 is a blot showing that STING is absent in numerous cancer cell-lines but not normal cells. Western blot of endogenous STING expression in normal human cells (PASMC, NHDF-ad) and cancer cells lines (293T-H1299): 30 µg of whole cell extracts were resolved by 10% SDS-PAGE followed by western blot using either rabbit-anti-human-STING antiserum or mouse-anti-beta-actin monoclonal antibody (Sigma). Names of cells tested are labeled above the image.
Figure 24 is a Western blot of endogenous STING expression in various human cell lines: 30 µg of whole cell extracts were resolved by 10% SDS-PAGE followed by western blot using either rabbit-anti-human-STING antiserum or mouse-anti-beta-actin monoclonal antibody (Sigma). Names of cell lines tested are labeled above the image.

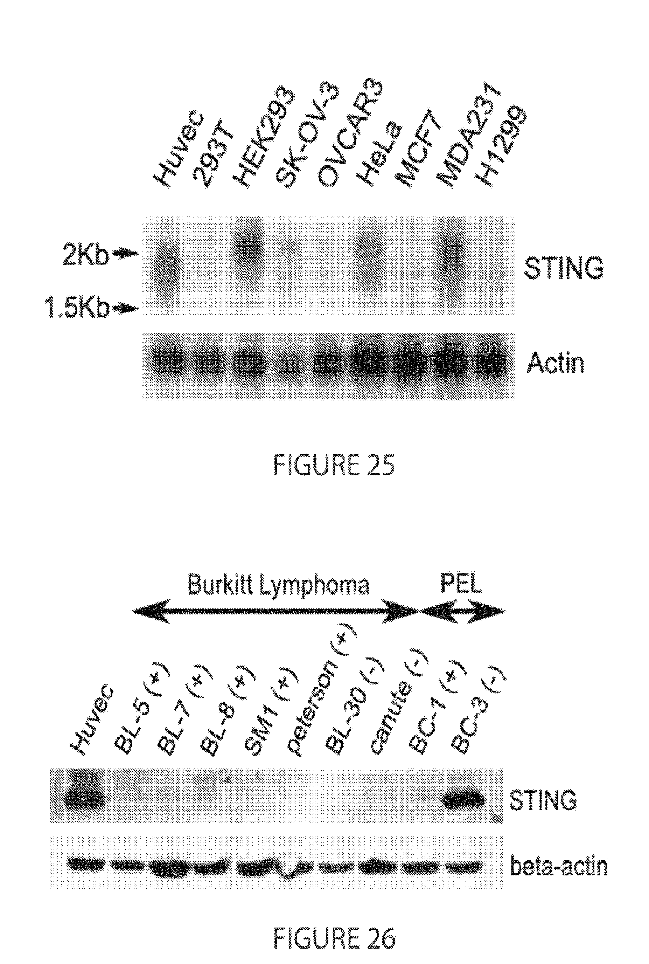

Figure 25 is a Northern blot of endogenous STING expression in various human cell lines: mRNAs were extracted using the MicroPoly(A)Purist™ Kit (Ambion). Northern blot was performed following the NorthernMax®-Gly Kit (Ambion) procedure. Briefly, 2 µg of each mRNA was resolved by 1% agarose gel, transferred and immobilized on Ambion BrightStar®-Plus Membrane, probed with 32P-labled human-STING or β-actin DNA probes, and finally, exposed for autoradiography. Names of cell lines tested are labeled above the image.
Figure 26 is a Western blot of endogenous STING expression in various human lymphoma cell lines: 30 µg of whole cell extracts were resolved by 10% SDS-PAGE followed by western blot using either rabbit-anti-human-STING antiserum or mouse-anti-beta-actin monoclonal antibody (Sigma). 7 human Burkitt lymphoma lines (BL-5, BL-7, BL-8, SM1, peterson, BL-30 and canute) and 2 primary effusion lymphoma lines (BC-1 and BC-3) were tested in this western blot. Huvec cell was used as a positive control. (+), EBV positive; (-), EBV negative.

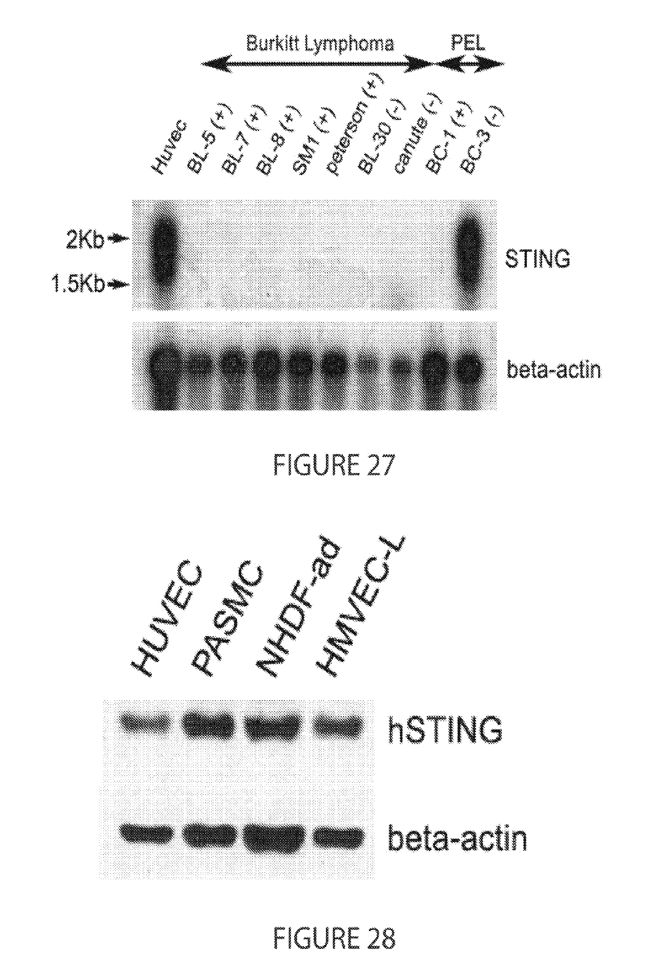

Figure 27 is a Northern blot of endogenous STING expression in various human lymphoma cell lines: mRNAs were extracted using the MicroPoly(A)Purist™ Kit (Ambion). Northern blot was performed following the NorthernMax®-Gly Kit (Ambion) procedure. Briefly, 2 µg of each mRNA was resolved by 1% agarose gel, transferred and immobilized on Ambion BrightStar®-Plus Membrane, probed with 32P-labled human-STING or β-actin DNA probes, and finally, exposed for autoradiography. 7 human Burkitt lymphoma lines (BL-5, BL-7, BL-8, SM1, peterson, BL-30 and canute) and 2 primary effusion lymphoma lines (BC-1 and BC-3) were tested in this northern blot. Huvec cell was used as a positive control. (+), EBV positive; (-), EBV negative.
Figure 28 is a Western blot of endogenous STING expression in various types of normal human cells: 30 µg of whole cell extracts were resolved by 10% SDS-PAGE followed by western blot using either rabbit-anti-human-STING antiserum or mouse-anti-beta-actin monoclonal antibody (Sigma). Normal human cells were purchased from Lonza and names of cells tested are labeled above the image.

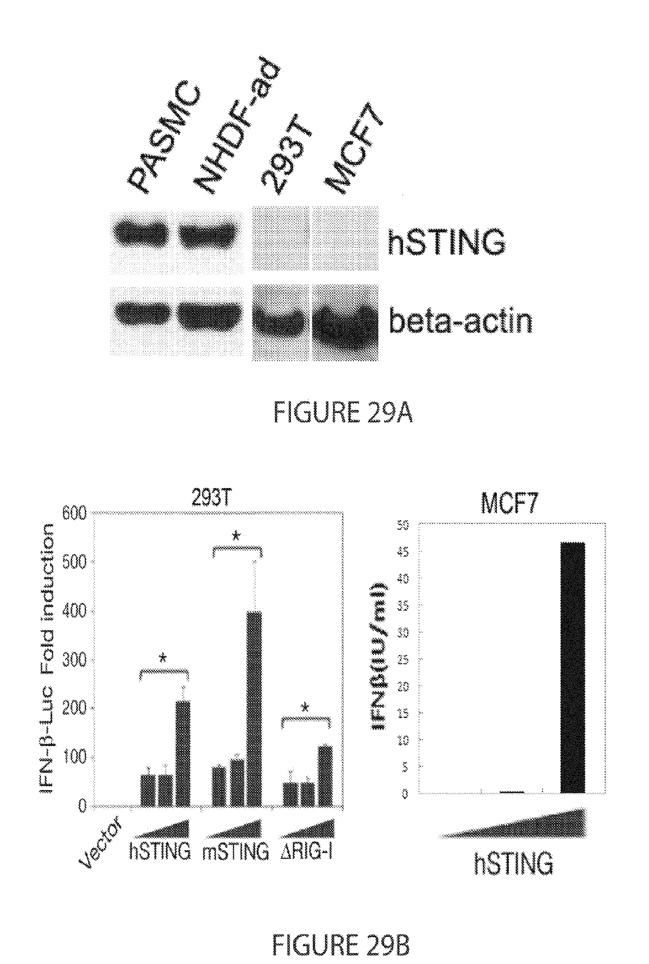

Figures 29A-29B: STING is lost from numerous cancer cells although reconstitution of STING can rescue the intracellular DNA-mediated type I IFN induced pathway. Figure 29A is an immunoblot blot analysis of hSTING expression in normal human cells (PASMC and NHDF-ad) or human cancer cells (293T and MCF7). Figure 29B : 293T cells were transfected with an IFN-β-Luc (p110-Luc) plasmid and increasing amounts of human STING (hSTING), murine STING (mSTING) or control ΔRIG-I, and analyzed for luciferase activity. MCF7 cells were transfected with increasing amount of hSTING and culture supernatants were collected and analyzed for IFN-β protein by ELISA.

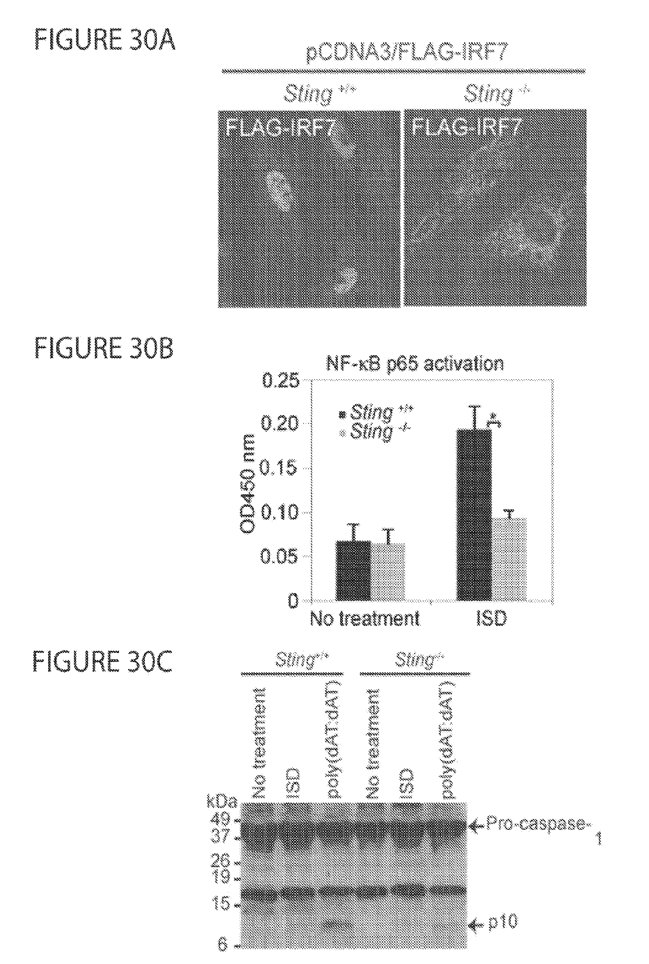

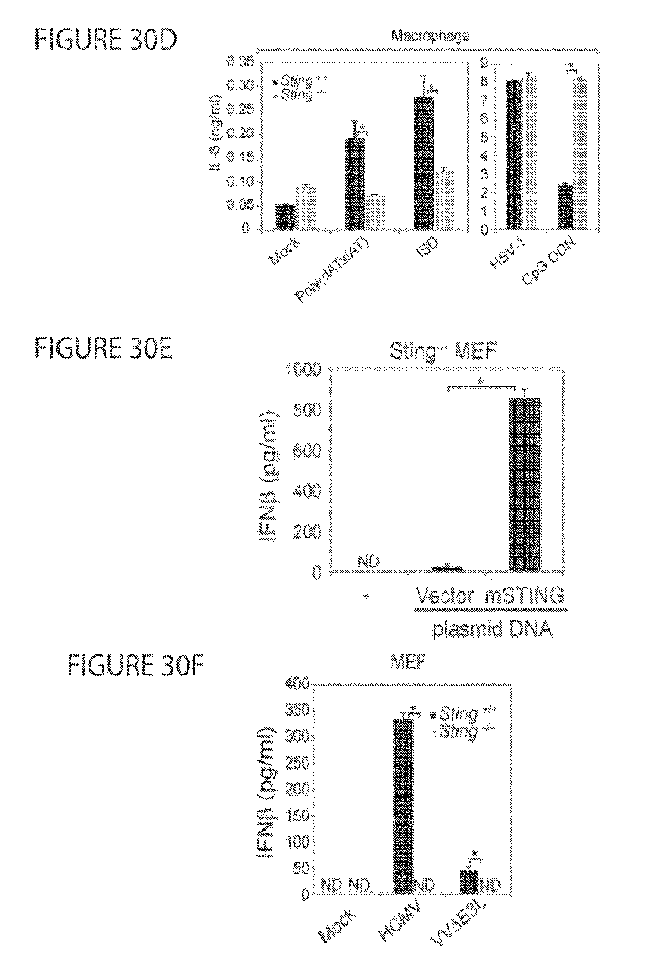

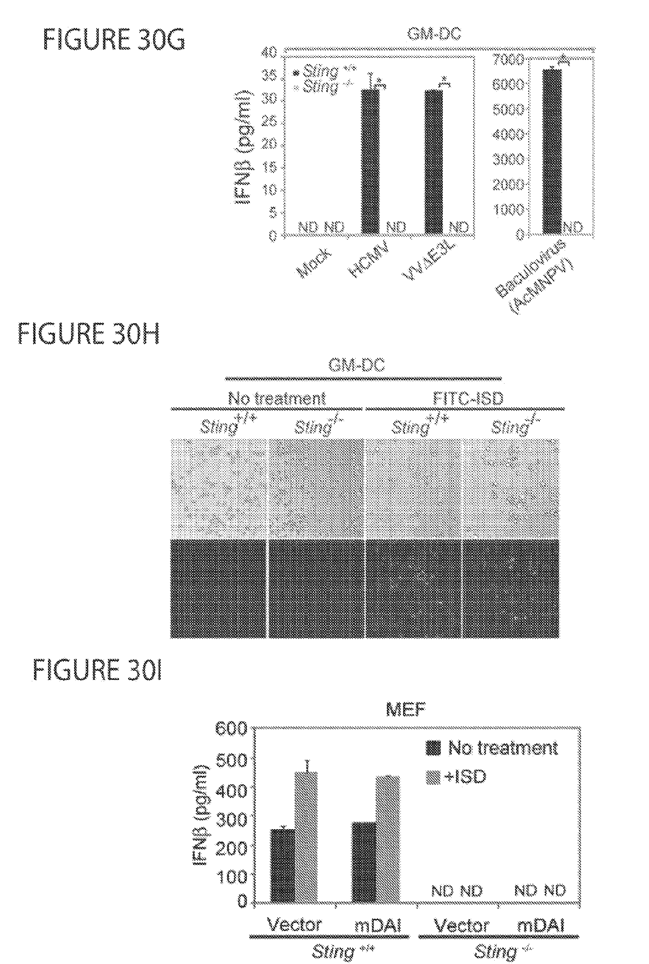

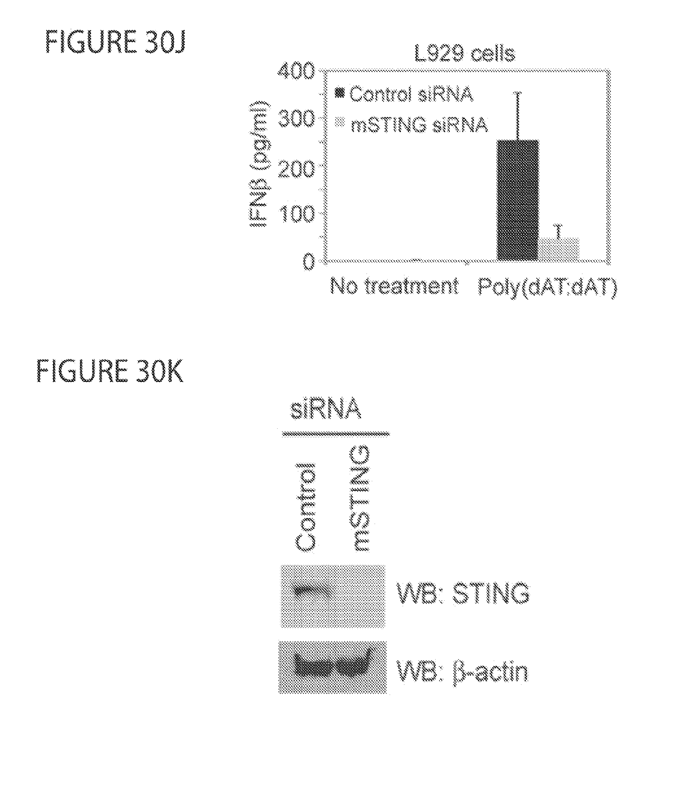

Figures 30A-30K show that STING is required for intracellular DNA-mediated type I IFN type production. Figure 30A: *Sting*^{+/+} or ^{-/-} MEFS were transfected with FLAG-IRF7 for 24 hours and immunostained using anti-FLAG antibody. Plasmid transfection stimulates the STING pathway, activating IRF7 and nuclear localization. This does not occur in the absence of STING. Figure 30B: *Sting* ^{+/+} or ^{-/-} MEFS were treated with ISD for 16 hours and cell lysates analyzed for NF-κB p65 activity using ELISA. Figure 30C: *Sting* ^{+/+} or ^{-/-} macrophage were treated with ISD or poly (dAT:dAT) for 16 hours and lysates analyzed for caspase-1 p10 fragment using antibody. Figure 30D: Macrophages were transfected with 1µg/ml of DNA ligands or infected for 16 hours with HSV-1 (moi 10). IL-6 was measured by ELISA. IL-6 production in the absence of STING is defective in response to poly(dAT:dAT) and ISD, but not HSV-1 or CpG ODN. Figure 30E: *Sting* ^{-/-} MEFS were reconstituted with STING and IFNβ production rescued as measured by ELISA. Figures 30F and 30G: *Sting* ^{+/+} or ^{-/-} MEFS were infected (moi 10) with human cytomegalovirus (HCMV) or vaccinia virus (VVΔE3L) or baculovirus and IFNβ production measured by ELISA after 16 hours. Figure 30H is a scan of a photograph showing GM-DCs from *Sting* ^{+/+} or ^{-/-} without treatment or treated with FITC-ISD. Figure 30I: *Sting* ^{+/+} or ^{-/-}MEFS were transfected with murine DAI for 16 hours and treated with ISD for 16 hours, and endogenous IFNβ was measured. Figure 30J: L929 cells were treated with RNAi to STING for 3 days, and treated with poly(dA:dT). After 16 hours, IFNβ production measured by ELISA. Figure 30K: Knockdown of STING in (Figure 30I) was confirmed by immunoblot using anti-STING antibody. Asterisks indicate significant difference (P<0.05) as determined by Student's t-test. Error bars indicate s.d.

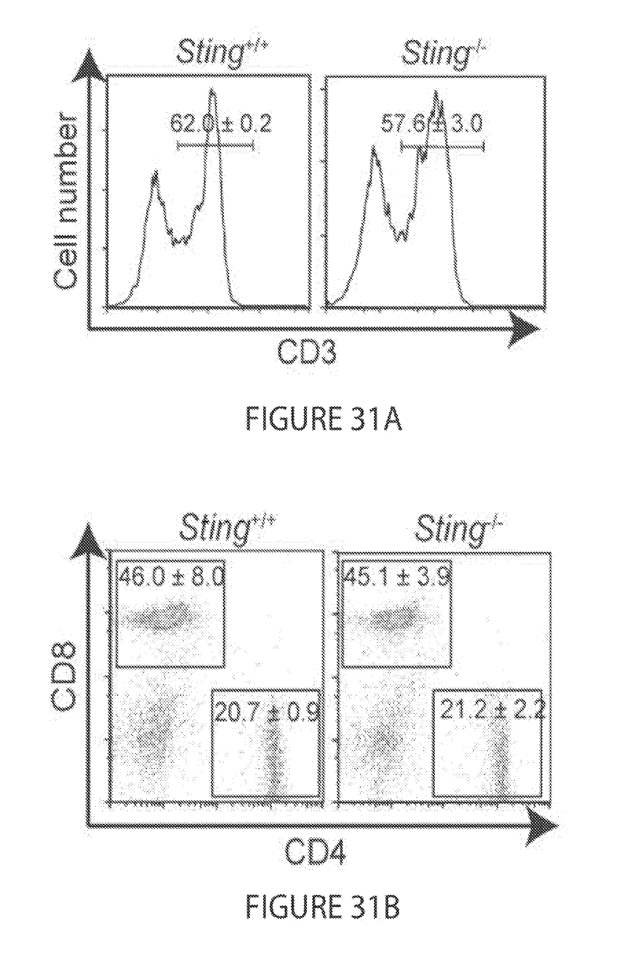

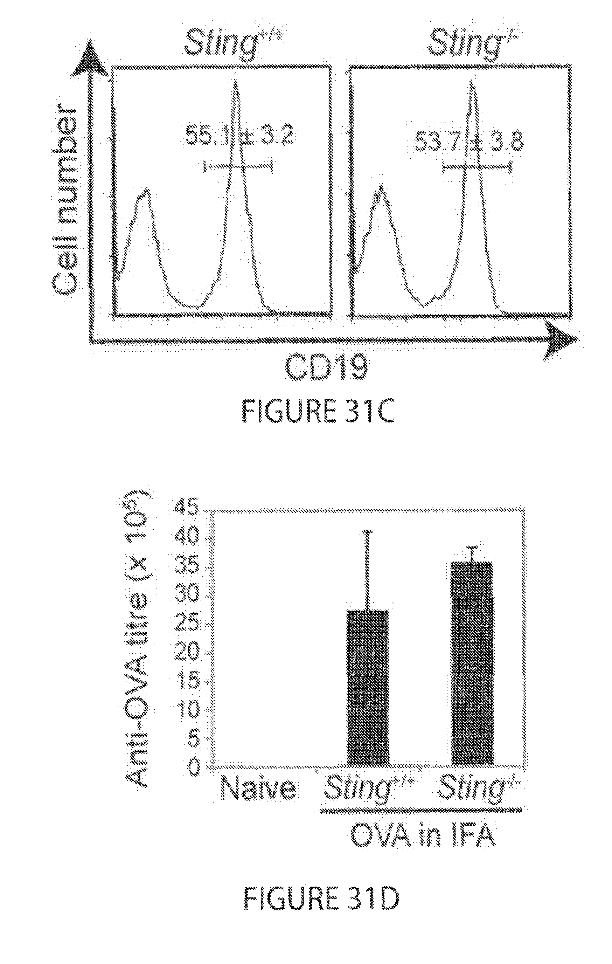

Figures 31A-31E: Normal T and B cell repertoire in STING ^{-/-} animals under non-stimulatory conditions. Figures 31A-31C: Splenocytes from STING ^{+/+} or ^{-/-} mice were analyzed by FACS using anti-CD3, anti- CD4 or CD8 (for T cell) or anti-CD 19 (for B cells) under non-stimulated conditions. Figure 31D: Ovalbumin (10 µg) in incomplete Freunds adjuvant (IFA) was inoculated i.p. into STING ^{+/+} or ^{-/-} mice (n =4). Animals were boosted after 14 days. Antibody production to OVA was measured 14 days after the last boost. Figure 30E: Splenocytes from (Figure 30D) were stimulated with anti-OVA peptide (SIINFEKL) and after 4 days IFNγ was measured by ELISA. Asterisks indicate significant difference (P<0.05) as determined by Student's t-test. Error bars indicate s.d.

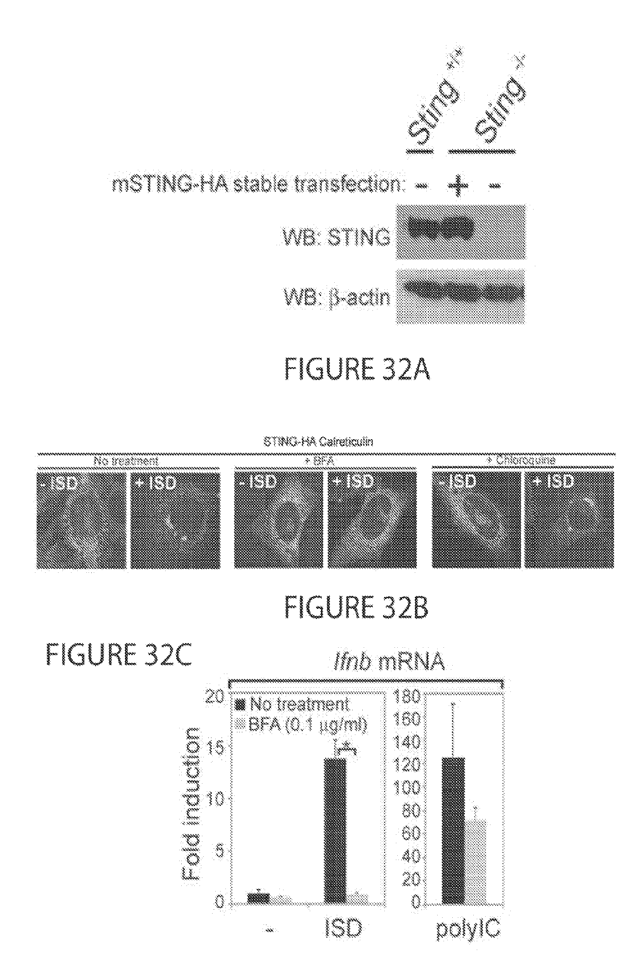

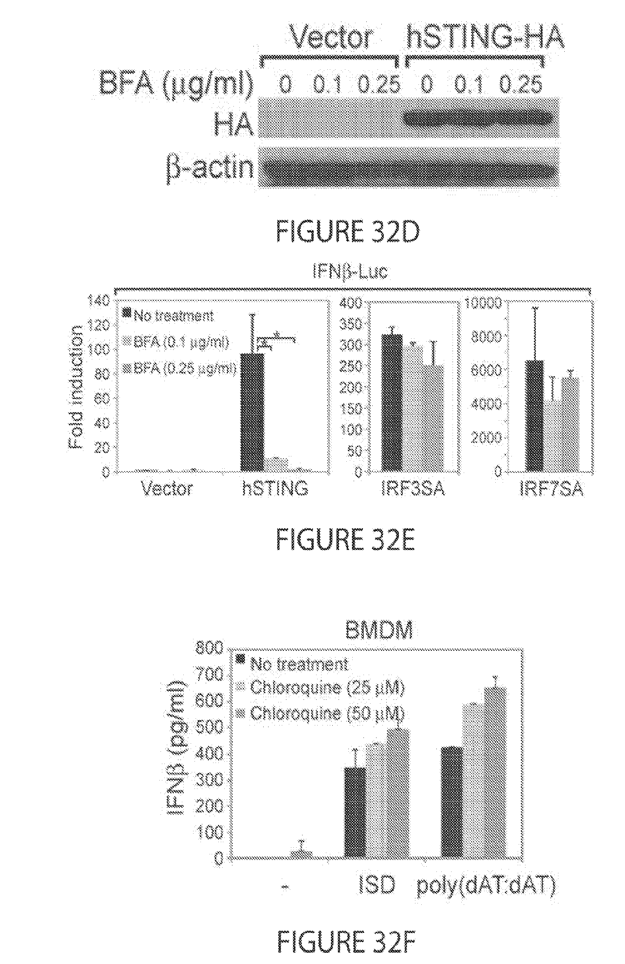

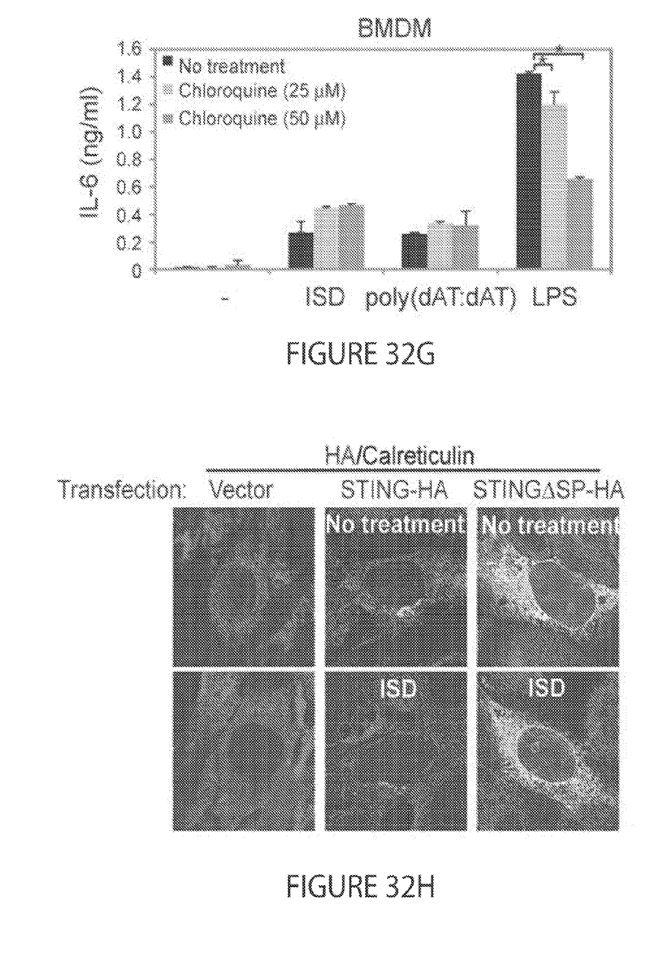

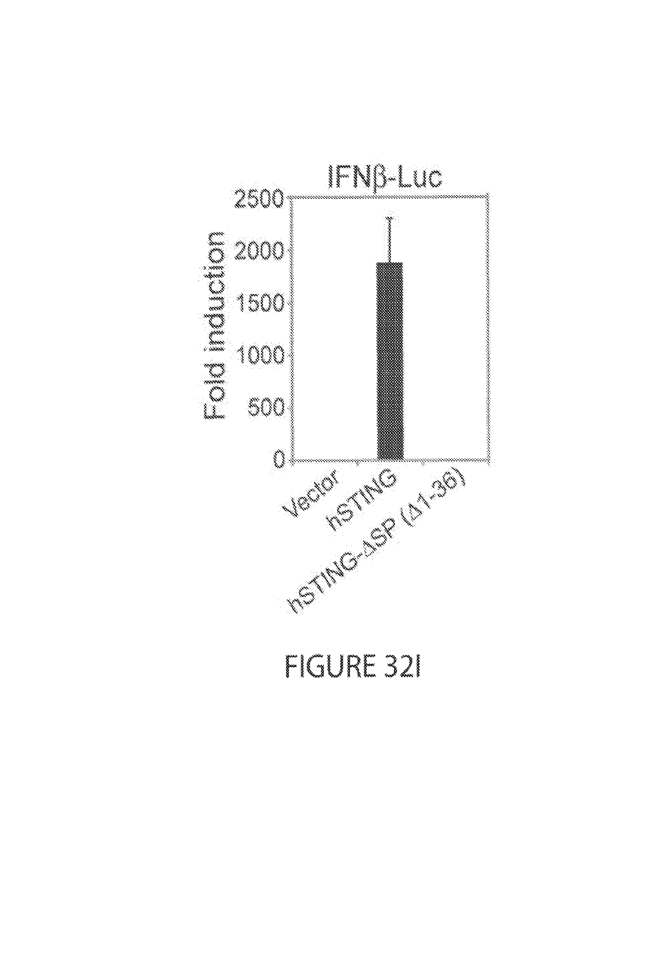

Figures 32A-32I: Brefeldin A blocks STING trafficking. Figure 32A: Immunoblot analysis of STING expression in *Sting* ^{-/-} MEFs stably transfected with HA-STING, to demonstrate comparable amounts of STING in both cell types. Figure 32B: MEFs stably transfected with HA-STING were treated with Brefeldin A (0.1 µg/ml) or chloroquine (0.1 mM) for 1 hour prior to ISD stimulation (3 hours 1 µg/ml in Lipofectamine 2000). Green = anti-HA and red = anti-calreticulin. Figure 32C: Wild type MEFs were treated with Brefeldin A as above and IFNβ mRNA measured. Figures 32D, 32E: 293T cells were treated with various concentrations of Brefeldin A as above were co-transfected with STING, constitutively active IRF3 or IRF7 and IFN-luciferase reporter plasmid. Luciferase was measured after 24 hours. Figures 32F, 32G: Macrophages treated with various chloroquine amounts for one hour were treated with ISD (1 µg/ml), poly (dAT-dAT) [1 µg/ml] or LPS (1µg/ml) and after 16 hours IFNβ or IL6 measured by ELISA. Figure 32H: STING ^{-/-} MEFS were transfected with HA-STING or HA-STINGΔSP (that lacks the first 36 amino acids representing a potential signal peptide motif) for 24 hours prior to treatment with ISD (1 µg/ml). After 3 hours STING localization was measured using immunofluorescence and an anti-HA antibody (green) or anti calreticulin antibody (red). Figure 32I: 293T cells were co-transfected with HA-STING or HA-STINGΔSP and an IFN-Luciferase plasmid and after 24 hours luciferase was measured. STINGΔSP does not traffic in response to ISD and cannot activate type I IFN. Asterisks indicate significant difference (P<0.05) as determined by Student's t-test. Error bars indicate s.d.

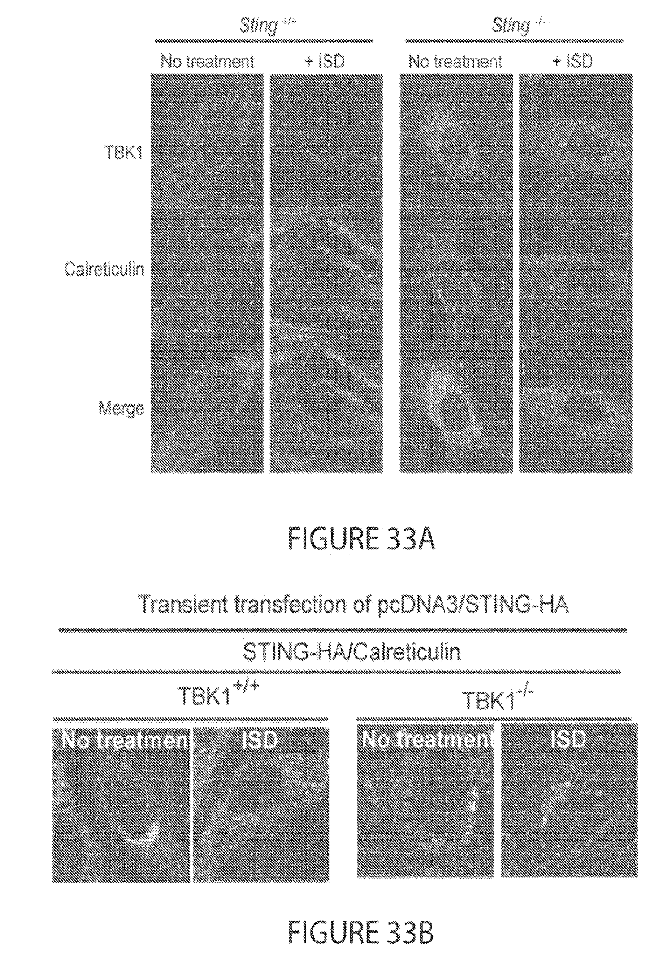

Figures33A-33B: STING is essential for TBK-1 trafficking in response to intracellular DNA. Figure 33A: STING ^{+/+} or ^{-/-} MEFS were treated with ISD (1 µg/ml with Lipofactamine 2000) and after 3 hours endogenous TBK1 localization was analyzed using immunofluorescence using and anti-TBK antibody (green) or anti-calreticulin (red). Figure 33B: TBK1^{+/+} or ^{-/-} MEFs were transfected with HA-STING and after 24 hours cells were treated with ISD for 3 hours and STING localization analyzed by immunofluorescence using an anti HA antibody (green) or anti-calreticulin (red). STING localizes in the absence of TBK1.

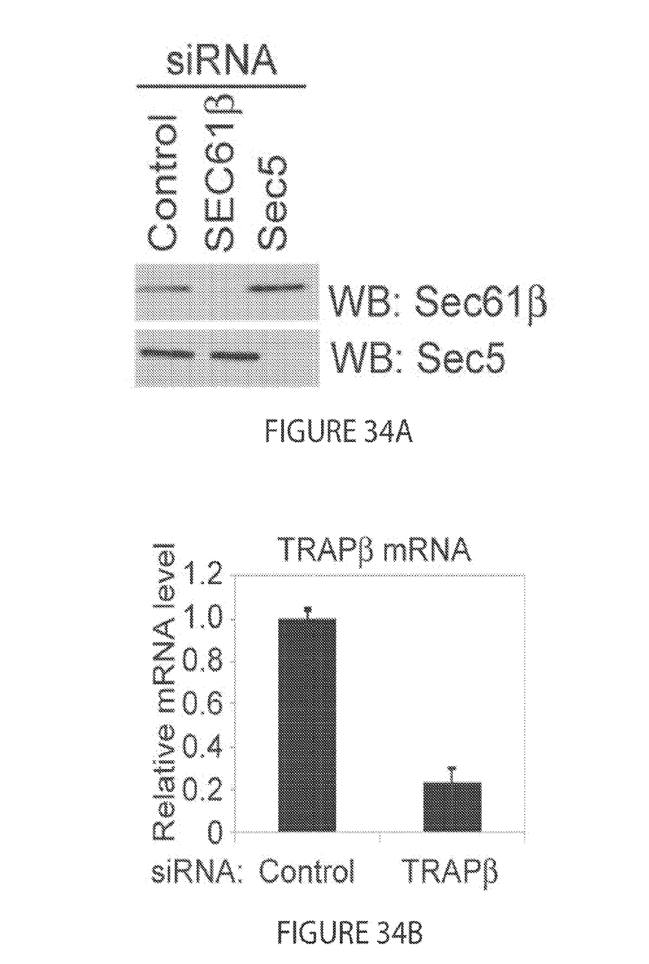

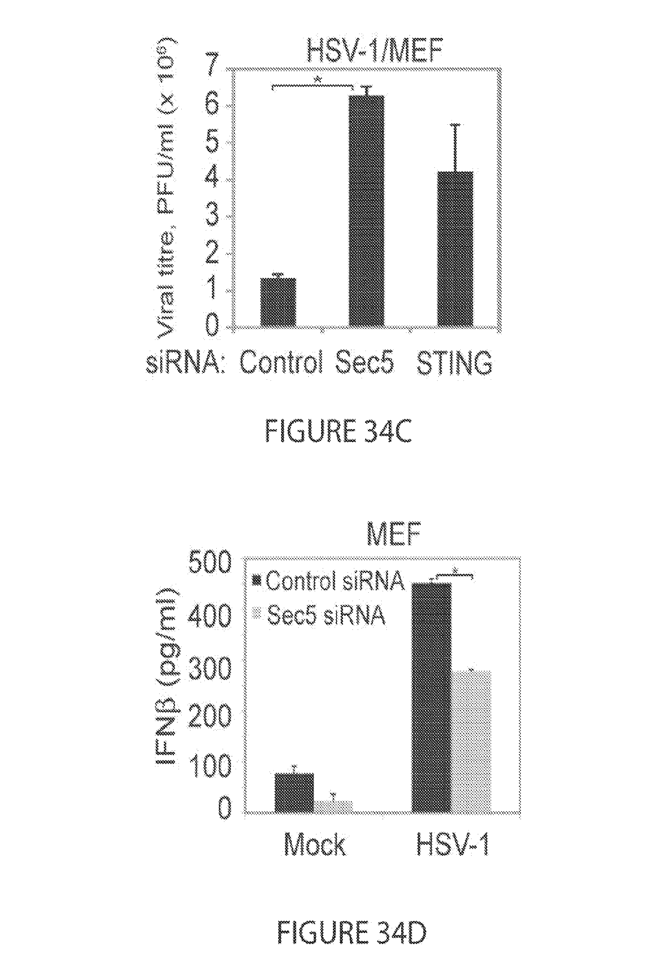

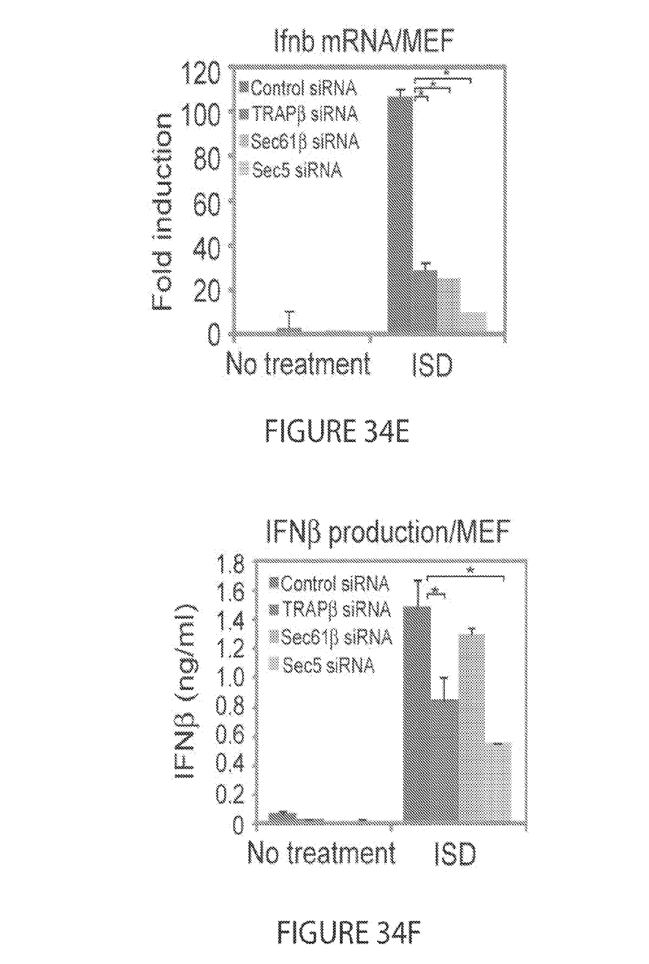

Figures 34A-34F: Sec5 is required for efficient intracellular DNA-mediated signaling. Figure 34A: Immunoblot of RNAi (to Sec5 or Sec61β) treated cells indicating knockdown of Sec5 and Sec61β. Figure 34B: RT-PCR measurements of RNAi (Trapβ) treated cells indicating knockdown of Trapβ. Figure 34C: MEFs treated with RNAi to Sec5 or STING for 3 days were infected with HSV1 (moi 1) for 24 hours and viral titers measured. Figure 34D: Same as in (Figure 34C) except that endogenous IFNβ was measured. Figure 34E: MEFs treated with RNAi to Trapβ, Sec61β or Sec5 for 3 days were treated with ISD for 3hours and Ifnb mRNA measured by RT-PCR. Figure 34F: Same as in (Figure 34E) except that endogenous IFNβ was measured.

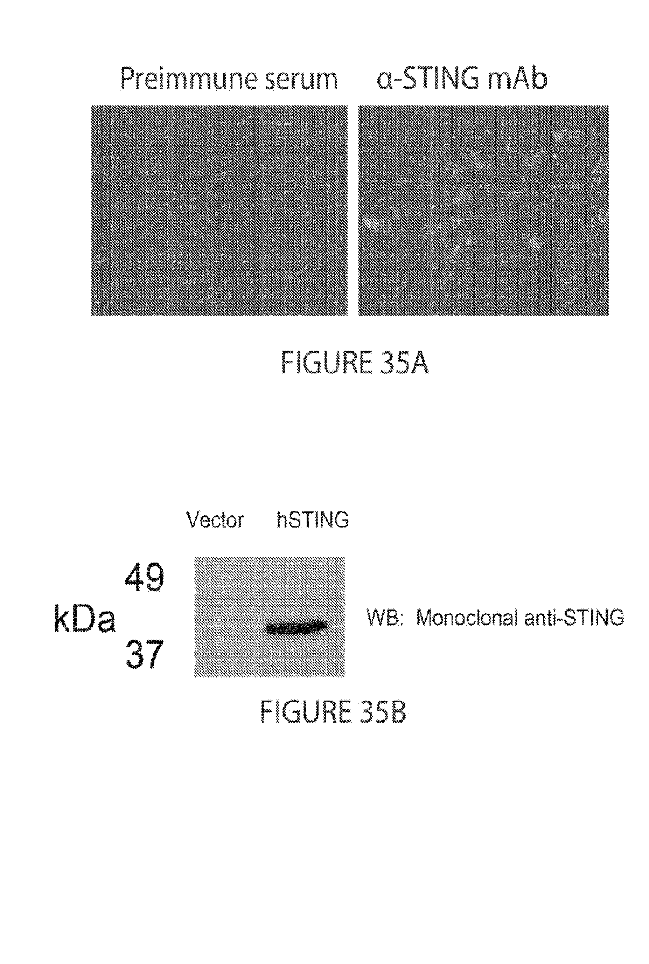

Figures 35A, 35B show immunostaining (Figure 35A) and immunobloting (Figure 35B) of 293T cells transfected with hSTING for 24 hours using anti-STING monoclonal antibody AS-1. The monoclonal antibody (Mab) was generated by inoculating/immunizing mice (Balb/c) with plasmid DNA (100 µg) expressing the human STING gene. Spleen cells from the immunized animals were fused to myeloma cells to generate hybridomas, which were isolated to generate clones. Secreted antibody was screened using 293T cells as controls or 293T cells expressing human STING, in immunofluorescence assays (Figure 35A). Antibody fluorescing the STING transfected cells but not controls was used to incubate immunoblots of lysates expressing human STING or control vector. Thus Mab AS-1 (for anti-STING 1 from clone 2g9) recognizes STING in immunofluorescence studies and by immunoblot.

### DETAILED DESCRIPTION

The present invention is described with reference to the attached figures, wherein like reference numerals are used throughout the figures to designate similar or equivalent elements. The figures are not drawn to scale and they are provided merely to illustrate the instant invention. Several aspects of the invention are described below with reference to example applications for illustration. It should be understood that numerous specific details, relationships, and methods are set forth to provide a full understanding of the invention. One having ordinary skill in the relevant art, however, will readily recognize that the invention can be practiced without one or more of the specific details or with other methods. The present invention is not limited by the illustrated ordering of acts or events, as some acts may occur in different orders and/or concurrently with other acts or events. Furthermore, not all illustrated acts or events are required to implement a methodology in accordance with the present invention.

All genes, gene names, and gene products disclosed herein are intended to correspond to homologs from any species for which the compositions and methods disclosed herein are applicable. Thus, the terms include, but are not limited to genes and gene products from humans and mice. It is understood that when a gene or gene product from a particular species is disclosed, this disclosure is intended to be exemplary only, and is not to be interpreted as a limitation unless the context in which it appears clearly indicates. Thus, for example, for the genes disclosed herein, which in some embodiments relate to mammalian nucleic acid and amino acid sequences are intended to encompass homologous and/or orthologous genes and gene products from other animals including, but not limited to other mammals, fish, amphibians, reptiles, and birds. In preferred embodiments, the genes or nucleic acid sequences are human.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

### Definitions

The terminology used herein is for the purpose of describing particular embodiments only and is not intended to be limiting of the invention. As used herein, the singular forms "a", "an" and "the" are intended to include the plural forms as well, unless the context clearly indicates otherwise. Furthermore, to the extent that the terms "including", "includes", "having", "has", "with", or variants thereof are used in either the detailed description and/or the claims, such terms are intended to be inclusive in a manner similar to the term "comprising."

The term "about" or "approximately" means within an acceptable error range for the particular value as determined by one of ordinary skill in the art, which will depend in part on how the value is measured or determined, *i.e.,* the limitations of the measurement system. For example, "about" can mean within 1 or more than 1 standard deviation, per the practice in the art. Alternatively, "about" can mean a range of up to 20%, preferably up to 10%, more preferably up to 5%, and more preferably still up to 1% of a given value. Alternatively, particularly with respect to biological systems or processes, the term can mean within an order of magnitude, preferably within 5-fold, and more preferably within 2-fold, of a value. Where particular values are described in the application and claims, unless otherwise stated the term "about" meaning within an acceptable error range for the particular value should be assumed.

The term "induces or enhances an immune response" is meant causing a statistically measurable induction or increase in an immune response over a control sample to which the peptide, polypeptide or protein has not been administered. Preferably the induction or enhancement of the immune response results in a prophylactic or therapeutic response in a subject. Examples of immune responses are increased production of type I IFN, increased resistance to viral and other types of infection by alternate pathogens. The enhancement of immune responses to tumors (anti-tumor responses), or the development of vaccines to prevent tumors or eliminate existing tumors.

The term "STING" is meant to include, without limitation, nucleic acids, polynucleotides, oligonucleotides, sense and antisense polynucleotide strands, complementary sequences, peptides, polypeptides, proteins, homologous and/or orthologous STING molecules, isoforms, precursors, mutants, variants, derivatives, splice variants, alleles, different species, and active fragments thereof.

The term "lacks a functional STING gene" is meant that a transgenic animal lacks a gene that encodes STING, or lacks other genetic components (e.g. promoters) required for expression of STING.

The term "active fragment or variant" is meant a fragment that is at least 380 amino acid residues in length and is 100% identical to a contiguous portion of the peptide, polypeptide or protein, or a variant that is at least 90%, preferably 95% identical to a fragment up to and including the full length peptide, polypeptide or protein. A variant, for example, may include conservative amino acid substitutions, as defined in the art, or nonconservative substitutions, providing that at least e.g. 10%, 25%, 50%, 75% or 90% of the activity of the original peptide, polypeptide or protein is retained. Also included are STING molecules, fragments or variants having post-translational modifications such as sumoylation, phosphorylation glycosylation, splice variants, and the like, all of which may effect the efficacy of STING function.

Unless otherwise indicated, the terms "peptide", "polypeptide" or "protein" are used interchangeably herein, although typically they refer to peptide sequences of varying sizes.

The term "variant," when used in the context of a polynucleotide sequence, may encompass a polynucleotide sequence related to a wild type gene. This definition may also include, for example, "allelic," "splice," "species," or "polymorphic" variants. A splice variant may have significant identity to a reference molecule, but will generally have a greater or lesser number of polynucleotides due to alternate splicing of exons during mRNA processing. The corresponding polypeptide may possess additional functional domains or an absence of domains. Species variants are polynucleotide sequences that vary from one species to another. Of particular utility in the invention are variants of wild type gene products. Variants may result from at least one mutation in the nucleic acid sequence and may result in altered mRNAs or in polypeptides whose structure or function may or may not be altered. Any given natural or recombinant gene may have none, one, or many allelic forms. Common mutational changes that give rise to variants are generally ascribed to natural deletions, additions, or substitutions of nucleotides. Each of these types of changes may occur alone, or in combination with the others, one or more times in a given sequence.

The resulting polypeptides generally will have significant amino acid identity relative to each other. A polymorphic variant is a variation in the polynucleotide sequence of a particular gene between individuals of a given species. Polymorphic variants also may encompass "single nucleotide polymorphisms" (SNPs) or single base mutations in which the polynucleotide sequence varies by one base. The presence of SNPs may be indicative of, for example, a certain population with a propensity for a disease state, that is susceptibility versus resistance.

Derivative polynucleotides include nucleic acids subjected to chemical modification, for example, replacement of hydrogen by an alkyl, acyl, or amino group. Derivatives, e.g., derivative oligonucleotides, may comprise non-naturally-occurring portions, such as altered sugar moieties or inter-sugar linkages. Exemplary among these are phosphorothioate and other sulfur containing species which are known in the art. Derivative nucleic acids may also contain labels, including radionucleotides, enzymes, fluorescent agents, chemiluminescent agents, chromogenic agents, substrates, cofactors, inhibitors, magnetic particles, and the like.

A "derivative" polypeptide or peptide is one that is modified, for example, by glycosylation, pegylation, phosphorylation, sulfation, reduction/alkylation, acylation, chemical coupling, or mild formalin treatment. A derivative may also be modified to contain a detectable label, either directly or indirectly, including, but not limited to, a radioisotope, fluorescent, and enzyme label.

The term "immunoregulatory" is meant a compound, composition or substance that is immunogenic (i.e. stimulates or increases an immune response) or immunosuppressive (i.e. reduces or suppresses an immune response).

"An antigen presenting cell" (APC) is a cell that is capable of activating T cells, and includes, but is not limited to, monocytes/macrophages, B cells and dendritic cells (DCs). The term "dendritic cell" or "DC" refers to any member of a diverse population of morphologically similar cell types found in lymphoid or non-lymphoid tissues. These cells are characterized by their distinctive morphology, high levels of surface MHC-class II expression. DCs can be isolated from a number of tissue sources. DCs have a high capacity for sensitizing MHC-restricted T cells and are very effective at presenting antigens to T cells *in situ.* The antigens may be self-antigens that are expressed during T cell development and tolerance, and foreign antigens that are present during normal immune processes.

The term "expression vector" as used herein refers to a vector containing a nucleic acid sequence coding for at least part of a gene product capable of being transcribed. In some cases, RNA molecules are then translated into a protein, polypeptide, or peptide. In other cases, these sequences are not translated, for example, in the production of antisense molecules, siRNA, ribozymes, and the like. Expression vectors can contain a variety of control sequences, which refer to nucleic acid sequences necessary for the transcription and possibly translation of an operatively linked coding sequence in a particular host organism. In addition to control sequences that govern transcription and translation, vectors and expression vectors may contain nucleic acid sequences that serve other functions as well.

By "encoding" or "encoded", "encodes", with respect to a specified nucleic acid, is meant comprising the information for translation into the specified protein. A nucleic acid encoding a protein may comprise non-translated sequences (e.g., introns) within translated regions of the nucleic acid, or may lack such intervening non-translated sequences (e.g., as in cDNA). The information by which a protein is encoded is specified by the use of codons. Typically, the amino acid sequence is encoded by the nucleic acid using the "universal" genetic code.

As used herein, "heterologous" in reference to a nucleic acid is a nucleic acid that originates from a foreign species, or, if from the same species, is substantially modified from its native form in composition and/or genomic locus by deliberate human intervention. For example, a promoter operably linked to a heterologous structural gene is from a species different from that from which the structural gene was derived, or, if from the same species, one or both are substantially modified from their original form. A heterologous protein may originate from a foreign species or, if from the same species, is substantially modified from its original form by deliberate human intervention.

"Sample" is used herein in its broadest sense. A sample comprising polynucleotides, polypeptides, peptides, antibodies and the like may comprise a bodily fluid; a soluble fraction of a cell preparation, or media in which cells were grown; a chromosome, an organelle, or membrane isolated or extracted from a cell; genomic DNA, RNA, or cDNA, polypeptides, or peptides in solution or bound to a substrate; a cell; a tissue; a tissue print; a fingerprint, skin or hair; and the like.

The terms "patient", "subject" or "individual" are used interchangeably herein, and refers to a mammalian subject to be treated, with human patients being preferred. In some cases, the methods of the invention find use in experimental animals, in veterinary application, and in the development of animal models for disease, including, but not limited to, rodents including mice, rats, and hamsters; and primates.

"Diagnostic" or "diagnosed" means identifying the presence or nature of a pathologic condition. Diagnostic methods differ in their sensitivity and specificity. The "sensitivity" of a diagnostic assay is the percentage of diseased individuals who test positive (percent of "true positives"). Diseased individuals not detected by the assay are "false negatives." Subjects who are not diseased and who test negative in the assay, are termed "true negatives." The "specificity" of a diagnostic assay is 1 minus the false positive rate, where the "false positive" rate is defined as the proportion of those without the disease who test positive. While a particular diagnostic method may not provide a definitive diagnosis of a condition, it suffices if the method provides a positive indication that aids in diagnosis.

"Treatment" is an intervention performed with the intention of preventing the development or altering the pathology or symptoms of a disorder. Accordingly, "treatment" refers to both therapeutic treatment and prophylactic or preventative measures. Those in need of treatment include those already with the disorder as well as those in which the disorder is to be prevented. In tumor (e.g., cancer) treatment, a therapeutic agent may directly decrease the pathology of tumor cells, or render the tumor cells more susceptible to treatment by other therapeutic agents, e.g., radiation and/or chemotherapy. As used herein, "ameliorated" or "treatment" refers to a symptom which is approaches a normalized value (for example a value obtained in a healthy patient or individual), e.g., is less than 50% different from a normalized value, preferably is less than about 25% different from a normalized value, more preferably, is less than 10% different from a normalized value, and still more preferably, is not significantly different from a normalized value as determined using routine statistical tests. For example the term "treat" or "treating" with respect to tumor cells refers to stopping the progression of said cells, slowing down growth, inducing regression, or amelioration of symptoms associated with the presence of said cells. Treatment of an individual suffering from an infectious disease organism refers to a decrease and elimination of the disease organism from an individual. For example, a decrease of viral particles as measured by plaque forming units or other automated diagnostic methods such as ELISA etc.

The "treatment of cancer", refers to one or more of the following effects: (1) inhibition, to some extent, of tumor growth, including, (i) slowing down and (ii) complete growth arrest; (2) reduction in the number of tumor cells; (3) maintaining tumor size; (4) reduction in tumor size; (5) inhibition, including (i) reduction, (ii) slowing down or (iii) complete prevention, of tumor cell infiltration into peripheral organs; (6) inhibition, including (i) reduction, (ii) slowing down or (iii) complete prevention, of metastasis; (7) enhancement of anti-tumor immune response, which may result in (i) maintaining tumor size, (ii) reducing tumor size, (iii) slowing the growth of a tumor, (iv) reducing, slowing or preventing invasion and/or (8) relief, to some extent, of the severity or number of one or more symptoms associated with the disorder.

As used herein, the term "safe and effective amount" refers to the quantity of a component which is sufficient to yield a desired therapeutic response without undue adverse side effects (such as toxicity, irritation, or allergic response) commensurate with a reasonable benefit/risk ratio when used in the manner of this invention. By "therapeutically effective amount" is meant an amount of a compound of the present invention effective to yield the desired therapeutic response. For example, an amount effective to delay the growth of or to cause a cancer, either a sarcoma or lymphoma, or to shrink the cancer or prevent metastasis. The specific safe and effective amount or therapeutically effective amount will vary with such factors as the particular condition being treated, the physical condition of the patient, the type of mammal or animal being treated, the duration of the treatment, the nature of concurrent therapy (if any), and the specific formulations employed and the structure of the compounds or its derivatives.

"Cells of the immune system" or "immune cells", is meant to include any cells of the immune system that may be assayed, including, but not limited to, B lymphocytes, also called B cells, T lymphocytes, also called T cells, natural killer (NK) cells, natural killer T (NK) cells, lymphokine-activated killer (LAK) cells, monocytes, macrophages, neutrophils, granulocytes, mast cells, platelets, Langerhans cells, stem cells, dendritic cells, peripheral blood mononuclear cells, tumor-infiltrating (TIL) cells, gene modified immune cells including hybridomas, drug modified immune cells, and derivatives, precursors or progenitors of the above cell types.

"Immune effector cells" refers to cells capable of binding an antigen and which mediate an immune response selective for the antigen. These cells include, but are not limited to, T cells (T lymphocytes), B cells (B lymphocytes), monocytes, macrophages, natural killer (NK) cells and cytotoxic T lymphocytes (CTLs), for example CTL lines, CTL clones, and CTLs from tumor, inflammatory, or other infiltrates.

"Immune related molecules" refers to any molecule identified in any immune cell, whether in a resting ("non-stimulated") or activated state, and includes any receptor, ligand, cell surface molecules, nucleic acid molecules, polypeptides, variants and fragments thereof.

"T cells" or "T lymphocytes" are a subset of lymphocytes originating in the thymus and having heterodimeric receptors associated with proteins of the CD3 complex (e.g., a rearranged T cell receptor, the heterodimeric protein on the T cell surfaces responsible for antigen/MHC specificity of the cells). T cell responses may be detected by assays for their effects on other cells (e.g., target cell killing, activation of other immune cells, such as B-cells) or for the cytokines they produce.

The phrase "T cell response" means an immunological response involving T cells. The T cells that are "activated" divide to produce antigen specific memory T cells or antigen specific cytotoxic T cells. The cytotoxic T cells bind to and destroy cells recognized as containing the antigen. The memory T cells are activated by the antigen and thus provide a response to an antigen already encountered. This overall response to the antigen is the antigen specific T cell response, e.g. tumor specific.

A "secondary immune response" or "adaptive immune response" may be active or passive, and may be humoral (antibody based) or cellular that is established during the life of an animal, is specific for an inducing antigen, and is marked by an enhanced immune response on repeated encounters with said antigen. A key feature of the T lymphocytes of the adaptive immune system is their ability to detect minute concentrations of pathogen-derived peptides presented by MHC molecules on the cell surface.

### STING Compositions

Embodiments of the invention are directed to nucleic acid sequences and encoded products thereof, to a molecule termed herein STING. The novel STING nucleic acids, polynucleotides, oligonucleotides, peptides, mutants, variants and active fragments thereof, can be used to modulate innate and adaptive immunity in a subject and/or for the treatment of an immune-related disorder, including treating and preventing infection by modulating immunity. Also provided are an agent reactive with the peptide, a pharmaceutical composition that includes the peptide, isolated nucleic acid molecules, an isolated nucleic acid molecules encoding the peptides, recombinant nucleic acid constructs that include the nucleic acid molecules, at least one host cell comprising the recombinant nucleic acid constructs, and a method of producing the peptide using the host cell. The present invention further provides a method for treating and/or preventing infection in a subject by administering the peptide of the invention to the subject, thereby modulating innate immunity in the subject. Additionally, the present invention provides a method for identifying candidate therapeutic agents.

In the Examples section which follows, the data provide for the identification following expression cloning and characterization of a novel molecule, STING (STimulator of INterferon Genes) that regulates innate immune signaling processes. Both the nucleic acid and peptide sequences are identified in humans and mice. STING, comprising 5 putative transmembrane (TM) regions, predominantly resides in the endoplasmic reticulum (ER) and is able to activate both NF-κB and IRF3 transcription pathways to induce type I IFN and to exert a potent anti-viral state following expression. In contrast, loss of STING reduced the ability of polyIC to activate type I IFN and rendered murine embryonic fibroblasts lacking STING (^{-/-} MEFs) generated by targeted homologous recombination, susceptible to vesicular stomatitis virus (VSV) infection. DNA-mediated ability to stimulate type I IFN responses in the absence of STING, was also greatly inhibited, indicating that STING may play an important role in recognizing DNA-virus and bacteria as well as other pathogen infection. Yeast-two hybrid and co-immunoprecipitation studies indicated that STING interacts with RIG-I and with Ssr2/TRAPβ, a member of the translocon-associated protein (TRAP) complex required for protein translocation across the ER membrane following translation. RNAi ablation of TRAPβ was subsequently found to inhibit STING function as well as impede the production of type I IFN in response to polyIC. Thus, aside from identifying a novel regulator of innate immune signaling, this data further implicates, for the first time, a potential role for the translocon in intracellular dsRNA/virus signaling and DNA /virus, plasmid DNA based pathways in mammalian cells which appears central for the activation of primary innate immune responses, including the production of Type I IFN.

In preferred embodiments, STING molecules and the molecules STING regulates in the various pathways described herein, provide the design of new adjuvants, vaccines and therapies to regulate the immune system and other systems. The molecule, and/or pathways it regulates, would be utilized in vaccine and therapies to combat disease. Modulation of the immune system by STING provides for the treatment of diseases, including diseases caused by foreign agents. Exemplary infections by foreign agents which may be treated and/or prevented by the method of the present invention include an infection by a bacterium (e.g., a Gram-positive or Gram-negative bacterium), an infection by a fungus, an infection by a parasite, and an infection by a virus. In one embodiment of the present invention, the infection is a bacterial infection (e.g., infection by *E. coli, Klebsiella pneumoniae, Pseudomonas aeruginosa,* Salmonella spp., *Staphylococcus aureus*, Streptococcus spp., or vancomycin-resistant enterococcus). In another embodiment, the infection is a fungal infection (e.g. infection by a mould, a yeast, or a higher fungus). In still another embodiment, the infection is a parasitic infection (e.g., infection by a single-celled or multicellular parasite, including *Giardia duodenalis, Cryptosporidium parvum*, *Cyclospora cayetanensis,* and *Toxoplasma gondiz).* In yet another embodiment, the infection is a viral infection (e.g., infection by a virus associated with AIDS, avian flu, chickenpox, cold sores, common cold, gastroenteritis, glandular fever, influenza, measles, mumps, pharyngitis, pneumonia, rubella, SARS, and lower or upper respiratory tract infection (e.g., respiratory syncytial virus)).

STING also associates with components of the proteosome (PSMB 1), which is essential for the elimination of misfolded proteins. Removal of misfolded proteins from the secretory pathway depends on their recognition in the endoplasmic reticulum ER). STING resides in the ER and associates with PSMB1 to potentially play a role in ERAD- ER-associated protein degradation. A functional ubiquitin proteosome system is essential and defects can lead to neurodegenerative diseases (Parkinson, Alzheimer's) and cardiac disorders. Up-regulation of proteosome activity can lead to wasting conditions (sepsis, cachexia). Immunoproteosomes occurring in the brain and muscle reflect persistent inflammation and are downregulated in cancer cells. The induction of apoptosis by proteosome inhibitors is a treatment for cancer (such as myeloma). Thus STING could play a role in these pathways and provide a target for cancer therapy and neurodegenerative disease.

In another embodiment, STING is associated with a molecule of interest (e.g. peptide, antigen, enzyme, etc) either by binding, as a fusion moiety, or linked via a chemical moiety to modulate the molecule of interest's half-life, retention in an intracellular body, degradation, altering of intracellular or surface expression of the molecule of interest, etc.

In a preferred embodiment, an isolated nucleic acid sequence encoding a protein or peptide comprises a sequence as set forth in SEQ ID NOS: 1 or 2, or an active fragment or variant thereof that induces or enhances an immune response.

In another preferred embodiment, an active fragment is of at least 380 consecutive amino acid residues in length in humans and induces or enhances an immune *response in vivo* or *in vitro.*

In another preferred embodiment, expression of SEQ ID NOS: 1 or 2 or active fragments thereof, induce type I IFN in a cell, tissue or organ, *in vivo or in vitro.*

In another preferred embodiment, the expression of SEQ ID NOS: 1 or 2 *in vivo* or *in vitro* induces an antiviral immune response.

In another preferred embodiment, STING induces an anti-tumor response. Cancer cells may activate the STING pathway by numerous ways, for example following virus infection (EBV, HPV, HBV ETC) of known or unknown viruses, by the re-activation of endogenous retroviruses (ERV), or other retroelements (retroelement-derived DNA, retrotranscription) or proteins or nucleic acids which activate the STING pathway (Endogenous retroviruses and cancer, Ruprecht K et al., Cell Mol Life Sci. 2008 Nov;65(21):3366-82) Gifford and Tristem Virus Genes 2003 26:3 291-315) (Dolei A, Perron H. Neurovirol. 2009 Jan;15(1):4-13)(Goodier JL, Kazazian HH Jr Cell. 2008 Oct 3;135(1):23-35).

In another preferred embodiment, treatment of cancer comprises administration of STING or agents which induce STING and pathways thereof. The data shown herein, evidences that the intracellular DNA pathway that is regulated by STING is defective in all cancer cells examined so far. Thus suppression of the STING pathway may be an early, critical and hitherto novel requirement for the cellular transformation process. The intracellular DNA pathway is defective and that this event involves STING or STING interacting molecules, or molecules upstream or downstream of STING, including TRAP complexes, vesicular pathways and endosome pathways.

In a preferred embodiment, STING and/or STING interacting molecules are administered to patients for the treatment of cancer. STING can be administered as a polynucleotide, polypeptide, peptides, antisense oligonucleotides, vectors expressing STING, antibodies and the like. The data herein, indicate that STING is absent in a variety of lymphoma such as Burkett's lymphoma, breast cancer, other leukemia, lymphoma, melanoma and so on. The data herein also show that there is defective intracellular DNA signaling in cancer cells but not normal cells. Thus, reconstitution of STING and/or STING associated molecules are used in treatments for cancer and other diseases or disorders. For example, the data show that reconstitution of STING into cancer cell-lines previously shown to be defective in intracellular DNA signaling such as 293T or MCF7, rescues the intracellular pathway. That is reconstitution of STING can induce type I IFN and perhaps other signal transduction pathways. STING would thus be useful to reconstitute in cancer cells, perhaps to kill cancer but not normal cells. STING or other molecules associated directly or indirectly with STING in the intracellular DNA pathway may be useful targets for therapeutic intervention. Without wishing to be bound by theory, it is also proposed that the STING pathway will be deregulated/suppressed in viral infected tumors such as HPV related cervical carcinoma, HCV or HBV-related hepatocellular carcinoma, herpes virus associated cancers (EBV positive Burkett's lines- (see, for example, Figures 26, 27). STING may also be suppressed in latently infected cells such as those mentioned above (EBV, HPV) as well as CMV, HIV etc.

In another preferred embodiment, STING is administered to a patient to prevent or treat cancer. Cancer refers to all types of cancer or neoplasm or malignant tumors found in mammals, including, but not limited to: leukemias, lymphomas, melanomas, carcinomas and sarcomas. Examples of cancers are cancer of the brain, breast, pancreas, cervix, colon, head and neck, kidney, lung, non-small cell lung, melanoma, mesothelioma, ovary, sarcoma, stomach, uterus and Medulloblastoma. As used herein, the terms "cancer," "neoplasm," and "tumor," are used interchangeably and in either the singular or plural form, refer to cells that have undergone a malignant transformation that makes them pathological to the host organism. Primary cancer cells (that is, cells obtained from near the site of malignant transformation) can be readily distinguished from non-cancerous cells by well-established techniques, particularly histological examination. The definition of a cancer cell, as used herein, includes not only a primary cancer cell, but any cell derived from a cancer cell ancestor. This includes metastasized cancer cells, and *in vitro* cultures and cell lines derived from cancer cells. When referring to a type of cancer that normally manifests as a solid tumor, a "clinically detectable" tumor is one that is detectable on the basis of tumor mass; e.g., by procedures such as CAT scan, MR imaging, X-ray, ultrasound or palpation, and/or which is detectable because of the expression of one or more cancer-specific antigens in a sample obtainable from a patient.

In a preferred embodiment, administration of STING prevents (neuroprotective) or treats neurological disorders. Neurological disorder refers to any disorder of the nervous system and/or visual system. "Neurological disorders" include disorders that involve the central nervous system (brain, brainstem and cerebellum), the peripheral nervous system (including cranial nerves), and the autonomic nervous system (parts of which are located in both central and peripheral nervous system). Neurodegenerative diseases, include, for example, Alzheimer's Disease, stroke, multiple sclerosis etc.

The following is a list of several neurological disorders, symptoms, signs and syndromes that can be treated using compositions and methods according to the present invention: acquired epileptiform aphasia; acute disseminated encephalomyelitis; adrenoleukodystrophy; age-related macular degeneration; agenesis of the corpus callosum; agnosia; Aicardi syndrome; Alexander disease; Alpers' disease; alternating hemiplegia; Alzheimer's disease; Vascular dementia; amyotrophic lateral sclerosis; anencephaly; Angelman syndrome; angiomatosis; anoxia; aphasia; apraxia; arachnoid cysts; arachnoiditis; Anronl-Chiari malformation; arteriovenous malformation; Asperger syndrome; ataxia telegiectasia; attention deficit hyperactivity disorder; autism; autonomic dysfunction; back pain; Batten disease; Behcet's disease; Bell's palsy; benign essential blepharospasm; benign focal; amyotrophy; benign intracranial hypertension; Binswanger's disease; blepharospasm; Bloch Sulzberger syndrome; brachial plexus injury; brain abscess; brain injury; brain tumors (including glioblastoma multiforme); spinal tumor; Brown-Sequard syndrome; Canavan disease; carpal tunnel syndrome; causalgia; central pain syndrome; central pontine myelinolysis; cephalic disorder; cerebral aneurysm; cerebral arteriosclerosis; cerebral atrophy; cerebral gigantism; cerebral palsy; Charcot-Marie-Tooth disease; chemotherapy-induced neuropathy and neuropathic pain; Chiari malformation; chorea; chronic inflammatory demyelinating polyneuropathy; chronic pain; chronic regional pain syndrome; Coffin Lowry syndrome; coma, including persistent vegetative state; congenital facial diplegia; corticobasal degeneration; cranial arteritis; craniosynostosis; Creutzfeldt-Jakob disease; cumulative trauma disorders; Cushing's syndrome; cytomegalic inclusion body disease; cytomegalovirus infection; dancing eyes-dancing feet syndrome; Dandy-Walker syndrome; Dawson disease; De Morsier's syndrome; Dejerine-Klumke palsy; dementia; dermatomyositis; diabetic neuropathy; diffuse sclerosis; dysautonomia; dysgraphia; dyslexia; dystonias; early infantile epileptic encephalopathy; empty sella syndrome; encephalitis; encephaloceles; encephalotrigeminal angiomatosis; epilepsy; Erb's palsy; essential tremor; Fabry's disease; Fahr's syndrome; fainting; familial spastic paralysis; febrile seizures; Fisher syndrome; Friedreich's ataxia; fronto-temporal dementia and other "tauopathies"; Gaucher's disease; Gerstmann's syndrome; giant cell arteritis; giant cell inclusion disease; globoid cell leukodystrophy; Guillain-Barre syndrome; HTLV-1-associated myelopathy; Hallervorden-Spatz disease; head injury; headache; hemifacial spasm; hereditary spastic paraplegia; heredopathia atactica polyneuritiformis; herpes zoster oticus; herpes zoster; Hirayama syndrome; HIV-associated dementia and neuropathy (also neurological manifestations of AIDS); holoprosencephaly; Huntington's disease and other polyglutamine repeat diseases; hydranencephaly; hydrocephalus; hypercortisolism; hypoxia; immune-mediated encephalomyelitis; inclusion body myositis; incontinentia pigmenti; infantile phytanic acid storage disease; infantile refsum disease; infantile spasms; inflammatory myopathy; intracranial cyst; intracranial hypertension; Joubert syndrome; Kearns-Sayre syndrome; Kennedy disease Kinsbourne syndrome; Klippel Feil syndrome; Krabbe disease; Kugelberg-Welander disease; kuru; Lafora disease; Lambert-Eaton myasthenic syndrome; Landau-Kleffner syndrome; lateral medullary (Wallenberg) syndrome; learning disabilities; Leigh's disease; Lennox-Gustaut syndrome; Lesch-Nyhan syndrome; leukodystrophy; Lewy body dementia; Lissencephaly; locked-in syndrome; Lou Gehrig's disease (i.e., motor neuron disease or amyotrophic lateral sclerosis); lumbar disc disease; Lyme disease--neurological sequelae; Machado-Joseph disease; macrencephaly; megalencephaly; Melkersson-Rosenthal syndrome; Menieres disease; meningitis; Menkes disease; metachromatic leukodystrophy; microcephaly; migraine; Miller Fisher syndrome; mini-strokes; mitochondrial myopathies; Mobius syndrome; monomelic amyotrophy; motor neuron disease; Moyamoya disease; mucopolysaccharidoses; milti-infarct dementia; multifocal motor neuropathy; multiple sclerosis and other demyelinating disorders; multiple system atrophy with postural hypotension; p muscular dystrophy; myasthenia gravis; myelinoclastic diffuse sclerosis; myoclonic encephalopathy of infants; myoclonus; myopathy; myotonia congenital; narcolepsy; neurofibromatosis; neuroleptic malignant syndrome; neurological manifestations of AIDS; neurological sequelae of lupus; neuromyotonia; neuronal ceroid lipofuscinosis; neuronal migration disorders; Niemann-Pick disease; O'Sullivan-McLeod syndrome; occipital neuralgia; occult spinal dysraphism sequence; Ohtahara syndrome; olivopontocerebellar atrophy; opsoclonus myoclonus; optic neuritis; orthostatic hypotension; overuse syndrome; paresthesia; Parkinson's disease; paramyotonia congenital; paraneoplastic diseases; paroxysmal attacks; Parry Romberg syndrome; Pelizaeus-Merzbacher disease; periodic paralyses; peripheral neuropathy; painful neuropathy and neuropathic pain; persistent vegetative state; pervasive developmental disorders; photic sneeze reflex; phytanic acid storage disease; Pick's disease; pinched nerve; pituitary tumors; polymyositis; porencephaly; post-polio syndrome; postherpetic neuralgia; postinfectious encephalomyelitis; postural hypotension; Prader-Willi syndrome; primary lateral sclerosis; prion diseases; progressive hemifacial atrophy; progressive multifocal leukoencephalopathy; progressive sclerosing poliodystrophy; progressive supranuclear palsy; pseudotumor cerebri; Ramsay-Hunt syndrome (types I and II); Rasmussen's encephalitis; reflex sympathetic dystrophy syndrome; Refsum disease; repetitive motion disorders; repetitive stress injuries; restless legs syndrome; retrovirus-associated myelopathy; Rett syndrome; Reye's syndrome; Saint Vitus dance; Sandhoff disease; Schilder's disease; schizencephaly; septo-optic dysplasia; shaken baby syndrome; shingles; Shy-Drager syndrome; Sjögren's syndrome; sleep apnea; Soto's syndrome; spasticity; spina bifida; spinal cord injury; spinal cord tumors; spinal muscular atrophy; Stiff-Person syndrome; stroke; Sturge-Weber syndrome; subacute sclerosing panencephalitis; subcortical arteriosclerotic encephalopathy; Sydenham chorea; syncope; syringomyelia; tardive dyskinesia; Tay-Sachs disease; temporal arteritis; tethered spinal cord syndrome; Thomsen disease; thoracic outlet syndrome; Tic Douloureux; Todd's paralysis; Tourette syndrome; transient ischemic attack; transmissible spongiform encephalopathies; transverse myelitis; traumatic brain injury; tremor; trigeminal neuralgia; tropical spastic paraparesis; tuberous sclerosis; vascular dementia (multi-infarct dementia); vasculitis including temporal arteritis; Von Hippel-Lindau disease; Wallenberg's syndrome; Werdnig-Hoffman disease; West syndrome; whiplash; Williams syndrome; Wildon's disease; and Zellweger syndrome.

In another preferred embodiment, administration of STING prevents or treats patients suffering from autoimmune diseases. Examples of autoimmune diseases comprise: rheumatoid arthritis, systemic lupus erythematosus, inflammatory bowel diseases (IBDs) comprising Crohn disease (CD) and ulcerative colitis (UC) which are chronic inflammatory conditions with polygenic susceptibility.

In another preferred embodiment, STING is a prognostic marker for disease progression or outcome or response or resistance to anti-cancer therapies. For example, loss of STING, change in expression and/or function of STING as compared to a normal cell, or detection of a mutant STING molecule would be an indication of a cell progressing towards being a tumor cell.

In another preferred embodiment, STING is prognostic of a disease state comprising cancer, autoimmunity, infection by an agent and the like.

In a preferred embodiment, a peptide, polypeptide or protein encoded by an isolated nucleic acid set forth as SEQ ID NOS: 1 or 2, or an active fragment or variant thereof. In certain embodiments, the peptide is at least 380 consecutive amino acid residues in length.

In another preferred embodiment, a biomarker comprises a nucleic acid sequence set forth as SEQ ID NOS: 1 or 2, an active fragment, variant, or mutant thereof.

In another preferred embodiment, a biomarker comprises a peptide, polypeptide or protein encoded by a nucleic acid sequence set forth as SEQ ID NOS: 1 or 2, an active fragment, variant, or mutant thereof.

*Role in Autoimmunity:* Antibodies to double-stranded DNA are present in roughly half of patients with systemic lupus erythematosus, and are highly specific to the diagnosis of SLE (Simón JA, et al. Rheumatology (Oxford). 2004 Feb;43(2):220-4). In addition, these antibodies have prominent prognostic importance: SLE patients with anti-dsDNA antibodies have a much greater likelihood of developing severe manifestations of lupus including lupus nephritis and vasculitis, and have a substantially poorer long-term prognosis. Despite the long-standing awareness of the importance of dsDNA antibodies to lupus, the relevance of these antibodies to disease pathogenesis has remained mysterious. Isolated dsDNA specificities have been found in some patients that directly interact with target tissue antigens, but patients without identified cross-reactivities of this sort still often target the kidneys with similarly poor prognoses. B and T cell tolerance protocols toward dsDNA antigens in patients or in animal models of lupus have had very limited success (Renaudineau Y, et al. Arthritics Rheum. 2006 Aug;54(8):2523-32; Cardiel MH, et al. Arthritis Rheum. 2008 Aug;58(8):2470-80; Kang HK, et al. J Immunol. 2007 Jun 15;178(12):7849-58.). Interest in the relevance of nucleosomal DNA to induction of innate immunity and in particular severe dsDNA-associated lupus continues to exist, though, and non-TLR9 innate immune recognition pathways for self DNA to induce immune reactivity have been identified. Given the potential role that STING may play in inducing/amplifying innate immune responses to products potentially including constituents of cell debris (such as nucleosomes or cellular RNAs), STING may also play a role in the expression of a broader array of autoimmune and auto-inflammatory conditions. It is thus notable that the STING gene is located in humans at 5q31.2, at or immediately adjacent to the IBD5 locus that has been linked to disease risk and/or to more severe disease in processes including Crohn's disease, rheumatoid arthritis, diabetes mellitus type I, and celiac disease (7-10). At least one report has also observed an association between this region and risk for lupus (De Jager PL, et al. Genes Immun. 2006 Jun;7(4):327-34). Without wishing to be bound by theory, it is hypothesized that increased induction of STING-associated pathways increases pro-immune signaling, increasing the likelihood and severity of autoimmune diseases, including the tendency for anti-dsDNA-associated lupus to target major organs and to lead to poorer outcomes. Other diseases regulated by the STING pathway include multiple sclerosis, the cause of which may involve DNA based retroelements (Dolei A, Perron H. J Neurovirol. 2009 Jan;15(1):4-13; Christensen T. Association of human endogenous retroviruses with multiple sclerosis and possible interactions with herpes viruses Rev Med Virol. 2005 May-Jun;15(3):179-211).

*STING Interacting Molecules:* STING interacts or associates with a variety, including, as yet unknown molecules and pathways.

In a preferred embodiment, STING, compositions thereof, active fragment, variant, or mutant thereof modulates expression and/or function of molecules which interact or associate with, directly or indirectly, with STING. This includes all pathways and molecules involved in those pathways, directly or indirectly, which STING may affect (directly or indirectly).

For example, TRAMP-β which binds to STING, interacts with (identified via UniProtKB, MINT, and/or STRING), for example: SSR2 (ENSP000003572953); SSR4 ENSP000003591033 STRING (score=.996); SSR1 ENSP000002447633 STRING (score=.995); SSR3 ENSP000002650443 STRING (score=.995); SRPR ENSP000003280233 STRING (score=.96); TRAM1 ENSP000002622133 STRING (score=.955) DDOST ENSP000003641883 STRING (score=.943); RPN2 ENSP000003459043 STRING (score=.931); SEC61B ENSP000002236413 STRING (score=.925); SEC11A ENSP000002682203 STRING (score=.918); SRP19 ENSP000002829993 STRING (score=.917); SEC61G ENSP000003415383 STRING (score=.916); SRP9 ENSP000003052303 STRING (score=.915); DAD1 ENSP000002504983 STRING (score=.91); SRP68 ENSP000003120663 STRING (score=.91); SPCS2 ENSP000002636723 STRING (score=.907); SEC11C ENSP000002997143 STRING (score=.906); STT3B ENSP000002957703 STRING (score=.905); SEC61A1 ENSP000002432533 STRING (score=.903); SEC61A2 ENSP000003683193 STRING (score=.903); SRP72 ENSP000003421813 STRING (score=.902); ERO1L ENSP000003520443 STRING (score=.899) INS ENSP000003707313 STRING (score=.899); RPN1 ENSP000002962553 STRING (score=.899); SPCS1 ENSP000002330253 STRING (score=.899); SPCS3 ENSP000002645973 STRING (score=.899); SRP14 ENSP000002678843 STRING (score=.899); SRP54 ENSP000002167743 STRING (score=.899); SRPRB ENSP000002734063 STRING (score=.899); TRAM2 ENSP000001825273 STRING (score=.899); ENSP000003515683 STRING (score=.905).

In another example, SEC61B interacting proteins (P604681, 2 ENSP000002236413) [via UniProtKB, MINT, and/or STRING; Interactant Interaction Details GeneCard External ID(s)] comprise: SSR1 P433072, ENSP000002447633 MINT-4546723 STRING (score=.96); SEC61G ENSP000003415383 STRING (score=.986); SEC61A1 ENSP000002432533 STRING (score=.981); TRAM1 ENSP000002622133 STRING (score=.964); SSR3 ENSP000002650443 STRING (score=.934); SSR4 ENSP000003591033 STRING (score=.934); SSR2 ENSP000003572953 STRING (score=.925); RPN2 ENSP000003459043 STRING (score=.923); SRP9 ENSP000003052303 STRING (score=.912); DAD1 ENSP000002504983 STRING (score=.911); SPCS2 ENSP000002636723 STRING (score=.908) DDOST ENSP000003641883 STRING (score=.906); TRAM2 ENSP000001825273 STRING (score=.904); SRP68 ENSP000003120663 STRING (score=.903); SEC61A2 ENSP000003683193 STRING (score=.9); ERO1L ENSP000003520443 STRING (score=.899) INS ENSP000003707313 STRING (score=.899); RPN1 ENSP000002962553 STRING (score=.899); SEC11A ENSP000002682203 STRING (score=.899); SEC11C ENSP000002997143 STRING (score=.899); SPCS1 ENSP000002330253 STRING (score=.899); SPCS3 ENSP000002645973 STRING (score=.899); SRP14 ENSP000002678843 STRING (score=.899); SRP19 ENSP000002829993 STRING (score=.899); SRP54 ENSP000002167743 STRING (score=.899); SRP72 ENSP000003421813 STRING (score=.899); SRPR ENSP000003280233 STRING (score=.899); SRPRB ENSP000002734063 STRING (score=.899); STT3B ENSP000002957703 STRING (score=.899); DERL2 ENSP000001587713 STRING (score=.762); DERL3 ENSP000003153033 STRING (score=.762); EDEM1 ENSP000002564973 STRING (score=.746); SEC62 ENSP000003376883 STRING (score=.732); BCAP31 P515721 EBI-1788819, EBI-77683; -- ENSP000003515683 STRING (score=.899).

Examples of interacting proteins for EXOC2/Sec 5 (Q96KP11 ENSP000002304493) [via UniProtKB, MINT, and/or STRING; Interactant Interaction Details GeneCard External ID(s)] comprise: SERGEF Q9UGK81, ENSP000002659653 EBI-465715, EBI-465368 STRING (score=.998); EXOC3 ENSP000003233773 STRING (score=.997); EXOC8 ENSP000003535643 STRING (score=.997); RALA ENSP000000052573 STRING (score=.997); EXOC7 ENSP000003341003 STRING (score=.987); EXOC4 ENSP000002538613 STRING (score=.973); EXOC5 ENSP000003421003 STRING (score=.973); RALB ENSP000003653983 STRING (score=.972); EXOC6 ENSP000002607623 STRING (score=.969); EXOC1 ENSP000003706953 STRING (score=.954); ARF6 ENSP000002983163 STRING (score=.935); DPH3 ENSP000002850823 STRING (score=.928); RHOQ ENSP000002387383 STRING (score=.899); TRIP10 ENSP000003201173 STRING (score=.899).

### Vectors Expressing STING:

In another preferred embodiment, a vector comprises a polynucleotide set forth as SEQ ID NOS: 1, 2, variants, mutants or active fragments thereof.

The vector can be administered to a patient wherein expression of SEQ ID NOS: 1, 2, variants, mutants or active fragments thereof, induces a response comprising at least one of: anti-viral activity, immune responses, immune signaling or intracellular B-form DNA.

A number of vectors are known to be capable of mediating transfer of gene products to mammalian cells, as is known in the art and described herein. A "vector" (sometimes referred to as gene delivery or gene transfer "vehicle") refers to a macromolecule or complex of molecules comprising a polynucleotide to be delivered to a host cell, either *in vitro* or *in vivo.* The polynucleotide to be delivered may comprise a coding sequence of interest in gene therapy. Vectors include, for example, viral vectors (such as adenoviruses ("Ad"), adeno-associated viruses (AAV), and vesicular stomatitis virus (VSV) and retroviruses), liposomes and other lipid-containing complexes, and other macromolecular complexes capable of mediating delivery of a polynucleotide to a host cell. Vectors can also comprise other components or functionalities that further modulate gene delivery and/or gene expression, or that otherwise provide beneficial properties to the targeted cells. As described and illustrated in more detail below, such other components include, for example, components that influence binding or targeting to cells (including components that mediate cell-type or tissue-specific binding); components that influence uptake of the vector nucleic acid by the cell; components that influence localization of the polynucleotide within the cell after uptake (such as agents mediating nuclear localization); and components that influence expression of the polynucleotide. Such components also might include markers, such as detectable and/or selectable markers that can be used to detect or select for cells that have taken up and are expressing the nucleic acid delivered by the vector. Such components can be provided as a natural feature of the vector (such as the use of certain viral vectors which have components or functionalities mediating binding and uptake), or vectors can be modified to provide such functionalities. Other vectors include those described by Chen et al; BioTechniques, 34: 167-171 (2003). A large variety of such vectors are known in the art and are generally available.

A "recombinant viral vector" refers to a viral vector comprising one or more heterologous gene products or sequences. Since many viral vectors exhibit size-constraints associated with packaging, the heterologous gene products or sequences are typically introduced by replacing one or more portions of the viral genome. Such viruses may become replication-defective, requiring the deleted function(s) to be provided in trans during viral replication and encapsidation (by using, e.g., a helper virus or a packaging cell line carrying gene products necessary for replication and/or encapsidation). Modified viral vectors in which a polynucleotide to be delivered is carried on the outside of the viral particle have also been described (see, e.g., Curiel, D T, et al. PNAS 88: 8850-8854, 1991).

Suitable nucleic acid delivery systems include viral vector, typically sequence from at least one of an adenovirus, adenovirus-associated virus (AAV), helper-dependent adenovirus, retrovirus, or hemagglutinating virus of Japan-liposome (HVJ) complex. Preferably, the viral vector comprises a strong eukaryotic promoter operably linked to the polynucleotide e.g., a cytomegalovirus (CMV) promoter.

Additionally preferred vectors include viral vectors, fusion proteins and chemical conjugates. Retroviral vectors include Moloney murine leukemia viruses and HIV-based viruses. One preferred HIV-based viral vector comprises at least two vectors wherein the gag and pol genes are from an HIV genome and the env gene is from another virus. DNA viral vectors are preferred. These vectors include pox vectors such as orthopox or avipox vectors, herpesvirus vectors such as a herpes simplex I virus (HSV) vector [Geller, A.I. et al., J. Neurochem, 64: 487 (1995); Lim, F., et al., in DNA Cloning: Mammalian Systems, D. Glover, Ed. (Oxford Univ. Press, Oxford England) (1995); Geller, A.I. et al., Proc Natl. Acad. Sci.: U.S.A.:90 7603 (1993); Geller, A.I., et al., Proc Natl. Acad. Sci USA: 87:1149 (1990)], Adenovirus Vectors [LeGal LaSalle et al., Science, 259:988 (1993); Davidson, et al., Nat. Genet. 3: 219 (1993); Yang, et al., J. Virol. 69: 2004 (1995)] and Adeno-associated Virus Vectors [Kaplitt, M.G., et al., Nat. Genet. 8:148 (1994)].

Pox viral vectors introduce the gene into the cells cytoplasm. Avipox virus vectors result in only a short term expression of the nucleic acid. Adenovirus vectors, adeno-associated virus vectors and herpes simplex virus (HSV) vectors may be an indication for some invention embodiments. The adenovirus vector results in a shorter term expression (e.g., less than about a month) than adeno-associated virus, in some embodiments, may exhibit much longer expression. The particular vector chosen will depend upon the target cell and the condition being treated. The selection of appropriate promoters can readily be accomplished. Preferably, one would use a high expression promoter. An example of a suitable promoter is the 763-base-pair cytomegalovirus (CMV) promoter. The Rous sarcoma virus (RSV) (Davis, et al., Hum Gene Ther 4:151 (1993)) and MMT promoters may also be used. Certain proteins can expressed using their native promoter. Other elements that can enhance expression can also be included such as an enhancer or a system that results in high levels of expression such as a tat gene and tar element. This cassette can then be inserted into a vector, e.g., a plasmid vector such as, pUC19, pUC118, pBR322, or other known plasmid vectors, that includes, for example, an *E. coli* origin of replication. See, Sambrook, et al., Molecular Cloning: A Laboratory Manual, Cold Spring Harbor Laboratory press, (1989). The plasmid vector may also include a selectable marker such as the β-lactamase gene for ampicillin resistance, provided that the marker polypeptide does not adversely effect the metabolism of the organism being treated. The cassette can also be bound to a nucleic acid binding moiety in a synthetic delivery system, such as the system disclosed in WO 95/22618.

If desired, the polynucleotides of the invention may also be used with a microdelivery vehicle such as cationic liposomes and adenoviral vectors. For a review of the procedures for liposome preparation, targeting and delivery of contents, see Mannino and Gould-Fogerite, BioTechniques, 6:682 (1988). See also, Felgner and Holm, Bethesda Res. Lab. Focus, 11(2):21 (1989) and Maurer, R.A., Bethesda Res. Lab. Focus, 11(2):25 (1989).

Replication-defective recombinant adenoviral vectors, can be produced in accordance with known techniques. See, Quantin, et al., Proc. Natl. Acad. Sci. USA, 89:2581-2584 (1992); Stratford-Perricadet, et al., J. Clin. Invest., 90:626-630 (1992); and Rosenfeld, et al., Cell, 68:143-155 (1992).

Another delivery method is to use single stranded DNA producing vectors which can produce the STING intracellularly. See for example, Chen et al, BioTechniques, 34: 167-171 (2003), which is incorporated herein, by reference, in its entirety.

Expression of STING may be controlled by any promoter/enhancer element known in the art, but these regulatory elements must be functional in the host selected for expression. Promoters which may be used to control STING gene expression include, but are not limited to, cytomegalovirus (CMV) promoter (U.S. Pat. Nos. 5,385,839 and 5,168,062), the SV40 early promoter region (Benoist and Chambon, 1981, Nature 290:304-310), the promoter contained in the 3' long terminal repeat of Rous sarcoma virus (Yamamoto, et al., Cell 22:787-797, 1980), the herpes thymidine kinase promoter (Wagner et al., Proc. Natl. Acad. Sci. U.S.A. 78:1441-1445, 1981), the regulatory sequences of the metallothionein gene (Brinster et al., Nature 296:39-42, 1982); prokaryotic expression vectors such as the .beta.-lactamase promoter (Villa-Kamaroff, et al., Proc. Natl. Acad. Sci. U.S.A. 75:3727-3731, 1978), or the tac promoter *(*DeBoer, et al., Proc. Natl. Acad. Sci. U.S.A. 80:21-25, 1983); see also "Useful proteins from recombinant bacteria" in Scientific American, 242:74-94, 1980; promoter elements from yeast or other fungi such as the Gal 4 promoter, the ADC (alcohol dehydrogenase) promoter, PGK (phosphoglycerol kinase) promoter, alkaline phosphatase promoter; and the animal transcriptional control regions, which exhibit tissue specificity and have been utilized in transgenic animals: elastase I gene control region which is active in pancreatic acinar cells (Swift et al., Cell 38:639-646, 1984; Ornitz et al., Cold Spring Harbor Symp. Quant. Biol. 50:399-409, 1986; MacDonald, Hepatology 7:425-515, 1987); insulin gene control region which is active in pancreatic beta cells (Hanahan, Nature 315:115-122, 1985), immunoglobulin gene control region which is active in lymphoid cells (Grosschedl et al., Cell 38:647-658, 1984; Adames et al., Nature 318:533-538, 1985; Alexander et al., Mol. Cell Biol. 7:1436-1444, 1987), mouse mammary tumor virus control region which is active in testicular, breast, lymphoid and mast cells (Leder et al., Cell 45:485-495, 1986), albumin gene control region which is active in liver (Pinkert et al., Genes and Devel. 1:268-276, 1987), alpha-fetoprotein gene control region which is active in liver (Krumlauf et al., Mol. Cell. Biol. 5:1639-1648, 1985; Hammer et al., Science 235:53-58, 1987), alpha 1-antitrypsin gene control region which is active in the liver (Kelsey et al., Genes and Devel. 1: 161-171, 1987), beta-globin gene control region which is active in myeloid cells (Mogram et al., Nature 315:338-340, 1985; Kollias et al., Cell 46:89-94, 1986), myelin basic protein gene control region which is active in oligodendrocyte cells in the brain (Readhead et al., Cell 48:703-712, 1987), myosin light chain-2 gene control region which is active in skeletal muscle (Sani, Nature 314:283-286, 1985), and gonadotropic releasing hormone gene control region which is active in the hypothalamus (Mason et al., Science 234:1372-1378, 1986).

A wide variety of host/expression vector combinations may be employed in expressing the DNA sequences of this invention. Useful expression vectors, for example, may consist of segments of chromosomal, non-chromosomal and synthetic DNA sequences. Suitable vectors include derivatives of SV40 and known bacterial plasmids, e.g., *E. coli* plasmids col E1, pCR1, pBR322, pMal-C2, pET, pGEX (Smith et al., Gene 67:31-40, 1988), pMB9 and their derivatives, plasmids such as RP4; phage DNAs, e.g., the numerous derivatives of phage 1, e.g., NM989, and other phage DNA, e.g., M13 and filamentous single stranded phage DNA; yeast plasmids such as the 2µ plasmid or derivatives thereof, vectors useful in eukaryotic cells, such as vectors useful in insect or mammalian cells; vectors derived from combinations of plasmids and phage DNAs, such as plasmids that have been modified to employ phage DNA or other expression control sequences; and the like.

Yeast expression systems can also be used according to the invention to express STING. For example, the non-fusion pYES2 vector (XbaI, SphI, ShoI, NotI, GstXI, EcoRI, BstXI, BamH1, SacI, Kpn1, and HindIII cloning sites; Invitrogen) or the fusion pYESHisA, B, C (XbaI, SphI, ShoI, NotI, BstXI, EcoRI, BamH1, SacI, KpnI, and HindIII cloning sites, N-terminal peptide purified with ProBond resin and cleaved with enterokinase; Invitrogen), to mention just two, can be employed according to the invention. A yeast two-hybrid expression system can be prepared in accordance with the invention.

One preferred delivery system is a recombinant viral vector that incorporates one or more of the polynucleotides therein, preferably about one polynucleotide. Preferably, the viral vector used in the invention methods has a pfu (plague forming units) of from about 10⁸ to about 5x 10¹⁰ pfu. In embodiments in which the polynucleotide is to be administered with a non-viral vector, use of between from about 0.1 nanograms to about 4000 micrograms will often be useful e.g., about 1 nanogram to about 100 micrograms.

### Immune modulation:

STING activates innate immune responses. Accordingly, it is expected that it can be attached to disease specific antigens and used in vaccines as an adjuvant, modulates the immune response and as such, applicable in a wide variety of disease therapies.

Immune systems are classified into two general systems, the "innate" or "primary" immune system and the "acquired/adaptive" or "secondary" immune system. It is thought that the innate immune system initially keeps the infection under control, allowing time for the adaptive immune system to develop an appropriate response. The various components of the innate immune system trigger and augment the components of the adaptive immune system, including antigen-specific B and T lymphocytes (Kos, Immunol. Res. 1998, 17:303; Romagnani, Immunol. Today. 1992, 13: 379; Banchereau and Steinman, Nature. 1988, 392:245). A "primary immune response" refers to an innate immune response that is not affected by prior contact with the antigen. The main protective mechanisms of primary immunity are the skin (protects against attachment of potential environmental invaders), mucous (traps bacteria and other foreign material), gastric acid (destroys swallowed invaders), antimicrobial substances such as interferon (IFN) (inhibits viral replication) and complement proteins (promotes bacterial destruction), fever (intensifies action of interferons, inhibits microbial growth, and enhances tissue repair), natural killer (NK) cells (destroy microbes and certain tumor cells, and attack certain virus infected cells), and the inflammatory response (mobilizes leukocytes such as macrophages and dendritic cells to phagocytose invaders).

Some cells of the innate immune system, including macrophages and dendritic cells (DC), function as part of the adaptive immune system as well by taking up foreign antigens through pattern recognition receptors, combining peptide fragments of these antigens with major histocompatibility complex (MHC) class I and class II molecules, and stimulating naive CD8⁺ and CD4⁺ T cells respectively (Banchereau and Steinman, *supra*; Holmskov et al., Immunol. Today. 1994, 15:67; Ulevitch and Tobias Annu. Rev. Immunol. 1995, 13:437). Professional antigen-presenting cells (APCs) communicate with these T cells, leading to the differentiation of naive CD4+ T cells into T-helper 1 (Th1) or T-helper 2 (Th2) lymphocytes that mediate cellular and humoral immunity, respectively (Trinchieri Annu. Rev. Immunol. 1995, 13:251; Howard and O'Garra, Immunol. Today. 1992, 13:198; Abbas et al., Nature. 1996, 383:787; Okamura et al, Adv. Immunol. 1998, 70:281; Mosmann and Sad, Immunol. Today. 1996, 17:138; O'Garra Immunity. 1998, 8:275).

In adaptive immunity, adaptive T and B cell immune responses work together with innate immune responses. The basis of the adaptive immune response is that of clonal recognition and response. An antigen selects the clones of cell which recognize it, and the first element of a specific immune response must be rapid proliferation of the specific lymphocytes. This is followed by further differentiation of the responding cells as the effector phase of the immune response develops. These effector T lymphocytes, in the context of the embodiments, are the tumor specific T lymphocytes.

In a preferred embodiment, an immunomodulatory composition comprises STING polynucleotides, peptides, mutants, variants or fragments thereof.

In another preferred embodiment, an immunomodulatory composition comprises an agent which modulates the expression and/or function of STING, thus affecting the intracellular pathways that STING is involved in (e.g. regulation of intracellular DNA-mediated type -1 interferon induction). The immuniomodulatory compositions are used in preventing or treating a patients varying diseases or disorders. Examples of diseases or disorders comprise: immune disorders, autoimmunity, cancer, inflammation, cachexia, neurodegenerative disease or disorders, neurological diseases or disorders, cardiac dysfunction, transplantation, or infection.

In another preferred embodiment, an agent inhibits or increases a function of STING as compared to a baseline control.

In another preferred embodiment, STING inhibits or increases an immune response as compared to a baseline control.

In another preferred embodiment, a partner molecule or a molecule which is part of a pathway whereby STING acts either directly, for example, binding to a partner molecule, or indirectly, for example, STING affects a molecule in one part of the pathway thereby affecting the entire pathway downstream of that interaction. Examples of partner molecules that STING may interact with, directly or indirectly comprise: RIG-I, Ssr2, TRAP, SEC61, NK-kB, TBK, IPS-1, MAM components, exocyst components, major histocompatibility complex (MHC) class I and II components, GARP components, translocon components, endosome components or IRF3, IRF7.

In another preferred embodiment, an adjuvant which can be administered with one or more vaccines (e.g. DNA vaccine), or other therapeutic agents comprises: (i) a peptide encoded by nucleic acid sequence set forth as SEQ ID NOS: 1 or 2, an active fragment, variant, or mutant thereof; or, (ii) a nucleic acid sequence set forth as SEQ ID NOS: 1 or 2, an active fragment, variant, or mutant thereof.

In another preferred embodiment, a method of modulating a DNA-induced innate immune response in a patient in need thereof, comprises administering to the patient, a therapeutically effective amount of: (i) a peptide encoded by nucleic acid sequence set forth as SEQ ID NOS: 1 or 2, an active fragment, variant, or mutant thereof; or, (ii) a nucleic acid sequence set forth as SEQ ID NOS: 1 or 2, an active fragment, variant, or mutant thereof.

In another preferred embodiment, a method of treating a viral infection in a patient comprises administering to the patient, a therapeutically effective amount of: (i) a peptide encoded by nucleic acid sequence set forth as SEQ ID NOS: 1 or 2, an active fragment, variant, or mutant thereof; or, (ii) a nucleic acid sequence *(Sting)* set forth as SEQ ID NOS: 1 or 2, an active fragment, variant, or mutant thereof.

In a preferred embodiment, STING regulates cross-presentation of antigen. STING also interacts with beta-2 microimmunoglobulin, a component of the MHC-I. β₂ microglobulin is necessary for cell surface expression of MHC class I and stability of the peptide binding groove. In the absence of β₂ microglobulin, very limited amounts of MHC class I (classical and non-classical) molecules can be detected on the surface. In the absence of MHC class I, CD8 T cells cannot develop. Type II IFN can upregulate components of the proteosome (immune subunits) that are incorporated into immunoproteosomes. These produce peptides that effectively, with antigen, bind to MHC class I receptors to stimulate cell-mediated immunity. Thus, in some preferred embodiments, STING regulate these processes and modulates immunity. Defects in this process could also involve STING and provide screens and targets for drugs.

STING also appears important for recognizing DNA's ability to stimulate the innate immune response (including DNA comprising vectors, plasmids, poly dA-dT, poly dC-dG and DNA of varying lengths and sequence composition including interferon stimulating DNA [ISD]). Thus, in another preferred embodiment, STING modulates the innate immune response.

In another preferred embodiment, a method of modulating a DNA-induced innate immune response in a patient in need thereof, comprises administering to the patient, a therapeutically effective amount of: (i) a peptide encoded by nucleic acid sequence set forth as SEQ ID NOS: 1 or 2, an active fragment, variant, or mutant thereof; or, (ii) a nucleic acid sequence *(Sting)* set forth as SEQ ID NOS: 1 or 2, an active fragment, variant, or mutant thereof.

All DNA vaccines function through the TBK1 pathway, activating the IFN pathway through TBK1, which is essential for an immune response. It appears that STING mediates this process, since plasmid DNA completely fails to activate IFN in the absence of STING. Thus, STING and related molecules should be important components in future vaccines and immunotherapeutic methods. STING was also found to activate the NF-kB pathway.

In another preferred embodiment, an adjuvant comprises: (i) a peptide encoded by nucleic acid sequence set forth as SEQ ID NOS: 1 or 2, an active fragment, variant, or mutant thereof; or, (ii) a nucleic acid sequence *(Sting)* set forth as SEQ ID NOS: 1 or 2, an active fragment, variant, or mutant thereof.

STING may regulate the translocon and proteosome and Golgi/endosome/exocyst function and provide a new mechanism for the strategic design of new vaccine and therapeutic concepts.

Regulation of the pathway by STING attached to antigens, or encapsulated, is expected to result in new vaccine concepts since this molecule potently stimulates the innate immune pathway. The pathway could also be stimulated by other molecules- to regulate function. The pathway further implicates the translocon in the mechanism of action. Thus, these molecules could also be targeted for vaccine and therapeutic purposes. These pathways or STING itself could be disregulated in cancers or by viruses following infection-contributing to pathogenesis.

STING may be targeted by pathogens such as flaviviruses, yellow fever virus, dengue virus for suppression, to evade host defense responses, and may be a targeted for anti-pathogen drugs.

STING may be useful in vaccines against pathogens and cancer, and could be targeted by drugs or used as a therapeutic target to boost immune system. As STING and intracellular DNA pathways may be disregulated in cancer (the data herein indicates it is not expressed in many cancer cells), it may also serve as a biomarker for disease and/or a prognostic marker for outcome of disease. STING may be frequently disregulated in cancer and provide a mechanism for viral oncolysis.

In a preferred embodiment, a vaccine comprises a nucleic acid sequence set forth as SEQ ID NOS: 1 or 2, or an active fragment, variant, or mutant thereof. The vaccine can be administered in the context of a vector or as a nucleic acid.

In another preferred embodiment, a vaccine comprises a peptide, polypeptide or protein encoded by a nucleic acid sequence set forth as SEQ ID NOS: 1 or 2, an active fragment, variant, or mutant thereof.

In another preferred embodiment, a method of modulating an immune response *in vivo,* comprises administering to a patient in need thereof, an immunoregulatory composition in a therapeutically effective amount. Preferably, the immunomodulatory composition comprises a nucleic acid sequence set forth as SEQ ID NOS: 1 or 2, an active fragment, variant, or mutant thereof.

In another preferred embodiment, the immunomodulatory composition comprises a peptide, polypeptide or protein encoded by a nucleic acid sequence set forth as SEQ ID NOS: 1 or 2, an active fragment, variant, or mutant thereof.

In yet another embodiment, a method of preventing or treating a disease or disorder in a subject in need thereof, comprises administering to a patient an immunoregulatory composition in a therapeutically effective amount wherein the immunomodulatory composition comprises a nucleic acid sequence set forth as SEQ ID NOS: 1 or 2, an active fragment, variant, or mutant thereof. In another preferred embodiment, the immunomodulatory composition comprises a peptide, polypeptide or protein encoded by a nucleic acid sequence set forth as SEQ ID NOS: 1 or 2, an active fragment, variant, or mutant thereof.

In another preferred embodiment, STING, mutants, variants and active fragments thereof, are administered to a patient to prevent or treat an infection from an infectious disease agent, for example, such as a viral, bacterial, fungal or protozoan agent.

Viruses such as the herpes family (HSV-1- 8) and other DNA viruses implicated in cancer such as HPV and HBV may also inadvertently activate STING to induce IFN and an innate and thus adaptive immune response. The same result is apparent following infection with bacteria such as Listeria. The DNA from Listeria normally activates IFN, but this does not occur in the absence of STING. Collectively, STING is required for innate signaling following infection with negative stranded RNA viruses, plausible positive-stranded viruses, as well as DNA viruses and certain bacteria and other DNA pathogens such as parasites and fungi. Thus, STING appears even more important than RIG-1 or MDA5 or IPS-1 (which collectively just modulate RNA virus innate immunity).

In a preferred embodiment, a method of treating a viral infection in a patient comprises administering to the patient, a therapeutically effective amount of: (i) a peptide encoded by nucleic acid sequence set forth as SEQ ID NOS: 1 or 2, an active fragment, variant, or mutant thereof; or, (ii) a nucleic acid sequence *(Sting)* set forth as SEQ ID NOS: 1 or 2, an active fragment, variant, or mutant thereof.

*Peptides:* Nucleic acids encoding for STING peptides are set forth as SEQ ID NOS: 1 and 2. The term "STING" will refer to both the nucleic acids and peptides. The invention is not limited to these two species but includes without limitation, allelic variants, species variants, splicing variants, mutants, fragments, and the like. For illustrative purposes only, these two sequences will be used throughout the specification but is not to be construed as a limitation.

In a preferred embodiment, peptides encoded by SEQ ID NOS: 1 or 2 comprise at least five consecutive amino acid residues with the understanding that they are "active" peptides. "Active" includes one or more functions of STING which includes known functions as described herein but also any other function that is innate to the STING molecule or including one which may be altered based on any manipulation by the end user.

In another preferred embodiment, STING peptide includes the peptide itself, chemical equivalents thereto, isomers thereof (e.g., isomers, stereoisomers, retro isomers, retro-inverso isomers, all-[D] isomers, all-[L] isomers, or mixed [L] and [D] isomers thereof), conservative substitutions therein, precursor forms thereof, endoproteolytically-processed forms thereof, such as cleavage of single amino acids from N or C terminals or immunologically active metabolites of the peptides of the invention, pharmaceutically-acceptable salts and esters thereof, and other forms resulting from post-translational modification. Also included is any parent sequence, up to and including 10, 9, 8, 7, 6, 5 and 4 amino acids in length (cyclized, or linear, or branched from the core parent sequence), for which the specified sequence is a subsequence. A person skilled in the art would appreciate that where the peptide can be a monomer, dimer, a trimer, etc. The use of the peptides of the present invention include use of peptides wherein the active fragment or fragments are complexed to one or more binding partners. Modified peptides which retain the activity of the peptides of the invention are encompassed within the scope of the present invention.

In another preferred embodiment, a STING peptide comprises at least one non-native amino acid residue or a non-amino acid molecule. A "non-native" amino acid residue comprises any change to an amino acid which is encoded by the STING nucleic acid sequence. Thus, a non-native amino acid residue or non-amino acid molecule comprises, without limitation: a chemical equivalent, analog, synthetic molecule, derivative, variant, substitution, peptide nucleic acid, a linker molecule, inorganic molecule etc.

The mutations can be introduced at the nucleic acid level or at the amino acid level. With respect to particular nucleic acid sequences, because of the degeneracy of the genetic code, a large number of functionally identical nucleic acids encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations," which are one species of conservatively modified variations. If mutations at the nucleic acid level are introduced to encode a particular amino acid, then one or more nucleic acids are altered. For example proline is encoded by CCC, CCA, CCG, CCU; thus, one base change, e.g. CCC (proline) to GCC gives rise to alanine. Thus by way of example every natural or non-natural nucleic acid sequence herein which encodes a natural or non-natural polypeptide also describes every possible silent variation of the natural or non-natural nucleic acid. One of skill will recognize that each codon in a natural or non-natural nucleic acid (except AUG, which is ordinarily the only codon for methionine, and TGG, which is ordinarily the only codon for tryptophan) can be modified to yield a functionally identical molecule or a different molecule. Accordingly, each silent variation of a natural and non-natural nucleic acid which encodes a natural and non-natural polypeptide is implicit in each described sequence.

As to amino acid sequences, individual substitutions, deletions or additions to a nucleic acid, peptide, polypeptide, or protein sequence which alters, adds or deletes a single natural and non-natural amino acid or a small percentage of natural and non-natural amino acids in the encoded sequence, the alteration results in the deletion of an amino acid, addition of an amino acid, or substitution of a natural and non-natural amino acid with a chemically similar amino acid. Conservative substitution tables providing functionally similar natural amino acids are well known in the art. Such conservatively modified variants are in addition to and do not exclude polymorphic variants, interspecies homologs, and alleles of the methods and compositions described herein.

A "non-natural amino acid" refers to an amino acid that is not one of the 20 common amino acids or pyrolysine or selenocysteine. Other terms that may be used synonymously with the term "non-natural amino acid" is "non-naturally encoded amino acid," "unnatural amino acid," "non-naturally-occurring amino acid," and variously hyphenated and non-hyphenated versions thereof. The term "non-natural amino acid" includes, but is not limited to, amino acids which occur naturally by modification of a naturally encoded amino acid (including but not limited to, the 20 common amino acids or pyrrolysine and selenocysteine) but are not themselves incorporated, without user manipulation, into a growing polypeptide chain by the translation complex. Examples of naturally-occurring amino acids that are not naturally-encoded include, but are not limited to, N-acetylglucosaminyl-L-serine, N-acetylglucosaminyl-L-threonine, and O-phosphotyrosine. Additionally, the term "non-natural amino acid" includes, but is not limited to, amino acids which do not occur naturally and may be obtained synthetically or may be obtained by modification of non-natural amino acids.

In some cases, the non-natural amino acid substitution(s) or incorporation(s) will be combined with other additions, substitutions, or deletions within the polypeptide to affect other chemical, physical, pharmacologic and/or biological traits. In some cases, the other additions, substitutions or deletions may increase the stability (including but not limited to, resistance to proteolytic degradation) of the polypeptide or increase affinity of the polypeptide for its appropriate receptor, ligand and/or binding proteins. In some cases, the other additions, substitutions or deletions may increase the solubility of the polypeptide. In some embodiments sites are selected for substitution with a naturally encoded or non-natural amino acid in addition to another site for incorporation of a non-natural amino acid for the purpose of increasing the polypeptide solubility following expression in recombinant host cells. In some embodiments, the polypeptides comprise another addition, substitution, or deletion that modulates affinity for the associated ligand, binding proteins, and/or receptor, modulates (including but not limited to, increases or decreases) receptor dimerization, stabilizes receptor dimers, modulates circulating half-life, modulates release or bioavailability, facilitates purification, or improves or alters a particular route of administration. Similarly, the non-natural amino acid polypeptide can comprise chemical or enzyme cleavage sequences, protease cleavage sequences, reactive groups, antibody-binding domains (including but not limited to, FLAG or poly-His) or other affinity based sequences (including but not limited to, FLAG, poly-His, GST, etc.) or linked molecules (including but not limited to, biotin) that improve detection (including but not limited to, GFP), purification, transport thru tissues or cell membranes, prodrug release or activation, size reduction, or other traits of the polypeptide.

The methods and compositions described herein include incorporation of one or more non-natural amino acids into a polypeptide. One or more non-natural amino acids may be incorporated at one or more particular positions which does not disrupt activity of the polypeptide. This can be achieved by making "conservative" substitutions, including but not limited to, substituting hydrophobic amino acids with non-natural or natural hydrophobic amino acids, bulky amino acids with non-natural or natural bulky amino acids, hydrophilic amino acids with non-natural or natural hydrophilic amino acids) and/or inserting the non-natural amino acid in a location that is not required for activity.

A variety of biochemical and structural approaches can be employed to select the desired sites for substitution with a non-natural amino acid within the polypeptide. Any position of the polypeptide chain is suitable for selection to incorporate a non-natural amino acid, and selection may be based on rational design or by random selection for any or no particular desired purpose. Selection of desired sites may be based on producing a non-natural amino acid polypeptide (which may be further modified or remain unmodified) having any desired property or activity, including but not limited to agonists, super-agonists, partial agonists, inverse agonists, antagonists, receptor binding modulators, receptor activity modulators, modulators of binding to binder partners, binding partner activity modulators, binding partner conformation modulators, dimer or multimer formation, no change to activity or property compared to the native molecule, or manipulating any physical or chemical property of the polypeptide such as solubility, aggregation, or stability. For example, locations in the polypeptide required for biological activity of a polypeptide can be identified using methods including, but not limited to, point mutation analysis, alanine scanning or homolog scanning methods. Residues other than those identified as critical to biological activity by methods including, but not limited to, alanine or homolog scanning mutagenesis may be good candidates for substitution with a non-natural amino acid depending on the desired activity sought for the polypeptide. Alternatively, the sites identified as critical to biological activity may also be good candidates for substitution with a non-natural amino acid, again depending on the desired activity sought for the polypeptide. Another alternative would be to make serial substitutions in each position on the polypeptide chain with a non-natural amino acid and observe the effect on the activities of the polypeptide. Any means, technique, or method for selecting a position for substitution with a non-natural amino acid into any polypeptide is suitable for use in the methods, techniques and compositions described herein.

The structure and activity of naturally-occurring mutants of a polypeptide that contain deletions can also be examined to determine regions of the protein that are likely to be tolerant of substitution with a non-natural amino acid. Once residues that are likely to be intolerant to substitution with non-natural amino acids have been eliminated, the impact of proposed substitutions at each of the remaining positions can be examined using methods including, but not limited to, the three-dimensional structure of the relevant polypeptide, and any associated ligands or binding proteins. X-ray crystallographic and NMR structures of many polypeptides are available in the Protein Data Bank (PDB, www.rcsb.org), a centralized database containing three-dimensional structural data of large molecules of proteins and nucleic acids, one can be used to identify amino acid positions that can be substituted with non-natural amino acids. In addition, models may be made investigating the secondary and tertiary structure of polypeptides, if three-dimensional structural data is not available. Thus, the identity of amino acid positions that can be substituted with non-natural amino acids can be readily obtained. Exemplary sites of incorporation of a non-natural amino acid include, but are not limited to, those that are excluded from potential receptor binding regions, or regions for binding to binding proteins or ligands may be fully or partially solvent exposed, have minimal or no hydrogen-bonding interactions with nearby residues, may be minimally exposed to nearby reactive residues, and/or may be in regions that are highly flexible as predicted by the three-dimensional crystal structure of a particular polypeptide with its associated receptor, ligand or binding proteins.

A wide variety of non-natural amino acids can be substituted for, or incorporated into, a given position in a polypeptide. By way of example, a particular non-natural amino acid may be selected for incorporation based on an examination of the three dimensional crystal structure of a polypeptide with its associated ligand, receptor and/or binding proteins, a preference for conservative substitutions

As further used herein, a "chemical equivalent" of a peptide of the invention is a molecule which possesses the same desired activity, e.g. immunological activity, as peptides described herein, and exhibits a trivial chemical different, or a molecule which is converted, under mild conditions, into a peptide of the invention (e.g., esters, ethers, reduction products, and complexes of the peptides of the invention).

Additionally, as used herein, "conservative substitutions" are those amino acid substitutions which are functionally equivalent to the substituted amino acid residue, either because they have similar polarity or steric arrangement, or because they belong to the same class as the substituted residue (e.g., hydrophobic, acidic, or basic). The term "conservative substitutions", as defined herein, includes substitutions having an inconsequential effect on the ability of the peptide of the invention to enhance innate immunity. Examples of conservative substitutions include the substitution of a polar (hydrophilic) residue for another (e.g., arginine/lysine, glutamine/asparagine, or threonine/serine); the substitution of a nonpolar (hydrophobic) residue (e.g. isoleucine, leucine, methionine, phenylalanine, tyrosine) for another, the substitution of an acidic residue (e.g., aspartic acid or glutamic acid) for another; or the substitution of a basic residue (e.g., arginine, histidine, lysine or ornithine) for another.

The term "analogue", as used herein, includes any peptide having an amino acid sequence substantially identical to a sequence described herein, in which at least one residue has been conservatively substituted with a functionally-similar residue. An "analogue" includes functional variants and obvious chemical equivalents of an amino acid sequence of a STING peptide. As further used herein, the term "functional variant" refers to the activity of a peptide that demonstrates an ability to modulate innate immunity, such as, for example by stimulating IFNβ; interacting with one or more molecules, such as for example, RIG-I, Ssr2/TRAPβ; activating a transcription pathway, such as for example, activating NF-κB, IRF3, etc. An "analogue" further includes any pharmaceutically-acceptable salt of an analogue as described herein.

A "derivative", as used herein, refers to a peptide of the invention having one or more amino acids chemically derivatized by reaction of a functional side group. Exemplary derivatized molecules include, without limitation, peptide molecules in which free amino groups have been derivatized to form salts or amides, by adding acetyl groups, amine hydrochlorides, carbobenzoxy groups, chloroacetyl groups, formyl groups, p-toluene sulfonyl groups, or t-butyloxycarbonyl groups. Free hydroxyl groups may be derivatized to form O-acyl or O-alkyl derivatives. Furthermore, free carboxyl groups may be derivatized to form salts, esters (e.g., methyl and ethyl esters), or hydrazides. Thus, a "derivative" further includes any pharmaceutically-acceptable salt of a derivative as described herein.

In one embodiment of the present invention, the isolated peptide of the invention has a modified C-terminus and/or a modified N-terminus. For example, the isolated peptide may have an amidated C-terminus. For example, the amino terminus can be acetylated (Ac) or the carboxy terminus can be amidated (NH₂). However, in one embodiment of the invention, the peptides of the invention are preferably not acetylated if such a modification would result in loss of desired immunological activity. Amino terminus modifications include methylating (i.e., --NHCH₃ or --NH(CH₃)₂, acetylating, adding a carbobenzoyl group, or blocking the amino terminus with any blocking group containing a carboxylate functionality defined by RCOO--, where R is selected from the group consisting of naphthyl, acridinyl, steroidyl, and similar groups. Carboxy terminus modifications include replacing the free acid with a carboxamide group or forming a cyclic lactam at the carboxy terminus to introduce structural constraints.

In one embodiment backbone substitutions can be made, such as NH to NCH₃. The isolated peptide may also be a modification (e.g., a point mutation, such as an insertion or a deletion, or a truncation). By way of example, the peptide may comprise an amino acid sequence comprising a modified residue by at least one point insertion of a D amino acid as long as desired STING activity is retained. In particular, proline analogs in which the ring size of the proline residue is changed from 5 members to 4, 6, or 7 members can be employed. Cyclic groups can be saturated or unsaturated, and if unsaturated, can be aromatic or non-aromatic.

In another preferred embodiment, the naturally occurring side chains of the 20 genetically encoded amino acids (or D amino acids) are replaced with other side chains with similar properties, for instance with groups such as alkyl, lower alkyl, cyclic 4-, 5-, 6-, to 7-membered alkyl amide, amide lower alkyl amide di(lower alkyl), lower alkoxy, hydroxy, carboxy and the lower ester derivatives thereof, and with 4-, 5-, 6-, to 7-membered heterocyclic.

Such substitutions can include but are not necessarily limited to: (1) non-standard positively charged amino acids, like: ornithine, Nlys; N-(4-aminobutyl)-glycine which has the lysine side chain attached to the "N-terminus" and compounds with aminopropyl or aminoethyl groups attached to the amino group of glycine. (2), Non-naturally occurring amino acids with no net charge and side-chains similar to arginine, such as, Cit; citrulline and Hci; citrulline with one more methylene group; (3) non-standard non-naturally occurring amino acids with OH (e.g., like serine), such as, hSer; homoserine (one more methylen group, Hyp; hydroxyproline, Val(βOH); hydroxyvaline, Pen; penicillamin, (Val(βSH); (4) proline derivatives, such as, D-Pro, such as, 3,4-dehydroproline, Pyr; pyroglutamine (proline with C=O in ring), Proline with fluorine substitutions on the ring, 1,3-thiazolidine-+carboxylic acid (proline with S in ring); (5) Histidine derivative, such as, Thi; beta-(2-thienyl)-alanine; or (6) alkyl derivatives, such as, Abu; 2-aminobutyric acid (ethyl group on Cα), Nva; norvaline (propyl group on Cα), Nle; norleucine (butyl group on Cα), Hol; homoleucine (propyl group on Cα), Aib, alpha-aminoisobutyric acid (valine without methylene group). A person skilled in the art would appreciate that those substitutions that retain the activity of the parent peptide/sequence.

In another alternative embodiment, the C-terminal carboxyl group or a C-terminal ester can be induced to cyclize by internal displacement of the --OH or the ester (--OR) of the carboxyl group or ester respectively with the N-terminal amino group to form a cyclic peptide. For example, after synthesis and cleavage to give the peptide acid, the free acid is converted to an activated ester by an appropriate carboxyl group activator such as dicyclohexylcarbodiimide (DCC) in solution, for example, in methylene chloride (CH₂Cl₂), dimethyl formamide (DMF) mixtures. The cyclic peptide is then formed by internal displacement of the activated ester with the N-terminal amine. Internal cyclization as opposed to polymerization can be enhanced by use of very dilute solutions. Such methods are well known in the art.

The peptides of the invention can be cyclized, or a desamino or descarboxy residue at the termini of the peptide can be incorporated, so that there is no terminal amino or carboxyl group, to decrease susceptibility to proteases or to restrict the conformation of the peptide. C-terminal functional groups of the compounds of the present invention include amide, amide lower alkyl, amide di(lower alkyl), lower alkoxy, hydroxy, and carboxy, and the lower ester derivatives thereof, and the pharmaceutically acceptable salts thereof.

The peptides of the invention can be cyclized by adding an N and/or C terminal cysteine and cyclizing the peptide through disulfide linkages or other side chain interactions.

A desamino or descarboxy residue at the termini of the peptide can be incorporated, so that there is no terminal amino or carboxyl group, to decrease susceptibility to proteases or to restrict the conformation of the peptide.

*Chimeric Molecules*: In a preferred embodiment, one or more STING nucleic acids, proteins or peptides can be linked or fused to another moiety. For example, a targeting sequence, such as, for example, an aptamer, antibody sequence; a therapeutic effector molecule, e.g. cytokines, antibiotics, toxins, radiolabels; signal leader peptide; intracellular targeting moiety and the like. The STING molecules can be genetically fused, or linked via linker molecules.

For example, STING fusion molecules can be fused or linked to a therapeutically effective domain which can be a modulatory or cytolytic moiety having a significant serum half-life (t_{1/2}) beyond that of either antibody or modulatory/cytolytic moiety alone.

Accordingly, embodiments of the invention are further directed to chimeric fusion molecules comprising single or multivalent therapeutically active domains which can be modulatory and/or cytolytic; an antigen-binding domain; compositions of single-chain and multivalent modulatory and cytolytic antigen-binding fusion proteins and the like. Other moieties include integrin motifs and NGR motifs.

Furthermore, the chimeric STING molecules to be used in the present invention, as described herein, may comprise at least one further domain, *inter alia*, domains which provide for purification means, like, e.g. histidine stretches. The further domain(s) may be linked by covalent or non-covalent bonds.

The linkage can be based on genetic fusion according to the methods known in the art and described herein or can be performed by, e.g., chemical cross-linking as described in, e.g., WO 94/04686. The additional domain present in the construct may be linked by a flexible linker, such as a polypeptide linker to one of the binding site domains; the polypeptide linker can comprise plural, hydrophilic or peptide-bonded amino acids of a length sufficient to span the distance between the C-terminal end of one of the domains and the N-terminal end of the other of the domains when the polypeptide assumes a conformation suitable for binding when disposed in aqueous solution.

In another preferred embodiment, the molecule comprises a label for detecting the fusion molecule *in vivo* and to monitor the effects of the chimeric molecule during therapy.

"Detectable moiety" or a "label" refers to a composition detectable by spectroscopic, photochemical, biochemical, immunochemical, or chemical means. For example, useful labels include ³²P, ³⁵S, fluorescent dyes, electron-dense reagents, enzymes (e.g., as commonly used in an ELISA), biotin-streptavidin, dioxigenin, haptens and proteins for which antisera or monoclonal antibodies are available, or nucleic acid molecules with a sequence complementary to a target. The detectable moiety often generates a measurable signal, such as a radioactive, chromogenic, or fluorescent signal, that can be used to quantify the amount of bound detectable moiety in a sample. Quantitation of the signal is achieved by, e.g., scintillation counting, densitometry, or flow cytometry.

In other embodiments, the molecules can be in any stereoisomeoric form, for example, enantiomers, diastereomers, tautomers and the like. The fusion molecule or parts thereof includes all variants, mutations, alleles, substitutes, fragments and analogs thereof.

In another preferred embodiment, a moiety which can be used in embodiments of the invention can be determined based on the desired treatment. For example, if the disease is cancer, various anticancer agents can be used. If a disease relates to transplantation or autoimmunity, immune suppressor molecules can be used. If the diseases is an infection, antibiotics, antiviral moieties, anti-inflammatory moieties can be used. The invention contemplates the use of one or more moieties with different properties can be used, for example, an anti-angiogenic moiety and a toxin. The various moieties and combinations are only limited by the imagination of the user. As illustrative examples, the moieties can include one or more molecules' comprising: endostatin, angiogenin, angiostatin, chemokines, angioarrestin, angiostatin (plasminogen fragment), basement-membrane collagen-derived anti-angiogenic factors (tumstatin, canstatin, or arrestin), anti-angiogenic antithrombin III, signal transduction inhibitors, cartilage-derived inhibitor (CDI), CD59 complement fragment, fibronectin fragment, gro-beta, heparinases, heparin hexasaccharide fragment, human chorionic gonadotropin (hCG), interferon alpha/beta/gamma, interferon inducible protein (IP-10), interleukin-12, kringle 5 (plasminogen fragment), metalloproteinase inhibitors (TIMPs), 2-methoxyestradiol, placental ribonuclease inhibitor, plasminogen activator inhibitor, platelet factor-4 (PF4), prolactin 16 kD fragment, proliferin-related protein (PRP), various retinoids, tetrahydrocortisol-S, thrombospondin-1 (TSP-1), transforming growth factor-beta (TGF-β), vasculostatin, vasostatin (calreticulin fragment) and the like. These molecules include all forms, variants, mutations, alleles, substitutes, fragments and analogs thereof.

In another preferred embodiment, the targeting domain comprises antibody, aptamer, a ligand for a receptor (e.g. VEGF), diabodies, peptides, lipopolysaccharides, integrins and the like.

In another preferred embodiment, the chimeric fusion molecules comprise an antigen binding domain specific for other tumor antigens. The antigen binding domain can be an antibody or aptamer, receptor, ligand etc.

In one preferred embodiment, the invention provides for antibody fusion molecules comprising: F_{c} region, C_{H}1, C_{H}2 and/or C_{H}3, Fab, Fab', F(ab')₂, single chain Fv (S_{c}Fv)and Fv fragments, as well as any portion of an antibody having specificity toward a desired target epitope or epitopes. Also preferred are antibodies or antibody fragments or to single chain, two-chain, and multi-chain proteins and glycoproteins belonging to the classes of polyclonal, monoclonal, chimeric, bispecific and hetero immunoglobulins (monoclonal antibodies being preferred); it also includes synthetic and genetically engineered variants of these immunoglobulins.

In another preferred embodiment, the antigen binding domain is an aptamer. In the preferred embodiment, the chimeric molecule comprises an aptamer fused to the STING molecule, variants, mutants and fragments thereof. The aptamer can be specific for any one or more tumor antigens.

As used herein, the term "aptamer" or "selected nucleic acid binding species" shall include non-modified or chemically modified RNA or DNA. The method of selection may be by, but is not limited to, affinity chromatography and the method of amplification by reverse transcription (RT) or polymerase chain reaction (PCR).

Many tumor antigens are well known in the art. See for example, Van den Eynde BJ, van der Bruggen P. Curr Opin Immunol 1997; 9: 684-93; Houghton AN, Gold JS, Blachere NE. Curr Opin Immunol 2001; 13: 134-140; van der Bruggen P, Zhang Y, Chaux P, Stroobant V, Panichelli C, Schultz ES, Chapiro J, Van den Eynde BJ, Brasseur F, Boon T. Immunol Rev 2002; 188: 51-64, which are herein incorporated by reference in their entirety. Alternatively, many antibodies directed towards tumor antigens are commercially available.

In another preferred embodiment, the present invention features a compound having a plurality of binding moieties, wherein at least two binding moieties have specificity for different binding sites on the same target. In preferred embodiments, the plurality of binding moieties includes a polypeptide. In other preferred embodiments, the targeting moieties are all binding polypeptides which bind to different sites on the desired target. In certain preferred embodiments, the target is a protein, a receptor, or a receptor/ligand complex and the binding polypeptides bind to different epitopes on the protein, the receptor, or the receptor/ligand complex.

In another preferred embodiment, the target is a receptor involved in angiogenesis, hyperproliferative disorders or wound healing. In another embodiment the target includes a family of receptors, such as, for example, protein-tyrosine kinase receptors. In one embodiment, the target is Flt-1 and KDR, VEGF (VEGF-1, -2 or -3) or the KDR/VEGF and Flt-1/VEGF complexes, and the binding moieties, particularly binding peptides, bind to different epitopes on Flt-1 and KDR or the KDR/VEGF and Flt-1/VEGF complexes. For example, VEGFR-2/KDR, VEGFR-1/Flt-1 and VEGFR-3/Flt-4.

In connection solid tumor treatment, the present invention may be used in combination with classical approaches, such as surgery, radiotherapy, chemotherapy, and the like. The invention therefore provides combined therapies in which the therapeutic compositions are used simultaneously with, before, or after surgery or radiation treatment; or are administered to patients with, before, or after conventional chemotherapeutic, radiotherapeutic or other anti-angiogenic agents, or targeted immunotoxins or coaguligands.

In another preferred embodiments, the chimeric molecule comprises one or more cytolytic or other effector molecules. Cytolytic molecules that can be used to fuse to an antibody or fragment thereof, include, but are not limited to TNF-α, TNF-β, suitable effector genes such as those that encode a peptide toxin--such as ricin, abrin, diphtheria, gelonin, Pseudomonasexotoxin A, *Crotalus durissus terrificus* toxin, *Crotalus adamenteus* toxin, *Naja naja* toxin, and *Naja mocambique* toxin. (Hughes et al., Hum. Exp. Toxicol. 15:443, 1996; Rosenblum et al., Cancer Immunol. Immunother. 42:115, 1996; Rodriguez et al., Prostate 34:259, 1998; Mauceri et al., Cancer Res. 56:4311; 1996).

Also suitable are genes that induce or mediate apoptosis--such as the ICE-family of cysteine proteases, the Bcl-2 family of proteins, Bax, BclXs and caspases (Favrot et al., Gene Ther. 5:728, 1998; McGill et al., Front. Biosci. 2:D353, 1997; McDonnell et al., Semin. Cancer Biol. 6:53, 1995). Another potential anti-tumor agent is apoptin, a protein that induces apoptosis even where small drug chemotherapeutics fail (Pietersen et al., Adv. Exp. Med. Biol. 465:153, 2000). Koga *et al.* (Hu. Gene Ther. 11: 1397, 2000) propose a telomerase-specific gene therapy using the hTERT gene promoter linked to the apoptosis gene Caspase-8 (FLICE).

Also of interest are enzymes present in the lytic package that cytotoxic T lymphocytes or LAK cells deliver to their targets. Perforin, a pore-forming protein, and Fas ligand are major cytolytic molecules in these cells (Brandau et al., Clin. Cancer Res. 6:3729, 2000; Cruz et al., Br. J. Cancer 81:881, 1999). CTLs also express a family of at least 11 serine proteases termed granzymes, which have four primary substrate specificities (Kam et al., Biochim. Biophys. Acta 1477:307, 2000). Low concentrations of streptolysin 0 and pneumolysin facilitate granzyme B-dependent apoptosis (Browne et al., Mol. Cell Biol. 19:8604, 1999).

Other suitable effectors encode polypeptides having activity that is not itself toxic to a cell, but renders the cell sensitive to an otherwise nontoxic compound--either by metabolically altering the cell, or by changing a non-toxic prodrug into a lethal drug. Exemplary is thymidine kinase (tk), such as may be derived from a herpes simplex virus, and catalytically equivalent variants. The HSV tk converts the anti-herpetic agent ganciclovir (GCV) to a toxic product that interferes with DNA replication in proliferating cells.

If desired, although not required, factors may also be included, such as, but not limited to, chemokines, cytokines, e.g. interleukins, e.g. IL-2, IL-3, IL-6, and IL-11, as well as the other interleukins, the colony stimulating factors, such as GM-CSF, interferons, e.g. γ-interferon, erythropoietin.

In another preferred embodiment, STING chimeric molecules can also comprise an antisense or siRNA domain.

In another preferred embodiment, the STING molecules, chimeric STING molecules, etc, can be radiolabeled. Uses include therapeutic and imaging for diagnostic purposes. The label may be a radioactive atom, an enzyme, or a chromophore moiety. Methods for labeling antibodies have been described, for example, by Hunter and Greenwood, Nature, 144:945 (1962) and by David et al. Biochemistry 13:1014-1021 (1974). Additional methods for labeling antibodies have been described in U.S. Pat. Nos. 3,940,475 and 3,645,090. Methods for labeling oligonucleotide probes have been described, for example, by Leary et al. Proc. Natl. Acad. Sci. USA (1983) 80:4045; Renz and Kurz, Nucl. Acids Res. (1984) 12:3435; Richardson and Gumport, Nucl. Acids Res. (1983) 11:6167; Smith et al. Nucl. Acids Res. (1985) 13:2399; and Meinkoth and Wahl, Anal. Biochem. (1984) 138:267.

The label may be radioactive. Some examples of useful radioactive labels include ³²P, ¹²⁵I, ¹³¹I, and ³H. Use of radioactive labels have been described in U.K. 2,034,323, U.S. Pat. No. 4,358,535, and U.S. Pat. No. 4,302,204.

Some examples of non-radioactive labels include enzymes, chromophores, atoms and molecules detectable by electron microscopy, and metal ions detectable by their magnetic properties.

Some useful enzymatic labels include enzymes that cause a detectable change in a substrate. Some useful enzymes and their substrates include, for example, horseradish peroxidase (pyrogallol and o-phenylenediamine), β-galactosidase (fluorescein β-D-galactopyranoside), and alkaline phosphatase (5-bromo-4-chloro-3-indolyl phosphate/nitro blue tetrazolium). The use of enzymatic labels has been described in U.K. 2,019,404, EP 63,879, and by Rotman, Proc. Natl. Acad. Sci. USA, 47, 1981-1991 (1961).

Useful chromophores include, for example, fluorescent, chemiluminescent, and bioluminescent molecules, as well as dyes. Some specific chromophores useful in the present invention include, for example, fluorescein, rhodamine, Texas red, phycoerythrin, umbelliferone, luminol.

The labels may be conjugated to the antibody or nucleotide probe by methods that are well known in the art. The labels may be directly attached through a functional group on the probe. The probe either contains or can be caused to contain such a functional group. Some examples of suitable functional groups include, for example, amino, carboxyl, sulfhydryl, maleimide, isocyanate, isothiocyanate. Alternatively, labels such as enzymes and chromophores may be conjugated to the antibodies or nucleotides by means of coupling agents, such as dialdehydes, carbodiimides, dimaleimides, and the like.

The label may also be conjugated to the probe by means of a ligand attached to the probe by a method described above and a receptor for that ligand attached to the label. Any of the known ligand-receptor combinations is suitable. Some suitable ligand-receptor pairs include, for example, biotin-avidin or biotin-streptavidin, and antibody-antigen.

In another preferred embodiment, the chimeric fusion molecules of the invention can be used for imaging. In imaging uses, the complexes are labeled so that they can be detected outside the body. Typical labels are radioisotopes, usually ones with short half-lives. The usual imaging radioisotopes, such as ¹²³I, ¹²⁴I, ¹²⁵I, ¹³¹I, ^{99m}TC, ¹⁸⁶Re, ¹⁸⁸Re, ⁶⁴Cu, ⁶⁷Cu, ²¹²Bi, ²¹³Bi, ⁶⁷Ga, ⁹⁰Y, ¹¹¹In, ¹⁸F, ³H, ¹⁴C, ³⁵S or ³²P can be used. Nuclear magnetic resonance (NMR) imaging enhancers, such as gadolinium-153, can also be used to label the complex for detection by NMR. Methods and reagents for performing the labeling, either in the polynucleotide or in the protein moiety, are considered known in the art.

It is also envisaged that the STING molecules disclosed for uses, compositions and methods of the present invention comprises (a) further domain(s) which may also function as immune-modulating molecule. The immune-modulating molecule comprise, but are not limited to cytokines, lymphokines, T cell co-stimulatory ligands, etc. Preferably, the chimeric fusion molecule targets and delivers a modulatory or cytolytic molecule to a target cell, such as for example, a tumor cell, an infected cell; and also recruits immune cells and/or activated immune cells to the target cell.

An immune-modulating effector molecule positively and/or negatively influences the humoral and/or cellular immune system, particularly its cellular and/or non-cellular components, its functions, and/or its interactions with other physiological systems. The immune-modulating effector molecule may be selected from the group comprising cytokines, chemokines, macrophage migration inhibitory factor (MIF; as described, *inter alia*, in Bernhagen (1998), Mol Med 76(3-4); 151-61 or Metz (1997), Adv Immunol 66, 197-223), T-cell receptors and soluble MHC molecules. Such immune-modulating effector molecules are well known in the art and are described, *inter alia*, in Paul, "Fundamental immunology", Raven Press, New York (1989). In particular, known cytokines and chemokines are described in Meager, "The Molecular Biology of Cytokines" (1998), John Wiley & Sons, Ltd., Chichester, West Sussex, England; (Bacon (1998). Cytokine Growth Factor Rev 9(2):167-73; Oppenheim (1997). Clin Cancer Res 12, 2682-6; Taub, (1994) Ther. Immunol. 1(4), 229-46 or Michiel, (1992). Semin Cancer Biol 3(1), 3-15).

Immune cell activity that may be measured include, but is not limited to, (1) cell proliferation by measuring the DNA replication; (2) enhanced cytokine production, including specific measurements for cytokines, such as IFN-γ, GM-CSF, or TNF-α; (3) cell mediated target killing or lysis; (4) cell differentiation; (5) immunoglobulin production; (6) phenotypic changes; (7) production of chemotactic factors or chemotaxis, meaning the ability to respond to a chemotactin with chemotaxis; (8) immunosuppression, by inhibition of the activity of some other immune cell type; and, (9) apoptosis, which refers to fragmentation of activated immune cells under certain circumstances, as an indication of abnormal activation.

### Transgenic Non-Human Animal

In yet another aspect, the invention provides transgenic non-human animal, e.g., a transgenic mouse, which lack STING. Details are provided in the examples which follow.

In a preferred embodiment, a nonhuman transgenic mammal whose genome lacks a functional STING gene as set forth in SEQ ID NOS: 1 or 2, species variants, or mutants thereof.

Transgenic mammals can be prepared for evaluating the molecular mechanisms of STING, and particularly human STING -induced signaling. Such mammals provide excellent models for screening or testing drug candidates. It is possible to evaluate compounds or diseases on "knockout" animals, e.g., to identify a compound that can compensate for a defect in STING activity. Alternatively, human STING, "knock-in" mammals can be prepared for evaluating the molecular biology of this system in greater detail than is possible with human subjects. Both technologies permit manipulation of single units of genetic information in their natural position in a cell genome and to examine the results of that manipulation in the background of a terminally differentiated organism.

A "knockout mammal" is a mammal (e.g., mouse) that contains within its genome a specific gene that has been inactivated by the method of gene targeting (see, e.g., U.S. Pat. Nos. 5,777,195 and 5,616,491). A knockout mammal includes both a heterozygote knockout (i.e., one defective allele and one wild-type allele) and a homozygous mutant. Preparation of a knockout mammal requires first introducing a nucleic acid construct that will be used to suppress expression of a particular gene into an undifferentiated cell type termed an embryonic stem cell. This cell is then injected into a mammalian embryo. A mammalian embryo with an integrated cell is then implanted into a foster mother for the duration of gestation. Zhou, et al. (Genes and Development, 9:2623-34, 1995) describes PPCA knock-out mice. Knockout mice can be used to study viral infections, immune system functions, STING regulated pathways, identification of new therapies, cancer susceptibility, etc. Disease phenotypes that develop can provide a platform for further drug discovery.

The term "knockout" refers to partial or complete suppression of the expression of at least a portion of a protein encoded by an endogenous DNA sequence in a cell. The term "knockout construct" refers to a nucleic acid sequence that is designed to decrease or suppress expression of a protein encoded by endogenous DNA sequences in a cell. The nucleic acid sequence used as the knockout construct is typically comprised of (1) DNA from some portion of the gene (exon sequence, intron sequence, and/or promoter sequence) to be suppressed and (2) a marker sequence used to detect the presence of the knockout construct in the cell. The knockout construct is inserted into a cell, and integrates with the genomic DNA of the cell in such a position so as to prevent or interrupt transcription of the native DNA sequence. Such insertion usually occurs by homologous recombination (i.e., regions of the knockout construct that are homologous to endogenous DNA sequences hybridize to each other when the knockout construct is inserted into the cell and recombine so that the knockout construct is incorporated into the corresponding position of the endogenous DNA). The knockout construct nucleic acid sequence may comprise 1) a full or partial sequence of one or more exons and/or introns of the gene to be suppressed, 2) a full or partial promoter sequence of the gene to be suppressed, or 3) combinations thereof. Typically, the knockout construct is inserted into an embryonic stem cell (ES cell) and is integrated into the ES cell genomic DNA, usually by the process of homologous recombination. This ES cell is then injected into, and integrates with, the developing embryo.

The phrases "disruption of the gene" and "gene disruption" refer to insertion of a nucleic acid sequence into one region of the native DNA sequence (usually one or more exons) and/or the promoter region of a gene so as to decrease or prevent expression of that gene in the cell as compared to the wild-type or naturally occurring sequence of the gene. By way of example, a nucleic acid construct can be prepared containing a DNA sequence encoding an antibiotic resistance gene which is inserted into the DNA sequence that is complementary to the DNA sequence (promoter and/or coding region) to be disrupted. When this nucleic acid construct is then transfected into a cell, the construct will integrate into the genomic DNA. Thus, many progeny of the cell will no longer express the gene at least in some cells, or will express it at a decreased level, as the DNA is now disrupted by the antibiotic resistance gene.

A "knock-in" mammal is a mammal in which an endogenous gene is substituted with a heterologous gene (Roemer et al., New Biol. 3:331, 1991). Preferably, the heterologous gene is "knocked-in" to a locus of interest, either the subject of evaluation (in which case the gene may be a reporter gene; see Elefanty et al., Proc Natl Acad Sci USA 95:11897, 1998) of expression or function of a homologous gene, thereby linking the heterologous gene expression to transcription from the appropriate promoter. This can be achieved by homologous recombination, transposon (Westphal and Leder, Curr Biol 7:530, 1997), using mutant recombination sites (Araki et al., Nucleic Acicls Res 25:868, 1997) or PCR (Zhang and Henderson, Biotechniques 25:784, 1998).

Generally, for homologous recombination, the DNA will be at least about 1 kilobase (kb) in length and preferably 3-4 kb in length, thereby providing sufficient complementary sequence for recombination when the knockout construct is introduced into the genomic DNA of the ES cell.

Included within the scope of this invention is a mammal in which two or more genes have been knocked out or knocked in, or both. Such mammals can be generated by repeating the procedures set forth herein for generating each knockout construct, or by breeding to mammals, each with a single gene knocked out, to each other, and screening for those with the double knockout genotype.

Regulated knockout animals can be prepared using various systems, such as the tet-repressor system (see U.S. Pat. No. 5,654,168) or the Cre-Lox system (see U.S. Pat. Nos. 4,959,317 and 5,801,030).

In another series of embodiments, transgenic animals are created in which (i) a human STING is stably inserted into the genome of the transgenic animal; and/or (ii) the endogenous STING genes are inactivated and replaced with their human counterparts. See, e.g., Coffman, Semin. Nephrol. 17:404, 1997; Esther et al., Lab. Invest. 74:953, 1996; Murakami et al., Blood Press. Suppl. 2:36, 1996. Such animals can be treated with candidate compounds and monitored for the effects of such drugs on STING activity.

In another preferred embodiment, cells from the transgenic animals both knock-in, knock-out and xenogeneic STING molecules, can be used in different assays, the cells can be transformed, immortalized etc.

### Antisense STING Oligonucleoticles

In another preferred embodiment, the functions and/or expression of STING in a cell or patient are modulated by targeting STING molecules. Examples of STING antisense, or siRNA oligonucleotides comprise SEQ ID NOS: 3 to 5.

In a preferred embodiment, an oligonucleotide comprises at least five consecutive bases complementary to a nucleic acid sequence set forth as SEQ ID NOS: 1 or 2, wherein the oligonucleotide specifically hybridizes to and modulates expression and/or function of STING *in vivo* or *in vitro.* In another preferred embodiment, the oligomeric compounds of the present invention also include variants in which a different base is present at one or more of the nucleotide positions in the compound. For example, if the first nucleotide is an adenosine, variants may be produced which contain thymidine, guanosine or cytidine at this position. This may be done at any of the positions of the oligonucleotide. These compounds are then tested using the methods described herein to determine their ability to inhibit expression of a target nucleic acid.

In some embodiments, homology, sequence identity or complementarity, between the oligonucleotide and target is from about 50% to about 60%. In some embodiments, homology, sequence identity or complementarity, is from about 60% to about 70%. In some embodiments, homology, sequence identity or complementarity, is from about 70% to about 80%. In some embodiments, homology, sequence identity or complementarity, is from about 80% to about 90%. In some embodiments, homology, sequence identity or complementarity, is about 90%, about 92%, about 94%, about 95%, about 96%, about 97%, about 98%, about 99% or about 100%.

In another preferred embodiment, an oligonucleotide comprises combinations of phosphorothioate internucleotide linkages and at least one internucleotide linkage selected from the group consisting of: alkylphosphonate, phosphorodithioate, alkylphosphonothioate, phosphoramidate, carbamate, carbonate, phosphate triester, acetamidate, carboxymethyl ester, and/or combinations thereof.

In another preferred embodiment, an oligonucleotide optionally comprises at least one modified nucleobase comprising, peptide nucleic acids, locked nucleic acid (LNA) molecules, analogues, derivatives and/or combinations thereof.

An oligonucleotide is specifically hybridizable when binding of the compound to the target nucleic acid interferes with the normal function of the target nucleic acid to cause a loss of activity, and there is a sufficient degree of complementarity to avoid non-specific binding of the oligonucleotide to non-target nucleic acid sequences under conditions in which specific binding is desired. Such conditions include, i.e., physiological conditions in the case of *in vivo* assays or therapeutic treatment, and conditions in which assays are performed in the case of *in vitro* assays.

An oligonucleotide, whether DNA, RNA, chimeric, substituted etc, is specifically hybridizable when binding of the compound to the target DNA or RNA molecule interferes with the normal function of the target DNA or RNA to cause a loss of utility, and there is a sufficient degree of complementarily to avoid non-specific binding of the oligonucleotide to non-target sequences under conditions in which specific binding is desired, i.e., under physiological conditions in the case of *in vivo* assays or therapeutic treatment, and in the case of *in vitro* assays, under conditions in which the assays are performed.

The specificity and sensitivity of antisense is also harnessed by those of skill in the art for therapeutic uses. Antisense oligonucleotides have been employed as therapeutic moieties in the treatment of disease states in animals and man. Antisense oligonucleotides have been safely and effectively administered to humans and numerous clinical trials are presently underway. It is thus established that oligonucleotides can be useful therapeutic modalities that can be configured to be useful in treatment regimes for treatment of cells, tissues and animals, especially humans.

In embodiments of the present invention oligomeric oligonucleotides, particularly oligonucleotides, bind to target nucleic acid molecules and modulate the expression and/or function of molecules encoded by a target gene. The functions of DNA to be interfered comprise, for example, replication and transcription. The functions of RNA to be interfered comprise all vital functions such as, for example, translocation of the RNA to the site of protein translation, translation of protein from the RNA, splicing of the RNA to yield one or more mRNA species, and catalytic activity which may be engaged in or facilitated by the RNA. The functions may be up-regulated or inhibited depending on the functions desired.

The oligonucleotides, include, antisense oligomeric compounds, antisense oligonucleotides, external guide sequence (EGS) oligonucleotides, alternate splicers, primers, probes, and other oligomeric compounds that hybridize to at least a portion of the target nucleic acid. As such, these compounds may be introduced in the form of single-stranded, double-stranded, partially single-stranded, or circular oligomeric compounds.

Targeting an oligonucleotide to a particular nucleic acid molecule (e.g. SEQ ID NOS: 1 or 2), in the context of this invention, can be a multistep process. The process usually begins with the identification of a target nucleic acid whose function is to be modulated. This target nucleic acid may be, for example, a cellular gene (or mRNA transcribed from the gene) whose expression is associated with a particular disorder or disease state, or a nucleic acid molecule from an infectious agent.

The targeting process usually also includes determination of at least one target region, segment, or site within the target nucleic acid for the antisense interaction to occur such that the desired effect, e.g., modulation of expression, will result. Within the context of the present invention, the term "region" is defined as a portion of the target nucleic acid having at least one identifiable structure, function, or characteristic. Within regions of target nucleic acids are segments. "Segments" are defined as smaller or sub-portions of regions within a target nucleic acid. "Sites," as used in the present invention, are defined as positions within a target nucleic acid.

Since, as is known in the art, the translation initiation codon is typically 5'-AUG (in transcribed mRNA molecules; 5'-ATG in the corresponding DNA molecule), the translation initiation codon is also referred to as the "AUG codon," the "start codon" or the "AUG start codon". A minority of genes has a translation initiation codon having the RNA sequence 5'-GUG, 5'-UUG or 5'-CUG; and 5'-AUA, 5'-ACG and 5'-CUG have been shown to function *in vivo.* Thus, the terms "translation initiation codon" and "start codon" can encompass many codon sequences, even though the initiator amino acid in each instance is typically methionine (in eukaryotes) or formylmethionine (in prokaryotes). Eukaryotic and prokaryotic genes may have two or more alternative start codons, any one of which may be preferentially utilized for translation initiation in a particular cell type or tissue, or under a particular set of conditions. In the context of the invention, "start codon" and "translation initiation codon" refer to the codon or codons that are used *in vivo* to initiate translation of an mRNA transcribed from a gene encoding STING, regardless of the sequence(s) of such codons. A translation termination codon (or "stop codon") of a gene may have one of three sequences, i.e., 5'-UAA, 5'-UAG and 5'-UGA (the corresponding DNA sequences are 5'-TAA, 5'-TAG and 5'-TGA, respectively).

The terms "start codon region" and "translation initiation codon region" refer to a portion of such an mRNA or gene that encompasses from about 25 to about 50 contiguous nucleotides in either direction (i.e., 5' or 3') from a translation initiation codon. Similarly, the terms "stop codon region" and "translation termination codon region" refer to a portion of such an mRNA or gene that encompasses from about 25 to about 50 contiguous nucleotides in either direction (i.e., 5' or 3') from a translation termination codon. Consequently, the "start codon region" (or "translation initiation codon region") and the "stop codon region" (or "translation termination codon region") are all regions that may be targeted effectively with the antisense compounds of the present invention.

The open reading frame (ORF) or "coding region," which is known in the art to refer to the region between the translation initiation codon and the translation termination codon, is also a region which may be targeted effectively. Within the context of the present invention, a targeted region is the intragenic region encompassing the translation initiation or termination codon of the open reading frame (ORF) of a gene.

Another target region includes the 5' untranslated region (5'UTR), known in the art to refer to the portion of an mRNA in the 5' direction from the translation initiation codon, and thus including nucleotides between the 5' cap site and the translation initiation codon of an mRNA (or corresponding nucleotides on the gene). Still another target region includes the 3' untranslated region (3'UTR), known in the art to refer to the portion of an mRNA in the 3' direction from the translation termination codon, and thus including nucleotides between the translation termination codon and 3' end of an mRNA (or corresponding nucleotides on the gene). The 5' cap site of an mRNA comprises an N7-methylated guanosine residue joined to the 5'-most residue of the mRNA via a 5'-5' triphosphate linkage. The 5' cap region of an mRNA is considered to include the 5' cap structure itself as well as the first 50 nucleotides adjacent to the cap site. Another target region for this invention is the 5' cap region.

Although some eukaryotic mRNA transcripts are directly translated, many contain one or more regions, known as "introns," which are excised from a transcript before it is translated. The remaining (and therefore translated) regions are known as "exons" and are spliced together to form a continuous mRNA sequence. In one embodiment, targeting splice sites, i.e., intron-exon junctions or exon-intron junctions, is particularly useful in situations where aberrant splicing is implicated in disease, or where an overproduction of a particular splice product is implicated in disease. An aberrant fusion junction due to rearrangement or deletion is another embodiment of a target site. mRNA transcripts produced via the process of splicing of two (or more) mRNAs from different gene sources are known as "fusion transcripts". Introns can be effectively targeted using antisense compounds targeted to, for example, DNA or pre-mRNA.

In another preferred embodiment, the antisense oligonucleotides bind to coding and/or non-coding regions of a target polynucleotide and modulate the expression and/or function of the target molecule.

In another preferred embodiment, the antisense oligonucleotides bind to natural antisense polynucleotides and modulate the expression and/or function of the target molecule.

In another preferred embodiment, the antisense oligonucleotides bind to sense polynucleotides and modulate the expression and/or function of the target molecule.

Alternative RNA transcripts can be produced from the same genomic region of DNA. These alternative transcripts or "pre-mRNA variants" are transcripts produced from the same genomic DNA that differ from other transcripts produced from the same genomic DNA in either their start or stop position and contain both intronic and exonic sequence.

Upon excision of one or more exon or intron regions, or portions thereof during splicing, pre-mRNA variants produce smaller "mRNA variants". Consequently, mRNA variants are processed pre-mRNA variants and each unique pre-mRNA variant must always produce a unique mRNA variant as a result of splicing. These mRNA variants are also known as "alternative splice variants". If no splicing of the pre-mRNA variant occurs then the pre-mRNA variant is identical to the mRNA variant.

Variants can be produced through the use of alternative signals to start or stop transcription. Pre-mRNAs and mRNAs can possess more that one start codon or stop codon. Variants that originate from a pre-mRNA or mRNA that use alternative start codons are known as "alternative start variants" of that pre-mRNA or mRNA. Those transcripts that use an alternative stop codon are known as "alternative stop variants" of that pre-mRNA or mRNA. One specific type of alternative stop variant is the "polyA variant" in which the multiple transcripts produced result from the alternative selection of one of the "polyA stop signals" by the transcription machinery, thereby producing transcripts that terminate at unique polyA sites. Within the context of the invention, the types of variants described herein are also embodiments of target nucleic acids.

The locations on the target nucleic acid to which the antisense compounds hybridize are defined as at least a 5-nucleobase portion of a target region to which an active antisense compound is targeted.

While the specific sequences of certain exemplary target segments are set forth herein, one of skill in the art will recognize that these serve to illustrate and describe particular embodiments within the scope of the present invention. Additional target segments are readily identifiable by one having ordinary skill in the art in view of this disclosure.

Target segments 5-100 nucleobases in length comprising a stretch of at least five (5) consecutive nucleobases selected from within the illustrative preferred target segments are considered to be suitable for targeting as well.

Target segments can include DNA or RNA sequences that comprise at least the 5 consecutive nucleobases from the 5'-terminus of one of the illustrative preferred target segments (the remaining nucleobases being a consecutive stretch of the same DNA or RNA beginning immediately upstream of the 5'-terminus of the target segment and continuing until the DNA or RNA contains about 5 to about 100 nucleobases). Similarly preferred target segments are represented by DNA or RNA sequences that comprise at least the 5 consecutive nucleobases from the 3'-terminus of one of the illustrative preferred target segments (the remaining nucleobases being a consecutive stretch of the same DNA or RNA beginning immediately downstream of the 3'-terminus of the target segment and continuing until the DNA or RNA contains about 5 to about 100 nucleobases). One having skill in the art armed with the target segments illustrated herein will be able, without undue experimentation, to identify further preferred target segments.

Once one or more target regions, segments or sites have been identified, antisense compounds are chosen which are sufficiently complementary to the target, i.e., hybridize sufficiently well and with sufficient specificity, to give the desired effect.

In embodiments of the invention the oligonucleotides bind to an antisense strand of a particular target. The oligonucleotides are at least 5 nucleotides in length and can be synthesized so each oligonucleotide targets overlapping sequences such that oligonucleotides are synthesized to cover the entire length of the target polynucleotide. The targets also include coding as well as non coding regions.

According to the present invention, antisense compounds include antisense oligonucleotides, ribozymes, external guide sequence (EGS) oligonucleotides, siRNA compounds, single- or double-stranded RNA interference (RNAi) compounds such as siRNA compounds, and other oligomeric compounds which hybridize to at least a portion of the target nucleic acid and modulate its function. As such, they may be DNA, RNA, DNA-like, RNA-like, or mixtures thereof, or may be mimetics of one or more of these. These compounds may be single-stranded, double-stranded, circular or hairpin oligomeric compounds and may contain structural elements such as internal or terminal bulges, mismatches or loops. Antisense compounds are routinely prepared linearly but can be joined or otherwise prepared to be circular and/or branched. Antisense compounds can include constructs such as, for example, two strands hybridized to form a wholly or partially double-stranded compound or a single strand with sufficient self-complementarity to allow for hybridization and formation of a fully or partially double-stranded compound. The two strands can be linked internally leaving free 3' or 5' termini or can be linked to form a continuous hairpin structure or loop. The hairpin structure may contain an overhang on either the 5' or 3' terminus producing an extension of single stranded character. The double stranded compounds optionally can include overhangs on the ends. Further modifications can include conjugate groups attached to one of the termini, selected nucleobase positions, sugar positions or to one of the internucleoside linkages. Alternatively, the two strands can be linked via a non-nucleic acid moiety or linker group. When formed from only one strand, dsRNA can take the form of a self-complementary hairpin-type molecule that doubles back on itself to form a duplex. Thus, the dsRNAs can be fully or partially double stranded. Specific modulation of gene expression can be achieved by stable expression of dsRNA hairpins in transgenic cell lines, however, in some embodiments, the gene expression or function is up regulated. When formed from two strands, or a single strand that takes the form of a self-complementary hairpin-type molecule doubled back on itself to form a duplex, the two strands (or duplex-forming regions of a single strand) are complementary RNA strands that base pair in Watson-Crick fashion.

Once introduced to a system, the compounds of the invention may elicit the action of one or more enzymes or structural proteins to effect cleavage or other modification of the target nucleic acid or may work via occupancy-based mechanisms. In general, nucleic acids (including oligonucleotides) may be described as "DNA-like" (i.e., generally having one or more 2'-deoxy sugars and, generally, T rather than U bases) or "RNA-like" (i.e., generally having one or more 2'-hydroxyl or 2'-modified sugars and, generally U rather than T bases). Nucleic acid helices can adopt more than one type of structure, most commonly the A- and B-forms. It is believed that, in general, oligonucleotides which have B-form-like structure are "DNA-like" and those which have A-form-like structure are "RNA-like." In some (chimeric) embodiments, an antisense compound may contain both A- and B-form regions.

In another preferred embodiment, the desired oligonucleotides or antisense compounds, comprise at least one of: antisense RNA, antisense DNA, chimeric antisense oligonucleotides, antisense oligonucleotides comprising modified linkages, interference RNA (RNAi), short interfering RNA (siRNA); a micro, interfering RNA (miRNA); a small, temporal RNA (stRNA); or a short, hairpin RNA (shRNA); small RNA-induced gene activation (RNAa); small activating RNAs (saRNAs), or combinations thereof.

dsRNA can also activate gene expression, a mechanism that has been termed "small RNA-induced gene activation" or RNAa. dsRNAs targeting gene promoters induce potent transcriptional activation of associated genes. RNAa was demonstrated in human cells using synthetic dsRNAs, termed "small activating RNAs" (saRNAs). It is currently not known whether RNAa is conserved in other organisms.

Small double-stranded RNA (dsRNA), such as small interfering RNA (siRNA) and microRNA (miRNA), have been found to be the trigger of an evolutionary conserved mechanism known as RNA interference (RNAi). RNAi invariably leads to gene silencing via remodeling chromatin to thereby suppress transcription, degrading complementary mRNA, or blocking protein translation. dsRNAs may also act as small activating RNAs (saRNA). Without wishing to be bound by theory, by targeting sequences in gene promoters, saRNAs would induce target gene expression in a phenomenon referred to as dsRNA-induced transcriptional activation (RNAa).

RNA interference (RNAi) has become a powerful tool for blocking gene expression in mammals and mammalian cells. This approach requires the delivery of small interfering RNA (siRNA) either as RNA itself or as DNA, using an expression plasmid or virus and the coding sequence for small hairpin RNAs that are processed to siRNAs. This system enables efficient transport of the pre-siRNAs to the cytoplasm where they are active and permit the use of regulated and tissue specific promoters for gene expression.

In a preferred embodiment, an oligonucleotide or antisense compound comprises an oligomer or polymer of ribonucleic acid (RNA) and/or deoxyribonucleic acid (DNA), or a mimetic, chimera, analog or homolog thereof. This term includes oligonucleotides composed of naturally occurring nucleobases, sugars and covalent internucleoside (backbone) linkages as well as oligonucleotides having non-naturally occurring portions which function similarly. Such modified or substituted oligonucleotides are often desired over native forms because of desirable properties such as, for example, enhanced cellular uptake, enhanced affinity for a target nucleic acid and increased stability in the presence of nucleases.

According to the present invention, the oligonucleotides or "antisense compounds" include antisense oligonucleotides (e.g. RNA, DNA, mimetic, chimera, analog or homolog thereof), ribozymes, external guide sequence (EGS) oligonucleotides, siRNA compounds, single- or double-stranded RNA interference (RNAi) compounds such as siRNA compounds, saRNA, aRNA, and other oligomeric compounds which hybridize to at least a portion of the target nucleic acid and modulate its function. As such, they may be DNA, RNA, DNA-like, RNA-like, or mixtures thereof, or may be mimetics of one or more of these. These compounds may be single-stranded, double-stranded, circular or hairpin oligomeric compounds and may contain structural elements such as internal or terminal bulges, mismatches or loops. Antisense compounds are routinely prepared linearly but can be joined or otherwise prepared to be circular and/or branched. Antisense compounds can include constructs such as, for example, two strands hybridized to form a wholly or partially double-stranded compound or a single strand with sufficient self-complementarity to allow for hybridization and formation of a fully or partially double-stranded compound. The two strands can be linked internally leaving free 3' or 5' termini or can be linked to form a continuous hairpin structure or loop. The hairpin structure may contain an overhang on either the 5' or 3' terminus producing an extension of single stranded character. The double stranded compounds optionally can include overhangs on the ends. Further modifications can include conjugate groups attached to one of the termini, selected nucleobase positions, sugar positions or to one of the internucleoside linkages. Alternatively, the two strands can be linked via a non-nucleic acid moiety or linker group. When formed from only one strand, dsRNA can take the form of a self-complementary hairpin-type molecule that doubles back on itself to form a duplex. Thus, the dsRNAs can be fully or partially double stranded. Specific modulation of gene expression can be achieved by stable expression of dsRNA hairpins in transgenic cell lines (Hammond et al., Nat. Rev. Genet., 1991, 2, 110-119; Matzke et al., Curr. Opin. Genet. Dev., 2001, 11, 221-227; Sharp, Genes Dev., 2001, 15, 485-490). When formed from two strands, or a single strand that takes the form of a self-complementary hairpin-type molecule doubled back on itself to form a duplex, the two strands (or duplex-forming regions of a single strand) are complementary RNA strands that base pair in Watson-Crick fashion.

The antisense compounds in accordance with this invention can comprise an antisense portion from about 5 to about 80 nucleobases (i.e. from about 5 to about 80 linked nucleosides) in length. This refers to the length of the antisense strand or portion of the antisense compound. In other words, a single-stranded antisense compound of the invention comprises from 5 to about 80 nucleobases, and a double-stranded antisense compound of the invention (such as a dsRNA, for example) comprises an antisense strand or portion of 5 to about 80 nucleobases in length. One of ordinary skill in the art will appreciate that this comprehends antisense portions of 5, 6, 7,8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, or 80 nucleobases in length, or any range therewithin.

In one embodiment, the antisense compounds of the invention have antisense portions of 10 to 50 nucleobases in length. One having ordinary skill in the art will appreciate that this embodies oligonucleotides having antisense portions of 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 nucleobases in length, or any range therewithin. In some embodiments, the oligonucleotides are 15 nucleobases in length.

In one embodiment, the antisense or oligonucleotide compounds of the invention have antisense portions of 12 or 13 to 30 nucleobases in length. One having ordinary skill in the art will appreciate that this embodies antisense compounds having antisense portions of 12, 13, 14, 15, 16, 17, 18, 19, 20, 21, 22, 23, 24, 25, 26, 27, 28, 29 or 30 nucleobases in length, or any range therewithin.

Certain preferred oligonucleotides of this invention are chimeric oligonucleotides. "Chimeric oligonucleotides" or "chimeras," in the context of this invention, are oligonucleotides which contain two or more chemically distinct regions, each made up of at least one nucleotide. These oligonucleotides typically contain at least one region of modified nucleotides that confers one or more beneficial properties (such as, for example, increased nuclease resistance, increased uptake into cells, increased binding affinity for the target) and a region that is a substrate for enzymes capable of cleaving RNA:DNA or RNA:RNA hybrids. By way of example, RNase H is a cellular endonuclease which cleaves the RNA strand of an RNA:DNA duplex. Activation of RNase H, therefore, results in cleavage of the RNA target, thereby greatly enhancing the efficiency of antisense modulation of gene expression. Consequently, comparable results can often be obtained with shorter oligonucleotides when chimeric oligonucleotides are used, compared to phosphorothioate deoxyoligonucleotides hybridizing to the same target region. Cleavage of the RNA target can be routinely detected by gel electrophoresis and, if necessary, associated nucleic acid hybridization techniques known in the art. In one preferred embodiment, a chimeric oligonucleotide comprises at least one region modified to increase target binding affinity, and, usually, a region that acts as a substrate for RNAse H. Affinity of an oligonucleotide for its target (in this case, a nucleic acid encoding ras) is routinely determined by measuring the Tₘ of an oligonucleotide/target pair, which is the temperature at which the oligonucleotide and target dissociate; dissociation is detected spectrophotometrically. The higher the Tₘ, the greater the affinity of the oligonucleotide for the target.

Chimeric antisense compounds of the invention may be formed as composite structures of two or more oligonucleotides, modified oligonucleotides, oligonucleosides and/or oligonucleotide mimetics as described above. Such; compounds have also been referred to in the art as hybrids or gapmers. Representative United States patents that teach the preparation of such hybrid structures comprise, but are not limited to, US patent nos. 5,013,830; 5,149,797; 5, 220,007; 5,256,775; 5,366,878; 5,403,711; 5,491,133; 5,565,350; 5,623,065; 5,652,355; 5,652,356; and 5,700,922, each of which is herein incorporated by reference.

In another preferred embodiment, the region of the oligonucleotide which is modified comprises at least one nucleotide modified at the 2' position of the sugar, most preferably a 2'-O-alkyl, 2'-O-alkyl-O-alkyl or 2'-fluoro-modified nucleotide. In other preferred embodiments, RNA modifications include 2'-fluoro, 2'-amino and 2' O-methyl modifications on the ribose of pyrimidines, abasic residues or an inverted base at the 3' end of the RNA. Such modifications are routinely incorporated into oligonucleotides and these oligonucleotides have been shown to have a higher Tₘ (i.e., higher target binding affinity) than; 2'-deoxyoligonucleotides against a given target. The effect of such increased affinity is to greatly enhance RNAi oligonucleotide inhibition of gene expression. RNAse H is a cellular endonuclease that cleaves the RNA strand of RNA:DNA duplexes; activation of this enzyme therefore results in cleavage of the RNA target, and thus can greatly enhance the efficiency of RNAi inhibition. Cleavage of the RNA target can be routinely demonstrated by gel electrophoresis. In another preferred embodiment, the chimeric oligonucleotide is also modified to enhance nuclease resistance. Cells contain a variety of exo- and endo-nucleases which can degrade nucleic acids. A number of nucleotide and nucleoside modifications have been shown to make the oligonucleotide into which they are incorporated more resistant to nuclease digestion than the native oligodeoxynucleotide. Nuclease resistance is routinely measured by incubating oligonucleotides with cellular extracts or isolated nuclease solutions and measuring the extent of intact oligonucleotide remaining over time, usually by gel electrophoresis. Oligonucleotides which have been modified to enhance their nuclease resistance survive intact for a longer time than unmodified oligonucleotides. A variety of oligonucleotide modifications have been demonstrated to enhance or confer nuclease resistance. Oligonucleotides which contain at least one phosphorothioate modification are presently more preferred. In some cases, oligonucleotide modifications which enhance target binding affinity are also, independently, able to enhance nuclease resistance. Some desirable modifications can be found in De Mesmaeker et al. Acc. Chem. Res. 1995, 28:366-374.

Specific examples of some preferred oligonucleotides envisioned for this invention include those comprising modified backbones, for example, phosphorothioates, phosphotriesters, methyl phosphonates, short chain alkyl or cycloalkyl intersugar linkages or short chain heteroatomic or heterocyclic intersugar linkages. Most preferred are oligonucleotides with phosphorothioate backbones and those with heteroatom backbones, particularly CH₂ --NH--O--CH₂, CH,--N(CH₃)--O--CH₂ [known as a methylene(methylimino) or MMI backbone], CH₂ --O--N (CH₃)--CH₂, CH₂ --N (CH₃)--N (CH₃)--CH₂ and O--N (CH₃)--CH₂ --CH₂ backbones, wherein the native phosphodiester backbone is represented as O--P--O--CH,). The amide backbones disclosed by De Mesmaeker et al. Acc. Chem. Res. 1995, 28:366-374) are also preferred. Also preferred are oligonucleotides having morpholino backbone structures (Summerton and Weller, U.S. Pat. No. 5,034,506). In other preferred embodiments, such as the peptide nucleic acid (PNA) backbone, the phosphodiester backbone of the oligonucleotide is replaced with a polyamide backbone, the nucleobases being bound directly or indirectly to the aza nitrogen atoms of the polyamide backbone (Nielsen et al. Science 1991, 254, 1497). Oligonucleotides may also comprise one or more substituted sugar moieties. Preferred oligonucleotides comprise one of the following at the 2' position: OH, SH, SCH₃, F, OCN, OCH₃ OCH₃, OCH₃ O(CH₂)ₙ CH₃, O(CH₂)ₙ NH₂ or O(CH₂)ₙ CH₃ where n is from 1 to about 10; C₁ to C₁₀ lower alkyl, alkoxyalkoxy, substituted lower alkyl, alkaryl or aralkyl; Cl; Br; CN; CF₃ ; OCF₃; O--, S--, or N-alkyl; O--, S--, or N-alkenyl; SOCH₃; SO₂ CH₃; ONO₂; NO₂; N₃; NH₂; heterocycloalkyl; heterocycloalkaryl; aminoalkylamino; polyalkylamino; substituted silyl; an RNA cleaving group; a reporter group; an intercalator; a group for improving the pharmacokinetic properties of an oligonucleotide; or a group for improving the pharmacodynamic properties of an oligonucleotide and other substituents having similar properties. A preferred modification includes 2'-methoxyethoxy [2'-O-CH₂ CH₂ OCH₃, also known as 2'-O-(2-methoxyethyl)] (Martin et al., Helv. Chim. Acta, 1995, 78, 486). Other preferred modifications include 2'-methoxy (2'-O--CH₃), 2'-propoxy (2'-OCH₂ CH₂CH₃) and 2'-fluoro (2'-F). Similar modifications may also be made at other positions on the oligonucleotide, particularly the 3' position of the sugar on the 3' terminal nucleotide and the 5' position of 5' terminal nucleotide. Oligonucleotides may also have sugar mimetics such as cyclobutyls in place of the pentofuranosyl group.

Oligonucleotides may also include, additionally or alternatively, nucleobase (often referred to in the art simply as "base") modifications or substitutions. As used herein, "unmodified" or "natural" nucleobases include adenine (A), guanine (G), thymine (T), cytosine (C) and uracil (U). Modified nucleobases include nucleobases found only infrequently or transiently in natural nucleic acids, e.g., hypoxanthine, 6-methyladenine, 5-Me pyrimidines, particularly 5-methylcytosine (also referred to as 5-methyl-2' deoxycytosine and often referred to in the art as 5-Me-C), 5-hydroxymethylcytosine (HMC), glycosyl HMC and gentobiosyl HMC, as well as synthetic nucleobases, e.g., 2-aminoadenine, 2-(methylamino)adenine, 2-(imidazolylalkyl)adenine, 2-(aminoalklyamino)adenine or other heterosubstituted alkyladenines, 2-thiouracil, 2-thiothymine, 5-bromouracil, 5-hydroxymethyluracil, 8-azaguanine, 7-deazaguanine, N₆ (6-aminohexyl)adenine and 2,6-diaminopurine. Kornberg, A., DNA Replication, W. H. Freeman & Co., San Francisco, 1980, pp75-77; Gebeyehu, G., et al. Nucl. Acids Res. 1987, 15:4513). A "universal" base known in the art, e.g., inosine, may be included. 5-Me-C substitutions have been shown to increase nucleic acid duplex stability by 0.6-1.2°C. (Sanghvi, Y. S., in Crooke, S. T. and Lebleu, B., eds., Antisense Research and Applications, CRC Press, Boca Raton, 1993, pp. 276-278) and are presently preferred base substitutions.

Another modification of the oligonucleotides of the invention involves chemically linking to the oligonucleotide one or more moieties or conjugates which enhance the activity or cellular uptake of the oligonucleotide. Such moieties include but are not limited to lipid moieties such as a cholesterol moiety, a cholesteryl moiety (Letsinger et al., Proc. Natl. Acad. Sci. USA 1989, 86, 6553), cholic acid (Manoharan et al. Bioorg. Med. Chem. Let. 1994, 4, 1053), a thioether, e.g., hexyl-S-tritylthiol (Manoharan et al. Ann. N.Y. Acad. Sci. 1992, 660, 306; Manoharan et al. Bioorg. Med. Chem. Let. 1993, 3, 2765), a thiocholesterol (Oberhauser et al., Nucl. Acids Res. 1992, 20, 533), an aliphatic chain, e.g., dodecandiol or undecyl residues (Saison-Behmoaras et al. EMBO J. 1991, 10, 111; Kabanov et al. FEBS Lett. 1990, 259, 327; Svinarchuk et al. Biochimie 1993, 75, 49), a phospholipid, e.g., di-hexadecyl-rac-glycerol or triethylammonium 1,2-di-O-hexadecyl-rac-glycero-3-H-phosphonate (Manoharan et al. Tetrahedron Lett. 1995, 36, 3651; Shea et al. Nucl. Acids Res. 1990, 18, 3777), a polyamine or a polyethylene glycol chain (Manoharan et al. Nucleosicles & Nucleotides 1995, 14, 969), or adamantane acetic acid (Manoharan et al. Tetrahedron Lett. 1995, 36, 3651). Oligonucleotides comprising lipophilic moieties, and methods for preparing such oligonucleotides are known in the art, for example, U.S. Pat. Nos. 5,138,045, 5,218,105 and 5,459,255.

It is not necessary for all positions in a given oligonucleotide to be uniformly modified, and in fact more than one of the aforementioned modifications may be incorporated in a single oligonucleotide or even at within a single nucleoside within an oligonucleotide. The present invention also includes oligonucleotides which are chimeric oligonucleotides as hereinbefore defined.

In another embodiment, the nucleic acid molecule of the present invention is conjugated with another moiety including but not limited to abasic nucleotides, polyether, polyamine, polyamides, peptides, carbohydrates, lipid, or polyhydrocarbon compounds. Those skilled in the art will recognize that these molecules can be linked to one or more of any nucleotides comprising the nucleic acid molecule at several positions on the sugar, base or phosphate group.

In accordance with the invention, use of modifications such as the use of LNA monomers to enhance the potency, specificity and duration of action and broaden the routes of administration of oligonucleotides comprised of current chemistries such as MOE, ANA, FANA, PS etc (ref: Recent advances in the medical chemistry of antisense oligonucleotide by Uhlman, Current Opinions in Drug Discovery & Development 2000 Vol 3 No 2). This can be achieved by substituting some of the monomers in the current oligonucleotides by LNA monomers. The LNA modified oligonucleotide may have a size similar to the parent compound or may be larger or preferably smaller. It is preferred that such LNA-modified oligonucleotides contain less than about 70%, more preferably less than about 60%, most preferably less than about 50% LNA monomers and that their sizes are between about 5 and 25 nucleotides, more preferably between about 12 and 20 nucleotides.

Preferred modified oligonucleotide backbones comprise, but not limited to, phosphorothioates, chiral phosphorothioates, phosphorodithioates, phosphotriesters, aminoalkylphosphotriesters, methyl and other alkyl phosphonates comprising 3'alkylene phosphonates and chiral phosphonates, phosphinates, phosphoramidates comprising 3'-amino phosphoramidate and aminoalkylphosphoramidates, thionophosphoramidates, thionoalkylphosphonates, thionoalkylphosphotriesters, and boranophosphates having normal 3'-5' linkages, 2'-5' linked analogs of these, and those having inverted polarity wherein the adjacent pairs of nucleoside units are linked 3'-5' to 5'-3' or 2'-5' to 5'-2'. Various salts, mixed salts and free acid forms are also included.

Preferred modified oligonucleotide backbones that do not include a phosphorus atom therein have backbones that are formed by short chain alkyl or cycloalkyl internucleoside linkages, mixed heteroatom and alkyl or cycloalkyl internucleoside linkages, or one or more short chain heteroatomic or heterocyclic internucleoside linkages. These comprise those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and others having mixed N, O, S and CH₂ component parts.

In other preferred oligonucleotide mimetics, both the sugar and the internucleoside linkage, i.e., the backbone, of the nucleotide units are replaced with novel groups. The base units are maintained for hybridization with an appropriate nucleic acid target compound. One such oligomeric compound, an oligonucleotide mimetic that has been shown to have excellent hybridization properties, is referred to as a peptide nucleic acid (PNA). In PNA compounds, the sugar-backbone of an oligonucleotide is replaced with an amide containing backbone, in particular an aminoethylglycine backbone. The nucleobases are retained and are bound directly or indirectly to aza nitrogen atoms of the amide portion of the backbone.

In another preferred embodiment of the invention the oligonucleotides with phosphorothioate backbones and oligonucleosides with heteroatom backbones, and in particular-CH₂-NH-O-CH₂-,-CH₂-N (CH₃)-O-CH₂-known as a methylene (methylimino) or MMI backbone,-CH₂-O-N (CH₃)-CH₂-,-CH₂N(CH₃)-N(CH₃) CH₂-and-O-N(CH₃)-CH₂-CH₂-wherein the native phosphodiester backbone is represented as-O-P-O-CH₂- of the above referenced US patent no. 5,489,677, and the amide backbones of the above referenced US patent no. 5,602,240. Also preferred are oligonucleotides having morpholino backbone structures of the above-referenced US patent no. 5,034,506.

Modified oligonucleotides may also contain one or more substituted sugar moieties. Preferred oligonucleotides comprise one of the following at the 2' position: OH; F; O-, S-, or N-alkyl; O-, S-, or N-alkenyl; O-, S-or N-alkynyl; or O alkyl-O-alkyl, wherein the alkyl, alkenyl and alkynyl may be substituted or unsubstituted C to CO alkyl or C₂ to CO alkenyl and alkynyl. Particularly preferred are O (CH₂)ₙ OₘCH₃, O(CH₂)ₙ,OCH₃, O(CH₂)ₙNH₂, O(CH₂)ₙCH₃, O(CH₂)ₙONH₂, and O(CH₂ₙON(CH₂)nCH₃)₂ where n and m can be from 1 to about 10. Other preferred oligonucleotides comprise one of the following at the 2' position: C to CO, (lower alkyl, substituted lower alkyl, alkaryl, aralkyl, O-alkaryl or O-aralkyl, SH, SCH₃, OCN, Cl, Br, CN, CF₃, OCF₃, SOCH₃, SO₂CH₃, ONO₂, NO₂, N₃, NH₂, heterocycloalkyl, heterocycloalkaryl, aminoalkylamino, polyalkylamino, substituted silyl, an RNA cleaving group, a reporter group, an intercalator, a group for improving the pharmacokinetic properties of an oligonucleotide, or a group for improving the pharmacodynamic properties of an oligonucleotide, and other substituents having similar properties. A preferred modification comprises 2'-methoxyethoxy (2'-O-CH₂CH₂OCH₃, also known as 2'-O-(2-methoxyethyl) or 2'-MOE) (Martin et al., Helv. Chim. Acta, 1995, 78, 486-504) i.e., an alkoxyalkoxy group. A further preferred modification comprises 2'-dimethylaminooxyethoxy, i.e. , a O(CH₂)₂ON(CH₃)₂ group, also known as 2'-DMAOE, as described in examples herein below, and 2'-dimethylaminoethoxyethoxy (also known in the art as 2'-O-dimethylaminoethoxyethyl or 2'-DMAEOE), i.e., 2'-O-CH₂-O-CH₂-N (CH₂)₂.

Other preferred modifications comprise 2'-methoxy (2'-O CH₃), 2'-aminopropoxy (2'-O CH₂CH₂CH₂NH₂) and 2'-fluoro (2'-F). Similar modifications may also be made at other positions on the oligonucleotide, particularly the 3' position of the sugar on the 3' terminal nucleotide or in 2'-5' linked oligonucleotides and the 5' position of 5' terminal nucleotide. Oligonucleotides may also have sugar mimetics such as cyclobutyl moieties in place of the pentofuranosyl sugar.

Oligonucleotides may also comprise nucleobase (often referred to in the art simply as "base") modifications or substitutions. As used herein, "unmodified" or "natural" nucleobases comprise the purine bases adenine (A) and guanine (G), and the pyrimidine bases thymine (T), cytosine (C) and uracil (U). Modified nucleobases comprise other synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl uracil and cytosine, 6-azo uracil, cytosine and thymine, 5-uracil (pseudo-uracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylquanine and 7-methyladenine, 8-azaguanine and 8-azaadenine, 7-deazaguanine and 7-deazaadenine and 3-deazaguanine and 3-deazaadenine.

Further, nucleobases comprise those disclosed in United States Patent No. 3,687,808, those disclosed in 'The Concise Encyclopedia of Polymer Science And Engineering', pages 858-859, Kroschwitz, J.I., ed. John Wiley & Sons, 1990, those disclosed by Englisch et al., 'Angewandle Chemie, International Edition', 1991, 30, page 613, and those disclosed by Sanghvi, Y.S., Chapter 15, 'Antisense Research and Applications', pages 289-302, Crooke, S.T. and Lebleu, B. ea., CRC Press, 1993. Certain of these nucleobases are particularly useful for increasing the binding affinity of the oligomeric compounds of the invention. These comprise 5-substituted pyrimidines, 6-azapyrimidines and N-2, N-6 and 0-6 substituted purines, comprising 2-aminopropyladenine, 5-propynyluracil and 5-propynylcytosine. 5-methylcytosine substitutions have been shown to increase nucleic acid duplex stability by 0.6-1.2°C (Sanghvi, Y.S., Crooke, S.T. and Lebleu, B., eds, 'Antisense Research and Applications', CRC Press, Boca Raton, 1993, pp. 276-278) and are presently preferred base substitutions, even more particularly when combined with 2'-O-methoxyethyl sugar modifications.

### Anti-STING Molecules

*Antibodies and Aptamers:* In addition to the detection of STING gene and gene expression using nucleic acid hybridization technology, immunoassays can also be used to detect STING proteins of the invention. Such assays are useful for screening for modulators of STING, as well as for therapeutic and diagnostic applications. Immunoassays can be used to qualitatively or quantitatively analyze STING protein. A general overview of the applicable technology can be found in Harlow & Lane, Antibodies: A Laboratory Manual (1988).

Antibodies that specifically bind to: STING, a STING complexed with another molecule, STING fragments, mutants, variants and complementary sequences thereof are provided.

As used herein, the term "antibody" is meant to refer to complete, intact antibodies, and Fab fragments and F(ab)₂ fragments thereof. Complete, intact antibodies include monoclonal antibodies such as murine monoclonal antibodies, chimeric antibodies and humanized antibodies. Antibodies that bind to an epitope which is present on, for example, Rad50, are useful to isolate and purify the Rad50 from both natural sources and recombinant expression systems using well known techniques such as affinity chromatography. Such antibodies are useful to detect the presence of such protein in a sample and to determine if cells are expressing the protein.

Once the specific antibodies against STING are available, STING can be detected by a variety of immunoassay methods. In addition, the antibody can be used therapeutically as a STING modulators. For a review of immunological and immunoassay procedures, see Basic and Clinical Immunology (Stites & Terr eds., 7th ed. 1991). Moreover, the immunoassays of the present invention can be performed in any of several configurations, which are reviewed extensively in Enzyme Immunoassay (Maggio, ed., 1980); and Harlow & Lane, *supra*.

In a preferred embodiment, an antibody is specific for STING, variants, mutants and fragments thereof. Preferred STING molecules are human STING molecules. For example, mouse anti-human STING; rabbit anti-human STING; mouse anti-human STING; goat-anti-, mouse STING, etc. The species of the antibody can be any species. However, human or humanized antibodies are preferred. The antibodies can be directed to epitopes on linearized STING peptides or peptides in a native conformation.

In another preferred embodiment, a monoclonal or polyclonal antibody is specific for one or more antigenic epitopes of a peptide, polypeptide or protein encoded by a nucleic acid sequence set forth as SEQ ID NOS: 1 or 2, an active fragment, variant, or mutant thereof.

In another preferred embodiment, aptamers to STING are also contemplated. As used herein, the term "aptamer" or "selected nucleic acid binding species" include non-modified or chemically modified RNA or DNA. The method of selection may be by, but is not limited to, affinity chromatography and the method of amplification by reverse transcription (RT) or polymerase chain reaction (PCR).

### Candidate Therapeutic Agents

High throughput functional genomics assays can be used to identify modulators of STING function and/or expression. Detailed methods for measuring functions of STING, such as for example, STING mediated interferon induction, are provided in the examples which follow. Typically, the cells are contacted with a cDNA or a random peptide library (encoded by nucleic acids). The cDNA library can comprise sense, antisense, full length, and truncated cDNAs. The peptide library is encoded by nucleic acids. The effect of the cDNA or peptide library on STING is then monitored. The effect of the cDNA or peptide can be validated and distinguished from somatic mutations, using, e.g., regulatable expression of the nucleic acid such as expression from a tetracycline promoter. cDNAs and nucleic acids encoding peptides can be rescued using techniques known to those of skill in the art, e.g., using a sequence tag.

Proteins interacting with the peptide or with the protein encoded by the cDNA (e.g., STING) can be isolated using a yeast two-hybrid system, mammalian two hybrid system, or phage display screen, etc. Targets so identified can be further used as bait in these assays to identify additional members of the STING functional pathways, which members are also targets for drug development (see, e.g., Fields et al., Nature 340:245 (1989); Vasavada et al., Proc. Nat'l Acad. Sci. USA 88:10686 (1991); Fearon et al., Proc. Nat'l Acad. Sci. USA 89:7958 (1992); Dang et al., Mol. Cell. Biol. 11:954 (1991); Chien et al., Proc. Nat'l Acad. Sci. USA 9578 (1991); and U.S. Pat. Nos. 5,283,173, 5,667,973, 5,468,614, 5,525,490, and 5,637,463).

In a preferred embodiment, a method of identifying therapeutic agents comprises contacting: (i) a peptide encoded by nucleic acid sequence set forth as SEQ ID NOS: 1 or 2, an active fragment, variant, or mutant thereof; or, (ii) a nucleic acid sequence (STING) set forth as SEQ ID NOS: 1 or 2, an active fragment, variant, or mutant thereof, with a candidate therapeutic agent; determining whether (i) the agent modulates a function of the peptide or interaction of the peptide with a partner molecule; or (ii) the agent modulates expression and/or function of the nucleic acid sequence (STING).

In another preferred embodiment, a method of identifying candidate therapeutic agents for treatment of disease, comprises culturing an isolated cell expressing STING, administering a candidate therapeutic agent to the cultured cell; correlating STING expression and/or function in the presence or absence of a candidate therapeutic agent as compared to control cells, wherein a drug is identified based on desirable therapeutic outcomes. For example, a drug which modulates expression of STING; a drug which modulates interferon production; association of STING with various molecules; modulation of pathways; β2-microglobulin expression and the like; thereby, identifying candidate therapeutic agents that regulates STING expression and/or function.

Another suitable method for diagnosis and candidate drug discovery includes contacting a test sample with a cell expressing STING, an allele or fragment thereof; and detecting interaction of the test sample with STING, an allele or fragment thereof, or expression product of STING, an allele or fragment thereof. STING, an allele or fragment thereof, or expression product of the gene, an allele or fragment thereof suitably can be detectably labeled e.g. with a fluorescent or radioactive component.

In another preferred embodiment, a cell from a patient is isolated and contacted with a candidate therapeutic molecule. The genes, expression products thereof, are monitored to identify which genes or expression products are regulated by the drug.

*Nucleic Acid Microarrays:* Identification of a nucleic acid sequence capable of binding to STING can be achieved by immobilizing a library of nucleic acids onto the substrate surface so that each unique nucleic acid is located at a defined position to form an array. Details are provided in the examples section which follows. In general, the immobilized library of nucleic acids are exposed to a biomolecule or candidate agent under conditions which favored binding of the biomolecule to the nucleic acids. Non-specifically binding biomolecules could be washed away using mild to stringent buffer conditions depending on the level of specificity of binding desired. The nucleic acid array would then be analyzed to determine which nucleic acid sequences bound to the biomolecule. Preferably the biomolecules would carry a fluorescent tag for use in detection of the location of the bound nucleic acids.

An assay using an immobilized array of nucleic acid sequences may be used for determining the sequence of an unknown nucleic acid; single nucleotide polymorphism (SNP) analysis; analysis of gene expression patterns from a particular species, tissue, cell type, etc.; gene identification; etc.

Additional diagnostic uses for oligonucleotides designed from the sequences encoding a desired gene expression product may involve the use of PCR. These oligomers may be chemically synthesized, generated enzymatically, or *produced in vitro.* Oligomers will preferably contain a fragment of a polynucleotide encoding the expression products, or a fragment of a polynucleotide complementary to the polynucleotides, and will be employed under optimized conditions for identification of a specific gene. Oligomers may also be employed under less stringent conditions for detection or quantitation of closely-related DNA or RNA sequences.

In further embodiments, oligonucleotides or longer fragments derived from any of the polynucleotide sequences, may be used as targets in a microarray. The microarray can be used to monitor the identity and/or expression level of large numbers of genes and gene transcripts simultaneously to identify genes with which target genes or its product interacts and/or to assess the efficacy of candidate therapeutic agents in regulating expression products of genes that mediate, for example, neurological disorders. This information may be used to determine gene function, and to develop and monitor the activities of therapeutic agents.

Microarrays may be prepared, used, and analyzed using methods known in the art (see, e.g., Brennan et al., 1995, U.S. Pat. No. 5,474,796; Schena et al., 1996, Proc. Natl. Acad. Sci. U.S.A. 93: 10614-10619; Baldeschweiler et al., 1995, PCT application WO95/251116; Shalon, et al., 1995, PCT application WO95/35505; Heller et al., 1997, Proc. Natl. Acad. Sci. U.S.A. 94: 2150-2155; and Heller et al., 1997, U.S. Pat. No. 5,605,662). In other embodiments, a microarray comprises peptides, or other desired molecules which can be assayed to identify a candidate agent.

Candidate agents include numerous chemical classes, though typically they are organic compounds including small organic compounds, nucleic acids including oligonucleotides, and peptides. Small organic compounds suitably may have e.g. a molecular weight of more than about 40 or 50 yet less than about 2,500. Candidate agents may comprise functional chemical groups that interact with proteins and/or DNA.

Candidate agents may be obtained from a wide variety of sources including libraries of synthetic or natural compounds. For example, numerous means are available for random and directed synthesis of a wide variety of organic compounds and biomolecules, including expression of randomized oligonucleotides. Alternatively, libraries of natural compounds in the form of e.g. bacterial, fungal and animal extracts are available or readily produced.

Therapeutic agent assays of the invention suitably include, animal models, cell-based systems and non-cell based systems.

Preferably, identified genes, variants, fragments, or oligopeptides thereof are used for identifying agents of therapeutic interest, e.g. by screening libraries of compounds or otherwise identifying compounds of interest by any of a variety of drug screening or analysis techniques. The gene, allele, fragment, or oligopeptide thereof employed in such screening may be free in solution, affixed to a solid support, borne on a cell surface, or located intracellularly.

Another technique for drug screening provides for high throughput screening of compounds having suitable binding affinity to the protein of interest (see, e.g., Geysen et al., 1984, PCT application WO84/03564). In this method, large numbers of different small test compounds are synthesized on a solid substrate. The test compounds are reacted with identified genes, or fragments thereof, and washed. Bound molecules are then detected by methods well known in the art. Alternatively, non-neutralizing antibodies can be used to capture the peptide and immobilize it on a solid support.

The methods of screening of the invention comprise using screening assays to identify, from a library of diverse molecules, one or more compounds having a desired activity. A "screening assay" is a selective assay designed to identify, isolate, and/or determine the structure of, compounds within a collection that have a preselected activity. By "identifying" it is meant that a compound having a desirable activity is isolated, its chemical structure is determined (including without limitation determining the nucleotide and amino acid sequences of nucleic acids and polypeptides, respectively) the structure of and, additionally or alternatively, purifying compounds having the screened activity). Biochemical and biological assays are designed to test for activity in a broad range of systems ranging from protein-protein interactions, enzyme catalysis, small molecule-protein binding, to cellular functions. Such assays include automated, semi-automated assays and HTS (high throughput screening) assays.

In HTS methods, many discrete compounds are preferably tested in parallel by robotic, automatic or semi-automatic methods so that large numbers of test compounds are screened for a desired activity simultaneously or nearly simultaneously. It is possible to assay and screen up to about 6,000 to 20,000, and even up to about 100,000 to 1,000,000 different compounds a day using the integrated systems of the invention.

Typically in HTS, target molecules are administered or cultured with isolated cells with modulated receptors, including the appropriate controls.

In one embodiment, screening comprises contacting each cell culture with a diverse library of member compounds, some of which are ligands of the target, under conditions where complexes between the target and ligands can form, and identifying which members of the libraries are present in such complexes. In another non limiting modality, screening comprises contacting a target enzyme with a diverse library of member compounds, some of which are inhibitors (or activators) of the target, under conditions where a product or a reactant of the reaction catalyzed by the enzyme produce a detectable signal. In the latter modality, inhibitors of target enzyme decrease the signal from a detectable product or increase a signal from a detectable reactant (or vice-versa for activators).

In one embodiment the invention provides soluble assays using a STING protein, or a cell or tissue expressing an STING protein, either naturally occurring or recombinant. In another embodiment, the invention provides solid phase based *in vitro* assays in a high throughput format, where the STING protein or fragment thereof, is attached to a solid phase substrate. Any one of the assays described herein can be adapted for high throughput screening, e.g., ligand binding, cellular proliferation, cell surface marker flux, e.g., CD-69, screening, radiolabeled GTP binding, second messenger flux, e.g., Ca²⁺, IP3, cGMP, or cAMP, cytokine production, etc. In one preferred embodiment, the cell-based system using β2-microglobulin modulation and FACS assays is used in a high throughput format for identifying modulators of STING proteins, and therefore modulators of T cell activation.

In the high throughput assays of the invention, either soluble or solid state, it is possible to screen up to several thousand different modulators or ligands in a single day. This methodology can be used for STING proteins *in vitro,* or for cell-based or membrane-based assays comprising an STING protein. In particular, each well of a microtiter plate can be used to run a separate assay against a selected potential modulator, or, if concentration or incubation time effects are to be observed, every 5-10 wells can test a single modulator. Thus, a single standard microtiter plate can assay about 100 (e.g., 96) modulators. If 1536 well plates are used, then a single plate can easily assay from about 100- about 1500 different compounds. It is possible to assay many plates per day; assay screens for up to about 6,000, 20,000, 50,000, or more than 100,000 different compounds are possible using the integrated systems of the invention.

For a solid state reaction, the protein of interest or a fragment thereof, e.g., an extracellular domain, or a cell or membrane comprising the protein of interest or a fragment thereof as part of a fusion protein can be bound to the solid state component, directly or indirectly, via covalent or non covalent linkage e.g., via a tag. The tag can be any of a variety of components. In general, a molecule which binds the tag (a tag binder) is fixed to a solid support, and the tagged molecule of interest is attached to the solid support by interaction of the tag and the tag binder.

A number of tags and tag binders can be used, based upon known molecular interactions well described in the literature. For example, where a tag has a natural binder, for example, biotin, protein A, or protein G, it can be used in conjunction with appropriate tag binders (avidin, streptavidin, neutravidin, the Fc region of an immunoglobulin, etc.) Antibodies to molecules with natural binders such as biotin are also widely available and appropriate tag binders; see, SIGMA Immunochemicals 1998 catalogue SIGMA, St. Louis Mo.).

Similarly, any haptenic or antigenic compound can be used in combination with an appropriate antibody to form a tag/tag binder pair. Thousands of specific antibodies are commercially available and many additional antibodies are described in the literature. For example, in one common configuration, the tag is a first antibody and the tag binder is a second antibody which recognizes the first antibody. In addition to antibody-antigen interactions, receptor-ligand interactions are also appropriate as tag and tag-binder pairs. For example, agonists and antagonists of cell membrane receptors (e.g., cell receptor-ligand interactions such as transferrin, c-kit, viral receptor ligands, cytokine receptors, chemokine receptors, interleukin receptors, immunoglobulin receptors and antibodies, the cadherein family, the integrin family, the selection family, and the like; see, e.g., Pigott & Power, The Adhesion Molecule Facts Book I (1993). Similarly, toxins and venoms, viral epitopes, hormones (e.g., opiates, steroids, etc.), intracellular receptors (e.g. which mediate the effects of various small ligands, including steroids, thyroid hormone, retinoids and vitamin D; peptides), drugs, lectins, sugars, nucleic acids (both linear and cyclic polymer configurations), oligosaccharides, proteins, phospholipids and antibodies can all interact with various cell receptors.

Synthetic polymers, such as polyurethanes, polyesters, polycarbonates, polyureas, polyamides, polyethyleneimines, polyarylene sulfides, polysiloxanes, polyimides, and polyacetates can also form an appropriate tag or tag binder. Many other tag/tag binder pairs are also useful in assay systems described herein, as would be apparent to one of skill upon review of this disclosure.

Common linkers such as peptides, polyethers, and the like can also serve as tags, and include polypeptide sequences, such as poly gly sequences of between about 5 and 200 amino acids. Such flexible linkers are known to persons of skill in the art. For example, poly(ethelyne glycol) linkers are available from Shearwater Polymers, Inc. Huntsville, Ala. These linkers optionally have amide linkages, sulfhydryl linkages, or heterofunctional linkages.

Tag binders are fixed to solid substrates using any of a variety of methods currently available. Solid substrates are commonly derivatized or functionalized by exposing all or a portion of the substrate to a chemical reagent which fixes a chemical group to the surface which is reactive with a portion of the tag binder. For example, groups which are suitable for attachment to a longer chain portion would include amines, hydroxyl, thiol, and carboxyl groups. Aminoalkylsilanes and hydroxyalkylsilanes can be used to functionalize a variety of surfaces, such as glass surfaces. The construction of such solid phase biopolymer arrays is well described in the literature. See, e.g., Merrifield, J. Am. Chem. Soc. 85:2149-2154 (1963) (describing solid phase synthesis of, e.g., peptides); Geysen et al., J. Immun. Meth. 102:259-274 (1987) (describing synthesis of solid phase components on pins); Frank & Doring, Tetrahedron 44:60316040 (1988) (describing synthesis of various peptide sequences on cellulose disks); Fodor et al., Science, 251:767-777 (1991); Sheldon et al., Clinical Chemistry 39(4):718-719 (1993); and Kozal et al., Nature Medicine 2(7):753759 (1996) (all describing arrays of biopolymers fixed to solid substrates). Non-chemical approaches for fixing tag binders to substrates include other common methods, such as heat, cross-linking by UV radiation, and the like.

*Chemical Libraries:* Developments in combinatorial chemistry allow the rapid and economical synthesis of hundreds to thousands of discrete compounds. These compounds are typically arrayed in moderate-sized libraries of small molecules designed for efficient screening. Combinatorial methods, can be used to generate unbiased libraries suitable for the identification of novel compounds. In addition, smaller, less diverse libraries can be generated that are descended from a single parent compound with a previously determined biological activity. In either case, the lack of efficient screening systems to specifically target therapeutically relevant biological molecules produced by combinational chemistry such as inhibitors of important enzymes hampers the optimal use of these resources.

A combinatorial chemical library is a collection of diverse chemical compounds generated by either chemical synthesis or biological synthesis, by combining a number of chemical "building blocks," such as reagents. For example, a linear combinatorial chemical library, such as a polypeptide library, is formed by combining a set of chemical building blocks (amino acids) in a large number of combinations, and potentially in every possible way, for a given compound length (i.e., the number of amino acids in a polypeptide compound). Millions of chemical compounds can be synthesized through such combinatorial mixing of chemical building blocks.

A "library" may comprise from 2 to 50,000,000 diverse member compounds. Preferably, a library comprises at least 48 diverse compounds, preferably 96 or more diverse compounds, more preferably 384 or more diverse compounds, more preferably, 10,000 or more diverse compounds, preferably more than 100,000 diverse members and most preferably more than 1,000,000 diverse member compounds. By "diverse" it is meant that greater than 50% of the compounds in a library have chemical structures that are not identical to any other member of the library. Preferably, greater than 75% of the compounds in a library have chemical structures that are not identical to any other member of the collection, more preferably greater than 90% and most preferably greater than about 99%.

The preparation of combinatorial chemical libraries is well known to those of skill in the art. For reviews, see Thompson et al., Synthesis and application of small molecule libraries, Chem Rev 96:555-600, 1996; Kenan et al., Exploring molecular diversity with combinatorial shape libraries, Tends Biochem Sci 19:57-64, 1994; Janda, Tagged versus untagged libraries: methods for the generation and screening of combinatorial chemical libraries, Proc Natl Acad Sci USA. 91:10779-85, 1994; Lebl et al., One-bead-one-structure combinatorial libraries, Biopolymers 37:177-98, 1995; Eichler et al., Peptide, peptidomimetic, and organic synthetic combinatorial libraries, Med Res Rev. 15:481-96, 1995; Chabala, Solid-phase combinatorial chemistry and novel tagging methods for identifying leads, Curr Opin Biotechnol. 6:632-9, 1995; Dolle, Discovery of enzyme inhibitors through combinatorial chemistry, Mol Divers. 2:223-36, 1997; Fauchere et al., Peptide and nonpeptide lead discovery using robotically synthesized soluble libraries, Can J. Physiol Pharmacol. 75:683-9, 1997; Eichler et al., Generation and utilization of synthetic combinatorial libraries, Mol Med Today 1: 174-80, 1995; and Kay et al., Identification of enzyme inhibitors from phage-displayed combinatorial peptide libraries, Comb Chem High Throughput Screen 4:535-43, 2001.

Other chemistries for generating chemical diversity libraries can also be used. Such chemistries include, but are not limited to, peptoids (PCT Publication No. WO 91/19735); encoded peptides (PCT Publication WO 93/20242); random bio-oligomers (PCT Publication No. WO 92/00091); benzodiazepines (U.S. Pat. No. 5,288,514); diversomers, such as hydantoins, benzodiazepines and dipeptides (Hobbs, et al., Proc. Nat. Acad. Sci. USA, 90:6909-6913 (1993)); vinylogous polypeptides (Hagihara, et al., J. Amer. Chem. Soc. 114:6568 (1992)); nonpeptidal peptidomimetics with .beta.-D-glucose scaffolding (Hirschmann, et al., J. Amer. Chem. Soc., 114:9217-9218 (1992)); analogous organic syntheses of small compound libraries (Chen, et al., J. Amer. Chem. Soc., 116:2661 (1994)); oligocarbamates (Cho, et al., Science, 261:1303 (1993)); and/or peptidyl phosphonates (Campbell, et al., J. Org. Chem. 59:658 (1994)); nucleic acid libraries (see, Ausubel, Berger and Sambrook, all *supra*); peptide nucleic acid libraries (see, e.g., U.S. Pat. No. 5,539,083); antibody libraries (see, e.g., Vaughn, et al., Nature Biotechnology, 14(3):309-314 (1996) and PCT/US96/10287); carbohydrate libraries (see, e.g., Liang, et al., Science, 274:1520-1522 (1996) and U.S. Pat. No. 5,593,853); small organic molecule libraries (see, e.g., benzodiazepines, Baum C&E News, January 18, page 33 (1993); isoprenoids (U.S. Pat. No. 5,569,588); thiazolidinones and metathiazanones (U.S. Pat. No. 5,549,974); pyrrolidines (U.S. Pat. Nos. 5,525,735 and 5,519,134); morpholino compounds (U.S. Pat. No. 5,506,337); benzodiazepines (U.S. Pat. No. 5,288,514); and the like.

Devices for the preparation of combinatorial libraries are commercially available (see, e.g., 357 MPS, 390 MPS, Advanced Chem. Tech, Louisville Ky., Symphony, Rainin, Woburn, Mass., 433A Applied Biosystems, Foster City, Calif., 9050 Plus, Millipore, Bedford, Mass.). In addition, numerous combinatorial libraries are themselves commercially available (see, e.g., ComGenex, Princeton, N.J., Asinex, Moscow, Ru, Tripos, Inc., St. Louis, Mo., ChemStar, Ltd., Moscow, RU, 3D Pharmaceuticals, Exton, Pa., Martek Bio sciences, Columbia, Md., etc.).

High throughput screening can be used to measure the effects of drugs on complex molecular events such as signal transduction pathways, as well as cell functions including, but not limited to, cell function, apoptosis, cell division, cell adhesion, locomotion, exocytosis, and cell-cell communication. Multicolor fluorescence permits multiple targets and cell processes to be assayed in a single screen. Cross-correlation of cellular responses will yield a wealth of information required for target validation and lead optimization.

In another aspect, the present invention provides a method for analyzing cells comprising providing an array of locations which contain multiple cells wherein the cells contain one or more fluorescent reporter molecules; scanning multiple cells in each of the locations containing cells to obtain fluorescent signals from the fluorescent reporter molecule in the cells; converting the fluorescent signals into digital data; and utilizing the digital data to determine the distribution, environment or activity of the fluorescent reporter molecule within the cells.

A major component of the new drug discovery paradigm is a continually growing family of fluorescent and luminescent reagents that are used to measure the temporal and spatial distribution, content, and activity of intracellular ions, metabolites, macromolecules, and organelles. Classes of these reagents include labeling reagents that measure the distribution and amount of molecules in living and fixed cells, environmental indicators to report signal transduction events in time and space, and fluorescent protein biosensors to measure target molecular activities within living cells. A multiparameter approach that combines several reagents in a single cell is a powerful new tool for drug discovery.

This method relies on the high affinity of fluorescent or luminescent molecules for specific cellular components. The affinity for specific components is governed by physical forces such as ionic interactions, covalent bonding (which includes chimeric fusion with protein-based chromophores, fluorophores, and lumiphores), as well as hydrophobic interactions, electrical potential, and, in some cases, simple entrapment within a cellular component. The luminescent probes can be small molecules, labeled macromolecules, or genetically engineered proteins, including, but not limited to green fluorescent protein chimeras.

Those skilled in this art will recognize a wide variety of fluorescent reporter molecules that can be used in the present invention, including, but not limited to, fluorescently labeled biomolecules such as proteins, phospholipids, RNA and DNA hybridizing probes. Similarly, fluorescent reagents specifically synthesized with particular chemical properties of binding or association have been used as fluorescent reporter molecules (Barak et al., (1997), J. Biol. Chem. 272:27497-27500; Southwick et al., (1990), Cytometry 11:418-430; Tsien (1989) in Methods in Cell Biology, Vol. 29 Taylor and Wang (eds.), pp. 127-156). Fluorescently labeled antibodies are particularly useful reporter molecules due to their high degree of specificity for attaching to a single molecular target in a mixture of molecules as complex as a cell or tissue.

The luminescent probes can be synthesized within the living cell or can be transported into the cell via several non-mechanical modes including diffusion, facilitated or active transport, signal-sequence-mediated transport, and endocytotic or pinocytotic uptake. Mechanical bulk loading methods, which are well known in the art, can also be used to load luminescent probes into living cells (Barber et al. (1996), Neuroscience Letters 207:17-20; Bright et al. (1996), Cytometry 24:226-233; McNeil (1989) in Methods in Cell Bioloy, Vol. 29, Taylor and Wang (eds.), pp. 153-173). These methods include electroporation and other mechanical methods such as scrape-loading, bead-loading, impact-loading, syringe-loading, hypertonic and hypotonic loading. Additionally, cells can be genetically engineered to express reporter molecules, such as GFP, coupled to a protein of interest as previously described (Chalfie and Prasher U.S. Pat. No. 5,491,084; Cubitt et al. (1995), Trends in Biochemical Science 20:448-455).

Once in the cell, the luminescent probes accumulate at their target domain as a result of specific and high affinity interactions with the target domain or other modes of molecular targeting such as signal-sequence-mediated transport. Fluorescently labeled reporter molecules are useful for determining the location, amount and chemical environment of the reporter. For example, whether the reporter is in a lipophilic membrane environment or in a more aqueous environment can be determined (Giuliano et al. (1995), Ann. Rev. of Biophysics and Biomolecular Structure 24:405-434; Giuliano and Taylor (1995), Methods in Neuroscience 27.1-16). The pH environment of the reporter can be determined (Bright et al. (1989), J. Cell Biology 104:1019-1033; Giuliano et al. (1987), Anal. Biochem. 167:362-371; Thomas et al. (1979), Biochemistry 18:2210-2218). It can be determined whether a reporter having a chelating group is bound to an ion, such as Ca⁺⁺, or not (Bright et al. (1989), In Methods in Cell Biology, Vol. 30, Taylor and Wang (eds.), pp. 157-192; Shimoura et al. (1988), J. of Biochemistry (Tokyo) 251:405-410; Tsien (1989) In Methods in Cell Biology, Vol. 30, Taylor and Wang (eds.), pp. 127-156).

Furthermore, certain cell types within an organism may contain components that can be specifically labeled that may not occur in other cell types. For example, neural cells often contain polarized membrane components. That is, these cells asymmetrically distribute macromolecules along their plasma membrane. Connective or supporting tissue cells often contain granules in which are trapped molecules specific to that cell type (e.g., heparin, histamine, serotonin, etc.). Most muscular tissue cells contain a sarcoplasmic reticulum, a specialized organelle whose function is to regulate the concentration of calcium ions within the cell cytoplasm. Many nervous tissue cells contain secretory granules and vesicles in which are trapped neurohormones or neurotransmitters. Therefore, fluorescent molecules can be designed to label not only specific components within specific cells, but also specific cells within a population of mixed cell types.

Those skilled in the art will recognize a wide variety of ways to measure fluorescence. For example, some fluorescent reporter molecules exhibit a change in excitation or emission spectra, some exhibit resonance energy transfer where one fluorescent reporter loses fluorescence, while a second gains in fluorescence, some exhibit a loss (quenching) or appearance of fluorescence, while some report rotational movements (Giuliano et al. (1995), Ann. Rev. of Biophysics and Biomol. Structure 24:405-434; Giuliano et al. (1995), Methods in Neuroscience 27:1-16).

The whole procedure can be fully automated. For example, sampling of sample materials may be accomplished with a plurality of steps, which include withdrawing a sample from a sample container and delivering at least a portion of the withdrawn sample to test cell culture (e.g., a cell culture wherein gene expression is regulated). Sampling may also include additional steps, particularly and preferably, sample preparation steps. In one approach, only one sample is withdrawn into the auto-sampler probe at a time and only one sample resides in the probe at one time. In other embodiments, multiple samples may be drawn into the auto-sampler probe separated by solvents. In still other embodiments, multiple probes may be used in parallel for auto sampling.

In the general case, sampling can be effected manually, in a semi-automatic manner or in an automatic manner. A sample can be withdrawn from a sample container manually, for example, with a pipette or with a syringe-type manual probe, and then manually delivered to a loading port or an injection port of a characterization system. In a semi-automatic protocol, some aspect of the protocol is effected automatically (e.g., delivery), but some other aspect requires manual intervention (e.g., withdrawal of samples from a process control line). Preferably, however, the sample(s) are withdrawn from a sample container and delivered to the characterization system, in a fully automated manner-for example, with an auto-sampler.

*Labels*: The particular label or detectable moiety or tag used in the assay is not a critical aspect of the invention, as long as it does not significantly interfere with the specific binding of the ORF phage to their ligands. The detectable group can be any material having a detectable physical or chemical property. Such detectable labels have been well developed in the field of immunoassays and, in general, most labels useful in such methods can be applied to the present invention. Thus, a label is any composition detectable by spectroscopic, photochemical, biochemical, immunochemical, electrical, optical, or chemical means. Useful labels in the present invention include magnetic beads (e.g., DYNABEADS^{™}), fluorescent dyes (e.g., fluorescein isothiocyanate, Texas red, rhodamine, and the like), radiolabels (e.g., ³H, ¹²⁵I, ³⁵S, ¹⁴C, or ³²P), enzymes (e.g., horseradish peroxidase, alkaline phosphatase and others commonly used in an ELISA), and colorimetric labels such as colloidal gold or colored glass or plastic beads (e.g., polystyrene, polypropylene, latex, etc.).

The label may be coupled directly or indirectly to the desired component of the assay according to methods well known in the art. As indicated above, a wide variety of labels may be used, with the choice of label depending on sensitivity required, ease of conjugation with the compound, stability requirements, available instrumentation, and disposal provisions.

Non-radioactive labels are often attached by indirect means. Generally, a ligand molecule (e.g., biotin) is covalently bound to the molecule. The ligand then binds to another molecules (e.g., streptavidin) molecule, which is either inherently detectable or covalently bound to a signal system, such as a detectable enzyme, a fluorescent compound, or a chemiluminescent compound.

The molecules can also be conjugated directly to signal generating compounds, e.g., by conjugation with an enzyme or fluorophore. Enzymes of interest as labels will primarily be hydrolases, particularly phosphatases, esterases and glycosidases, or oxidotases, particularly peroxidases. Fluorescent compounds include fluorescein and its derivatives, rhodamine and its derivatives, dansyl, umbelliferone, etc. Chemiluminescent compounds include luciferin, and 2,3-dihydrophthalazinediones, e.g., luminol. For a review of various labeling or signal producing systems that may be used, see U.S. Pat. No. 4,391,904.

Means of detecting labels are well known to those of skill in the art. Thus, for example, where the label is a radioactive label, means for detection include a scintillation counter or photographic film as in autoradiography. Where the label is a fluorescent label, it may be detected by exciting the fluorochrome with the appropriate wavelength of light and detecting the resulting fluorescence. The fluorescence may be detected visually, by means of photographic film, by the use of electronic detectors such as charge-coupled devices (CCDs) or photomultipliers and the like. Similarly, enzymatic labels may be detected by providing the appropriate substrates for the enzyme and detecting the resulting reaction product. Finally simple colorimetric labels may be detected simply by observing the color associated with the label. Thus, in various dipstick assays, conjugated gold often appears pink, while various conjugated beads appear the color of the bead.

### Formulations and Administration of STING Compositions

The compounds are useful for the *in vivo* treatment or prevention of diseases in which STING is implicated. These include viral diseases, bacterial infections, parasitic of protozoan infections, cancer, autoimmune diseases, inflammation, transplantation, neurological diseases or disorders, Parkinson's disease, Amyotrophic Lateral Sclerosis (ALS), chronic obstructive pulmonary disease (COPD), multiple sclerosis, Alzheimer's Disease, hepatic diseases or disorders, gastrointestinal diseases or disorders, diabetes, cancer, autoimmunity, immune related diseases or disorders, neurological diseases or disorders, neurodegenerative diseases or disorders, nerve repair and paralysis, neuroendocrine differentiation, inflammatory diseases, muscular diseases or disorders, diseases or disorders associated with infectious organisms, and the like.

In one embodiment of the invention, there is provided a compound for use in a medicament, especially for preventing or treating diseases in which the disease pathology may be modified by modulating the immune response e.g. cancer, infections, autoimmunity, transplantations etc. In those diseases or conditions where it is desired to suppress an immune response (e.g. inflammation, autoimmune diseases or disorders, transplantation etc), STING may be down-regulated, e.g. antisense, siRNA, antibodies, aptamers etc. In those conditions whereby it is desired to increase or enhance an immune response, STING may be reconstituted e.g. cancer, viral infection etc, by administration of vector expressing STING, peptides, polynucleotides, oligonucleotides etc.

STING compounds or identified agents can be evaluated by various means. Cellular assays evaluate the ability of a compound to interfere with the infectious life cycle of a parasite or the migration of a cancer or inflammatory cell *ex vivo,* while not exhibiting toxicity against the host cell. Cellular assays measure the survival of a parasite-infected or viral infected cell in culture. *In vivo* assays evaluate the efficacy of test compounds to prevent or ameliorate disease symptoms, such as those associated with for example, infection, cancer invasion or growth, or inflammatory cell migration. Further, preferred compounds delay, interfere with, prevent or eliminate the completion of the infectious life cycle of a disease agent, the migratory ability of a cancer cell or an inflammation cell. etc Additionally, preferred compounds prevent or diminish a an infection in an individual or the migration of cancer cells or inflammatory cells in an individual, thereby preventing or ameliorating the pathogenic symptoms associated with such infections or the migration of rogue cells.

The compositions and methods of the invention are preferably employed for treatment or prophylaxis against diseases caused abnormal cell growth and by infectious agents, particularly for treatment of infections as may occur in tissue such as lung, heart, liver, prostate, brain, testes, stomach, intestine, bowel, spinal cord, sinuses, urinary tract or ovaries of a subject. The methods of the invention also may be employed to treat systemic conditions such as viremia or septicemia. The methods of the invention are also preferably employed for treatment of diseases and disorders associated with viral infections or bacterial infections, as well as any other disorder caused by an infectious agent.

To examine the extent of the effects of the compounds, samples, assays, cultures or test subjects comprising or lacking STING, are treated with a potential compound and are compared to negative control samples without the test compound, and positive control samples, treated with a compound known to inhibit the STING. Negative control samples (not treated with a test compound), are assigned a relative STING activity level of 100%. Inhibition of STING expression, activity, and/or function is achieved when the STING expression, activity, and/or function relative to the control is about 90%, preferably 75% or 50%, more preferably 25-0%.

The invention is useful in the prevention and/or treatment of the disease states mentioned or implied herein. The present invention also is useful in a method of treatment or prevention of diseases caused by deregulation of STING expression, activity, and/or function, which methods comprise administering to an animal, particularly a mammal, most particularly a human, in need thereof a compound of the present invention. The present invention particularly provides methods for treating diseases include infections by *Entamoeba histolytica*; *Pneumocystis carinii, Trypanosoma cruzi*, *Trypanosoma brucei*, *Leishmania mexicana*, *Clostridium histolyticum*, *Staphylococcus aureus*, foot-and-mouth disease virus and *Crithidia fasciculata;* as well as in osteoporosis, autoimmunity, schistosomiasis, malaria, tumor metastasis, metachromatic leukodystrophy, muscular dystrophy and amytrophy.

Other examples include veterinary and human pathogenic protozoa, intracellular active parasites of the phylum Apicomplexa or Sarcomastigophora, Trypanosoma, Plasmodia, Leishmania, Babesia and Theileria, Cryptosporidia, Sacrocystida, Amoeba, Coccidia and Trichomonadia. These compounds are also suitable for the treatment of *Malaria tropica,* caused by, for example, *Plasimodium falciparum, Malaria tentiana,* caused by *Plasmodium vivax* or *Plasmodium ovale* and for the treatment of *Malaria quartana,* caused by *Plasmodium malariae.* They are also suitable for the treatment of Toxoplasmosis, caused by *Toxoplasma gondii,* Coccidiosis, caused for instance by *Isospooa belli,* intestinal Sarcosporidiosis, caused by *Sarcocystis suihominis,* dysentery caused by *Entamoeba histolytica,* Cryptosporidiosis, caused by *Cryptosporidium parvum,* Chagas' disease, caused by *Trypanosoma cruzi,* sleeping sickness, caused by *Trypanosoma brucei rhoclesiense* or *gambiense*, the cutaneous and visceral as well as other forms of Leishmaniosis. They are also suitable for the treatment of animals infected by veterinary pathogenic protozoa, like *Theileria parva*, the pathogen causing bovine East coast fever, *Trypanosoma congolense congolense* or *Trypanosoma vivax vivax*, *Trypanosoma brucei brucei*, pathogens causing Nagana cattle disease in Africa, *Trypanosoma brucei evansi* causing Surra, *Babesia bigemina,* the pathogen causing Texas fever in cattle and buffalos, *Babesia bovis,* the pathogen causing European bovine Babesiosis as well as Babesiosis in dogs, cats and sheep, *Sarcocystis ovicanis* and ovifelis pathogens causing Sarcocystiosis in sheep, cattle and pigs, Cryptosporidia, pathogens causing Cryptosporidioses in cattle and birds, Eimeria and Isospora species, pathogens causing Coccidiosis in rabbits, cattle, sheep, goats, pigs and birds, especially in chickens and turkeys. Rickettsia comprise species such as *Rickettsia felis, Rickettsia prowazekii, Rickettsia rickettsii*, *Rickettsia typhi*, *Rickettsia conorii*, *Rickettsia africae* and cause diseases such as typhus, rickettsialpox, Boutonneuse fever, African Tick Bite Fever, Rocky Mountain spotted fever, Australian Tick Typhus, Flinders Island Spotted Fever and Queensland Tick Typhus. In the treatment of these diseases, the compounds of the present invention may be combined with other agents.

Particularly preferred bacteria causing serious human diseases are the Gram positive organisms: *Staphylococcus atreus*, *Staphylococcus epidermidis, Enterococcus faecalis* and *E. faecium*, *Streptococcus pneumoniae* and the Gram negative organisms: *Pseudomonas aeruginosa*, *Burkholdia cepacia*, *Xanthomonas maltophila*, *Escherichia coli*, *Enterobacter spp*, *Klebsiella pneumoniae* and *Salmonella spp*.

In another preferred embodiment, the bacteria are Gram negative bacteria. Examples, comprise: *Pseudomonas aeruginosa*; *Burkholdia cepacia*; *Xanthomonas maltophila*; *Escheoichia coli*; *Enterobacter* spp.; *Klebsiella pneumoniae; Salmonella* spp.

Particularly preferred fungi causing or associated with human diseases according to the present invention include (but not restricted to) *Candida albicans*, *Histoplasma neoformans*, *Coccidioides immitis* and *Penicillium marneffei.*

Non-limiting examples of viral organisms include, but not restricted to, those listed in Table 1. For information about the viral organisms see Fields of Virology, 3. ed., vol 1 and 2, BN Fields et al. (eds*.*).

**Table 1. Selected viral organisms causing human diseases.**

| **Herpesviruses** | |
|---|---|
| | Alpha-herpesviruses: |
| | Herpes simplex virus 1 (HSV-1) |
| | Herpes simplex vims 2 (HSV-2) |
| | Varicella Zoster virus (VZV) |
| | Beta-herpesviruses: |
| | Cytomegalovirus (CMV) |
| | Herpes virus 6 (HHV-6) |
| | Gamma-herpesviruses: |
| | Epstein-Barr virus (EBV) |
| | Herpes virus 8 (HHV-8) |

| **Hepatitis viruses** | |
|---|---|
| | Hepatitis A virus |
| | Hepatitis B virus |
| | Hepatitis C virus |
| | Hepatitis D virus |
| | Hepatitis E virus |

| **Retroviruses** | |
|---|---|
| | Human Immunodeficiency 1 (HIV-1) |

| **Orthomyxoviruses** | |
|---|---|
| | Influenzaviruses A, B and C |

| **Paramyxoviruses** | |
|---|---|
| | Respiratory Syncytial virus (RSV) |
| | Parainfluenza viruses (PI) |
| | Mumps virus |
| | Measles virus |

| **Togaviruses** | |
|---|---|
| | Rubella virus |

| **Picornaviruses** | |
|---|---|
| | Enteroviruses |
| | Rhinoviruses |
| | Coronaviruses |

| **Papovaviruses** | |
|---|---|
| | Human papilloma viruses (HPV) |
| | Polyomaviruses (BKV and JCV) |
| **Gastroenteritisviruses** | |
| **Filoviridae** | |
| **Bunyaviridae** | |
| **Rhabdoviridae** | |
| **Flaviviridae** | |

In another preferred embodiment, the compounds of the invention can be administered to patients for prevention or treatment, with combinations of one or more the compounds and other known therapies. For example, in the case of cancer, the STING compositions can be administered with chemotherapeutic or surgical treatments.

The compounds of this invention may also be administered orally to the patient, in a manner such that the concentration of drug is sufficient to inhibit bone resorption or to achieve any other therapeutic indication as disclosed herein. Typically, a pharmaceutical composition containing the compound is administered at an oral dose of between about 0.1 to about 50 mg/kg in a manner consistent with the condition of the patient. Preferably the oral dose would be about 0.5 to about 20 mg/kg.

An intravenous infusion of the compound in 5% dextrose in water or normal saline, or a similar formulation with suitable excipients, is most effective, although an intramuscular bolus injection is also useful. Typically, the parenteral dose will be about 0.01 to about 100 mg/kg; preferably between 0.1 and 20 mg/kg, in a manner to maintain the concentration of drug in the plasma at a concentration effective to inhibit a cysteine protease. The compounds may be administered one to four times daily at a level to achieve a total daily dose of about 0.4 to about 400 mg/kg/day. The precise amount of an inventive compound which is therapeutically effective, and the route by which such compound is best administered, is readily determined by one of ordinary skill in the art by comparing the blood level of the agent to the concentration required to have a therapeutic effect. Prodrugs of compounds of the present invention may be prepared by any suitable method. For those compounds in which the prodrug moiety is a ketone functionality, specifically ketals and/or hemiacetals, the conversion may be effected in accordance with conventional methods.

No unacceptable toxicological effects are expected when compounds, derivatives, salts, compositions etc, of the present invention are administered in accordance with the present invention. The compounds of this invention, which may have good bioavailability, may be tested in one of several biological assays to determine the concentration of a compound which is required to have a given pharmacological effect.

In another preferred embodiment, there is provided a pharmaceutical or veterinary composition comprising one or more STING compounds and a pharmaceutically or veterinarily acceptable carrier. Other active materials may also be present, as may be considered appropriate or advisable for the disease or condition being treated or prevented.

The carrier, or, if more than one be present, each of the carriers, must be acceptable in the sense of being compatible with the other ingredients of the formulation and not deleterious to the recipient.

The compounds described herein are suitable for use in a variety of drug delivery systems described above. Additionally, in order to enhance the *in vivo* serum half-life of the administered compound, the compounds may be encapsulated, introduced into the lumen of liposomes, prepared as a colloid, or other conventional techniques may be employed which provide an extended serum half-life of the compounds. A variety of methods are available for preparing liposomes, as described in, e.g., Szoka, et al., U.S. Pat. Nos. 4,235,871, 4,501,728 and 4,837,028 each of which is incorporated herein by reference. Furthermore, one may administer the drug in a targeted drug delivery system, for example, in a liposome coated with a tissue-specific antibody. The liposomes will be targeted to and taken up selectively by the organ.

The formulations include those suitable for rectal, nasal, topical (including buccal and sublingual), vaginal or parenteral (including subcutaneous, intramuscular, intravenous and intradermal) administration, but preferably the formulation is an orally administered formulation. The formulations may conveniently be presented in unit dosage form, e.g. tablets and sustained release capsules, and may be prepared by any methods well known in the art of pharmacy.

Such methods include the step of bringing into association the above defined active agent with the carrier. In general, the formulations are prepared by uniformly and intimately bringing into association the active agent with liquid carriers or finely divided solid carriers or both, and then if necessary shaping the product. The invention extends to methods for preparing a pharmaceutical composition comprising bringing a compound, for example, of general formula (I) in conjunction or association with a pharmaceutically or veterinarily acceptable carrier or vehicle.

The STING or any other compound identified using these methods can be formulated according to known methods to prepare pharmaceutically useful compositions, whereby the compound is combined in admixture with a pharmaceutically acceptable carrier vehicle. Therapeutic formulations are prepared for storage by mixing the active ingredient having the desired degree of purity with optional physiologically acceptable carriers, excipients or stabilizers (Remington's Pharmaceutical Sciences 16th edition, Osol, A. Ed. (1980)), in the form of lyophilized formulations or aqueous solutions. Acceptable carriers, excipients or stabilizers are nontoxic to recipients at the dosages and concentrations employed, and include buffers such as phosphate, citrate and other organic acids; antioxidants including ascorbic acid; low molecular weight (less than about 10 residues) polypeptides; proteins, such as serum albumin, gelatin or immunoglobulins; hydrophilic polymers such as polyvinylpyrrolidone, amino acids such as glycine, glutamine, asparagine, arginine or lysine; monosaccharides, disaccharides and other carbohydrates including glucose, mannose, or dextrins; chelating agents such as EDTA; sugar alcohols such as mannitol or sorbitol; salt-forming counterions such as sodium; and/or nonionic surfactants such as TWEEN™. (ICI Americas Inc., Bridgewater, N.J.), PLURONICS™. (BASF Corporation, Mount Olive, N.J.) or PEG.

The formulations to be used for *in vivo* administration must be sterile and pyrogen free. This is readily accomplished by filtration through sterile filtration membranes, prior to or following lyophilization and reconstitution.

The route of administration is in accord with known methods, e.g. injection or infusion by intravenous, intraperitoneal, intracerebral, intramuscular, intraocular, intraarterial or intralesional routes, topical administration, or by sustained release systems.

Dosages and desired drug concentrations of pharmaceutical compositions of the present invention may vary depending on the particular use envisioned. The determination of the appropriate dosage or route of administration is well within the skill of an ordinary physician. Animal experiments provide reliable guidance for the determination of effective doses for human therapy. Interspecies scaling of effective doses can be performed following the principles laid down by Mordenti, J. and Chappell, W. "The use of interspecies scaling in toxicokinetics" In Toxicokinetics and New Drug Development, Yacobi et al., Eds., Pergamon Press, New York 1989, pp. 42-96.

Formulations for oral administration in the present invention may be presented as: discrete units such as capsules, cachets or tablets each containing a predetermined amount of the active agent; as a powder or granules; as a solution or a suspension of the active agent in an aqueous liquid or a non-aqueous liquid; or as an oil-in-water liquid emulsion or a water in oil liquid emulsion; or as a bolus etc.

For compositions for oral administration (e.g. tablets and capsules), the term "acceptable carrier" includes vehicles such as common excipients e.g. binding agents, for example syrup, acacia, gelatin, sorbitol, tragacanth, polyvinylpyrrolidone (Povidone), methylcellulose, ethylcellulose, sodium carboxymethylcellulose, hydroxypropylmethylcellulose, sucrose and starch; fillers and carriers, for example corn starch, gelatin, lactose, sucrose, microcrystalline cellulose, kaolin, mannitol, dicalcium phosphate, sodium chloride and alginic acid; and lubricants such as magnesium stearate, sodium stearate and other metallic stearates, glycerol stearate stearic acid, silicone fluid, talc waxes, oils and colloidal silica. Flavoring agents such as peppermint, oil of wintergreen, cherry flavoring and the like can also be used. It may be desirable to add a coloring agent to make the dosage form readily identifiable. Tablets may also be coated by methods well known in the art.

A tablet may be made by compression or molding, optionally with one or more accessory ingredients. Compressed tablets may be prepared by compressing in a suitable machine the active agent in a free flowing form such as a powder or granules, optionally mixed with a binder, lubricant, inert diluent, preservative, surface-active or dispersing agent. Molded tablets may be made by molding in a suitable machine a mixture of the powdered compound moistened with an inert liquid diluent. The tablets may be optionally be coated or scored and may be formulated so as to provide slow or controlled release of the active agent.

Other formulations suitable for oral administration include lozenges comprising the active agent in a flavored base, usually sucrose and acacia or tragacanth; pastilles comprising the active agent in an inert base such as gelatin and glycerin, or sucrose and acacia; and mouthwashes comprising the active agent in a suitable liquid carrier.

Parenteral formulations will generally be sterile.

### Kits

Kits according to the present invention include at least one of STING molecules, antibodies, etc.. The kits may also include buffer and/or excipient solutions (in liquid or frozen form)--or buffer and/or excipient powder preparations to be reconstituted with water. Thus, preferably the kits containing the STING peptides, are frozen, lyophilized, pre-diluted, or pre-mixed at such a concentration that the addition of a predetermined amount of heat, of water, or of a solution provided in the kit will result in a formulation of sufficient concentration and pH as to be effective assaying any compound for therapeutic value in the treatment of disease. Preferably, such a kit will also comprise instructions for reconstituting and using the components of the assay. The kit may also comprise two or more component parts for the reconstituted active composition. For example, a second component part may be anti-tag antibodies labeled with Europium cryptate and allophycocyanin. The above-noted buffers, excipients, and other component parts can be sold separately or together with the kit.

While various embodiments of the present invention have been described above, it should be understood that they have been presented by way of example only, and not limitation. Numerous changes to the disclosed embodiments can be made in accordance with the disclosure herein without departing from the spirit or scope of the invention. Thus, the breadth and scope of the present invention should not be limited by any of the above described embodiments.

All documents mentioned herein are incorporated herein by reference. All publications and patent documents cited in this application are incorporated by reference for all purposes to the same extent as if each individual publication or patent document were so individually denoted. By their citation of various references in this document, Applicants do not admit any particular reference is "prior art" to their invention. Embodiments of inventive compositions and methods are illustrated in the following examples.

### EXAMPLES

The following non-limiting Examples serve to illustrate selected embodiments of the invention. It will be appreciated that variations in proportions and alternatives in elements of the components shown will be apparent to those skilled in the art and are within the scope of embodiments of the present invention.

### Examples 1: STING, an Endoplasmic Reticulum Adaptor that Facilitates Innate Immune Signaling

Cellular host defense responses to pathogen invasion principally involves the detection of pathogen associated molecular patterns (PAMPs) such as viral nucleic acid or bacterial cell wall components including lipopolysaccharide or flagellar proteins that results in the induction of anti-pathogen genes. For example, viral RNA can be detected by membrane bound Toll-like receptors (TLR's) present in the endoplasmic reticulum (ER) and/or endosomes (e.g. TLR 3 and 7/8) or by TLR-independent intracellular DExD/H box RNA helicases referred to as retinoic acid inducible gene 1 (RIG-I) or melanoma differentiation associated antigen 5 (MDA5, also referred to as IFIH1 and helicard). These events culminate in the activation of downstream signaling events, much of which remains unknown, leading to the transcription of NF-κB and IRF3/7- dependent genes, including type IIFN.

### methods

*Cells, viruses, and reagents*: 293T and HEK293 cells were obtained from the ATCC and were maintained in DMEM medium supplemented with 10% FBS. Human

Umbilical Vein Endothelial Cells (HUVEC) were obtained from Lonza (Basel, Switzerland), and were maintained in endothelial growth medium (EGM-2) (Lonza). FADD+/+, FADD-/-, TBK-1+/+ and TBK-1-/- MEFs (Balachandran, S., E. et al., Nature, 2004. 432(7015): p. 401-5). VSV-GFP was used in infections and titered as described previously (Balachandran, S., *E. et al*, 2004). VSV-ΔM was constructed as described previously (Faria, P.A., et al., Mol Cell, 2005. 17(1): p. 93-102). Murine IFN-β ELISA Kit was obtained from PBL (Piscataway, NJ). EMCV was purchased from ATCC.

*Plasmid constructs*: Human STING (hSTING), murine STING (mSTING), hSTING-N (amino acids 1-230) and hSTING-C (amino acids 173-379) sequences were amplified by PCR and were cloned into pcDNA3 plasmids to generate C-terminally HA-tagged expression constructs. Expression plasmids encoding Flag-tagged RIG-I, ΔRIG-1 (amino acids 1-284), IPS-1 and TBK-1 were described previously (Balachandran, S., et al., J Immunol, 2007. 178(4): p. 2429-39). GFP-tagged RIG-I and TBK-1 were generated by cloning into pAcGFP1-C1 (Clontech, CA). Other plasmids were obtained as follows: p110-Luc and PRD-III-I-Luc (T. Maniatis), IFNβ-Luc and ISRE-Luc (J. Hiscott), NFκB-Luc (Stratagene, CA), ER-dsRED and Golgi-dsRED (Clontech, CA).

*Antibodies:* Rabbit polyclonal antibody against a synthetic peptide corresponding to residues 324-340 of human STING was obtained from EvoQuest Custom Antibody Services (Invitrogen, CA). Other antibodies were obtained from following sources: SEC61β (Upstate, NY), β-actin, HA, FLAG (Sigma, MO), IRF3 and RIG-I (Abcam, MA).

*Northern blot*: Human multiple tissue RNA blots (Clontech, CA) were hybridized with a ³²P-labelled full-length human STING probe.

*Real time PCR*: Total RNA was isolated from cells using the RNeasy RNA extraction kit (Qiagen, Valencia, CA) and cDNA synthesis was performed using 1µg of total RNA (Roche, Indianapolis, IN). Fluorescence real time PCR analysis was performed using a LightCycler 2.0 instrument (Roche Molecular Biochemicals, Indianapolis, IN) and TaqMan Gene Expression Assays (Applied Biosystems, CA). Relative amounts of mRNA were normalized to the 18S ribosomal RNA levels in each sample.

*DNA Microarray analysis*: Total RNA was extracted from 293T cells transfected with STING expressing vectors. Preparation of cDNA and microarray analysis was performed at the W.M. Keck Foundation Biotechnology Research Laboratory DNA microarray facility at Yale University (New Haven, CT). The Human Genome U133 Plus 2.0 Array (Affymetrix, CA) was used. Data analysis was performed with GeneSpring software (Silicon Genetics, CA).

*Confocal microscopy*: ER-DsRED and Golgi-DsRED (Clontech, CA) were used for ER and Golgi marker, respectively. For mitochondria staining, living cells were incubated with 300 nM of Mito Tracker Red (Invitrogen, CA) for 45 min at 37°C.

*Mitochondria and ER fraction isolation*: Mitochondria and ER membranes were purified on discontinuous sucrose gradients as previously described (Bozidis, P., C.D. Williamson, and A.M. Colberg-Poley, Isolation of endoplasmic reticulum, mitochondria, and mitochondria-associated membrane fractions from transfected cells and from human cytomegalovirus-infected primary fibroblasts. Curr Protoc Cell Biol, 2007. Chapter 3: p. Unit 3 27). Briefly, HUVEC cells in MTE buffer (0.27M mannitol, 10 mM Tris-HCl, 0.1mM EDTA, pH 7.4) were lysed by sonication. Lysed cells were centrifuged at 700 x g for 10 min to remove nuclei and cellular debris. Mitochondria were obtained by centrifugation at 15,000 x g for 10 min, and post mitochondrial supernatant was used for purification of ER fractions. Mitochondria pellet was resuspended in MTE buffer, and was layered on discontinuous sucrose gradients consisting of 1.0 M and 1.7M sucrose and banded by centrifugation at 40,000 x g for 22 min. Mitochondria fraction was collected and pelleted by centrifugation at 15,000 x g for 10 min. Purified mitochondria were resuspended in PBS and used for Western blot analysis. To isolate ER fractions, post-mitochondrial supernatant described above was layered on discontinuous sucrose gradients consisting of 1.3M, 1.5 M and 2.0M sucrose, and banded by centrifugation at 100,000 x g for 70 min. The ER fraction at the interface between the supernatant and the 1.3 M sucrose was collected, and pelleted by centrifugation at 100,000 x g for 45 min. The ER membranes were resuspended in PBS and were used for Western blot analysis.

*RNA interference:* Chemically synthesized 21-nucleotide siRNA duplexes were obtained from Dharmacon, Inc (Lafayette, CO). RNA oligonucleotides used for human and murine STING were as follows: hSTING(a), GCAUCAAGGAUCGGGUUU (SEQ ID NO: 3); hSTING(b), GGUCAUAUUACAUCGGAU (SEQ ID NO: 3); mSTING, CCAACAGCGUCUACGAGA (SEQ ID NO: 5). 293T cells were plated on 24-well plates at 5 × 10⁴ cells per well and transfected with 10 pmol of siRNA duplex per well using Lipofectamine RNAiMAX (Invitrogen, CA). MEFs were transfected by using an Amaxa nucleofector apparatus (program A-023) and Amaxa MEF nucleofector kit 1 according to the manufacturer's recommendations. At 72 hours after transfection, cells were used for further experiments.

*Co*-*immunoprecipitation*, *native PAGE and immunoblot analysis*: 293T cells were seeded on 100-mm dishes at 1 × 10⁶ cells per dish. Cells were transfected with a total of 10 µg of empty plasmid or various expression plasmids using Lipofectamine 2000. At 36 hours after transfection, cells were lysed in M-PER buffer (PIERCE, IL) or 1% digitonin (Calbiochem, CA) buffer (20 mM Tris-HCl, 150 mM NaCl, 1% digitonin) containing protease inhibitors (Roche, IN). Lysates were incubated with HA affinity matrix (COVANCE, WI) or FLAG affinity beads (SIGMA) overnight. The beads were washed three times by TBS containing 0.05% Tween-20 and immunoprecipitates were eluted with non-reducing sample buffer by boiling for 5 min. For endogenous STING immunoprecipitation, HUVEC were lysed in 1% digitonin lysis buffer. Cleared supernatants were incubated with 10µg of anti-STING antibody, followed by incubation with immobilized protein G (PIERCE). The beads were washed four times by 1% digitonin lysis buffer and immunoprecipitates were eluted with SDS sample buffer by boiling for 5 min. Native PAGE and Immunoblotting was carried out as previously described (Balachandran, S., et al., J Immunol, 2007. 178(4): p. 2429-39).

*Generation of the STING knockout mice*: The linearized targeting vector was electroporated into E14.1 ES cells originated from 129SvEv strain, followed by the selection in G418. Targeted clones were screened by PCR. From 90 clones, 1 positive clone was identified. This ES clone was subjected to the generation of chimera mice by injection using C57BL/6J blastocysts as the host. The resulting male chimeras were further mated with C57BL/6J female mice for germline transmission. The heterozygous mice (F1 mice) were interbred to obtain wild-type, heterozygous and homozygous littermates (F2). The genotypes of the mice were determined by PCR. Animals were generated at the University of Miami School of Medicine Transgenic Core Facility. Mice were allowed to freely access to food and water and housed at an ambient temperature of 23°C and at a 12 h light/dark cycle. Animal care and handling was performed as per IACUC guidelines.

*Preparation of Dendritic Cells and Macrophages:* Bone marrow derived macrophage (BMDM) and dendritic cells (GM-DCs) were prepared from femurs and tibia isolated from wild-type and knockout mice. A single cell suspension (per mouse) was prepared and red blood cells were lysed. For GM-DCs, cells were cultured in DMEM supplemented with 10% FCS, 50 M 2-Mercaptoethanol, 100 U/ml Penicillin, 100 g/ml Streptomycin and GM-CSF (20ng/ml). For BMDM, cells were cultured in RPMI 1649 supplemented with 10% FBS, antibiotics, and 20% conditioned medium from NIH3T3 cells. Medium was replaced every two days. After 8 days, cells are used for experiments.

*IFN induced library construction*: To screen for interacting partners of STING we used the Yeast two Hybrid approach. A novel IFN-induced cDNA library from human cells was prepared. To construct this library, telomerase immortalized human fibroblast cells were treated overnight with 1000 units each of human IFN-α and -β to induce interferon dependent genes. Poly(A)+ RNA was extracted from the pelleted cells and confirmed to be of high quality. After confirmation, cDNA synthesis was carried out using 'BD POWERSCRIPT™' RT (Clontech). T his procedure allows tagging either side of the resultant first strand with adaptors containing distinct SfiI sites (A and B) for directional cloning. This also simultaneously ensures that the cDNA population is enriched for full length clones. The cDNA was then amplified by PCR, digested with SfiI and ligated to the yeast prey vector pGADT7-RecAB. The ligated mixture was transformed into *E. coli* strain DH10B. The number of independent clones in the unamplified library was estimated to be 3.2 × 10⁶, with an average size of 1.52 kb and inserts ranging in size from 0.8 to 3.0 kb. 15 independent colonies were isolated and DNA extracted to check for size by PCR.

*Yeast two-hybrid analyses*: hSTING-C (amino acids 173-379) sequence was amplified by PCR and was cloned into pGBK-T7 (Clontech) to generate a bait plasmid pGBK-T7-hSTING-C. 100µg of the IFN-induced library's plasmid DNA was used to perform a library-scale transformation of yeast strain Y187. Yeast library screens were performed by yeast mating. Briefly, pGBK-T7-hSTING-C was transformed into yeast strain AH109 and grown overnight in 50ml minimal medium lacking Tryptophan. A 1ml aliquot of pre-transformed library in Y187 was mixed with the above and yeast mating was allowed to proceed overnight before being plated. Positive colonies from the high-stringency plates were isolated and plasmid DNA extracted by the 'glass-bead' protocol. Briefly, yeast colonies were grown in minimal medium lacking Leucine overnight. 1.5ml of culture was spun down and the pellet was resuspended in 300µl of Yeast Breaking Buffer (2% Triton X-100, 1% SDS, 100mM NaCl, 10mM Tris 8.0, 1 mM EDTA) along with an equal volume of acid washed glass beads (Sigma Cat# G-8772). 300µl of Phenol: Chloroform (1:1, pH 8.0) was added and the tube was vortexed for at least 15 minutes. Samples were centrifuged and the aqueous phase was transferred to a new tube and the DNA was precipitated with 2 volumes of Ethanol. The recovered pellet was washed in 70% Ethanol, dried and dissolved in 50µl of Milli-Q water. 2-5µl were used to transform bacterial competent cells and plated on LB-Ampicillin plates. Individual colonies were grown and plasmid DNA extracted. Extracted DNA was used to re-transform yeast to reconfirm interactions and eliminate auto-activating false positives. Positive clones were sequenced for identification.

### Results and Discussion :

To further determine the mechanisms of innate immune signaling, an expression screening system was employed in which approximately 5,500 human and 9,000 murine full length cDNA's were individually transfected into 293T cells harboring a luciferase gene under control of the IFNβ promoter (IFNβ-Luc). Five genes whose overexpression lead to the induction of IFNβ-Luc approximately 250-fold but not control vectors was found to be IPS-1 (also referred to as VISA/CARDIF/MAVS), a putative CARD containing mitochondria protein and known inducer of type I IFN that plays a role in facilitating the RIG-I/MDA5 pathway. However, a previously uncharacterized molecule (gi:38093659/NP_938023/2610307008RIK) was also isolated by this method which is referred to herein, as STING (for STimulator of INterferon Genes) that harbored 5 predicted TM motifs (in humans) and existed as a 379 amino acid protein in human cells and 378 amino acids in murine cells (Figure 1A). A putative signal cleavage motif was found to exist at position 1-36 and a leucine rich region was apparent between amino acids 21-139 (Figures 1A, 1B). The predicted molecular weight of human STING (hSTING) was 42,192 Da, which approximately corresponded to its observed molecular weight in human 293 cells following immunoblot analysis using a rabbit antiserum raised to a STING peptide (Figure 1D). RNAi studies confirmed that the observed 42kDa band was indeed STING (Figure 1D). STING was found to be ubiquitously expressed in a variety of tissues as determined by northern analysis and was found to predominantly reside in the ER region of the cell as determined by confocal microscopy and fractionation analysis (Figures 1E, 1F).

**Table 2**

| **Sample Type** | **Gene Name** | **Fold Induction (Average)** | **Std Dev** | **Gene Description** |
|---|---|---|---|---|
| MAVS Control | MAVS Control | 276,55 | 44.81 | Control |
| Library Sample | MAVS/IPS-1 | 234.54 | 11.40 | MGC25836D430028G 21RIK |
| Library Sample | MAVS/IPS-1 | 221.91 | 1.44 | MGC25836.D430028G 21RIK |
| Library Sample | MAVS/IPS-1 | 221.89 | 3.74 | MC5C25836.D430028G 21RIK |
| Library Sample | MAVS/IPS-1 | 203.60 | 15.91 | MGC25866.D430028G 21RIK |
| Library Sample | MAVS/IPS-1 | 171.08 | 14.79 | KIAA127I |
| Library Sample | IRFI,IRF-1 | 152.22 | 13.42 | IRFI,IRF-1 |
| Library Sample | P65,RELA | 23.11 | 1.10 | P65,RELA |
| TRIF Control | TRIF Control | 22.52 | 11.51 | Control |
| Library Sample | STING | 19.03 | 0.75 | 2610307008RIK |

Table 2 shows the list of genes that induced the IFN-β promoter above 15-fold compared to a 'vector alone' control. The fold induction was calculated as an average of an experiment performed in duplicate. Approximately 5,500 human and 9,000 mouse full-length cDNAs cloned into pCMV-Sport6 vector (Invitrogen) generated by the Mammalian Gene Collection (MGC) were purchased from Open Biosystems (Huntsville, AL). To identify cDNAs that encode protein that alter IFNβ-luciferase reporter activity, 40 ng of empty, IPS-1 or TRIF cDNA positive control expression plasmids or cDNA library were aliquoted into designated control wells of the 384-well library screening plate. 35 ng of IFN reporter were dispensed into each well with a MultiDrop robot (TiterTek). After incubation, HEK293T cells were dispensed into each well. After 24 hrs, 40 µl of BriteLite luciferin reagent (Perkin Elmer) was added to each well with the MultiDrop, incubated for 1 minute at room temperature, and luminescence levels quantified with an Analyst GT multimode reader (Molecular Devices). All screens were performed in duplicate. This approach allowed for the isolation of new molecules, hitherto unknown, as regulators of the IFN pathway. Interestingly, the top 5 hits were IPS-1, which activated the IFNβ promoter significantly. Another top hit was IRF1 (150-fold). This data demonstrated that the screen was functional and was highly specific. Interestingly, a new molecule was isolated which is referred to herein as refer to as STING (STimulator of IFN Genes).

Overexpression of STING in 293T cells was subsequently confirmed to robustly induce the expression of the IFN promoter (IFNβ-Luc) up to 400-fold, but not a control TK promoter driving luciferase (pRL-TK), interferon regulatory factor 3 (IRF3) responsive promoters (PRD-III-I-Luc) up to 1000-fold, an NF-κB responsive promoter (NF-κB-Luc) 12-fold and interferon-inducible promoters (interferon sensitive response element- ISRE-Luc) up to 800-fold (Figures 2A-2D). STING did not activate control promoters driving luciferase reporters such as those derived from the Rb, p53 or E2F genes (Figures 5A-5D). Increased dimerization of IRF3 was also observed in STING expressing 293T cells confirming that STING regulates the induction of type I IFN at upstream of IRF3 activation, dimerization and translocation (Figure 2E). This data was verified by observing that endogenous IFNβ mRNA and IFNβ protein was induced significantly in MEFs transiently transfected with STING as determined by quantitative RT-PCR analysis and ELISA respectively (Figures 2F, 2G). DNA microarray analysis of STING expression in 293T cells further emphasized STING's ability to induce primary innate immune response genes as well as IFN-inducible genes (Figure 2H). Accordingly, MEFs cells expressing STING or IPS-1 were significantly resistant to VSV infection due to the induction of innate immune response genes (Figures 2I, 2J). Subsequent analysis indicated that STING function was ablated in the absence of the IκB kinase family member TBK-1, confirming that STING's activity involved activation of IRF3 and was indeed upstream of this kinase (Figure 2K). Finally, it was observed that STING did not exert robust activity in the absence of FADD, which is important for efficient innate immune signaling processes (Figures21). It was also established that while STING, HA tagged at the carboxyl region, retained activity, this activity was lost when STING was tagged at the amino terminus or carboxyl terminus with GFP. Subsequent analysis indicated that the amino terminus region of STING containing the 5 putative TM regions (amino acids 1-230) or just the carboxyl region of STING (amino acids 173-379) did not exhibit significant ability, alone, to induce the IFNβ-Luc promoter when transiently overexpressed in 293T cells (Figures 2M, 2N). Thus, full length intact STING is required for efficient function. However, it was further observed that the carboxyl region of STING exerted a dominant-negative inhibitory effect and could impede the ability of full-length STING to stimulate p110-Luc (Figure 20). Collectively, this data indicated that expression of STING activated the innate immune response including type I IFN leading to the induction of an antiviral state.

To further analyze STING function, an RNAi approach was utilized to ablate STING in a number of cell-types. The data indicated that knockdown of STING in HEK 293 cells, as confirmed by immunoblot analysis using an anti-STING antibody, modestly reduced the ability of the negative-stranded rhabdovirus VSV-GFP to induce IFNβ, presumably since these viruses are only weak activators of IFNβ (Figures 6A, 6B). However, while ablation of STING by RNAi in MEFs also lead to a slight reduction in IFNβ mRNA production in response to VSV-GFP, such cells were rendered extremely susceptible to virus infection and replication (Figures 3B-3D). To confirm a requirement for STING in the regulation of type I IFN induction and in host defense, STING deficient (STING ^{-/-}) mice were generated by targeted homologous recombination in ES cells (Figure 3E). Deletion of STING was confirmed by Southern and Northern analysis as well as by RT-PCR analysis and immunoblotting of STING -/- MEFs (Figures 3F, 3G). STING ^{-/-} animals were born at the Mendelian ratio and developed and bred normally. Accordingly, MEFs from wild type and STING ^{-/-} animals were infected with VSV-GFP at varying MOI's (0.01-1) for up to 36 hours post infection. This study confirmed that VSV-GFP generated more progeny virus (2 logs; 24-36 hours) in MEFs lacking STING compared to controls (Figures 3H, 3I). This data was verified using VSV expressing a luciferase reporter gene (Figures 7A-7E) and VSV-ΔM, which exhibits a defect in the viral matrix protein normally responsible for inhibiting cellular mRNA export from the nucleus (Figure 3J). VSVΔM replicates inefficiently in normal cells since it is incapable of preventing IFNβ mRNA export from the nucleus following infection, a consequence which results in the enhanced amplification of type I IFN transcription and antiviral activity. Reconstitution of STING to STING^{-/-} MEF's rescued the susceptibility to VSV infection (Figures 7A-7E). To evaluate whether loss of STING effected the production of IFNβ in response to negative-stranded viruses other than VSV, which is largely governed by the intracellular receptor helicase RIG-I, control or STING ^{-/-} MEFs were infected with the negative-stranded Sendai Virus (SeV), a member of the paramyxovirus family. This analysis indicated that loss of STING also reduced the ability of SeV to induce IFNβ (Figure 3K). In contrast, a strong requirement was not observed for STING to mediate the ability of transfected poly I:C to induce IFNβ induction, which is largely governed by the intracellular RIG-I homologue MDA5 (Figures 8A-8B). It was also observed that the positive-stranded encephalomyocarditis virus (EMCV), a member of the picornavirus family, did not effectively induce IFNβ in MEFs, regardless of the presence or absence of STING. In addition, no significant differences in viral titers were observed when comparing EMCV replication in infected STING ^{+/+} or ^{-/-} MEFs (Figures 8A-8B). Thus, it was concluded that STING may play a more predominant role in facilitating RIG-I mediated innate signaling rather than MDA5. Interestingly, a significant defect was not detected (>5-fold) in the ability of transfected B-form DNA (poly dA-dT or non CpG containing interferon stimulating DNA [ISD]) to induce IFNβ in MEFs lacking STING compared to controls (Figures 3L, 3M; Figures 9A-9E). TLR9 is considered to govern CpG DNA-mediated induction of IFNβ, but is not active in MEFs. Thus, it is plausible that STING may function in TLR9-independent, DNA-mediated induction of type I IFN. This effect was similarly observed in murine STING lacking bone marrow derived macrophages (BMDM) or bone marrow derived dendritic cells (GM-DC) cultured using granulocyte-monocyte colony stimulating factor (GM-CSF) (Figures 3N, 3O). However, no difference was observed in the ability of exogenous poly I:C or lipopolysaccharide (LPS) to induce IFNβ, when comparing STING ^{-/-} BMDM or GM-DC's to controls, events which depend on TLR 3 and 4 respectively (Figures 3N, 3O). While, loss of STING rendered MEFs highly susceptible to VSV-GFP, less susceptibility was observed following VSV-GFP infection of STING ^{-/-} GM-DC's or BMDM's, indicating that STING may be more important in facilitating negative-stranded virus-mediated innate signaling in fibroblasts (Figures 9A-9E). Collectively, the data would indicate that loss of STING leads to a defect in RIG-I mediated type I IFN induction but does not affect the TLR pathway. In addition, it is concluded that STING functions in the pathway utilized by B-form DNA to induce IFNβ.

To further examine the mechanisms of STING's function in innate signaling, it was attempted to determine if STING interacted with RIG-I and/or MDA5, which are putative innate immune signaling receptors for negative or positive stranded viral RNA respectively. As mentioned, recent data has indicated that RIG-I may recognize short, uncapped ssRNA species harboring 5'-triphosphates, generated by many negative-stranded viruses, such as VSV, while MDA5 may recognize longer RNA's such as those generated by EMCV as well as poly IC. Co-immunoprecipitation experiments in 293T cells principally indicated that FLAG-tagged RIG-I but not MDA5 could associate with HA-tagged STING in co-transfection experiments (Figure 4A). The binding of RIG-I to STING was augmented upon infection of 293T cells with SeV (Figure 4A). It was then confirmed in normal human umbilical vein endothelial cells (HUVECs) that endogenous RIG-I could associate directly, or indirectly as a complex, with endogenous STING (Figure 4B). This data indicate that STING seems to preferentially modulate the RIG-I, rather than the MDA5 regulated pathway. To extend this analysis, which region of RIG-I may associate with STING was next focused on. A smaller RIG-I construct was constructed comprising the CARD domains (amino acids 1-284) fused to a FLAG sequence. It was noted that the CARD domains of RIG-I, but not the carboxyl region of RIG-I (amino acids 218-925) were preferentially able to associate with HA-STING in transfected 293T cells (Figure 4C).

It was observed that transfected STING was able to modestly augment the ability of RIGI to activate the IFNβ promoter. However, it was noted that the carboxyl region of STING could inhibit the function of RIG-I, again indicating that this region of STING can exhibit a dominant-inhibitory effect (Figure 4D). RIG-I was subsequently shown to colocalize with STING in the co-transfected 293T cells (Figure 4E). The association of the RIG-I downstream adaptor IPS-1 with STING was noted although similar to the situation with RIG-I, it is not clear whether IPS-1 directly interacts with STING or exists as a complex with RIG-I/STING (Supplemental Fig 7). Accordingly, it was observed that STING was able to induce the induction of an IFNβ driven luciferase construct in MEFs lacking RIG-I or IPS-1, likely confirming that that STING functions downstream of these latter molecules (Supplemental Fig 8). The ability of ΔRIG-I or IPS-1 to induce IFNβ appeared diminished in STING -/- MEFs (Figure 4F). However, there was also a marked reduction in the induction of IFNβ by all transfected plasmids (including vector alone) in the absence of STING compared to control MEFs, since endogenous DNA innate signaling pathways are likely defective. Collectively, the data indicate that STING may be an important downstream adaptor molecule that facilitates RIG-I and perhaps IPS-1 function, as well as intracellular DNA-mediated innate signaling pathways.

To identify other proteins that may interact with STING and to shed further insight into the molecular mechanisms of STING action, a yeast-two hybrid approach was adopted where an IFN-induced human fibroblast cDNA library, developed by this laboratory, was screened using STING (amino acids 173-379) as a bait. Following this approach, it was determined that one molecule repeatedly found to associate with STING was Ssr2/TRAPβ, a member of the TRAP complex comprising four subunits (α-Δ) that facilitates translocation of proteins into the ER following translation (Figure 4G). The TRAP complex associates with the translocon, comprising three subunits, Sec61 α, β and γ. It was confirmed that TRAPβ can indeed associate with endogenous STING in HEK 293 cells following co-immunoprecipitation experiments (Figure 4H). Using this approach, it was confirmed that TRAPβ also co-immunoprecipitated with endogenous Sec61β, likely as a complex with TRAPβ (Figure 4H). It was next verified that STING could also associate not only with TRAPβ but also Sec61β (Figure 4I). This was achieved by co-transfecting FLAG-TRAPβ and HA-STING and immunoprecipitating with an HA antibody. Immunoblot analysis with an antibody to FLAG confirmed association of STING and TRAPβ. An antibody to endogenous Sec61β confirmed association with the STING/TRAPβ complex (Figure 4I). STING was also observed to co-localize with TRAPβ in the ER region of the cell (Figure 4K). Taken together, this data would indicate that the STING may be involved in translocon function and that the translocon may be able to influence the induction of type I IFN. It was therefore evaluated whether loss of TRAPβ or Sec61β eliminated STING function and/or affected type I IFN production using an RNAi approach to ablate these translocon molecules from 293T cells. Loss of TRAPβ or SEC61β was confirmed by quantitative RT-PCR or immunoblot and did not affect cell viability (Figures 12A-12C). This data indicated that loss of TRAPβ or SEC61β reduced STING's, ability to induce an IFNβ promoter driving luciferase (Figure 4J). Collectively, this data provides novel information indicating that the translocon may be involved in innate immune signaling processes in the cell. Thus, STING is predominantly an ER resident protein that may link RIG-I and DNA-mediated intracellular innate signaling to the translocon. Speculatively, RIG-I may innate immune signaling receptors for negative or positive stranded viral RNA detect translating viral RNA's at the intersection of ribosome/ER translocon association and require STING to exert its function. Alternatively, STING may participate in mediating ER stress response pathways an event that remains to be verified. While it is not clear how signaling from the translocon to IRF-3/NF-kB occurs, translocon functionally and physically associates with the exocyst - the octomeric Sec6-Sec8 complex that also associates with the ER and tethers secretory vesicles to membranes, in preparation for membrane fusion, and facilitates protein synthesis and secretion. Recently, the exocyst complex was found to recruit and activate TBK1 and play a role in type I IFN-β induction. The preliminary analysis herein, indicates that STING also co-immunprecipitates with TBK1 and that RNAi ablation of Sec5 also rendered cells defective in the STING function (Figure 4L). Thus, STING may facilitate the detection of intracellular viral RNA species as well as B-form DNA indicating convergence of these PAMP recognition pathways. While these mechanisms need to be clarified it is evident that a number of infectious agents and toxins target the ER. Accordingly, the ER has evolved to recognize and respond to these cellular stresses which include mediating innate immune signaling in the cell following infection.

### Example 2: STING Regulates Intracellular DNA-Mediated, Type-I Interferon-Dependent Innate Immunity

### Methods:

*Mice, cells, viruses and reagents:* Mutant mice lacking STING on a 129SvEv x C57BL/6J background, has been described previously (Ishikawa, H. & Barber, G.N. Nature 455, 674-678 (2008)). MEFs, bone marrow-derived macrophage, and GM-CSF-induced dendritic cells (GM-DCs) were prepared as described previously (Ishikawa, H. & Barber, G.N. Nature 455, 674-678 (2008)). To prepare FLT3 ligand-induced DCs (FLT3-DCs), bone marrow cells were cultured in RPMI 1640 medium supplemented with 10% FBS, 50µM 2-Mercaptoethanol, 10mM HEPES (pH7.4), and 100 ng/ml human FLT3 ligand (Peprotech) for 8 days. 293T cells were obtained from the ATCC and were maintained in DMEM medium supplemented with 10% FBS. VSV (Indiana strain) and EMCV were described previously (Ishikawa *et al.,* 2008)). HSV-1 (KOS strain) and *Listeria monocytogenes* (10403 serotype) were obtained from ATCC. Genomic DNA was obtained from following sources: HSV-1, HSV-2, adenovirus type 5 (ATCC); *E. coli,* Calf thymus (Sigma), poly(dA:dT) and poly(I:C) were obtained from Amersham biosciences. poly(dG:dC) and poly(dA) were obtained from Sigma. CpG ODN (ODN 1585) was obtained from Invivogen. For stimulation of cells, genomic DNA, polydeoxynucleotides or poly(I:C) were mixed with Lipofectamine 2000 (Invitrogen) at a ratio of 1:1 (vol/wt), and then added to cells at a final concentration of 1 µg/ml.

*Plasmids:* Yellow fever virus NS4B sequence was amplified by PCR using pYFM5.2 encoding the complete YFV-17D sequence as template, and was cloned into pcDNA3 (Invitrogen) plasmid to generate C-terminally HA-tagged expression construct. The expression plasmid containing chicken ovalbumin (OVA) cDNA was constructed by cloning of PCR-amplified OVA cDNA into pCDNA3. Expression plasmids encoding HA-tagged murine STING (mSTING-HA), FLAG-tagged ΔRIG-I (amino acids 1-284) and ΔMDA5 (amino acids 1-284) were described previously (Ishikawa *et al.,* 2008)). p110-Luc (IFNβ-Luc) was obtained from T. Maniatis. pUNO-hsaIRF3 (IRF3SA) and pUNO-hsaIRF7Δ (IRF7SA) were obtained from Invivogen. pCMV-SPORT6 containing murine DAI was obtained from Open Biosystems.

*Antibodies and ELISA*: Rabbit polyclonal antibody against STING was described previously (Ishikawa *et al.*, 2008)). Other antibodies were obtained from following sources: Calreticulin (ab14234; Abcam), Sigma1 receptor ([Sigma1R] ab53852; Abcam), TBK1 (EP611Y, Abcam), COX IV (ab16056, Abcam), rabbit polyclonal HA (ab9110; Abcam), Transferrin receptor ([TfR] H68.4; Invitrogen), mouse monoclonal HA (Sigma), FLAG (M2; Sigma), IRF3 (ZM3; Zymed), TGN46 (ab16059; Abcam), Giantin (ab24586; Abcam), EEA1 (#2441; Cell signaling), LAMP1 (NB120; Novus Biologicals), SEC61β (Upstate). ELISA kits were obtained from following sources: Murine IFN-β and IFN-α (PBL), murine IL-6 (R&D systems or Quansys biosciences), murine IFN-γ (R&D systems), murine RANTES (Quansys biosciences).

*RT-PCR:* Fluorescence real time PCR analysis was performed using a LightCycler 2.0 instrument (Roche Molecular Biochemicals, Indianapolis, IN) and the following TaqMan Gene Expression Assays (Applied biosystems): IFNβ (Mm00439546_s1), IFNα2 (Mm00833961_s1) and TRAPβ (Mm00481383_m1). Relative amounts of mRNA were normalized to the 18S ribosomal RNA levels in each sample.

*Reporter analysis*: 293T cells seeded on 24-well plates were transiently transfected with 50 ng of the luciferase reporter plasmid together with a total of 600 ng of various expression plasmids or empty control plasmids. As an internal control, 10 ng of pRL-TK was transfected simultaneously. Then, 24 hours later, the luciferase activity in the total cell lysate was measured.

*DNA vaccine:* Mice were immunized with a plasmid encoding OVA by intramuscular (i.m.) electroporation (100 µg per mouse). The booster immunization was given by 2 or 4 weeks after the primary immunization.

*Measurement of OVA-specific immune response*: Spleens were extracted 2 weeks after the second immunization and 5 × 10⁵ spleen cells were seeded on 96-well plates and then stimulated with synthetic peptide for OVA (H-2Kb SIINFEKL, Proimmune) at 10 µg/ml. After 3 days, the cell culture supernatants were collected and analyzed for the IFN-γ titer by ELISA (R&D systems). For intracellular IFN-γ staining, stimulated splenocytes were stained using FITC-labeled anti-CD8 antibody (BD). After washing, cells were fixed and permeablized. Then cells were stained using PE-labeled anti-IFN-γ antibody (BD). Flow cytometric analysis was performed on s FACScaliber instrument (BD). The serum anti-OVA antibody titer was measured by ELISA. Briefly, 96-well plates were coated with a OVA protein at 1 µg/ml and then were blocked with PBS containing 5% skimmed milk. Plates were washed and overlaid with serially diluted serum for 1 h at room temperature. After washing, antibodies were detected using goat anti-mouse IgG conjugated to horseradish peroxidase (Jackson Immuno Research). After an additional washing, the plates were stained using 3,3',5,5'-tetramethylbenzidine (TMB, Sigma) as a substrate. The reaction was stopped with 1M H₂SO₄ and the absorbance was measured. Antibody titers were expressed as the reciprocal of the endpoint dilution following background subtraction.

*Fractionation*: MAM, mitochondria, and microsomes were isolated from *Sting* ^{-/-} MEFs stably transfected with mSTING-HA plasmid. Briefly, cells were washed in phosphate-buffered saline (PBS) and pelleted by centrifugation at 1,000 x g for 10 min. The pellet was resuspended in sucrose homogenization buffer (0.25 M sucrose, 10 mM Hepes [pH 7.4]), and cells were lysed by using a dounce homogenizer. Lysed cells were centrifuged at 500g for 10 min, and the supernatant was collected. The supernatant was then centrifuged at 10,300g for 10 min to separate the crude microsomal (microsome and cytosol) from the crude mitochondrial (MAM and mitochondria), and the crude microsomal fraction (supernatant) was subjected to an ultracentrifugation at 100,000g for 60 min. The crude mitochondrial fraction (pellet) was resuspended in ice-cold mannitol buffer A (0.25M mannitol, 5 mM Hepes, 0.5 mM EDTA) and layered on top of a 30% Percoll in mannitol buffer B (0.225M mannitol, 25 mM Hepes, 1 mM EDTA). Mitochondria and MAM fractions were separated by ultracentrifugation at 95,000g for 65 min. Both isolated fractions were diluted with mannitol buffer B and were centrifuged at 6,300g for 10 min. The supernatant of MAM centrifugation was further separated by centrifugation at 100,000g for 60 min and the pellet was used for the MAM fraction, while the pellet of the mitochondria centrifugation was used as the mitochondria fraction. All of the fractions were resuspended in mannitol buffer B.

*Confocal microscopy*: For localization of Sec5 and LAMP1, cells grown on cover-slips were fixed in 80%/20% methanol/acetone at -20°C for 5 min. For EEA1 staining, cells were fixed with 4% paraformaldehyde in PBS for 15 min at 37°C, and were permeabilized in 0.2% Triton-X100. For staining of other proteins, cells were fixed by 4% formaldehyde in DMEM for 15 min at 37°C, and were permeabilized by 0.2% Triton-X100. For mitochondria staining, living cells were incubated with 300 nM of Mito Tracker Red (Invitrogen) for 45 min at 37°C. Fixed and permeabilized cells were preincubated with 0.1% BSA in PBS and were incubated with primary antibodies. Cells were then incubated with secondary antibodies conjugated with FITC, Cy3 or Cy5 (Sigma).

*RNA interference:* Chemically synthesized 21-nucleotide siRNA duplexes were obtained from Dharmacon, Inc (Lafayette, CO). The sequences of each siRNA oligonucleotide used in this study are follows: murine TRAPβ siRNA, 5'-UGAAAGAGAGGACGGGUUAUU-3' (SEQ ID NO: 6); murine Sec5 siRNA, 5'-AGAAGUAUUAGGUCGGAAA-3' (SEQ ID NO: 7), 5'-UCAACGUACUUCAGCGAUU-3' (SEQ ID NO: 8), 5'-CAGCAGAGAUUACACGUCA-3' (SEQ ID NO: 10), 5'-GUGAGUGGCUUGCGCAGUA-3' (SEQ ID NO: 11). MEFs were transfected by using an Amaxa nucleofector apparatus (program A-023) and Amaxa MEF nucleofector kit 1 according to the manufacturer's instructions. At 72 h after transfection, cells were used for further experiments.

### Statistics: Students t-test was used to analyze data.

In the previous example, Example 1, demonstrated for the first time a role for STING (also referred to as MPYS/MITA), an endoplasmic reticulum (ER) resident transmembrane protein, in facilitating the production of type I IFN in murine embryonic fibroblasts (MEFs) response to intracellular B-form DNA (Jin, L. et al. Mol Cell Biol 28, 5014-5026 (2008); Zhong, B. et al. Immunity 29, 538-550 (2008)). To extend the analysis into evaluating the importance of STING in mediating DNA-induced innate immune responses, WT ^{(+/+)} or *Sting* ^{*(-*/*-)*} low passage number MEFs and compared the induction of type I IFN (IFNβ) in response to a variety of DNA ligands. These results indicated that STING was essential for inducing IFNβ in response to transfected viral DNA (Adenovirus, Ad5; Herpes simplex virus, HSV-1 and 2), purified *E. coli* DNA; calf thymus DNA, CT DNA; and interferon stimulatory DNA, ISD (Figure 13A). Complete abrogation of IFNβ production was also observed following transfection of poly dGC:dGC) in *Sting* ^{-/-} MEF's and slight IFNβ production was observed using poly dAT:dAT likely due to STING-independent, RIG-I dependent signalling. Loss of STING did not significantly affect poly(IC)-mediated type I IFN production, which is largely governed by MDA5. Concomitant analysis further indicated a dramatic reduction in IL-6 production in STING lacking MEFs compared to controls following similar DNA transfections (Figure 13A). ISD mediated production of *Ifn*β and *Ifn2a* mRNA was not detectable in STING lacking MEFs compared to controls (Figure 13B). Translocation of IRF3 or IRF7 was thus not observed in ISD transfected STING lacking MEFs indicating that STING likely functions in mediating intracellular DNA triggered IFN production upstream of TBK-1 (Figure 13C). NF-κB signaling was also moderately defective in *Sting*

^{-/-} MEFs following exposure to transfected ISD. Collectively, these data indicate that STING plays an essential role in the recognition of intracellular DNA in fibroblast type cells. Given this, the importance of STING in facilitating intracellular DNA-mediated production of type I IFN in antigen presenting cells including macrophages, was examined. This analysis indicated that *Sting* ^{*(-*/*-)*} macrophages transfected with ISD, or infected with the DNA pathogens HSV-1 or *Listeria monocytogenes* were greatly defective in their ability to manufacture type I IFN (Figure 13D). However, the cleavage of pro-caspase 1 and production of active IL-1β, which is AIMII dependent, was unaffected by the loss of STING (Figure 13E). Thus, STING is essential for the production of IFN in response to intracellular DNA, though functions independent of the AIMII 'inflammasome' pathway. Further analysis similarly indicated that STING was required for efficient DNA-mediated production of type I IFN in GMDC's, as well as plasmacytoid derived DC's (Flt3-DC) (Figures 13E, 13F). However, exogenous CpG DNA remained able to induce type I IFN in Flt-DC's lacking STING compared to controls, indicating that TLR9 functions independent of the STING pathway (Figure 13F). The induction of IL-6 in response to intracellular DNA was also eliminated, in *Sting* ^{(-/-)} macrophages (Figure 17). However, HSV-1 and CpG DNA was able to induce IL-6 (Figure 17), but not type I IFN in macrophages devoid of STING (Figure 13D), likely in a TLR9-dependent manner. STING thus appears important for intracellular DNA-mediated production of Type I IFN in fibroblasts, macrophages, conventional DC's as well as pDC's. Finally, it was further noted that STING was required for the efficient production of type I IFN by cytomegalovirus (CMV), vaccinia virus (VVΔE3L) and baculovirus. STING thus appears critical for intracellular DNA-mediated production of Type I IFN in fibroblasts, macrophages, conventional DC's as well as pDC's.

Since a significant defect in the production of type I IFN in STING lacking cells, following infection using HSV-1 was observed, the *in vivo* importance of STING in facilitating effective host defense against virus infection was evaluated. Thus, *Sting* ^{(-/-)} or control mice were infected intravenously with HSV-1 and survival monitored. This study indicated that animals lacking STING died within 7 days of HSV-1 infection (Figure 14A). In contrast, 80% of similarly infected WT mice survived. Significant amounts of HSV-1 were detected in the brain of infected *Sting* ^{(-/-)} mice, but not in controls at 5 days post-infection (Figure 14B). Analysis of serum from the *Sting* ^{(-/-)} infected animals indicated a profound defect in the production of type I IFN, at 6 hours post infection, compared to infected control animals suggesting that IFN is responsible for preventing HSV-1 from reaching regions of the central nervous system including brain (Figures 14C, 14D). RANTES and IL-6 levels were similarly dramatically reduced in STING lacking animals at the same time point (Figure 14E, 14F). *Sting* ^{*-*/*-*} mice were also found to be more sensitive to HSV-1 following intravaginal administration of HSV-1. This data indicated that STING is important for the effective production of type I IFN and is essential *in vivo* for protection against HSV-1 infection.

Since it was observed that there is an *in vitro* defect in the ability of the negative-stranded virus vesicular stomatitis virus (VSV) to induce type I IFN in the absence of STING, the in vivo importance of STING in protecting against VSV-related disease was examined. It was observed that *Sting* ^{-/-} animals infected with VSV was also significantly sensitive to lethal infection compared to controls (Figure 14G). Defects in type I IFN production were seen in STING lacking animals at early time points (6 hours), though less so at 24 hours (Figures 14H, 14I and Figures 18A-18F). Thus, STING is required for efficient, early induction of type I IFN production and is required for protection against the negative-stranded virus, VSV infection, possibly through regulating the RIG-I/IPS-1 pathway.

Encephalomyocarditis virus (EMCV), a positive-stranded virus of the flavivirus family or synthetic poly(IC) induces the induction of type I IFN through triggering MDA5 activity. A requirement for STING was not observed in facilitating poly(IC) activity indicating that STING may not influence MDA5 function (Figure 13A). However, STING knockout mice were confirmed to be slightly more sensitive to lethal EMCV infection compared to controls indicating that STING may exert some influence on host defense pathways triggered by these viruses (Figure 14J). Nevertheless, significant defects were not observed in type I IFN production in *Sting* ^{*(-*/*-)*} mice compared to controls following EMCV infection (Figures 18A-18F). However, it is known that some flaviviruses such as hepatitis C virus (HCV) can activate the RIG-I pathway, signaling which is facilitated by STING. Interestingly, data bank analysis indicated that the flaviviruses yellow fever virus (YFV) and Dengue virus (DV) encoded a product NS4b, that exhibited strong homology with the amino terminus of STING (amino acids 125- 222) [Figures 19A-19C]. The importance of this region in STING activity was emphasized since loss of amino acids 153-173 eliminated STING function. Various flavivirus NS4b products have been shown to localize in the ER of the cell and to suppress the induction of type I IFN, although the mechanisms remain unclear. Given these observations, it was next determined whether YFV NS4b can interfere with STING's function. These experiments indicated that NS4b was able to inhibit, not only STING's ability to induce type I IFN, but also RIG-I and MDA5, and VSVΔM (Figure14K). The effect was not due to NS4b inducing apoptosis. YFV NS4b was shown to co localize with STING and was subsequently found to directly associate

with STING, preventing efficient STING function (Figures 18A-18F). These data indicate that STING is essential for effective type I IFN production in response to select DNA viruses as well as the negative-stranded virus VSV. Moreover, STING may be targeted by select flaviviruses for suppression.

STING partially exerts its function through the non-canonical IkB kinase TBK-1, which triggers interferon-regulatory factor 3 (IRF3) dependent stimulation of type-I IFN4. TBK-1 plays an important role in mediating the adjuvant activity of DNA vaccines *in vivo.* TBK-1 activation in response to plasmid DNA was found to occur in the absence of the DNA sensors, TLR9 or DAI/ZBP-1, indicating that other pathways exist to facilitate DNA-mediated immunization. To evaluate if STING played a role in this signaling pathway, STING^{-/-} or control mice were immunized with plasmid DNA encoding the ovalbumin gene. While normal B and T cell subsets were noted in unstimulated STING -/- animals, following immunization *Sting* ^{-/-} mice exhibited significantly less serum ovalbumin (OVA) specific immunoglobulin (Ig)G's compared to controls. (Figure 15A). In addition, spleen CD8⁺ T-cell frequency and IFN-γ secretion was markedly reduced in *Sting* ^{*-*/*-*} mice following immunization, compared to wild type mice (Figures 15B, 15C). Since immunoglobulin responses to OVA peptide were normal, these data emphasized that the STING-governed DNA sensor pathway is essential for efficient DNA vaccine-induced T-cell responses to antigen. Given this information, it was next evaluated whether STING played a role in facilitating T-cell responses following infection with the DNA virus vaccinia that expresses ovalbumin (VV-OVA). This study indicated that control mice, but not *Sting* ^{*(-*/*-)*} mice elicited strong T-cell responses to viral encoded OVA, verifying the importance of STING in innate immune signaling processes required for DNA adjuvant activity.

STING is a predominantly an ER resident protein that can associate with RIG-I and the mitochondrial innate immune signaling adaptor IPS-1, events that may explain STINGs importance in facilitating negative-stranded virus mediated type-I IFN production. STING associates with the translocon-associated protein complex (TRAP) in the ER. Physical association of mitochondria and the ER, referred to as mitochondria -associated ER membrane (MAM), is important for transmission of Ca²⁺ to the mitochondria and for oxidative metabolism. The next experiments examined whether STING could associate with MAM's, interactions that may help explain IPS-1/STING interactions. First, HA-tagged STING was reconstituted into *Sting* ^{*(-*/*-)*} MEFs, to follow endogenous STING localization using HA antibody. This analysis confirmed that STING is predominantly associated with the ER as determined by calreticulin marker co-staining (Figure 16A). Mitotracker co-staining also indicated that STING may co-localize with mitochondria associated with the ER (Figure 13B). Fractionation analysis subsequently indicated that STING is associated with microsomes, a complex of continuous membranes that comprise the ER, Golgi and transport vesicles. Endogenous STING was also found to fractionate with MAM and mitochondria fractions under unstimulated conditions in MEF's (Figure 14C). Calreticulin, known to be a chaperone involved in regulating the association of the ER and mitochondria, was observed to fractionate similarly. This data may indicate that in the case of VSV infection, STING could associate with IPS-1 through MAM interaction, to facilitate RIG-I signaling. Interestingly, following HSV-1 infection, STING was observed to become predominantly associated only with microsome fractions only (Figure 14C). To clarify these observations, HA-STING MEFs were infected with HSV-1 or transfected these cells with stimulatory ISD or negative-control ssDNA. These results indicated that in response to HSV-1 infection or ISD transfection, STING translocated from the ER and predominantly congregated to perinuclear, non-ER microsome compartments in the cell (Figure 16D). That STING trafficked from the ER and MAM fractions was confirmed by staining cells with calreticulin and Mitotracker markers (Figure 20). Brefeldin A but not chloroquine blocked STING trafficking indicating that STING locates from the ER via the Golgi to vesicles in the perinuclear region. This trafficking, in response to intracellular DNA, was similarly observed for TBK-1 which associates with STING. Significantly, in the absence of STING, TBK-1 failed to relocate to perinuclear regions in response to ISD transfection. Loss of STING's predicted 36 amino acid amino terminal signal peptide sequence eliminated STING trafficking and function, emphasizing the importance of this region in STING activity. Collectively, it is plausible that STING translocates as a complex with TBK-1 (Figure 16E).

To evaluate the sub-cellular localization of STING after DNA mediated intracellular stimulation, antibodies that recognize molecules associated with specific cellular organelles were used. It was observed that in the presence of DNA, STING most predominantly localized with the early endosome marker protein EEA1 and recycling endosome marker TfR (Figure 16F and Figure 20). TBK-1, which trafficked similarly in response to intracellular DNA, has also been demonstrated to associate with Sec 5, a component of the exocyst 8 subunit complex that facilitates vesicular transport processes. Upon intracellular DNA stimulation, STING was found to strongly co-localize with Sec5, which has also been demonstrated to associate in perinuclear endosome compartments (Figure 16G). The RalB/Sec5 pathway is required for efficient Sendai Virus (SV)-mediated type I IFN production. However, the data herein indicates that STING/TBK-1 complexes may traffic to endosome compartments to associate with Sec5/exocyst components and facilitate the production of type I IFN in response to intracellular DNA. To evaluate whether Sec5 also modulates the production of IFNβ in response to ISD, Sec5 production in normal MEF's was suppressed using RNAi (Figures 21A, 21B). This study indicated that in the absence of Sec5, ISD-mediated IFN production was significantly impaired (Figure 16G). A similar effect was observed upon knockdown of TRAPβ and Sec61β, components of the TRAP complex. The data thus indicates that intracellular DNA may induce STING to complex with TBK-1 and traffic to Sec5 containing endosome compartments, events that facilitate the production of type I IFN.

In conclusion, it was demonstrated that STING is essential for the recognition of intracellular DNA and efficient production of type I IFN in all cell types examined. Loss of STING renders mice susceptible to lethal DNA virus infection (HSV-1). However STING also facilitates host defense responses to negative-stranded viruses such as VSV, plausibly through RIG-I and IPS-1/MAM-ER translocon interactions. While STING-independent, VSV-mediated type I IFN-induction pathways exist, they do not appear sufficient on their own to protect mice against lethal VSV infection. It was concluded that in response to intracellular DNA, STING/TBK-1 complexes traffic to endosomal compartments to associate with exocyst components including Sec5, resulting in the induction of type I IFN. These events are impeded by select viruses such as YFV, implicating a new mechanism of viral suppression of host defense.

Although the invention has been illustrated and described with respect to one or more implementations, equivalent alterations and modifications will occur to others skilled in the art upon the reading and understanding of this specification and the annexed drawings. In addition, while a particular feature of the invention may have been disclosed with respect to only one of several implementations, such feature may be combined with one or more other features of the other implementations as may be desired and advantageous for any given or particular application.

The Abstract of the disclosure will allow the reader to quickly ascertain the nature of the technical disclosure. It is submitted with the understanding that it will not be used to interpret or limit the scope or meaning of the following claims.

The invention further relates to the following embodiments:
Embodiment 1: An isolated nucleic acid sequence encoding a protein or peptide comprising a sequence as set forth in SEQ ID NOS: 1 or 2, or an active fragment or variant thereof that modulates an immune response.
Embodiment 2: The isolated nucleic acid sequence of embodiment 1, wherein the active fragment is of at least 380 consecutive amino acid residues in length in humans and induces or enhances an immune response in vivo or in vitro.
Embodiment 3: The isolated nucleic acid sequence of embodiment 1, wherein expression of SEQ ID NOS: 1 or 2 or active fragments thereof, induce type I IFN in a cell, tissue or organ, in vivo or in vitro.
Embodiment 4: The isolated nucleic acid sequence of embodiment 1, wherein expression of SEQ ID NOS: 1 or 2 in vivo or in vitro induce an antiviral immune response.
Embodiment 5: A peptide, polypeptide or protein encoded by an isolated nucleic acid set forth as SEQ ID NOS: 1 or 2, or an active fragment or variant thereof.
Embodiment 6: The peptide, polypeptide or protein of embodiment 5, wherein the peptide is at least 380 consecutive amino acid residues in length.
Embodiment 7. The peptide of embodiment 5, wherein one or more amino acid molecules are optionally substituted with a non native molecule.
Embodiment 8: An immunoregulatory composition comprising a peptide, polypeptide or protein encoded by an isolated nucleic acid set forth as SEQ ID NOS: 1 or 2, or an active fragment or variant thereof.
Embodiment 9: The immunoregulatory composition of embodiment 8, wherein the protein or active fragment of peptide is linked or fused to a second domain.
Embodiment 10: The immunoregulatory composition of embodiment 9, wherein the second domain comprising at least one of: an antigen binding domain, a hormone, an enzyme, a cytokine, toxin, radioactive molecule, a detectable molecule, a membrane translocation sequence, a signal peptide, an organic molecule or inorganic molecule.
Embodiment 11: A vector comprising a polynucleotide set forth as SEQ ID NOS: 1, 2, variants, mutants or active fragments thereof.
Embodiment 12: The vector of embodiment 11, wherein expression of SEQ ID NOS: 1, 2, variants, mutants or active fragments thereof, induce responses in vivo or in vitro comprising at least one of: antiviral activity, immune responses, immune signaling or intracellular B-form DNA.
Embodiment 13: An isolated cell comprising nucleic acid sequence set forth as SEQ ID NOS: 1 or 2, or an active fragment or variant thereof.
Embodiment 14: The isolated cell of embodiment 13, wherein the nucleic acid sequence set forth as SEQ ID NOS: 1 or 2, or an active fragment or variant thereof is expressed in the cell.
Embodiment 15: The isolated cell of embodiment 13, wherein the cell is a mammalian cell.
Embodiment 16: A vaccine comprising a nucleic acid sequence set forth as SEQ ID NOS: 1 or 2, or an active fragment, variant, or mutant thereof.
Embodiment 17: A vaccine comprising a peptide, polypeptide or protein encoded by a nucleic acid sequence set forth as SEQ ID NOS: 1 or 2, an active fragment, variant, or mutant thereof.
Embodiment 18: A method of modulating an immune response in vivo, comprising: administering to a patient in need thereof, an immunoregulatory composition in a therapeutically effective amount; and, modulating the immune response.
Embodiment 19: The method of embodiment 18, wherein the immunomodulatory composition comprises a nucleic acid sequence set forth as SEQ ID NOS: 1 or 2, an active fragment, variant, or mutant thereof.
Embodiment 20: The method of embodiment 18, wherein the immunomodulatory composition comprises a peptide, polypeptide or protein encoded by a nucleic acid sequence set forth as SEQ ID NOS: 1 or 2, an active fragment, variant, or mutant thereof.
Embodiment 21: A method of preventing or treating a disease or disorder in a subject in need thereof, comprising: administering to a subject an immunoregulatory composition in a therapeutically effective amount; and, preventing or treating a disease or disorder in a subject.
Embodiment 22: The method of embodiment 21, wherein the immunomodulatory composition comprises a nucleic acid sequence set forth as SEQ ID NOS: 1 or 2, an active fragment, variant, or mutant thereof.
Embodiment 23: The method of embodiment 21, wherein the immunomodulatory composition comprises a peptide, polypeptide or protein encoded by a nucleic acid sequence set forth as SEQ ID NOS: 1 or 2, an active fragment, variant, or mutant thereof.
Embodiment 24: The method of embodiment 21 wherein the disease or disorder comprising: immune disorders, autoimmunity, cancer, inflammation, cachexia, neurodegenerative disease or disorders, neurological diseases or disorders, cardiac dysfunction, transplantation, or infection.
Embodiment 25: A nonhuman transgenic mammal whose genome lacks a functional STING gene as set forth in SEQ ID NOS: 1 or 2, species variants, or mutants thereof.
Embodiment 26: The transgenic nonhuman mammal of embodiment 25, wherein the nonhuman mammal is a mouse.
Embodiment 27: A monoclonal or polyclonal antibody specific for one or more antigenic epitopes of a peptide, polypeptide or protein encoded by a nucleic acid sequence set forth as SEQ ID NOS: 1 or 2, an active fragment, variant, or mutant thereof.
Embodiment 28: A method of identifying therapeutic agents comprising: contacting: (i) a peptide encoded by nucleic acid sequence set forth as SEQ ID NOS: 1 or 2, an active fragment, variant, or mutant thereof; or, (ii) a nucleic acid sequence (Sting) set forth as SEQ ID NOS: 1 or 2, an active fragment, variant, or mutant thereof, with a candidate therapeutic agent; determining whether (i) the agent modulates a function of the peptide or interaction of the peptide with a partner molecule; or (ii) the agent modulates expression and/or function of the nucleic acid sequence (Sting); and, identifying a therapeutic agent.
Embodiment 29: The method of embodiment 28, wherein the therapeutic agent inhibits or increases a function of the peptide or nucleic acid sequence (Sting) as compared to a baseline control.
Embodiment 30: The method of embodiment 28, wherein the peptide or nucleic acid sequence inhibits or increases an immune response as compared to a baseline control.
Embodiment 31: The method of embodiment 28, wherein a partner molecule comprises at least one or more molecules comprising: RIG-I, Ssr2, TRAP, SEC61, NK-kB, TBK, IPS-1, MAM components, exocyst components, Major Histocompatibility complex (MHC) class I and II components, GARP components, translocon components, endosome components or IRF3, IRF7.
Embodiment 32: An adjuvant comprising: (i) a peptide encoded by nucleic acid sequence set forth as SEQ ID NOS: 1 or 2, an active fragment, variant, or mutant thereof; or, (ii) a nucleic acid sequence (Sting) set forth as SEQ ID NOS: 1 or 2, an active fragment, variant, or mutant thereof.
Embodiment 33: A method of modulating a DNA-induced innate immune response in a patient in need thereof, comprising: administering to the patient, a therapeutically effective amount of: (i) a peptide encoded by nucleic acid sequence set forth as SEQ ID NOS: 1 or 2, an active fragment, variant, or mutant thereof; or, (ii) a nucleic acid sequence (Sting) set forth as SEQ ID NOS: 1 or 2, an active fragment, variant, or mutant thereof; and, modulating a DNA-induced innate immune response in a patient.
Embodiment 34: A method of treating a viral infection in a patient comprising: administering to the patient, a therapeutically effective amount of: (i) a peptide encoded by nucleic acid sequence set forth as SEQ ID NOS: 1 or 2, an active fragment, variant, or mutant thereof; or, (ii) a nucleic acid sequence (Sting) set forth as SEQ ID NOS: 1 or 2, an active fragment, variant, or mutant thereof; and, treating a viral infection in a patient.
Embodiment 35: An oligonucleotide comprising at least five consecutive bases complementary to a nucleic acid sequence set forth as SEQ ID NOS: 1 or 2, wherein the oligonucleotide specifically hybridizes to and modulates expression and/or function of STING in vivo or in vitro.
Embodiment 36: The oligonucleotide of embodiment 35, comprising combinations of phosphorothioate internucleotide linkages and at least one internucleotide linkage selected from the group consisting of: alkylphosphonate, phosphorodithioate, alkylphosphonothioate, phosphoramidate, carbamate, carbonate, phosphate triester, acetamidate, carboxymethyl ester, and/or combinations thereof.
Embodiment 37: The oligonucleotide of embodiment 35, wherein the oligonucleotide optionally comprises at least one modified nucleobase comprising, peptide nucleic acids, locked nucleic acid (LNA) molecules, analogues, derivatives and/or combinations thereof.
Embodiment 38: The oligonucleotide of embodiment 35, wherein the oligonucleotide comprises a modified sugar moiety comprising a 2'-O-methoxyethyl modified sugar moiety, a 2'-methoxy modified sugar moiety, a 2'-O-alkyl modified sugar moiety, or a bicyclic sugar moiety.
Embodiment 39: A biomarker comprising a nucleic acid sequence set forth as SEQ ID NOS: 1 or 2, fragments, variants, or mutants thereof.
Embodiment 40: A biomarker comprising a peptide, polypeptide or protein encoded by a nucleic acid sequence set forth as SEQ ID NOS: 1 or 2, fragments, variants, or mutants thereof.
Embodiment 41: An antisense oligonucleotide comprising any one of SEQ ID NOS: 3 to 5.
Embodiment 42: The antisense oligonucleotide of embodiment 41, wherein anyone of SEQ ID NOS: 3 to 5 specifically hybridize to the nucleic acid sequence set forth as SEQ ID NOS: 1 and 2.
Embodiment 43: A method of treating cancer comprising: administering to the patient, a therapeutically effective amount of: (i) a peptide encoded by nucleic acid sequence set forth as SEQ ID NOS: 1 or 2, an active fragment, variant, or mutant thereof; or, (ii) a nucleic acid sequence (Sting) set forth as SEQ ID NOS: 1 or 2, an active fragment, variant, or mutant thereof; and, treating cancer in a patient.
Embodiment 44: A method of reconstituting STING in a cell or patient, comprising: administering to the cell or patient, a therapeutically effective amount of: (i) a peptide encoded by nucleic acid sequence set forth as SEQ ID NOS: 1 or 2, an active fragment, variant, or mutant thereof; or, (ii) a nucleic acid sequence (Sting) set forth as SEQ ID NOS: 1 or 2, an active fragment, variant, or mutant thereof; and, reconstituting STING in a cell or patient.
Embodiment 45: The method of embodiment 44, wherein the cell is an abnormal cell comprising: a transformed cell, tumor cell, an infected cell or combinations thereof.
Embodiment 46: The method of embodiment 44, wherein a patient is suffering from a condition comprising: an autoimmune disease or disorder, an inflammatory response, cancer, infected by an agent, genetic disorder, neurological disease or disorder.
Embodiment 47: A prognostic biomarker comprising: STING, fragments, variants, or mutants thereof.
Embodiment 48: The prognostic biomarker of embodiment 47, wherein STING comprises: polynucleotides, oligonucleotides, polypeptides, peptides, or combinations thereof.
Embodiment 49: The prognostic biomarker of embodiment 47, wherein detection or absence of STING as compared to a control, is prognostic of an early disease state.
Embodiment 50: A composition comprising: STING, TRAP-β, SSR2; SSR4; SSR1; SSR3; SRPR; TRAM1; DDOST; RPN2; SEC61B; SEC11A; SRP19; SEC61G; SRP9; DAD1; SRP68; SPCS2; SEC11C; STT3B; SEC61A1; SEC61A2; SRP72; ERO1L; INS; RPN1; SPCS1; SPCS3; SRP14; SRP54; SRPRB; TRAM2; SSR1; SEC61G; SEC61A1; TRAM1; SRP68; SRP19; SRP72; SRPR; STT3B; DERL2; DERL3; EDEM1; SEC62; BCAP31; EBI-1788819; EBI-77683; EXOC2/Sec 5 SERGEF; EXOC3; EXOC8; RALA; EXOC7; EXOC4; EXOC5; RALB; EXOC6; EXOC1; ARF6; DPH3; RHOQ; TRIP10, fragments, variants, mutants, or combinations thereof.
Embodiment 51: An isolated molecule which interacts or associates with STING, directly or indirectly comprising: TRAP-β, SSR2; SSR4; SSR1; SSR3; SRPR; TRAM1; DDOST; RPN2; SEC61B; SEC11A; SRP19; SEC61G; SRP9; DAD1; SRP68; SPCS2; SEC11C; STT3B; SEC61A1; SEC61A2; SRP72; ERO1L; INS; RPN1; SPCS1; SPCS3; SRP14; SRP54; SRPRB; TRAM2; SSR1; SEC61G; SEC61A1; TRAM1; SRP68; SRP19; SRP72; SRPR; STT3B; DERL2; DERL3; EDEM1; SEC62; BCAP31; EBI-1788819; EBI-77683; EXOC2/Sec 5 SERGEF; EXOC3; EXOC8; RALA; EXOC7; EXOC4; EXOC5; RALB; EXOC6; EXOC1; ARF6; DPH3; RHOQ; TRIP10, fragments, variants, mutants, or combinations thereof.

## Claims

1. An immunomodulatory composition comprising an agent which modulates the expression and/or function of STING for use in treating or preventing a disease or disorder selected from the group consisting of cancer, an immune disorder, autoimmunity, inflammation, cachexia, a neurodegenerative disease or disorder, a neurological disease or disorder, cardiac dysfunction, transplantation, or infection, wherein the agent regulates intracellular DNA-mediated type-1 interferon induction.

2. The immunomodulatory composition for the use according to claim 1, wherein the agent is a small organic molecule.

3. The immunomodulatory composition for the use according to claim 1, wherein the agent is a nucleic acid sequence.

4. The immunomodulatory composition for the use according to claim 3, wherein the nucleic acid sequence is comprised in an expression vector.

5. The immunomodulatory composition for the use according to any one of claims 3 and 4, wherein the nucleic acid sequence encodes a STING protein as set forth by SEQ ID NO:1 or 2 or an active variant of any thereof which is at least 90% identical to the full-length STING protein.

6. The method of claim 3, wherein the agent is a double-stranded DNA which increases STING function in the cell.

7. The method of claim 6, wherein the double-stranded DNA comprises a non-native nucleotide.

8. The immunomodulatory composition for the use according to any one of claims 3 and 4, wherein the nucleic acid sequence encodes an interference RNA or an antisense oligonucleotide which downregulates STING expression in a cell.

9. The immunomodulatory composition for the use according to claim 1, wherein the agent is a peptide which modulates STING activity.

10. The immunomodulatory composition for the use according to claim 1, wherein the disease or disorder is a viral infection.

11. The immunomodulatory composition for the use according to claim 1, wherein the disease or disorder is a bacterial infection.

12. The immunomodulatory composition for the use according to claim 1, wherein the disease or disorder is cancer.

13. The immunomodulatory composition for the use according to claim 1, wherein the disease or disorder is an autoimmune disease.

14. The method of claim 1, wherein the agent increases STING expression and/or function.

15. The method of claim 1, wherein the agent inhibits STING expression and/or function.
